# EUROPEAN PATENT SPECIFICATION

(11) **EP 3 565 897 B1**
(45) Date of publication and mention of the grant of the patent: **11.12.2024**
(21) Application number: 18704637.0
(22) Date of filing: 05.01.2018
(51) Int. Cl.: C12N 15/113, A61P 21/00

(54) **OPTIMIZED STRATEGY FOR EXON SKIPPING MODIFICATIONS USING CRISPR/CAS9 WITH TRIPLE GUIDE SEQUENCES**
OPTIMIERTE STRATEGIE FÜR EXON-SKIPPING-MODIFIKATIONEN MITTELS CRISPR/CAS9 MIT TRIPLE-FÜHRUNGSSEQUENZEN
STRATÉGIE OPTIMISÉE POUR DES MODIFICATIONS PAR SAUT D'EXON À L'AIDE DE CRISPR/CAS9 AVEC DES SÉQUENCES DE GUIDAGE TRIPLE

(30) Priority: 05.01.2017 US 201762442606 P; 11.08.2017 US 201762544449 P; 08.12.2017 US 201762596298 P
(43) Date of publication of application: 13.11.2019
(73) Proprietor: The Board Of Regents Of The University Of Texas System, Austin, TX 78701 (US)
(72) Inventor: AMOASII, Leonela, Dallas, TX 75206 (US); OLSON, Eric, University Park, TX 75225 (US)
(74) Representative: Potter Clarkson
(86) International application number: PCT/US2018/012558
(87) International publication number: WO 2018/129296

(56) References cited:
- WO-A1-2016/025469
- WO-A1-2016/174056
- WO-A2-2014/197748
- WO-A2-2016/115543
- WO-A2-2017/095967
- US-A1- 2017 362 635
- NICLAS E. BENGTSSON ET AL: "Progress and prospects of gene therapy clinical trials for the muscular dystrophies", HUMAN MOLECULAR GENETICS, vol. 25, no. R1, 8 October 2015 (2015-10-08), gb, pages R9 - R17, XP055460661, ISSN: 0964-6906, DOI: 10.1093/hmg/ddv420
- YUDA WEI ET AL: "Prevention of Muscle Wasting by CRISPR/Cas9-mediated Disruption of Myostatin In Vivo", MOLECULAR THERAPY : THE JOURNAL OF THE AMERICAN SOCIETY OF GENE THERAPY, vol. 24, no. 11, 1 November 2016 (2016-11-01), US, pages 1889 - 1891, XP055460686, ISSN: 1525-0016, DOI: 10.1038/mt.2016.192
- LEONELA AMOASII ET AL: "Single-cut genome editing restores dystrophin expression in a new mouse model of muscular dystrophy", SCIENCE TRANSLATIONAL MEDICINE, vol. 9, no. 418, 29 November 2017 (2017-11-29), US, pages eaan8081, XP055452628, ISSN: 1946-6234, DOI: 10.1126/scitranslmed.aan8081

## Description

### FEDERAL FUNDING SUPPORT CLAUSE

This invention was made with government support under grant no. U54 HD 087351 awarded by National Institutes of Health. The government has certain rights in the invention.

### FIELD OF THE DISCLOSURE

The present disclosure relates to the fields of molecular biology, medicine and genetics. More particularly, the disclosure relates to compositions and uses thereof for genome editing to correct mutations *in vivo* using an exon-skipping approach.

### BACKGROUND

Muscular dystrophies (MD) are a group of more than 30 genetic diseases characterized by progressive weakness and degeneration of the skeletal muscles that control movement. Duchenne muscular dystrophy (DMD) is one of the most severe forms of MD that affects approximately 1 in 5000 boys and is characterized by progressive muscle weakness and premature death. Cardiomyopathy and heart failure are common, incurable and lethal features of DMD. The disease is caused by mutations in the gene encoding dystrophin (*DMD*), a large intracellular protein that links the dystroglycan complex at the cell surface with the underlying cytoskeleton, thereby maintaining integrity of the muscle cell membrane during contraction. Mutations in the dystrophin gene result in loss of expression of dystrophin, causing muscle membrane fragility and progressive muscle wasting.

WO 2014/197748A2 was published on 11 December 2014 and describes Clustered Regularly Interspaced Short Palindromic Repeats (CRISPR)/CRISPR-associated (Cas) 9-based system related compositions and methods of using said CRISPR/Cas9-based system related compositions for altering gene expression and genome engineering, as well as compositions and methods of using said compositions for altering gene expression and genome engineering in muscle, such as skeletal muscle and cardiac muscle.

### SUMMARY

Despite intense efforts to find cures through a variety of approaches, including myoblast transfer, viral delivery, and oligonucleotide-mediated exon skipping, there remains no cure for any type of muscular dystrophy. The present inventors recently used clustered regularly interspaced short palindromic repeat/Cas9 (CRISPR/Cas9)-mediated genome editing to correct the dystrophin gene (DMD) mutation in postnatal *mdx* mice, a model for DMD. *In vivo* AAV-mediated delivery of gene-editing components successfully removed the mutant genomic sequence by exon skipping in the cardiac and skeletal muscle cells of *mdx* mice. Using different modes of AAV9 delivery, the inventors restored dystrophin protein expression in cardiac and skeletal muscle of *mdx* mice. The mdx mouse model and the correction exon 23 using AAV delivery of myoediting machinery has been useful to show proof-of concept of exon skipping approach using several cuts in the genomic region encompassing the mutation *in vivo.* However, more efficient and safe approaches to genome editing and DMD would provide a more powerful means to intervene in this disease.

Accordingly, the present invention provides a nucleic acid comprising a sequence encoding a first DMD guide RNA targeting a first genomic target sequence, a sequence encoding a second DMD guide RNA targeting a second genomic target sequence, a sequence encoding a third DMD guide RNA targeting a third genomic target sequence, a first promoter wherein the first promoter drives expression of the sequence encoding the first DMD guide RNA, a second promoter wherein the second promoter drives expression of the sequence encoding the second DMD guide RNA, and a third promoter wherein the third promoter drives expression of the sequence encoding the third DMD guide RNA, wherein the first genomic target sequence, the second genomic target sequence and the third genomic sequence each comprise a dystrophin splice acceptor site, and wherein the sequence encoding the first DMD guide RNA, the sequence encoding the second DMD guide RNA genomic, and the sequence encoding the third DMD guide RNA target sequence are identical. Consequently, the first genomic target sequence, the second genomic target sequence, and the third genomic target sequence are also identical. In some embodiments, the sequence encoding the first promoter and the sequence encoding the second promoter are identical. In some embodiments, the sequence encoding the first promoter and the sequence encoding the second promoter not identical. In some embodiments, the sequence encoding the first promoter and the sequence encoding the second promoter share at least 50%, 60%, 70%, 80%, 90%, or 95% sequence identity. In some embodiments, at least two of the sequences encoding the first promoter, the sequence encoding the second promoter, and the sequence encoding the third promoter are identical. In some embodiments, at least two of the sequence encoding the first promoter, the sequence encoding the second promoter, and the sequence encoding the third promoter are not identical. In some embodiments, at least two of the sequences encoding the first promoter, the sequence encoding the second promoter, and the sequence encoding the third promoter share at least 50%, 60%, 70%, 80%, 90%, or 95% sequence identity. In some embodiments, the nucleic acid further comprises a sequence encoding a fourth DMD guide RNA targeting a fourth genomic target sequence, and a sequence encoding a fourth promoter, wherein the fourth promoter drives expression of the fourth sequence encoding a DMD guide RNA, wherein the fourth genomic target sequence comprises a dystrophin splice acceptor site. In some embodiments, at least two of the sequence encoding the first promoter, the sequence encoding the second promoter, the sequence encoding the third promoter, and the sequence encoding the fourth promoter are identical. In some embodiments, at least two of the sequence encoding the first promoter, the sequence encoding the second promoter, the sequence encoding the third promoter, and the sequence encoding the fourth promoter are not identical. In some embodiments, at least two of the sequence encoding the first promoter, the sequence encoding the second promoter, the sequence encoding the third promoter, and the sequence encoding the fourth promoter share at least 50%, 60%, 70%, 80%, 90%, or 95% sequence identity. In some embodiments, the first genomic target sequence, the second genomic target sequence, the third genomic target sequence, and the fourth genomic target sequence are identical. In some embodiments, the fourth genomic target sequence is not identical to the the first genomic target sequence, the second genomic target sequence, and the third genomic target sequence. In some embodiments, the fourth genomic target sequence shares at least 50%, 60%, 70%, 80%, 90%, or 95% sequence identity with the first genomic target sequence, the second genomic target sequence, and the third genomic target sequence. In some embodiments, the fourth genomic target sequence is complementary to the first genomic target sequence, the second genomic target sequence, and the third genomic target sequence. In some embodiments, the nucleic acid further comprises a sequence encoding a fifth DMD guide RNA targeting a fifth genomic target sequence, and a sequence encoding a fifth promoter, wherein the fifth promoter drives expression of the sequence encoding the fifth DMD guide RNA, wherein the fifth genomic target sequence comprises a dystrophin splice acceptor site. In some embodiments, at least two of the sequence encoding the first promoter, the sequence encoding the second promoter, the sequence encoding the third promoter, the sequence encoding the fourth promoter, and the sequence encoding the fifth promoter are identical. In some embodiments, at least two of the sequence encoding the first promoter, the sequence encoding the second promoter, the sequence encoding the third promoter, the sequence encoding the fourth promoter, and the sequence encoding the fifth promoter are not identical. In some embodiments, at least two of the sequence encoding the first promoter, the sequence encoding the second promoter, the sequence encoding the third promoter, the sequence encoding the fourth promoter, and the sequence encoding the fifth promoter share at least 50%, 60%, 70%, 80%, 90%, or 95% sequence identity. In some embodiments, either or both of the fourth genomic target sequence, and the fifth genomic target sequence, are identical to the first genomic target sequence, the second genomic target sequence, and the third genomic target sequence. In some embodiments, either or both of the fourth genomic target sequence, and the fifth genomic target sequence, are not identical to the first genomic target sequence, the second genomic target sequence, and the third genomic target sequence. In some embodiments, either or both of the fourth genomic target sequence, and the fifth genomic target sequence, share at least 50%, 60%, 70%, 80%, 90%, or 95% sequence identity with the first genomic target sequence, the second genomic target sequence, and the third genomic target sequence. In some embodiments, either or both of the fourth genomic target sequence, and the fifth genomic target sequence, are complementary to the first genomic target sequence, the second genomic target sequence, and the third genomic target sequence. In some embodiments, the nucleic acid further comprises at least one sequence encoding an additional DMD guide RNA targeting a genomic target sequence, and at least one additional promoter, wherein the additional promoter drives expression of the sequence encoding the additional DMD guide RNA, wherein the additional genomic target sequence comprises a dystrophin splice acceptor site. In some embodiments, the dystrophin splice acceptor site comprises the 5' splice acceptor site of exon 51. In some embodiments, the sequence encoding the first promoter or the sequence encoding the second promoter comprises a sequence encoding a constitutive promoter. In some embodiments, the first promoter or the second promoter comprises a constitutive promoter. In some embodiments, at least one of the sequence encoding the first promoter, the sequence encoding the second promoter, the sequence encoding the third promoter, the sequence encoding the fourth promoter, and the sequence encoding the fifth promoter comprises a sequence encoding a constitutive promoter. In some embodiments, at least one of the first promoter, the second promoter, the third promoter, the fourth promoter, and the fifth promoter comprises a constitutive promoter. In some embodiments, the sequence encoding the first promoter or the sequence encoding the second promoter comprises a sequence encoding an inducible promoter. In some embodiments, at least one of the first promoter, the second promoter, the third promoter, the fourth promoter, and the fifth promoter comprises an inducible promoter. In some embodiments, the sequence encoding the first promoter or the sequence encoding the second promoter comprises a sequence encoding a cell-type specific promoter. In some embodiments, at least one of the sequence encoding the first promoter, the sequence encoding the second promoter, the sequence encoding the third promoter, the sequence encoding the fourth promoter, and the sequence encoding the fifth promoter comprises a cell-type specific promoter. In some embodiments, the cell type specific promoter comprises a muscle-specific promoter. In some embodiments, the sequence encoding the first promoter or the sequence encoding the second promoter comprises a sequence encoding a U6 promoter, an H1 promoter, or a 7SK promoter. In some embodiments, at least one of the sequence encoding the first promoter, the sequence encoding the second promoter, the sequence encoding the third promoter, the sequence encoding the fourth promoter, and the sequence encoding the fifth promoter comprises a U6 promoter, an H1 promoter, or a 7SK promoter. In some embodiments, at least one of the sequences encoding the first promoter, the sequence encoding the second promoter, the sequence encoding the third promoter, the sequence encoding the fourth promoter, and the sequence encoding the fifth promoter comprises a U6 promoter. In some embodiments, at least one of the sequences encoding the first promoter, the sequence encoding the second promoter, the sequence encoding the third promoter, the sequence encoding the fourth promoter, and the sequence encoding the fifth promoter comprises an H1 promoter. In some embodiments, at least one of the sequences encoding the first promoter, the sequence encoding the second promoter, the sequence encoding the third promoter, the sequence encoding the fourth promoter, and the sequence encoding the fifth promoter comprises a 7SK promoter. The sequence encoding the first DMD guide RNA, the sequence encoding the second DMD guide RNA, and sequence encoding the third DMD guide RNA are identical, and in some embodiments, the 5' splice acceptor site comprises a 5' splice acceptor site of exon 51. In some embodiments, the sequence encoding the first promoter comprises a sequence encoding a U6 promoter, the sequence encoding the second promoter comprises a sequence encoding an H1 promoter, and the sequence encoding the third promoter comprises a 7SK promoter. In some embodiments, the nucleic acid comprises a DNA sequence. In some embodiments, the nucleic acid comprises an RNA sequence. In some embodiments, the nucleic acid further comprises one or more sequences encoding an inverted terminal repeat (ITR). In some embodiments, the nucleic acid further comprises a sequence encoding a 5' inverted terminal repeat (ITR) and a sequence encoding a 3' ITR. In some embodiments, the sequence encoding the 5' inverted terminal repeat (ITR) or the sequence encoding a 3' ITR comprises a sequence isolated or derived from an adeno-associated virus (AAV). In some embodiments, the sequence encoding the 5' inverted terminal repeat (ITR) or the sequence encoding a 3' ITR comprises a sequence isolated or derived from an adeno-associated virus (AAV) of serotype 2 (AAV2). In some embodiments, the sequence encoding the 5' inverted terminal repeat (ITR) and the sequence encoding a 3' ITR comprises a sequence isolated or derived from an AAV2. In some embodiments, the sequence encoding the 5' inverted terminal repeat (ITR) or the sequence encoding a 3' ITR comprises a sequence isolated or derived from an adeno-associated virus (AAV) of serotype 4 (AAV4). In some embodiments, the sequence encoding the 5' inverted terminal repeat (ITR) and the sequence encoding a 3' ITR comprises a sequence isolated or derived from an AAV4. In some embodiments, the sequence encoding the 5' inverted terminal repeat (ITR) or the sequence encoding a 3' ITR comprises or consists of 145 nucleotides. In some embodiments, the sequence encoding the 5' inverted terminal repeat (ITR) or the sequence encoding a 3' ITR comprises or consists of 115 nucleotides. In some embodiments, the sequence encoding the 5' inverted terminal repeat (ITR) or the sequence encoding a 3' ITR comprises or consists of 141 nucleotides. In some embodiments, the nucleic acid further comprises a polyadenosine (poly A) sequence. In some embodiments, the poly A sequence is a mini poly A sequence. In some embodiments, the sequence encoding the first DMD guide RNA or the sequence encoding the second DMD guide RNA comprises the sequence of any one of SEQ ID NOs. 60-382, 706-708 and 712-789. In some embodiments, the sequence encoding the first DMD guide RNA, the sequence encoding the second DMD guide RNA, and the sequence encoding the third DMD guide RNA each comprises the sequence of SEQ ID NO. 714.

The nucleic acid of the present invention may also be provided in the form of a vector comprising a nucleic acid comprising a sequence encoding a first DMD guide RNA targeting a first genomic target sequence, a sequence encoding a second DMD guide RNA targeting a second genomic target sequence, a sequence encoding a third DMD guide RNA targeting a third genomic target sequence, a first promoter wherein the first promoter drives expression of the sequence encoding the first DMD guide RNA, a second promoter wherein the first promoter drives expression of the sequence encoding the second DMD guide RNA, and a third promoter wherein the third promoter drives expression of the sequence encoding the third DMD guide RNA, wherein the first genomic target sequence, the second genomic target sequence and the third genomic target sequence each comprise a dystrophin splice acceptor site, and wherein the sequence encoding the first DMD guide RNA, the sequence encoding the second DMD guide RNA genomic, and the sequence encoding the third DMD guide RNA target sequence are identical. In some embodiments, the vector further comprises a sequence encoding an inverted terminal repeat of a transposable element. In some embodiments, the transposable element is a transposon. In some embodiments, the transposon is a Tn7 transposon. In some embodiments, the vector is a non-viral vector. In some embodiments, the non-viral vector is a plasmid. In some embodiments, the vector is a viral vector. In some embodiments, the viral vector is an adeno-associated viral (AAV) vector. In some embodiments, the AAV vector is replication-defective or conditionally replication defective. In some embodiments, the AAV vector is a recombinant AAV vector. In some embodiments, the AAV vector comprises a sequence isolated or derived from an AAV vector of serotype 1 (AAV1), 2 (AAV2), 3 (AAV3), 4 (AAV4), 5 (AAV5), 6 (AAV6), 7 (AAV7), 8 (AAV8), 9 (AAV9), 10 (AAV10), 11 (AAV11) or any combination thereof. In some embodiments, the AAV vector comprises a sequence isolated or derived from an AAV vector of serotype 9 (AAV9). In some embodiments, the AAV vector comprises a sequence isolated or derived from an AAV vector of serotype 2 (AAV2). In some embodiments, the AAV vector comprises a sequence isolated or derived from an AAV2 and a sequence isolated or derived from an AAV9. In some embodiments, the AAV vector comprises a sequence isolated or derived from an AAV4 and a sequence isolated or derived from an AAV9. In some embodiments, the vector is optimized for expression in mammalian cells. In some embodiments, the vector is optimized for expression in human cells. In some embodiments, the vector comprises the sequence of SEQ ID NO. 914, SEQ ID NO. 915, SEQ ID NO. 916, or SEQ ID NO. 917.

Also described herein is a nucleic acid comprising a sequence encoding a promoter and a sequence encoding a Cas9 or a nuclease domain thereof, wherein the sequence encoding the promoter comprises a sequence encoding a muscle-specific promoter. In some embodiments of the present disclosure, the sequence encoding the muscle-specific promoter comprises a sequence encoding a CK8 promoter. In some embodiments of the present disclosure, the sequence encoding the muscle-specific promoter comprises a sequence encoding a CK8e promoter. In some embodiments of the present disclosure, the sequence encoding the Cas9 or the nuclease domain thereof is isolated or derived from a sequence encoding an *S. pyogenes* Cas9 or a nuclease domain thereof. In some embodiments of the present disclosure, the sequence encoding the Cas9 or the nuclease domain thereof is isolated or derived from a sequence encoding *S. aureus* Cas9 or a nuclease domain thereof. In some embodiments of the present disclosure, the sequence encoding the Cas9 or the nuclease domain thereof is codon optimized for expression in a mammal. In some embodiments of the present disclosure, the sequence encoding the Cas9 or the nuclease domain thereof is codon optimized for expression in a human. In some embodiments of the present disclosure, the nucleic acid further comprises a polyA sequence. In some embodiments of the present disclosure, the polyA sequence is a mini polyA sequence. In some embodiments of the present disclosure, the nucleic acid further comprises one or more sequences encoding an inverted terminal repeat (ITR). In some embodiments of the present disclosure, the nucleic acid further comprises a sequence encoding a 5' inverted terminal repeat (ITR) and a sequence encoding a 3' ITR. In some embodiments of the present disclosure, the sequence encoding the 5' inverted terminal repeat (ITR) or the sequence encoding a 3' ITR comprises a sequence isolated or derived from an adeno-associated virus (AAV). In some embodiments of the present disclosure, the sequence encoding the 5' inverted terminal repeat (ITR) or the sequence encoding a 3' ITR comprises a sequence isolated or derived from an adeno-associated virus (AAV) of serotype 2 (AAV2). In some embodiments, the sequence encoding the 5' inverted terminal repeat (ITR) and the sequence encoding a 3' ITR comprises a sequence isolated or derived from an AAV2. In some embodiments of the present disclosure, the sequence encoding the 5' inverted terminal repeat (ITR) or the sequence encoding a 3' ITR comprises a sequence isolated or derived from an adeno-associated virus (AAV) of serotype 4 (AAV4). In some embodiments of the present disclosure, the sequence encoding the 5' inverted terminal repeat (ITR) and the sequence encoding a 3' ITR comprises a sequence isolated or derived from an AAV4. In some embodiments of the present disclosure, the sequence encoding the 5' inverted terminal repeat (ITR) or the sequence encoding a 3' ITR comprises or consists of 145 nucleotides, 115 nucleotides, or 141 nucleotides. In some embodiments of the present disclosure, the nucleic acid further comprises a nuclear localization signal. In some embodiments of the present disclosure, the nucleic acid is optimized for expression in mammalian cells. In some embodiments of the present disclosure, the nucleic acid is optimized for expression in human cells.

Also described herein is a vector comprising a nucleic acid comprising a sequence encoding a promoter and a sequence encoding a Cas9 or a nuclease domain thereof, wherein the sequence encoding the promoter comprises a sequence encoding a muscle-specific promoter such as the CK8 or CK8e promoter. In some embodiments of the present disclosure, the vector further comprises a sequence encoding an inverted terminal repeat (ITR) of a transposable element. In some embodiments of the present disclosure, the transposable element is a transposon. In some embodiments of the present disclosure, the transposon is a Tn7 transposon. In some embodiments of the present disclosure, the vector further comprises a sequence encoding a 5' ITR of a T7 transposon and a sequence encoding a 3' ITR of a T7 transposon. In some embodiments of the present disclosure, the vector is a non-viral vector. In some embodiments of the present disclosure, the non-viral vector is a plasmid. In some embodiments of the present disclosure, the vector is a viral vector. In some embodiments of the present disclosure, the viral vector is an adeno-associated viral (AAV) vector. In some embodiments of the present disclosure, the AAV vector is replication-defective or conditionally replication defective. In some embodiments of the present disclosure, the AAV vector is a recombinant AAV vector. In some embodiments of the present disclosure, the AAV vector comprises a sequence isolated or derived from an AAV vector of serotype 1 (AAV1), 2 (AAV2), 3 (AAV3), 4 (AAV4), 5 (AAV5), 6 (AAV6), 7 (AAV7), 8 (AAV8), 9 (AAV9), 10 (AAV10), 11 (AAV11) or any combination thereof. In some embodiments of the present disclosure, the AAV vector comprises a sequence isolated or derived from an AAV vector of serotype 9 (AAV9). In some embodiments of the present disclosure, the AAV vector comprises a sequence isolated or derived from an AAV vector of serotype 2 (AAV2). In some embodiments of the present disclosure, the AAV vector comprises a sequence isolated or derived from an AAV2 and a sequence isolated or derived from an AAV9. In some embodiments of the present disclosure, the AAV vector comprises a sequence isolated or derived from an AAV vector of serotype 4 (AAV4). In some embodiments of the present disclosure, the AAV vector comprises a sequence isolated or derived from an AAV4 and a sequence isolated or derived from an AAV9. In some embodiments of the present disclosure, wherein the vector is optimized for expression in mammalian cells. In some embodiments of the present disclosure, the vector is optimized for expression in human cells. In some embodiments of the present disclosure, the vector comprises the nucleic acid sequence of SEQ ID NO. 899, SEQ ID NO. 900, SEQ ID NO. 901, or SEQ ID NO. 902.

The nucleic acids of the present invention, and/or the other nucleic acids as disclosed herein may be present in is a cell that comprises one or more said nucleic acids. In some embodiments, the cell is a human cell. In some embodiments, the cell is a muscle cell or satellite cell. In some embodiments, the cell is an induced pluripotent stem (iPS) cell.

The nucleic acids of the present invention, and/or the other nucleic acids as disclosed herein may be provided in the form of a composition comprising one or more of said nucleic acids. In some embodiments, the composition further comprises a pharmaceutically acceptable carrier.

The nucleic acids of the present invention, and/or the other nucleic acids as disclosed herein may be provided in the form of a composition that comprises a cell comprising one or more of said nucleic acids. In some embodiments, the composition further comprises a pharmaceutically acceptable carrier.

The nucleic acids of the present invention, and/or the other nucleic acids as disclosed herein may also be provided in the form of a composition that comprises one or more vectors, wherein the one or more vectors comprise said nucleic acids. In some embodiments, the composition further comprises a pharmaceutically acceptable carrier.

The nucleic acids of the present invention, and/or the other nucleic acids as disclosed herein may be provided in a cell comprising a composition comprising one or more vectors, wherein the one or more vectors comprise said nucleic acids. In some embodiments, the cell is a human cell. In some embodiments, the cell is a muscle cell or satellite cell. In some embodiments, the cell is an induced pluripotent stem (iPS) cell.

In some embodiments, the nucleic acids of the present invention may be presented in a composition that comprises (i) a first nucleic acid sequence comprising a sequence encoding a first DMD guide RNA targeting a first genomic target sequence, a sequence encoding a second DMD guide RNA targeting a second genomic target sequence, a sequence encoding a third DMD guide RNA targeting a third genomic target sequence, a first promoter wherein the first promoter drives expression of the sequence encoding the first DMD guide RNA, a second promoter wherein the first promoter drives expression of the sequence encoding the second DMD guide RNA, and a third promoter wherein the first promoter drives expression of the sequence encoding the third DMD guide RNA, wherein the first genomic target sequence, the second genomic target sequence and the third genomic target sequence each comprise a dystrophin splice acceptor, wherein the sequence encoding the first DMD guide RNA, the sequence encoding the second DMD guide RNA genomic, and the sequence encoding the third DMD guide RNA target sequence are identical and (ii) a second nucleic acid sequence comprising a sequence encoding a promoter and a sequence encoding a Cas9 or a nuclease domain thereof, wherein the sequence encoding the promoter comprises a sequence encoding a muscle-specific promoter such as the CK8 or CK8e promoter. In some embodiments, the composition further comprises a pharmaceutically acceptable carrier.

In some embodiments, the nucleic acids of the present invention may be presented in a composition that comprises (i) a first vector comprising a nucleic acid sequence comprising a sequence encoding a first DMD guide RNA targeting a first genomic target sequence, a sequence encoding a second DMD guide RNA targeting a second genomic target sequence, a sequence encoding a third DMD guide RNA targeting a third genomic target sequence, a first promoter wherein the first promoter drives expression of the sequence encoding the first DMD guide RNA, a second promoter wherein the second promoter drives expression of the sequence encoding the second DMD guide RNA, and a third promoter wherein the third promoter drives expression of the sequence encoding the third DMD guide RNA, wherein the first genomic target sequence, the second genomic target sequence, and the third genomic target sequence each comprise a dystrophin splice acceptor, wherein the sequence encoding the first DMD guide RNA, the sequence encoding the second DMD guide RNA genomic, and the sequence encoding the third DMD guide RNA target sequence are identical and (ii) a second vector comprising a nucleic acid sequence comprising a sequence encoding a promoter and a sequence encoding a Cas9 or a nuclease domain thereof, wherein the sequence encoding the promoter comprises a sequence encoding a muscle-specific promoter such as the CK8 or CK8e promoter. In some embodiments, at least one of the first vector and the second vectors are AAVs. In some embodiments, the composition further comprises a pharmaceutically acceptable carrier.

The nucleic acids of the present invention may be for use in a method for correcting a dystrophin defect, the method comprising contacting a cell with one or more compositions of the disclosure under conditions suitable for expression of the first DMD guide RNA, the second DMD guide RNA, the third DMD guide RNA and the Cas9 protein or a nuclease domain thereof, wherein at least one of first DMD guide RNA, the second DMD guide RNA or the third DMD guide RNA forms a complex with the Cas9 protein or the nuclease domain thereof to form at least one DMD guide RNA-Cas9 complex, wherein the at least one DMD guide RNA-Cas9 complex disrupts a dystrophin splice site and induces selective skipping of a DMD exon.

The nucleic acids of the present invention may be for use in a method for correcting a dystrophin defect, the method comprising contacting a cell with one or more compositions of the disclosure under conditions suitable for expression of the first DMD guide RNA, the second DMD guide RNA, the third DMD guide RNA and the Cas9 protein or a nuclease domain thereof, wherein at least one of first DMD guide RNA, the second DMD guide RNA or the third DMD guide RNA forms a complex with the Cas9 protein or the nuclease domain thereof to form at least one DMD guide RNA-Cas9 complex, wherein the at least one DMD guide RNA-Cas9 complex induces a reframing of a dystrophin reading frame. In some embodiments, the reframing of a dystrophin reading frame induces an insertion. In some embodiments, the insertion comprises or consists of a single adenosine nucleotide.

The nucleic acids of the present invention may be for use in a method for inducing selective skipping of a DMD exon, the method comprising contacting a cell with one or more compositions of the disclosure under conditions suitable for expression of the first DMD guide RNA, the second DMD guide RNA, the third DMD guide RNA and the Cas9 protein or a nuclease domain thereof, wherein at least one of first DMD guide RNA, the second DMD guide RNA, or the third DMD guide RNA forms a complex with the Cas9 protein or the nuclease domain thereof to form at least one DMD guide RNA-Cas9 complex, wherein the at least one DMD guide RNA-Cas9 complex disrupts a dystrophin splice site and induces selective skipping of a DMD exon.

The nucleic acids of the present invention may be for use in a method for inducing a reframing event in the dystrophin reading frame, the method comprising contacting a cell with one or more compositions of the disclosure under conditions suitable for expression of the first DMD guide RNA, the second DMD guide RNA, the third DMD guide RNA and the Cas9 protein or a nuclease domain thereof, wherein at least one of first DMD guide RNA, the second DMD guide RNA or the third DMD guide RNA forms a complex with the Cas9 protein or the nuclease domain thereof to form at least one DMD guide RNA-Cas9 complex, wherein the at least one DMD guide RNA-Cas9 complex disrupts a dystrophin splice site and induces selective skipping of a DMD exon. In some embodiments, the at least one DMD guide RNA-Cas9 complex disrupts a dystrophin splice site and induces selective skipping of exon 51 of a human DMD gene.

The nucleic acids of the present invention may be for use in a method of treating muscular dystrophy in a subject in need thereof comprising administering to the subject a therapeutically effective amount of one or more compositions of the disclosure. In some embodiments, the composition is administered locally. In some embodiments, the composition is administered directly to a muscle tissue. In some embodiments, the composition is administered by an intramuscular infusion or injection. In some embodiments, the muscle tissue comprises a tibialis anterior tissue, a quadriceps tissue, a soleus tissue, a diaphragm tissue, or a heart tissue. In some embodiments, the composition is administered by an intracardiac injection. In some embodiments, the composition is administered systemically. In some embodiments, the composition is administered by an intravenous infusion or injection. In some embodiments, following administration of the composition, the subject exhibits normal dystrophin-positive myofibers, and mosaic dystrophin-positive myofibers containing centralized nuclei, or a combination thereof. In some embodiments, following administration of the composition, the subject exhibits an emergence or an increase in a level of abundance of normal dystrophin-positive myofibers when compared to an absence or a level of abundance of normal dystrophin-positive myofibers prior to administration of the composition. In some embodiments, following administration of the composition, the subject exhibits an emergence or an increase in a level of abundance of mosaic dystrophin-positive myofibers containing centralized nuclei when compared to an absence or an level of abundance of mosaic dystrophin-positive myofibers containing centralized nuclei prior to administration of the composition. In some embodiments, following administration of the composition, the subject exhibits a decreased serum CK level when compared to a serum CK level prior to administration of the composition. In some embodiment, following administration of the composition, the subject exhibits improved grip strength when compared to a grip strength prior to administration of the composition. In some embodiments, the subject is a neonate, an infant, a child, a young adult, or an adult. In some embodiments, the subject has muscular dystrophy. In some embodiments, the subject is a genetic carrier for muscular dystrophy. In some embodiments, the subject is male. In some embodiments, the subject is female. In some embodiments, the subject appears to be asymptomatic and wherein a genetic diagnosis reveals a mutation in one or both copies of a *DMD* gene that impairs function of the *DMD* gene product. In some embodiments, the subject presents an early sign or symptom of muscular dystrophy. In some embodiments, the early sign or symptom of muscular dystrophy comprises loss of muscle mass or proximal muscle weakness. In some embodiments, the loss of muscle mass or proximal muscle weakness occurs in one or both leg(s) and/or a pelvis, followed by one or more upper body muscle(s). In some embodiments, the early sign or symptom of muscular dystrophy further comprises pseudohypertrophy, low endurance, difficulty standing, difficulty walking, difficulty ascending a staircase or a combination thereof. In some embodiments, the subject presents a progressive sign or symptom of muscular dystrophy. In some embodiments, the progressive sign or symptom of muscular dystrophy comprises muscle tissue wasting, replacement of muscle tissue with fat, or replacement of muscle tissue with fibrotic tissue. In some embodiments, the subject presents a later sign or symptom of muscular dystrophy. In some embodiments, the later sign or symptom of muscular dystrophy comprises abnormal bone development, curvature of the spine, loss of movement, and paralysis. In some embodiments, the subject presents a neurological sign or symptom of muscular dystrophy. In some embodiments, the neurological sign or symptom of muscular dystrophy comprises intellectual impairment and paralysis. In some embodiments, administration of the composition occurs prior to the subject presenting one or more progressive, later or neurological signs or symptoms of muscular dystrophy. In some embodiments, the subject is less than 10 years old, less than 5 years old, or less than 2 years old.

The nucleic acids of the present invention may be provided for the use of a therapeutically-effective amount of one or more compositions comprising said nucleic acids for treating muscular dystrophy in a subject in need thereof.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, for the method being employed to determine the value, or that exists among the study subjects. Such an inherent variation may be a variation of ±10% of the stated value.

Throughout this application, nucleotide sequences are listed in the 5' to 3' direction, and amino acid sequences are listed in the N-terminal to C-terminal direction, unless indicated otherwise.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The following drawings form part of the present specification and are included to further demonstrate certain aspects of the present disclosure. The disclosure may be better understood by reference to one or more of these drawings in combination with the detailed description of specific embodiments presented herein.

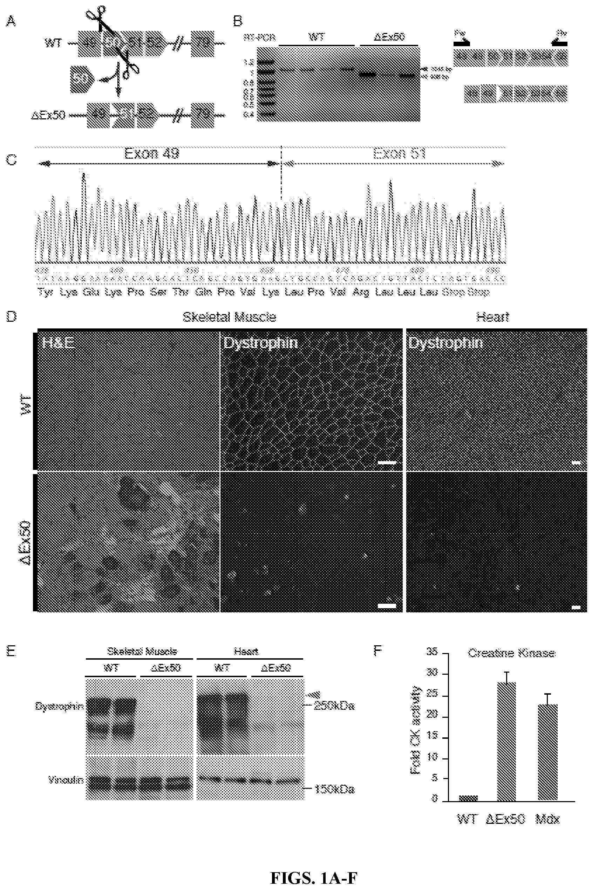

**FIGS. 1A-F**. "Humanized"-ΔEx50 mouse model. (FIG. 1A) Strategy showing CRISPR/Cas9-mediated genome editing approach to generate a humanized mouse model. (FIG. 1B) RT-PCR analysis to validate deletion of exon 50 (ΔEx50). (FIG. 1C) Sequence of RT-PCR product to validate exon 50 deletion and generation of an out-of-frame sequence (nucleic acid sequence = SEQ ID NO: 1; amino acid sequence = SEQ ID NO: 2). (FIG. 1E) Western blot analysis of dystrophin and vinculin expression in tibialis anterior and heart tissues. (FIG. 1F) Levels of serum CK, a marker of muscle dystrophy that reflects muscle damage and membrane leakage were measured in wildtype (WT), ΔEx50 and mdx mice. (FIG. 1D) Histochemistry of cardiac and skeletal muscle by hematoxylin and eosin (H&E) staining, and immunohistochemistry using dystrophin antibody. Scale bar: 50 µm.

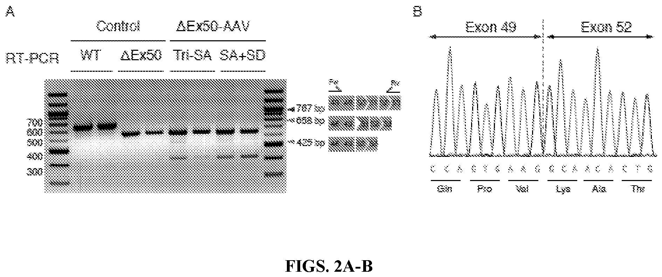

**FIG. 2A-B**. Exon 51 skipping. (FIG. 2A) RT-PCR of RNA from ΔEx50 mice 3 weeks after intramuscular injection indicates deletion of exon 51 (termed ΔEx50-51, lower band). (FIG. 2B) Sequence of the RT-PCR products of ΔEx50-51 band confirmed that exon 49 spliced directly to exon 52, excluding exon 51 (nucleic acid sequence = SEQ ID NO: 3; amino acid sequence = SEQ ID NO: 4).

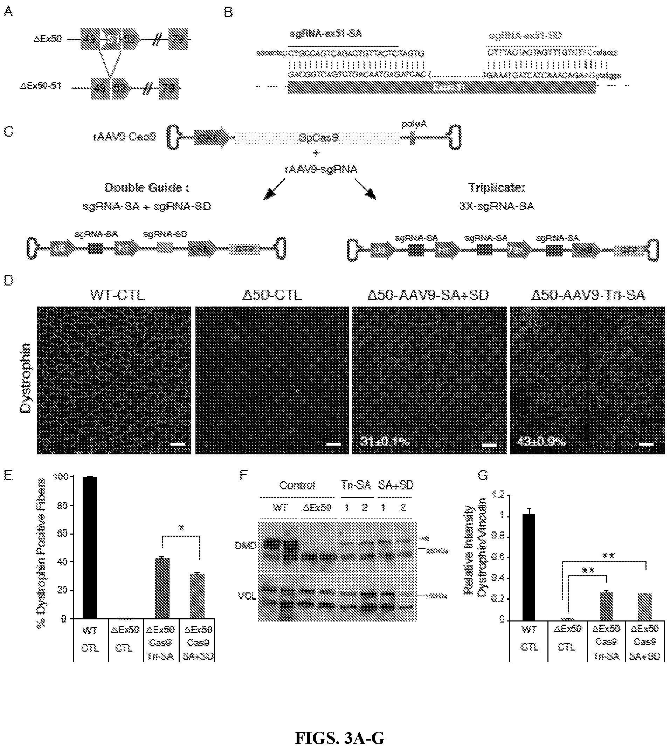

**FIG. 3A-G**. Correction of dystrophin expression using triplicate gRNA strategy 3 **weeks after intra-muscular injection.** (FIG. 3A) Strategy showing CRISPR/Cas9-mediated genome editing approach to correct the reading frame in ΔEx50 mouse model. (FIG. 3B) sgRNA targeting the splice acceptor site (sgRNA-ex51-SA) sequence and schematic illustration of sgRNA binding position. Fig. 3B discloses SEQ ID NOS 954-957, respectively, in order of appearance. (FIG. 3C) Schematic illustration of AAV injection plasmids and strategy. Double guide strategy using rAAV9-sgRNA plasmid for sgRNA-ex51-SA and sgRNA-ex51-SD. Triplicate using rAAV9-sgRNA plasmid containing 3 copies of sgRNA-ex51-SA. Muscle creatine kinase 8 (CK8) promoter to express SpCas9. U6, H1 and 7SK promoter for RNA polymerase III to express sgRNA. (FIG. 3D) Dystrophin immunohistochemistry staining of tibialis anterior muscle. (FIG. 3E) Quantification of dystrophin positive fibers normalized by area. (FIG. 3F) Western blot analysis of dystrophin (DMD) and vinculin (VCL) expression 3 weeks after intramuscular injection. (FIG. 3G) Quantification of dystrophin expression after normalization to vinculin. Data are represented as mean ± SEM. ***P*<0.005. Scale bar: 50 µm

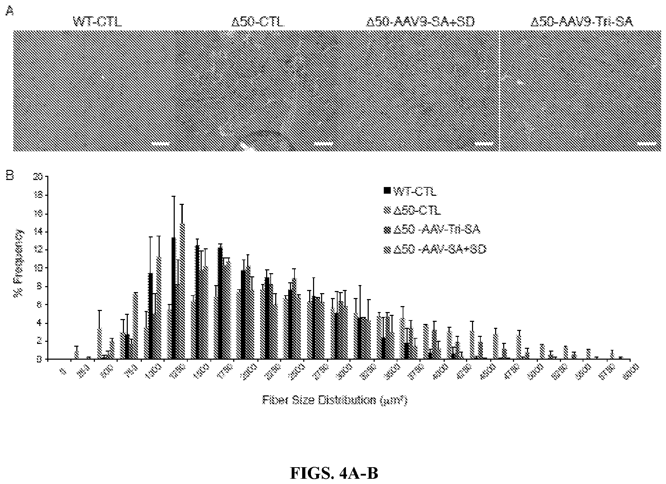

**FIG. 4A-B**. Histological improvement of injected muscle after 3 **weeks.** (FIG. 4A) Histochemistry of tibialis anterior muscle by hematoxylin and eosin (H&E) staining. (FIG. 4B) Quantification of fiber size and percentage of frequency. Data are represented as mean ± SEM. Scale bar: 50 µm. Each set of four data points for a given fiber size are Δ50-CTL, WT-CTL, Δ50-AAV-TriSA and Δ50-AAV-SA+SD left to right.

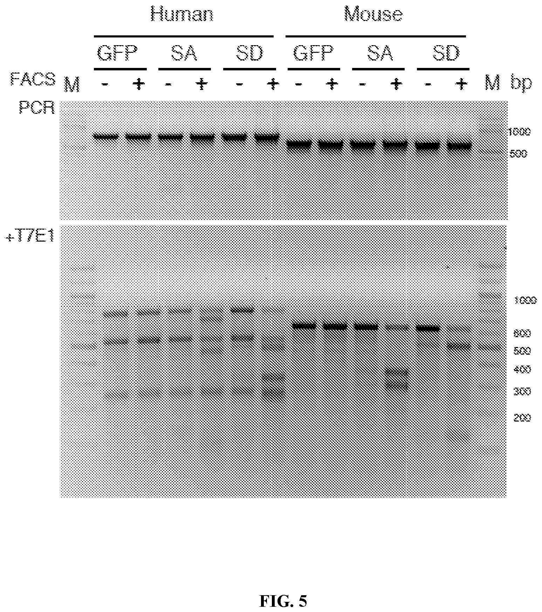

**FIG. 5****. Screen of sgRNA in human 293 cells and mouse 10T cells.** Undigested PCR products (upper panel) and T7E1 digestion (lower panel) on a 2% agarose gel. M denotes size marker lane. bp indicates the length of the marker bands.

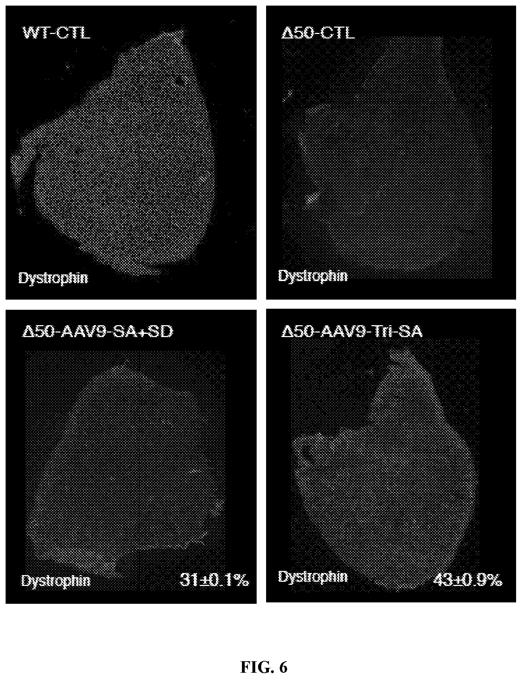

**FIG. 6****. Dystrophin immunohistochemistry staining of entire tibialis anterior muscle.**

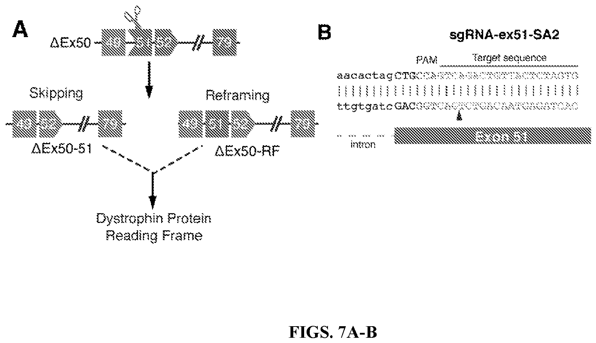

**FIGS. 7A-B**. Strategy for CRISPR/Cas9-mediated genome editing in ΔEx50 mice. (FIG. 7A) Strategy showing CRISPR/Cas9-mediated genome editing approach to correct the reading frame in ΔEx50 mouse model. (FIG. 7B) sgRNA targeting the splice acceptor site (sgRNA-ex51-SA2) sequence and schematic illustration of sgRNA binding position. Fig. 7B discloses SEQ ID NOS 958-959, respectively, in order of appearance.

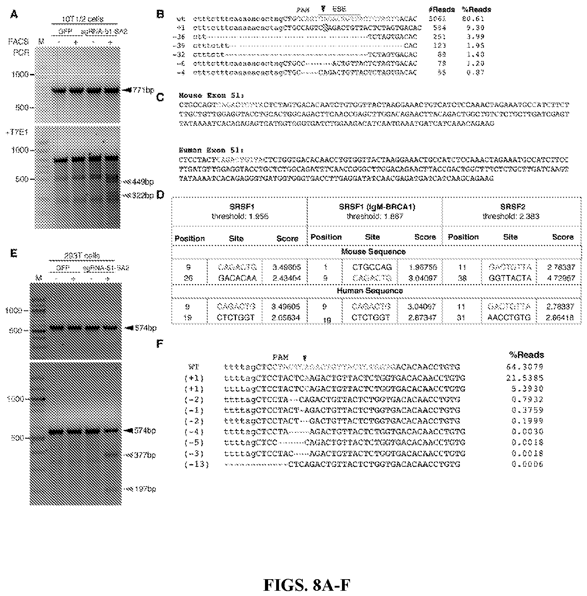

**FIG. 8A-F**. sgRNA genomic analysis in mouse and human cells (FIG. 8A) Cas9 was expressed in the presence or absence of mouse sgRNA-sgRNA-51-SA2 in 10T1/2 cells and gene editing was monitored by T7E1 assay in fluorescence-based cell sorted (FACS) (+) and non-sorted cells (-). GFP was used as a control. Undigested PCR products (upper panel) and T7E1 digestion (lower panel) are shown on a 2% agarose gel. Black arrowhead indicates the undigested 771bp PCR band. Green arrowheads in the lower panel indicate the cut bands by T7E1 assay. M denotes size marker lane. bp indicates the length of the marker bands. (FIG. 8B) Genomic deep sequencing analysis of PCR amplicons generated across the exon 51 target site in 10T1/2 cells. Sequence of representative indels aligned with sgRNA sequence (indicated in blue) revealing insertions (highlighted in green) and deletions (highlighted in red) (SEQ ID NOS 960-966, respectively, in order of appearance). The line indicates the predicted exon splicing enhancers (ESEs) sequence located at the site of sgRNA. Black arrow indicates the cleavage site. (FIG. 8C) Mouse Exon 51 sequence (SEQ ID NO: 967) with the predicted exon splicing enhancers (ESEs) located at the site of sgRNA is indicated in red. Human Exon 51 sequence (SEQ ID NO: 968) with the predicted exon splicing enhancers (ESEs) located at the site of sgRNA is indicated in red. (FIG. 8D) Mouse and human ESE sites of exon 51 predicted using ESEfinder3. (FIG. 8E) Cas9 was expressed in the presence or absence of mouse sgRNA-sgRNA-51-SA2 in 293 T human cells and gene editing was monitored by T7E1 assay in fluorescence-based cell sorted (FACS) (+) and non-sorted cells (-). GFP was used as a control. Undigested PCR products (upper panel) and T7E1 digestion (lower panel) are shown on a 2% agarose gel. Black arrowhead indicates the undigested 771bp PCR band. Green arrowheads in the lower panel indicate the cut bands by T7E1 assay. M denotes size marker lane. bp indicates the length of the marker bands. (FIG. 8F) Sequence of representative indels aligned with sgRNA sequence (indicated in blue) revealing deletions and insertions (SEQ ID NOS 969-978, respectively, in order of appearance). Black arrowhead indicates the cleavage site.

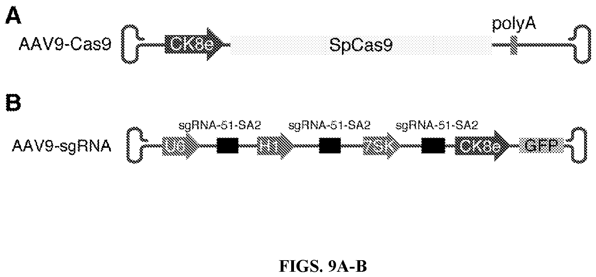

**FIG. 9A-B**. Schematic illustration of AAV injection plasmids and strategy. (FIG. 9A) Muscle creatine kinase 8 (CK8) promoter to express SpCas9. (FIG. 9B) Triplicate using rAAV9-sgRNA plasmid containing 3 copies of sgRNA-ex51-SA2. U6, H1 and 7SK promoter for RNA polymerase III to express sgRNA.

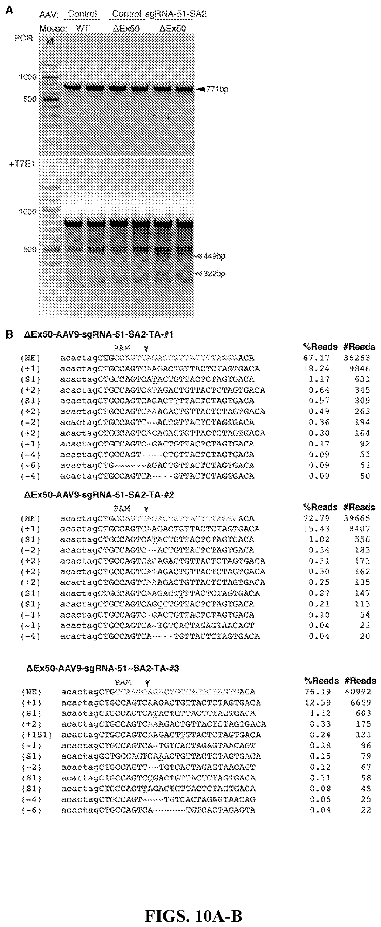

**FIG. 10A-B**. In vivo ***Dmd* gene editing.** (FIG. 10A) Undigested PCR products (upper panel) and T7E1 digestion (lower panel) are shown on a 2% agarose gel of TA (tibialis anterior) muscle samples from WT and ΔEx50 mice 3 weeks after intramuscular injection with AAV9-sgRNA-SA2 and AAV9-Cas9 expression vectors. Controls were injected with only AAV9-Cas9 not AAV9-sgRNA-SA2. Black arrowhead in the upper panel indicates the 771bp PCR band. Green arrowheads in the lower panel indicate the cut bands by T7E1 assay. M denotes size marker lane. bp indicates the length of the marker bands. N=4. (FIG. 10B) Genomic deep sequencing analysis of PCR amplicons generated across the exon 51 target site in ΔEx50 mice injected with AAV9-sgRNA-51-SA2 and AAV9-Cas9. Sequence of representative indels aligned with sgRNA sequence (indicated in blue) revealing insertions (highlighted in green) and deletions (highlighted in red). (SEQ ID NOS 979-1014, respectively, in order of appearance). Black arrowheads indicate the cleavage site. n=3.

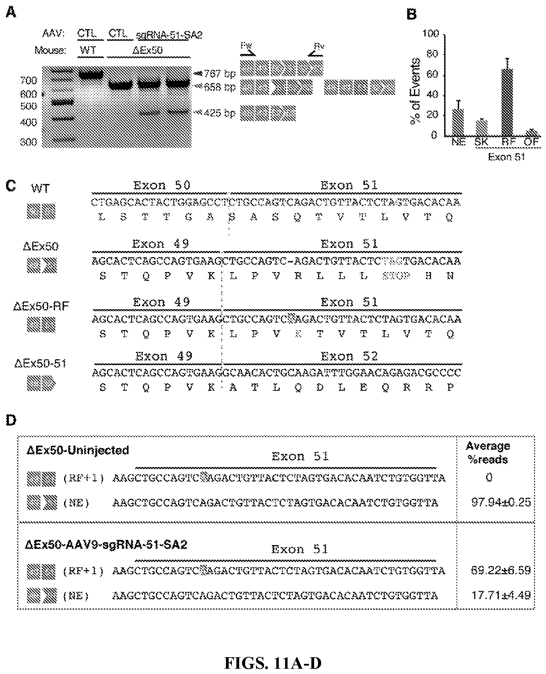

**FIG. 11A-D**. RT-PCR analysis of correction of reading frame. (FIG. 11A) RT-PCR of RNA from tibialis anterior muscles of wildtype (WT) and ΔEx50 mice 3 weeks after intramuscular injection of the sgRNA-51-SA2 and Cas9 expression vectors. Lower dystrophin bands indicate deletion of exon 51. Primer positions in exons 48 and 53 are indicated (Fw, Rv). (FIG. 11B) Percentage of events detected at exon 51 after AAV9-sgRNA-51-SA2 treatment using RT-PCR sequence analysis of TOPO-TA generated clones. For each of 4 different samples, we generated and sequenced 40 clones. RT-PCR products were divided into 4 groups: not-edited (NE), exon51-skipped (SK), reframed (RF) and out of frame (OF). (FIG. 11C) Sequence of the RT-PCR products of the ΔEx50-51 mouse dystrophin lower band confirmed that exon 49 spliced directly to exon 52, excluding exon 51. Sequence of RT-PCR products of ΔEx50 reframed (ΔEx50-RF). Fig. 11C discloses SEQ ID NOS 1015-1022, respectively, in order of appearance. (FIG. 11D) Deep sequencing analysis of RT-PCR products from the upper band containing ΔEx50 not-edited (NE) and ΔEx50-RF. Sequence of RT-PCR products revealing insertions (highlighted in green) and deletions (highlighted in red). n=4. Data are represented as mean ± SEM. Fig. 11D discloses SEQ ID NOS 1023-1026, respectively, in order appearance.

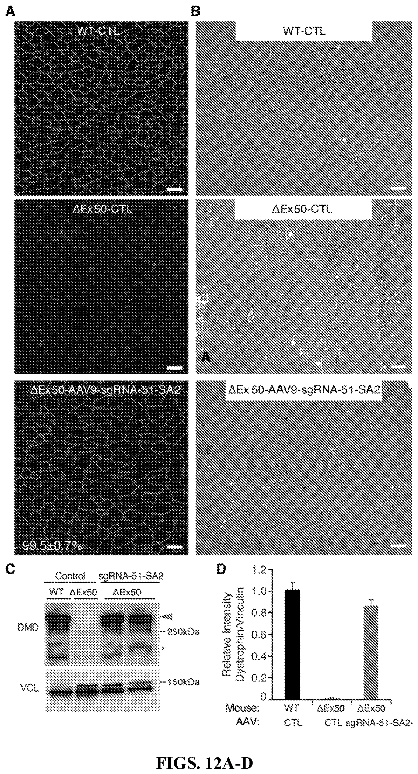

**FIG. 12A-D**. Intramuscular injection of AAV9-Cas9 and AAV9-sgRNA-51-SA2 **corrects dystrophin expression.** (FIG. 12A) Tibialis anterior muscles of ΔEx50 mice were injected with AAV9 vector encoding sgRNA and Cas9 and analyzed 3 weeks later by immunostaining for dystrophin. Wildtype control (WT-CTL) mice and ΔEx50 mice control (ΔEx50-CTL) were injected with AAV9-Cas9 alone without sgRNAs. Percentages of dystrophin-positive myofibers in ΔEx50-CTL mice and in treated ΔEx50 mice (ΔEx50-AAV9-sgRNA-51-SA2 and AAV9-Cas9) compared to WT-CTL are indicated in each panel. (FIG. 12B) Hematoxylin and eosin (H&E) staining of tibialis anterior muscles. (FIG. 12C) Western blot analysis of dystrophin (DMD) and vinculin (VCL) expression in tibialis anterior muscles 3 weeks after intramuscular injection. (FIG. 12D) Quantification of dystrophin expression from blots after normalization to vinculin. Asterisk indicates non-specific immunoreactive bands. n=5 for AAV9-sgRNA-51-SA2. Scale bar: 50µm

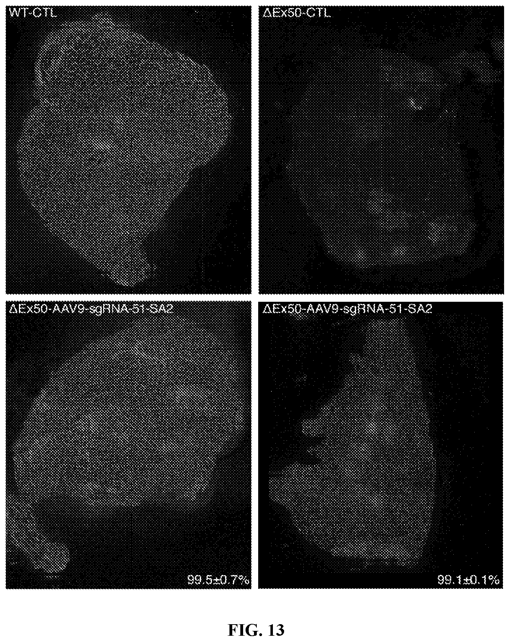

**FIG. 13****. Rescue of dystrophin expression following intramuscular injections of AAV9-Cas9 and AAV9-sgRNA-51-SA2 in ΔEx50 mouse model.** Dystrophin immunohistochemistry of entire tibialis anterior muscle. CTL mice were injected with AAV9-Cas9 alone without AAV9-sgRNA-51-SA2. n=5

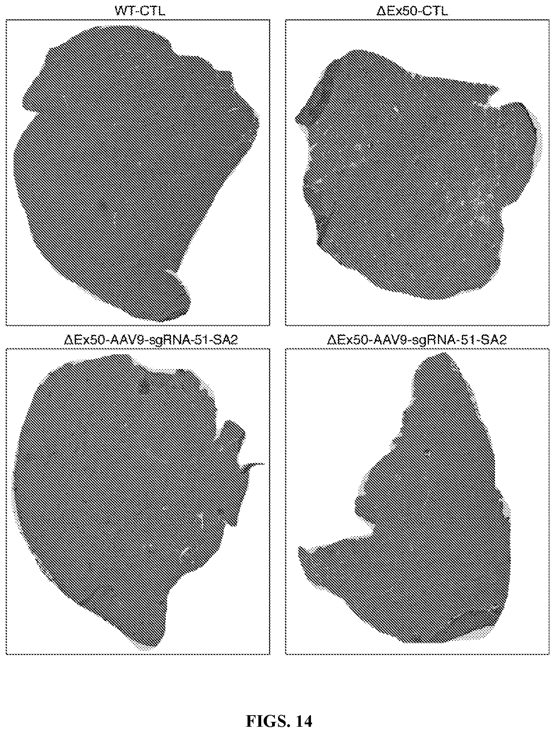

**FIG. 14****. Histological improvement of injected muscle after 3 weeks.** Histochemistry of tibialis anterior muscle by hematoxylin and eosin (H&E) staining.

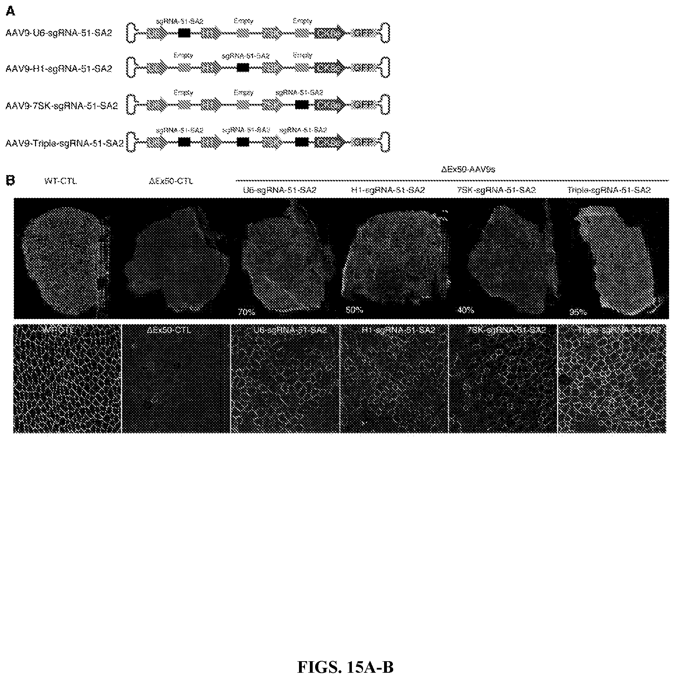

**FIG. 15A-B**. Rescue of dystrophin expression following intramuscular injections **of AAV9-Cas9 combined with different AAV9s expressing single copy or triple copy of sgRNA in ΔEx50 mouse model.** (FIG. 15A) The U6, H1 and 7SK promoters for RNA polymerase III were each individually used to express sgRNA in a single copy (AAV9-U6-sgRNA-51-SA2; AAV9-H1-sgRNA-51-SA2; AAV9-7SK-sgRNA-51-SA2) or triple copy. (FIG. 15B) Dystrophin immunohistochemistry of entire tibialis anterior muscle. Control (CTL) mice were injected with AAV9-Cas9 alone without AAV9-sgRNA-51-SA2.

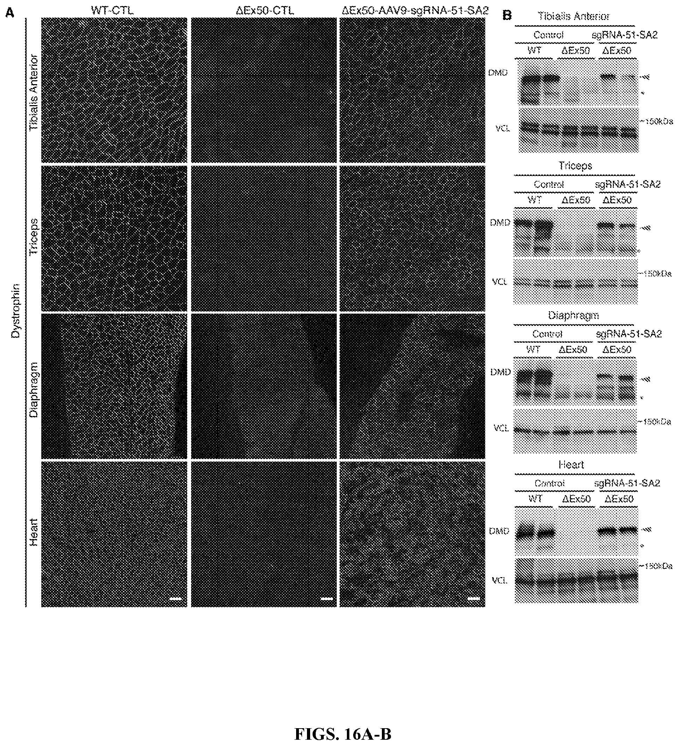

**FIG. 16A-B**. Rescue of dystrophin expression 4 weeks after systemic delivery of **AAV9-Cas9 and AAV9-sgRNA-51-SA2 in ΔEx50 mice.** (FIG. 16A) Dystrophin immunostaining of tibialis anterior (TA), triceps, diaphragm and cardiac muscles 8 weeks after systemic injection of AAV9-sgRNA-51. (FIG. 16B) Western blot analysis of dystrophin (DMD) and vinculin (VCL) expression in TA, triceps, diaphragm muscles and heart. n=5 for each group. Scale bar: 50µm.

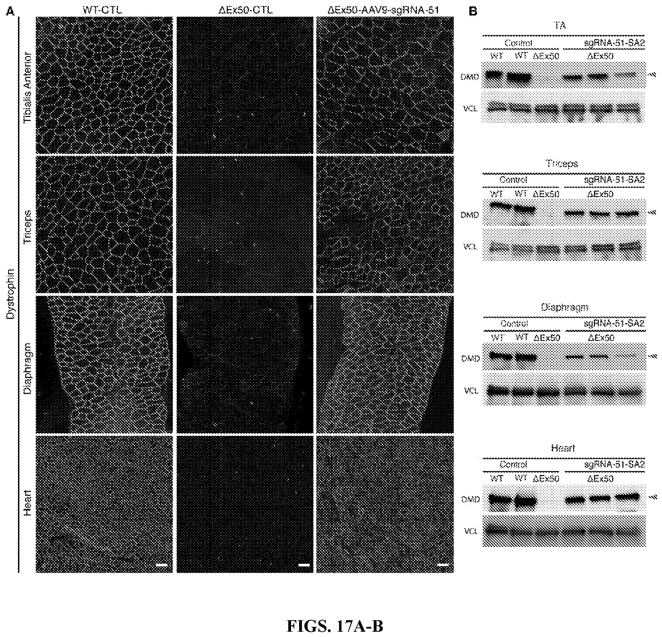

**FIG. 17A-B**. Rescue of dystrophin expression 8 weeks after systemic delivery of **AAV9-Cas9 and AAV9-sgRNA-51-SA2 in ΔEx50 mice.** (FIG. 17A) Dystrophin immunostaining of tibialis anterior (TA), triceps, diaphragm and cardiac muscles 8 weeks after systemic injection of AAV9-sgRNA-51. (FIG. 17B) Western blot analysis of dystrophin (DMD) and vinculin (VCL) expression in TA, triceps, diaphragm muscles and heart. n=5 for each group. Scale bar: 50µm.

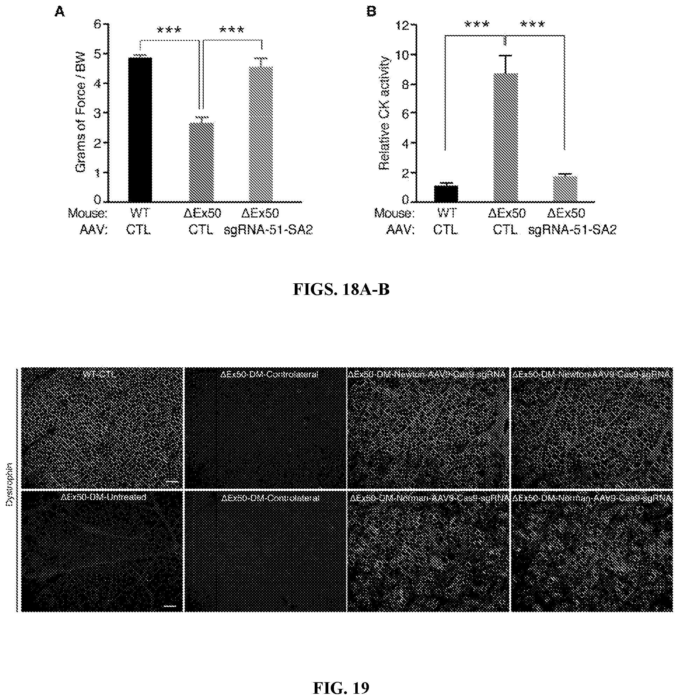

**FIG. 18A-B**. Functional improvement 4 weeks after systemic delivery of AAV9-**Cas9 and AAV9-sgRNA-51-SA2 in ΔEx50 mice.** (FIG. 18A) Wildtype (WT) mice, control ΔEx50 mice and ΔEx50 mice treated with AAV9-sgRNA-51 (ΔEx50-AAV9-sgRNA-51) were subjected to grip strength testing to measure muscle performance (grams of force). (FIG. 18B) Serum creatine kinase (CK) was measured in WT, ΔEx50 and ΔEx50-AAV9-sgRNA-51 mice. n=5. Asterisk indicates non-specific immunoreactive bands. Data are represented as mean ± SEM.
**FIG. 19****. Correction of dystrophin expression 6 weeks after intra-muscular injection in ΔEx50-MD Dog.** Dystrophin immunohistochemistry staining of cranial tibialis muscle of a wild-type dog (Nathan), a ΔEx50-MD Dog untreated, and two ΔEx50-MD Dogs (Norman and Newton) contralateral uninjected and AAV9-Cas9-sgRNA injected cranial tibialis muscle. Scale bar: 50µm.

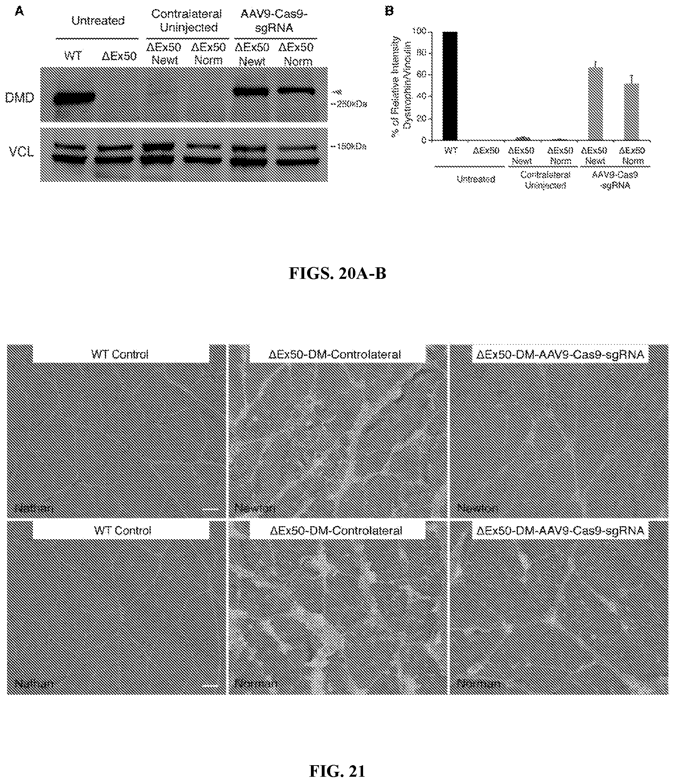

**FIGS. 20A-B**. Correction of dystrophin expression 6 weeks after intra-muscular **injection in ΔEx50-MD Dog.** (FIG. 20A) Western blot analysis of dystrophin (DMD) and vinculin (VCL) of cranial tibialis muscle of a wild-type dog (Nathan), a ΔEx50-MD Dog untreated, and two ΔEx50-MD Dogs (Norman and Newton) contralateral uninjected and AAV9-Cas9-sgRNA injected cranial tibialis muscle. (FIG. 20B) Quantification of dystrophin expression from two independent blots after normalization to vinculin.
**FIG. 21****. Histological improvement of injected muscle after 6 weeks in ΔEx50-MD Dog.** Histochemistry by hematoxylin and eosin (H&E) staining of cranial tibialis muscle of wild-type dog (Nathan), ΔEx50-MD Dogs (Norman and Newton) contralateral uninjected and AAV9-Cas9-sgRNA injected cranial tibialis muscle. Scale bar: 50 µm.

### DETAILED DESCRIPTION

DMD is a new mutation syndrome with more than 4,000 independent mutations that have been identified in humans (world-wide web at dmd.nl). The majority of patient mutations include deletions that cluster in a hotspot, and thus a therapeutic approach for skipping certain exon applies to large group of patients. The rationale of the exon skipping approach is based on the genetic difference between DMD and Becker muscular dystrophy (BMD) patients. In DMD patients, the reading frame of dystrophin mRNA is disrupted resulting in prematurely truncated, non-functional dystrophin proteins. BMD patients have mutations in the DMD gene that maintain the reading frame allowing the production of internally deleted, but partially functional dystrophins leading to much milder disease symptoms compared to DMD patients.

The present invention provides a nucleic acid comprises a sequence encoding a first DMD guide RNA targeting a first genomic target sequence, a sequence encoding a second DMD guide RNA targeting a second genomic target sequence, a sequence encoding a third DMD guide RNA targeting a third genomic target sequence, a first promoter wherein the first promoter drives expression of the sequence encoding the first DMD guide RNA, a second promoter wherein the second promoter drives expression of the sequence encoding the second DMD guide RNA, and a third promoter wherein the third promoter drives expression of the sequence encoding the third DMD guide RNA, wherein the first genomic target sequence, the second genomic target sequence and the third genomic sequence each comprise a dystrophin splice acceptor site, and wherein the sequence encoding the first DMD guide RNA, the sequence encoding the second DMD guide RNA genomic, and the sequence encoding the third DMD guide RNA target sequence are identical. The nucleic acids of the present invention may be presented in the form of compositions and may be for use in methods for treating DMD. In some embodiments, nucleic acids of the present invention may be provided in the form of an AAV construct, wherein the AAV construct comprises a nucleic acid encoding three promoters that each drive expression of a DMD guide RNA. Using compositions and methods disclosed herein, a more robust and safe form of genome editing was achieved in a humanized mouse model for DMD with a deletion in exon 50, and in a ΔEx50-MD Dog. These and other aspects of the disclosure are reproduced below.

### I. Duchenne Muscular Dystrophy

### A. Background

Duchenne muscular dystrophy (DMD) is a recessive X-linked form of muscular dystrophy, affecting around 1 in 5000 boys, which results in muscle degeneration and premature death. The disorder is caused by a mutation in the gene dystrophin (see GenBank Accession NO. NC_000023.11), located on the human X chromosome, which codes for the protein dystrophin (GenBank Accession No. AAA53189; SEQ ID NO: 5), the sequence of which is reproduced below:
1 mlwweevedc yeredvqkkt ftkwvnaqfs kfgkqhienl fsdlqdgrrl ldllegltgq
61 klpkekgstr vhalnnvnka lrvlqnnnvd lvnigstdiv dgnhkltlgl iwniilhwqv
121 knvmknimag lqqtnsekil lswvrqstrn ypqvnvinft tswsdglaln alihshrpdl
181 fdwnsvvcqq satqrlehaf niaryqlgie klldpedvdt typdkksilm yitslfqvlp
241 qqvsieaiqe vemlprppkv tkeehfqlhh qmhysqqitv slaqgyerts spkprfksya
301 ytqaayvtts dptrspfpsq hleapedksf gsslmesevn ldryqtalee vlswllsaed
361 tlqaqgeisn dvevvkdqfh thegymmdlt ahqgrvgnil qlgskligtg klsedeetev
421 qeqmnllnsr weclrvasme kqsnlhrvlm dlqnqklkel ndwltkteer trkmeeeplg
481 pdledlkrqv qqhkvlqedl eqeqvrvnsl thmvvvvdes sgdhataale eqlkvlgdrw
541 anicrwtedr wvllqdillk wqrlteeqcl fsawlseked avnkihttgf kdqnemlssl
601 qklavlkadl ekkkqsmgkl yslkqdllst lknksvtqkt eawldnfarc wdnlvqklek
661 staqisqavt ttqpsltqtt vmetvttvtt reqilvkhaq eelpppppqk krqitvdsei
721 rkrldvdite lhswitrsea vlqspefaif rkegnfsdlk ekvnaierek aekfrklqda
781 srsaqalveq mvnegvnads ikqaseqlns rwiefcqlls erlnwleyqn niiafynqlq
841 qleqmtttae nwlkiqpttp septaiksql kickdevnrl sglqpqierl kiqsialkek
901 gqgpmfldad fvaftnhfkq vfsdvqarek elqtifdtlp pmryqetmsa irtwvqqset
961 klsipqlsvt dyeimeqrlg elqalqsslq eqqsglyyls ttvkemskka pseisrkyqs
1021 efeeiegrwk klssqlvehc qkleeqmnkl rkiqnhiqtl kkwmaevdvf lkeewpalgd
1081 seilkkqlkq crllvsdiqt iqpslnsvne ggqkikneae pefasrlete lkelntqwdh
1141 mcqqvyarke alkgglektv slqkdlsemh ewmtqaeeey lerdfeyktp delqkaveem
1201 krakeeaqqk eakvklltes vnsviaqapp vaqealkkel etlttnyqwl ctrlngkckt
1261 leevwacwhe llsylekank wlnevefklk ttenipggae eisevldsle nlmrhsednp
1321 nqirilaqtl tdggvmdeli neeletfnsr wrelheeavr rqklleqsiq saqetekslh
1381 liqesltfid kqlaayiadk vdaaqmpqea qkiqsdltsh eisleemkkh nqgkeaaqrv
1441 lsqidvaqkk lqdvsmkfrl fqkpanfelr lqeskmilde vkmhlpalet ksveqevvqs
1501 qlnhcvnlyk slsevkseve mviktgrqiv qkkqtenpke ldervtalkl hynelgakvt
1561 erkqqlekcl klsrkmrkem nvltewlaat dmeltkrsav egmpsnldse vawgkatqke
1621 iekqkvhlks itevgealkt vlgkketlve dklsllnsnw iavtsraeew lnllleyqkh
1681 metfdqnvdh itkwiiqadt lldesekkkp qqkedvlkrl kaelndirpk vdstrdqaan
1741 lmanrgdhcr klvepqisel nhrfaaishr iktgkasipl keleqfnsdi qkllepleae
1801 iqqgvnlkee dfnkdmnedn egtvkellqr gdnlqqritd erkreeikik qqllqtkhna
1861 lkdlrsqrrk kaleishqwy qykrqaddll kclddiekkl aslpeprder kikeidrelq
1921 kkkeelnavr rqaeglsedg aamaveptqi qlskrwreie skfaqfrrln faqihtvree
1981 tmmvmtedmp leisyvpsty lteithvsqa lleveqllna pdlcakdfed lfkqeeslkn
2041 ikdslqqssg ridiihskkt aalqsatpve rvklqealsq ldfqwekvnk mykdrqgrfd
2101 rsvekwrrfh ydikifnqwl teaeqflrkt qipenwehak ykwylkelqd gigqrqtvvr
2161 tlnatgeeii qqssktdasi lqeklgslnl rwqevckqls drkkrleeqk nilsefqrdl
2221 nefvlwleea dniasiplep gkeqqlkekl eqvkllveel plrqgilkql netggpvlvs
2281 apispeeqdk lenklkqtnl qwikvsralp ekqgeieaqi kdlgqlekkl edleeqlnhl
2341 llwlspirnq leiynqpnqe gpfdvqetei avqakqpdve eilskgqhly kekpatqpvk
2401 rkledlssew kavnrllqel rakqpdlapg lttigasptq tvtlvtqpvv tketaiskle
2461 mpsslmlevp aladfnrawt eltdwlslld qviksqrvmv gdledinemi ikqkatmqdl
2521 eqrrpqleel itaaqnlknk tsnqeartii tdrieriqnq wdevqehlqn rrqqlnemlk
2581 dstqwleake eaeqvlgqar akleswkegp ytvdaiqkki tetkqlakdl rqwqtnvdva
2641 ndlalkllrd ysaddtrkvh miteninasw rsihkrvser eaaleethrl lqqfpldlek
2701 flawlteaet tanvlqdatr kerlledskg vkelmkqwqd lqgeieahtd vyhnldensq
2761 kilrslegsd davllqrrld nmnfkwselr kkslnirshl eassdqwkrl hlslqellvw
2821 lqlkddelsr qapiggdfpa vqkqndvhra fkrelktkep vimstletvr iflteqpleg
2881 leklyqepre lppeeraqnv trllrkqaee vnteweklnl hsadwqrkid etlerlqelq
2941 eatdeldlkl rqaevikgsw qpvgdllids lqdhlekvka lrgeiaplke nvshvndlar
3001 qlttlgiqls pynlstledl ntrwkllqva vedrvrqlhe ahrdfgpasq hflstsvqgp
3061 weraispnkv pyyinhetqt tcwdhpkmte lyqsladlnn vrfsayrtam klrrlqkalc
3121 ldllslsaac daldqhnlkq ndqpmdilqi inclttiydr leqehnnlvn vplcvdmcln
3181 wllnvydtgr tgrirvlsfk tgiislckah ledkyrylfk qvasstgfcd qrrlglllhd
3241 siqiprqlge vasfggsnie psvrscfqfa nnkpeieaal fldwmrlepq smvwlpvlhr
3301 vaaaetakhq akcnickecp iigfryrslk hfnydicqsc ffsgrvakgh kmhypmveyc
3361 tpttsgedvr dfakvlknkf rtkryfakhp rmgylpvqtv legdnmetpv tlinfwpvds
3421 apasspqlsh ddthsriehy asrlaemens ngsylndsis pnesiddehl liqhycqsln
3481 qdsplsqprs paqilisles eergeleril adleeenrnl qaeydrlkqq hehkglsplp
3541 sppemmptsp qsprdaelia eakllrqhkg rlearmqile dhnkqlesql hrlrqlleqp
3601 qaeakvngtt vsspstslqr sdssqpmllr vvgsqtsdsm geedllsppq dtstgleevm
3661 eqlnnsfpss rgrntpgkpm redtm.

In humans, dystrophin mRNA contains 79 exons. Dystrophin mRNA is known to be alternatively spliced, resulting in various isoforms. Exemplary dystrophin isoforms are listed in Table 1.

**Table 1: Dystrophin isoforms**

| **Sequence Name** | **Nucleic Acid Accession No.** | **Nucleic Acid SEQ ID NO:** | **Protein Accession No.** | **Protein SEQ ID NO:** | **Description** |
|---|---|---|---|---|---|
| DMD Genomic Sequence | NC_000023.11 (positions 31119219 to 33339609) | None | None | None | Sequence from Human X Chromosome (at positions Xp21.2 to p21.1) from Assembly GRCh38.p7 (GCF 000001405.33) |
| Dystrophin Dp427c isoform | NM_000109.3 | 6 | NP_000100.2 | 7 | Transcript Variant: transcript Dp427c is expressed predominantly in neurons of the cortex and the CA regions of the hippocampus. |
| | | | | | It uses a unique promoter/exon 1 located about 130 kb upstream of the Dp427m transcript promoter. |
| | | | | | The transcript includes the common exon 2 of transcript Dp427m and has a similar length of 14 kb. The Dp427c isoform contains a unique N-terminal MED sequence, instead of the MLWWEEVEDCY sequence (SEQ ID NO: 949) of isoform Dp427m. The remainder of isoform Dp427c is identical to isoform Dp427m. |
| Dystrophin Dp427m isoform | NM_004006.2 | 8 | NP_003997.1 | 9 | Transcript Variant: transcript Dp427m encodes the main dystrophin protein found in muscle. As a result of alternative promoter use, exon 1 encodes a unique N-terminal MLWWEEVEDCY (SEQ ID NO: 949) aa sequence. |
| Dystrophin Dp427p 1 isoform | NM_004009.3 | 10 | NP_004000.1 | 11 | Transcript Variant: transcript Dp427p1 initiates from a unique promoter/exon 1 located in what corresponds to the first intron of transcript Dp427m. The transcript adds the common exon 2 of Dp427m and has a similar length (14 kb). The Dp427p 1 isoform replaces the MLWWEEVEDCY (SEQ ID NO: 949) -start of Dp427m with a unique N-terminal MSEVSSD (SEQ ID NO: 950) aa sequence. |
| Dystrophin Dp260-1 isoform | NM _ 004011.3 | 12 | NP_004002.2 | 13 | Transcript Variant: transcript Dp260-1 uses exons 30-79, and originates from a promoter/exon 1 sequence located in intron 29 of the dystrophin gene. As a result, Dp260-1 contains a 95 bp exon 1 encoding a unique N-terminal 16 aa MTEIILLIFFPAYFLN-sequence (SEQ ID NO: 951) that replaces amino acids 1-1357 of the full-length dystrophin product (Dp427m isoform). |
| Dystrophin Dp260-2 isoform | NM_004012.3 | 14 | NP_004003.1 | 15 | Transcript Variant: transcript Dp260-2 uses exons 30-79, starting from a promoter/exon 1 sequence located in intron 29 of the dystrophin gene that is alternatively spliced and lacks N-terminal amino acids 1-1357 of the full length dystrophin (Dp427m isoform). The Dp260-2 transcript encodes a unique N-terminal MSARKLRNLSYKK sequence (SEQ ID NO: 952). |
| Dystrophin Dp140 isoform | NM_004013.2 | 16 | NP_004004.1 | 17 | Transcript Variant: Dp140 transcripts use exons 45-79, starting at a promoter/exon 1 located in intron 44. Dp 140 transcripts have a long (1 kb) 5' UTR since translation is initiated in exon 51 (corresponding to aa 2461 of dystrophin). In addition to the alternative promoter and exon 1, differential splicing of exons 71-74 and 78 produces at least five Dp 140 isoforms. Of these, this transcript (Dp 140) contains all of the exons. |
| Dystrophin Dp116 isoform | NM_004014.2 | 18 | NP_004005.1 | 19 | Transcript Variant: transcript Dp116 uses exons 56-79, starting from a promoter/exon 1 within intron 55. As a result, the Dp116 isoform contains a unique N-terminal MLHRKTYHVK aa sequence (SEQ ID NO: 953), instead of aa 1-2739 of dystrophin. Differential splicing produces several Dp116-subtypes. The Dp116 isoform is also known as S-dystrophin or apo-dystrophin-2. |
| Dystrophin Dp71 isoform | NM_004015.2 | 20 | NP_004006.1 | 21 | Transcript Variant: Dp71 transcripts use exons 63-79 with a novel 80- to 100-nt exon containing an ATG start site for a new coding sequence of 17 nt. The short coding sequence is in-frame with the consecutive dystrophin sequence from exon 63. Differential splicing of exons 71 and 78 produces at least four Dp71 isoforms. Of these, this transcript (Dp71) includes both exons 71 and 78. |
| Dystrophin Dp71b isoform | NM_004016.2 | 22 | NP_004007.1 | 23 | Transcript Variant: Dp71 transcripts use exons 63-79 with a novel 80- to 100-nt exon containing an ATG start site for a new coding sequence of 17 nt. The short coding sequence is in-frame with the consecutive dystrophin sequence from exon 63. Differential splicing of exons 71 and 78 produces at least four Dp71 isoforms. Of these, this transcript (Dp71b) lacks exon 78 and encodes a protein with a different C-terminus than Dp71 and Dp71a isoforms. |
| Dystrophin Dp71a isoform | NM_004017.2 | 24 | NP_004008.1 | 25 | Transcript Variant: Dp71 transcripts use exons 63-79 with a novel 80- to 100-nt exon containing an ATG start site for a new coding sequence of 17 nt. The short coding sequence is in-frame with the consecutive dystrophin sequence from exon 63. Differential splicing of exons 71 and 78 produces at least four Dp71 isoforms. Of these, this transcript (Dp71a) lacks exon 71. |
| Dystrophin Dp71ab isoform | NM_004018.2 | 26 | NP_004009.1 | 27 | Transcript Variant: Dp71 transcripts use exons 63-79 with a novel 80- to 100-nt exon containing an ATG start site for a new coding sequence of 17 nt. The short coding sequence is in-frame with the consecutive dystrophin sequence from exon 63. Differential splicing of exons 71 and 78 produces at least four Dp71 isoforms. Of these, this transcript (Dp71ab) lacks both exons 71 and 78 and encodes a protein with a C-terminus like isoform Dp71b. |
| Dystrophin Dp40 isoform | NM_004019.2 | 28 | NP_004010.1 | 29 | Transcript Variant: transcript Dp40 uses exons 63-70. The 5' UTR and encoded first 7 aa are identical to that in transcript Dp71, but the stop codon lies at the splice junction of the exon/intron 70. The 3' UTR includes nt from intron 70 which includes an alternative polyadenylation site. The Dp40 isoform lacks the normal C-terminal end of full-length dystrophin (aa 3409-3685). |
| Dystrophin Dp 140c isoform | NM_004020.3 | 30 | NP_004011.2 | 31 | Transcript Variant: Dp140 transcripts use exons 45-79, starting at a promoter/exon 1 located in intron 44. Dp 140 transcripts have a long (1 kb) 5' UTR since translation is initiated in exon 51 (corresponding to aa 2461 of dystrophin). In addition to the alternative promoter and exon 1, differential splicing of exons 71-74 and 78 produces at least five Dp 140 isoforms. Of these, this transcript (Dp140c) lacks exons 71-74. |
| Dystrophin Dp 140b isoform | NM_004021.2 | 32 | NP_004012.1 | 33 | Transcript Variant: Dp140 transcripts use exons 45-79, starting at a promoter/exon 1 located in intron 44. Dp 140 transcripts have a long (1 kb) 5' UTR since translation is initiated in exon 51 (corresponding to aa 2461 of dystrophin). In addition to the alternative promoter and exon 1, differential splicing of exons 71-74 and 78 produces at least five Dp 140 isoforms. Of these, this transcript (Dp 140b) lacks exon 78 and encodes a protein with a unique C-terminus. |
| Dystrophin Dp 140ab isoform | NM_004022.2 | 34 | NP_004013.1 | 35 | Transcript Variant: Dp140 transcripts use exons 45-79, starting at a promoter/exon 1 located in intron 44. Dp 140 transcripts have a long (1 kb) 5' UTR since translation is initiated in exon 51 (corresponding to aa 2461 of dystrophin). In addition to the alternative promoter and exon 1, differential splicing of exons 71-74 and 78 produces at least five Dp 140 isoforms. Of these, this transcript (Dp140ab) lacks exons 71 and 78 and encodes a protein with a unique C-terminus. |
| Dystrophin Dp 140bc isoform | NM_004023.2 | 36 | NP_004014.1 | 37 | Transcript Variant: Dp140 transcripts use exons 45-79, starting at a promoter/exon 1 located in intron 44. Dp 140 transcripts have a long (1 kb) 5' UTR since translation is initiated in exon 51 (corresponding to aa 2461 of dystrophin). In addition to the alternative promoter and exon 1, differential splicing of exons 71-74 and 78 produces at least five Dp 140 isoforms. Of these, this transcript (Dp140bc) lacks exons 71-74 and 78 and encodes a protein with a unique C-terminus. |
| Dystrophin isoform X2 | XM_006724469.3 | 38 | XP_006724532.1 | 39 | |
| Dystrophin isoform X5 | XM_011545467.1 | 40 | XP_011543769.1 | 41 | |
| Dystrophin isoform X6 | XM_006724473.2 | 42 | XP_006724536.1 | 43 | |
| Dystrophin isoform X8 | XM_006724475.2 | 44 | XP_006724538.1 | 45 | |
| Dystrophin isoform X4 | XM_017029328.1 | 46 | XP_016884817.1 | 47 | |
| Dystrophin isoform X1 | XM_006724468.2 | 48 | XP_006724531.1 | 49 | |
| Dystrophin isoform X13 | XM_017029331.1 | 50 | XP_016884820.1 | 51 | |
| Dystrophin isoform X3 | XM_006724470.3 | 52 | XP_006724533.1 | 53 | |
| Dystrophin isoform X7 | XM_006724474.3 | 54 | XP_006724537.1 | 55 | |
| Dystrophin isoform X9 | XM_011545468.2 | 56 | XP_011543770.1 | 57 | |
| Dystrophin isoform X11 | XM_017029330.1 | 58 | XP_016884819.1 | 59 | |
| Dystrophin isoform X10 | XM_017029329.1 | 865 | XP_016884818.1 | 866 | |
| Dystrophin isoform X12 | XM_011545469.1 | 867 | XP_011543771.1 | 868 | |

The murine dystrophin protein has the following amino acid sequence (Uniprot Accession No. P11531, SEQ. ID. NO: 869):

1 MWWVDCYRDV KKTTKWNASK GKHDNSDDGK RDGTGKKKGS
TRVHANNVNK ARVKNNVDVN

61 GSTDVDGNHK TGWNHWVKNV MKTMAGTNSK SWVRSTRNYV
NVNTSSWSDG ANAHSHRDDW

121 NSVVSHSATR HANAKCGKDD VATTYDKKSM YTSVVSAVMR
TSSKVTRHHH MHYSTVSAGY

181 TSSSKRKSYA TAAYVATSDS TSYSHARDKS DSSMTVNDSY TAVSWSADTR
AGSNDVVKHA

241 HGMMDTSHGV GNVGSVGKGK SDAVMNNSRW CRVASMKSKH
KVMDNKKDDW TKTRTKKMGD

301 DKCVHKVDVR VNSTHMVVVV DSSGDHATAA KVGDRWANCR
WTDRWVDKWH TCSTWSKDAM

361 KNTSGKDNMM SSHKSTKDKK KTMKSSNDSA KNKSVTKMWM
NARWDNTKKS SASAVTTTST

421 TTVMTVTMVT TRMVKHAKKR TVDSRKRDVD THSWTRSAVS
SAVYRKGNSD KVNAARKAKR

481 KDASRSAAVM ANGVNASRAS NSRWTCSRVN WYTNTYNMTT
TANKTSTTST AKSKCKDVNR

541 SAKSKKGGMD ADVATNHNHD GVRAKKTDTM RYTMSSRTWS
SKSVYSVTYM RGKASSKNGN

601 YSDTVKMAKK ASCKYSGHWK KSSVSCKHMN KRKNHKTKWM
AVDVKWAGDA KKKCRVGDTS

661 NSVNGGKKSA ASRTRNTWDH CRVYTRKAKA GDKTVSKDSM
HWMTAYRDYK TDTAVMKRAK

721 AKTKVKTTVN SVAHASAAKK TTTNYWCTRN GKCKTVWACW
HSYKANKWNV KKTMNVAGTV

781 SNMHHSNNRA TTDGGVMDNT NSRWRHAVRK KSSAKSHSDK
AAYTDKVDAA MAKSDTSHSM

841 KKHNGKDANR VSDVAKKDVS MKRKANRSKM DVKMHATKSV
VSSHCVNYKS SVKSVMVKTG

901 RVKKTNKDRV TAKHYNGAKV TRKKCKSRKM RKMNVTWAAT
DTTKRSAVGM SNDSVAWGKA

961 TKKKAHKSVT GSKMVGKKTV DKSNSNWAVT SRVWNYKHMT
DNTKWHADDS KKKKDKRKAM

1021 NDMRKVDSTR DAAKMANRGD HCRKVVSNRR AASHRKTGKA
SKNSDKAGVN KDNKDMSDNG

1081 TVNRGDNRTD RKRKKTKHNA KDRSRRKKAS HWYYKRADDK
CDKKASRDRK KDRKKKNAVR

1141 RAGSNGAAMA VTSKRWRSNA RRNAHTHTMV VTTDMDVSYV
STYTSHASVD HNTCAKDDKS

1201 KNKDNSGRDH KKKTAASATS MKVKVAVAMD GKHRMYKRGR
DRSVKWRHHY DMKVNWNVKK

1261 TNNWHAKYKW YKDGGRAVVR TNATGSSKTD VNKGSSRWHD
CKARRKRKNV SRDNVWADNA

1321 TGDKVKARGK NTGGAVVSAR DKKKKTNWKV SRAKGVHKDR
DHWSRNYNSA GDKVTVHGKA

1381 DVRSKGHYKK STVKRKDRSW AVNHRRTKDR AGSTTGASAS
TVTVTSVVTK TVSKMSSVAA

1441 DNRAWTTDWS DRVKSRVMVG DDNMKKATDR RTAANKNKTS
NARTTDRRWD VNRRNMKDST

1501 WAKAVGVRGK DSWKGHTVDA KKTTKAKDRR SVDVANDAKR
DYSADDTRKV HMTNNTSWGN

1561 HKRVSAATHR DKSWTATTAN VDASRKKDSR GVRMKWDGTH
TDYHNDNGKR SGSDARRDNM

1621 NKWSKKSNRS HASSDWKRHS VWKDDSRAGG DAVKNDHRAK
RKTKVMSTTV RTGKYRRANV

1681 TRRKAVNAWD KNRSADWRKD ARAADDKRAV KGSWVGDDSD
HKVKARGAKN VNRVNDAHTT

1741 GSYNSTDNTR WRVAVDRVRH AHRDGASHST SVGWRASNKV
YYNHTTTCWD HKMTYSADNN

1801 VRSAYRTAMK RRKACDSSAA CDADHNKNDM DNCTTYDRHN
NVNVCVDMCN WNVYDTGRTG

1861 RRVSKTGSCK AHDKYRYKVA SSTGCDRRGH DSRGVASGGS
NSVRSCANNK AADWMRSMVW

1921 VHRVAAATAK HAKCNCKCGR YRSKHNYDCS CSGRVAKGHK
MHYMVYCTTT SGDVRDAKVK

1981 NKRTKRYAKH RMGYVTVGDN MTVTNWVDSA ASSSHDDTHS
RHYASRAMNS NGSYNDSSNS

2041 DDHHYCSNDS SRSASSRGRA DNRNAYDRKH HKGSSMMTSS
RDAAAKRHKG RARMDHNKSH

2101 RRAAKVNGTT VSSSTSRSDS SMRVVGSTSS MGDSDTSTGV MNNSSSRGRN
AGKMRDTM

Dystrophin is an important component within muscle tissue that provides structural stability to the dystroglycan complex (DGC) of the cell membrane. While both sexes can carry the mutation, females are rarely affected with the skeletal muscle form of the disease.

Mutations vary in nature and frequency. Large genetic deletions are found in about 60-70% of cases, large duplications are found in about 10% of cases, and point mutants or other small changes account for about 15-30% of cases. Bladen *et al.* (2015), who examined some 7000 mutations, catalogued a total of 5,682 large mutations (80% of total mutations), of which 4,894 (86%) were deletions (1 exon or larger) and 784 (14%) were duplications (1 exon or larger). There were 1,445 small mutations (smaller than 1 exon, 20% of all mutations), of which 358 (25%) were small deletions and 132 (9%) small insertions, while 199 (14%) affected the splice sites. Point mutations totaled 756 (52% of small mutations) with 726 (50%) nonsense mutations and 30 (2%) missense mutations. Finally, 22 (0.3%) mid-intronic mutations were observed. In addition, mutations were identified within the database that would potentially benefit from novel genetic therapies for DMD including stop codon read-through therapies (10% of total mutations) and exon skipping therapy (80% of deletions and 55% of total mutations).

### B. Symptoms

Symptoms usually appear in boys between the ages of 2 and 3 and may be visible in early infancy. Even though symptoms do not appear until early infancy, laboratory testing can identify children who carry the active mutation at birth. Progressive proximal muscle weakness of the legs and pelvis associated with loss of muscle mass is observed first. Eventually this weakness spreads to the arms, neck, and other areas. Early signs may include pseudohypertrophy (enlargement of calf and deltoid muscles), low endurance, and difficulties in standing unaided or inability to ascend staircases. As the condition progresses, muscle tissue experiences wasting and is eventually replaced by fat and fibrotic tissue (fibrosis). By age 10, braces may be required to aid in walking but most patients are wheelchair dependent by age 12. Later symptoms may include abnormal bone development that lead to skeletal deformities, including curvature of the spine. Due to progressive deterioration of muscle, loss of movement occurs, eventually leading to paralysis. Intellectual impairment may or may not be present but if present, does not progressively worsen as the child ages. The average life expectancy for males afflicted with DMD is around 25.

The main symptom of Duchenne muscular dystrophy, a progressive neuromuscular disorder, is muscle weakness associated with muscle wasting with the voluntary muscles being first affected, especially those of the hips, pelvic area, thighs, shoulders, and calves. Muscle weakness also occurs later, in the arms, neck, and other areas. Calves are often enlarged. Symptoms usually appear before age 6 and may appear in early infancy. Other physical symptoms are:
1. Awkward manner of walking, stepping, or running - (patients tend to walk on their forefeet, because of an increased calf muscle tone. Also, toe walking is a compensatory adaptation to knee extensor weakness.)
2. Frequent falls.
3. Fatigue.
4. Difficulty with motor skills (running, hopping, jumping).
5. Lumbar hyperlordosis, possibly leading to shortening of the hip-flexor muscles. This has an effect on overall posture and a manner of walking, stepping, or running.
6. Muscle contractures of Achilles tendon and hamstrings impair functionality because the muscle fibers shorten and fibrose in connective tissue.
7. Progressive difficulty walking.
8. Muscle fiber deformities.
9. Pseudohypertrophy (enlarging) of tongue and calf muscles. The muscle tissue is eventually replaced by fat and connective tissue, hence the term pseudohypertrophy.
10. Higher risk of neurobehavioral disorders (*e.g.,* ADHD), learning disorders (dyslexia), and non-progressive weaknesses in specific cognitive skills (in particular short-term verbal memory), which are believed to be the result of absent or dysfunctional dystrophin in the brain.
11. Eventual loss of ability to walk (usually by the age of 12).
12. Skeletal deformities (including scoliosis in some cases).
13. Trouble getting up from lying or sitting position.

The condition can often be observed clinically from the moment the patient takes his first steps, and the ability to walk usually completely disintegrates between the time the boy is 9 to 12 years of age. Most men affected with DMD become essentially "paralyzed from the neck down" by the age of 21. Muscle wasting begins in the legs and pelvis, then progresses to the muscles of the shoulders and neck, followed by loss of arm muscles and respiratory muscles. Calf muscle enlargement (pseudohypertrophy) is quite obvious. Cardiomyopathy particularly (dilated cardiomyopathy) is common, but the development of congestive heart failure or arrhythmia (irregular heartbeat) is only occasional.

A positive Gowers' sign reflects the more severe impairment of the lower extremities muscles. The child helps himself to get up with upper extremities: first by rising to stand on his arms and knees, and then "walking" his hands up his legs to stand upright. Affected children usually tire more easily and have less overall strength than their peers. Creatine kinase (CPK-MM) levels in the bloodstream are extremely high. An electromyography (EMG) shows that weakness is caused by destruction of muscle tissue rather than by damage to nerves. Genetic testing can reveal genetic errors in the Xp21 gene. A muscle biopsy (immunohistochemistry or immunoblotting) or genetic test (blood test) confirms the absence of dystrophin, although improvements in genetic testing often make this unnecessary.

DMD patients may suffer from:
1. Abnormal heart muscle (cardiomyopathy).
2. Congestive heart failure or irregular heart rhythm (arrhythmia).
3. Deformities of the chest and back (scoliosis).
4. Enlarged muscles of the calves, buttocks, and shoulders (around age 4 or 5). These muscles are eventually replaced by fat and connective tissue (pseudohypertrophy).
5. Loss of muscle mass (atrophy).
6. Muscle contractures in the heels, legs.
7. Muscle deformities.
8. Respiratory disorders, including pneumonia and swallowing with food or fluid passing into the lungs (in late stages of the disease).

### C. Causes

Duchenne muscular dystrophy (DMD) is caused by a mutation of the dystrophin gene at locus Xp21, located on the short arm of the X chromosome. Dystrophin is responsible for connecting the cytoskeleton of each muscle fiber to the underlying basal lamina (extracellular matrix), through a protein complex containing many subunits. The absence of dystrophin permits excess calcium to penetrate the sarcolemma (the cell membrane). Alterations in calcium and signaling pathways cause water to enter into the mitochondria, which then burst.

In skeletal muscle dystrophy, mitochondrial dysfunction gives rise to an amplification of stress-induced cytosolic calcium signals and an amplification of stress-induced reactive-oxygen species (ROS) production. In a complex cascading process that involves several pathways and is not clearly understood, increased oxidative stress within the cell damages the sarcolemma and eventually results in the death of the cell. Muscle fibers undergo necrosis and are ultimately replaced with adipose and connective tissue.

DMD is inherited in an X-linked recessive pattern. Females will typically be carriers for the disease while males will be affected. Typically, a female carrier will be unaware they carry a mutation until they have an affected son. The son of a carrier mother has a 50% chance of inheriting the defective gene from his mother. The daughter of a carrier mother has a 50% chance of being a carrier and a 50% chance of having two normal copies of the gene. In all cases, an unaffected father will either pass a normal Y to his son or a normal X to his daughter. Female carriers of an X-linked recessive condition, such as DMD, can show symptoms depending on their pattern of X-inactivation.

Exon deletions preceding exon 51 of the human *DMD* gene, which disrupt the open reading frame (ORF) by juxtaposing out of frame exons, represent the most common type of human DMD mutation. Skipping of exon 51 can, in principle, restore the DMD ORF in 13% of DMD patients with exon deletions.

Duchenne muscular dystrophy has an incidence of 1 in 5000 male infants. Mutations within the dystrophin gene can either be inherited or occur spontaneously during germline transmission. A table of exemplary but non-limiting mutations and corresponding models are set forth below:

**Table 2: Dystrophin mutations and corresponding mouse models**

| **Deletion, small insertion and nonsense mutations** | **Name of Mouse Model** |
|---|---|
| Exon 44 | ΔEx44 |
| Exon 52 | ΔEx52 |
| Exon 43 | ΔEx43 |

### D. Diagnosis

Genetic counseling is advised for people with a family history of the disorder. Duchenne muscular dystrophy can be detected with about 95% accuracy by genetic studies performed during pregnancy.

**DNA test.** The muscle-specific isoform of the dystrophin gene is composed of 79 exons, and DNA testing and analysis can usually identify the specific type of mutation of the exon or exons that are affected. DNA testing confirms the diagnosis in most cases.

**Muscle biopsy.** If DNA testing fails to find the mutation, a muscle biopsy test may be performed. A small sample of muscle tissue is extracted (usually with a scalpel instead of a needle) and a dye is applied that reveals the presence of dystrophin. Complete absence of the protein indicates the condition.

Over the past several years DNA tests have been developed that detect more of the many mutations that cause the condition, and muscle biopsy is not required as often to confirm the presence of Duchenne's.

**Prenatal tests.** DMD is carried by an X-linked recessive gene. Males have only one X chromosome, so one copy of the mutated gene will cause DMD. Fathers cannot pass X-linked traits on to their sons, so the mutation is transmitted by the mother.

If the mother is a carrier, and therefore one of her two X chromosomes has a DMD mutation, there is a 50% chance that a female child will inherit that mutation as one of her two X chromosomes, and be a carrier. There is a 50% chance that a male child will inherit that mutation as his one X chromosome, and therefore have DMD.

Prenatal tests can tell whether their unborn child has the most common mutations. There are many mutations responsible for DMD, and some have not been identified, so genetic testing only works when family members with DMD have a mutation that has been identified.

Prior to invasive testing, determination of the fetal sex is important; while males are sometimes affected by this X-linked disease, female DMD is extremely rare. This can be achieved by ultrasound scan at 16 weeks or more recently by free fetal DNA testing. Chorion villus sampling (CVS) can be done at 11-14 weeks, and has a 1% risk of miscarriage. Amniocentesis can be done after 15 weeks, and has a 0.5% risk of miscarriage. Fetal blood sampling can be done at about 18 weeks. Another option in the case of unclear genetic test results is fetal muscle biopsy.

### E. Treatment

There is no current cure for DMD, and an ongoing medical need has been recognized by regulatory authorities. Phase 1-2a trials with exon skipping treatment for certain mutations have halted decline and produced small clinical improvements in walking. Treatment is generally aimed at controlling the onset of symptoms to maximize the quality of life, and may include the following:
1. Corticosteroids such as prednisolone and deflazacort increase energy and strength and defer severity of some symptoms.
2. Randomized control trials have shown that beta-2-agonists increase muscle strength but do not modify disease progression. Follow-up time for most RCTs on beta2-agonists is only around 12 months and hence results cannot be extrapolated beyond that time frame.
3. Mild, non-jarring physical activity such as swimming is encouraged. Inactivity (such as bed rest) can worsen the muscle disease.
4. Physical therapy is helpful to maintain muscle strength, flexibility, and function.
5. Orthopedic appliances (such as braces and wheelchairs) may improve mobility and the ability for self-care. Form-fitting removable leg braces that hold the ankle in place during sleep can defer the onset of contractures.
6. Appropriate respiratory support as the disease progresses is important.
Comprehensive multi-disciplinary care standards/guidelines for DMD have been developed by the Centers for Disease Control and Prevention (CDC), and are available at www.treatnmd.eu/dmd/care/diagnosis-management-DMD.

DMD generally progresses through five stages, as outlined in Bushby et al., Lancet Neurol., 9(1): 77-93 (2010) and Bushby et al., Lancet Neurol., 9(2): 177-198 (2010). During the presymptomatic stage, patients typically show developmental delay, but no gait disturbance. During the early ambulatory stage, patients typically show the Gowers' sign, waddling gait, and toe walking. During the late ambulatory stage, patients typically exhibit an increasingly labored gait and begin to lose the ability to climb stairs and rise from the floor. During the early non-ambulatory stage, patients are typically able to self-propel for some time, are able to maintain posture, and may develop scoliosis. During the late non-ambulatory stage, upper limb function and postural maintenance is increasingly limited.

In some embodiments, treatment is initiated in the presymptomatic stage of the disease. In some embodiments, treatment is initiated in the early ambulatory stage. In some embodiments, treatment is initiated in the late ambulatory stage. In embodiments, treatment is initiated during the early non-ambulatory stage. In embodiments, treatment is initiated during the late non-ambulatory stage.

### 1. Physical Therapy

Physical therapists are concerned with enabling patients to reach their maximum physical potential. Their aim is to:
1. minimize the development of contractures and deformity by developing a programme of stretches and exercises where appropriate,
2. anticipate and minimize other secondary complications of a physical nature by recommending bracing and durable medical equipment, and
3. monitor respiratory function and advise on techniques to assist with breathing exercises and methods of clearing secretions.

### 2. Respiration Assistance

Modern "volume ventilators/respirators," which deliver an adjustable volume (amount) of air to the person with each breath, are valuable in the treatment of people with muscular dystrophy related respiratory problems. The ventilator may require an invasive endotracheal or tracheotomy tube through which air is directly delivered, but, for some people non-invasive delivery through a face mask or mouthpiece is sufficient. Positive airway pressure machines, particularly bi-level ones, are sometimes used in this latter way. The respiratory equipment may easily fit on a ventilator tray on the bottom or back of a power wheelchair with an external battery for portability.

Ventilator treatment may start in the mid to late teens when the respiratory muscles can begin to collapse. If the vital capacity has dropped below 40% of normal, a volume ventilator/respirator may be used during sleeping hours, a time when the person is most likely to be under ventilating ("hypoventilating"). Hypoventilation during sleep is determined by a thorough history of sleep disorder with an oximetry study and a capillary blood gas (See Pulmonary Function Testing). A cough assist device can help with excess mucus in lungs by hyperinflation of the lungs with positive air pressure, then negative pressure to get the mucus up. If the vital capacity continues to decline to less than 30 percent of normal, a volume ventilator/respirator may also be needed during the day for more assistance. The person gradually will increase the amount of time using the ventilator/respirator during the day as needed.

### F. Prognosis

Duchenne muscular dystrophy is a progressive disease which eventually affects all voluntary muscles and involves the heart and breathing muscles in later stages. The life expectancy is currently estimated to be around 25, but this varies from patient to patient. Recent advancements in medicine are extending the lives of those afflicted. The *Muscular Dystrophy Campaign,* which is a leading UK charity focusing on all muscle disease, states that "with high standards of medical care young men with Duchenne muscular dystrophy are often living well into their 30s."

In rare cases, persons with DMD have been seen to survive into the forties or early fifties, with the use of proper positioning in wheelchairs and beds, ventilator support (via tracheostomy or mouthpiece), airway clearance, and heart medications, if required. Early planning of the required supports for later-life care has shown greater longevity in people living with DMD.

Curiously, in the mdx mouse model of Duchenne muscular dystrophy, the lack of dystrophin is associated with increased calcium levels and skeletal muscle myonecrosis. The intrinsic laryngeal muscles (ILM) are protected and do not undergo myonecrosis. ILM have a calcium regulation system profile suggestive of a better ability to handle calcium changes in comparison to other muscles, and this may provide a mechanistic insight for their unique pathophysiological properties. The ILM may facilitate the development of novel strategies for the prevention and treatment of muscle wasting in a variety of clinical scenarios.

### II. Other Diseases Benefiting from Genome Editing

In addition to finding application in the treatment of DMD, the inventors' multiple-guide approach can be advantageously applied to other disease states where such intervention could result in correction of the underlying causal genetic defects. Some of these are described briefly below.

**Limb Girdle Muscular Dystrophy.** Limb-girdle muscular dystrophy (LGMD) or Erb's muscular dystrophy is a genetically and clinically heterogeneous group of rare muscular dystrophies. It is characterized by progressive muscle wasting which affects predominantly hip and shoulder muscles. LGMD has an autosomal pattern of inheritance and currently has no known cure. The symptoms of an individual with Limb-girdle muscular dystrophy (LGMD) generally has difficulty walking, going both up and down stairs and raising from a chair. Difficulty bending over and falling on a regular basis are also common. Difficulty lifting certain objects is also a common presentation of LGMD as well as difficulty holding your arms out or above your head. Eventually the ability to walk/run deteriorates.

The disease inevitably gets worse over time, although progression is more rapid in some patients than others. Eventually the disease can affect other muscles such as the ones located in the face. The disease commonly leads to dependence on a wheelchair within years of symptom onset, but there is high inter-patient variability, with some patients maintaining mobility.

The muscle weakness is generally symmetric, proximal, and slowly progressive. In most cases, pain is not present with LGMD, and mental function is not affected. LGMD can begin in childhood, adolescence, young adulthood or even later, the age of onset is usually between 10 and 30. Both genders are affected equally, when limb-girdle muscular dystrophy begins in childhood the progression appears to be faster and the disease more disabling. When the disorder begins in adolescence or adulthood the disease is generally not as severe and progresses more slowly. There is no sensory neuropathy or autonomic or visceral dysfunction at presentation.

In terms of the genetics of LGMD is an inherited disorder, though it may be inherited as a dominant or recessive genetic defect. The result of the defect is that the muscles cannot properly form certain proteins needed for normal muscle function. Several different proteins can be affected, and the specific protein that is absent or defective identifies the specific type of muscular dystrophy. Among the proteins affected in LGMD are α, β, γ and δ sarcoglycans. The sarcoglycanopathies could be possibly amenable to gene therapy.

**Dysferlinopathy.** Dysferlinopathy is an autosomal recessive neuromuscular disorder caused by a deficiency of functional dysferlin protein due to mutations in the dysferlin gene. Dysferlinopathy is characterized by progressive muscle wasting and is most often clinically diagnosed as Limb-girdle muscular dystrophy type 2B (LGMD2B) or Miyoshi muscular dystrophy 1 (MMD1; a type of distal muscular dystrophy), depending on the initial pattern of muscle involvement at diagnosis. Dysferlinopathy is a rare disease, the exact incidence of which has not yet been determined.

The symptoms of dysferlinopathy usually manifest in early adulthood between the ages of 16 and 25 and primarily affect the skeletal muscle of the limbs and the limb girdles (hips and shoulders), leaving critical muscles such as the heart and diaphragm largely unaffected. The majority of dysferlinopathy patients become non-ambulant within 10-20 years of diagnosis, but life expectancy is normal. There is a large amount of variability in the age of onset and progression of the disease.

Although LGMD2B and MMD1 are both caused by dysferlin deficiency, a diagnosis of LGMD2B is given when weakness initially presents in the proximal muscles (thighs and upper arms) while a diagnosis of MMD1 is given when weakness initially presents in the distal muscles (calves and lower arms). In both cases, weakness eventually progresses to include both distal and proximal muscles. Both LGMD2B and MMD1 are very difficult to diagnose, and patients are often misdiagnosed many times before they are successfully diagnosed with dysferlinopathy.

While "dysferlinopathy" simply refers to the absence of the dysferlin protein, LGMD2B and Miyoshi myopathy 1 are the confirmed diagnosis only when mutations in the dysferlin gene can be detected. It is essential that patients receive a genetic diagnosis for participation in clinical studies and trials specific to dysferlinopathy. The Jain Foundation is a non-profit family organization (www.jain-foundation.org) that helps patients with limb girdle muscular dystrophy to achieve a genetic diagnosis by organizing testing and covering the cost.

**Titin Myopathy.** Titin, also known as connectin, is a protein that, in humans, is encoded by the *TTN* gene. Titin is a giant protein, greater than 1 µm in length, that functions as a molecular spring which is responsible for the passive elasticity of muscle. It is composed of 244 individually folded protein domains connected by unstructured peptide sequences. These domains unfold when the protein is stretched and refold when the tension is removed.

Titin is important in the contraction of striated muscle tissues. It connects the Z line to the M line in the sarcomere. The protein contributes to force transmission at the Z line and resting tension in the I band region. It limits the range of motion of the sarcomere in tension, thus contributing to the passive stiffness of muscle. Variations in the sequence of titin between different types of muscle (*e.g.,* cardiac or skeletal) have been correlated with differences in the mechanical properties of these muscles.

Titin is a large abundant protein of striated muscle. Titin's primary functions are to stabilize the thick filament, center it between the thin filaments, prevent overstretching of the sarcomere, and to recoil the sarcomere like a spring after it is stretched. An N-terminal Z-disc region and a C-terminal M-line region bind to the Z-line and M-line of the sarcomere, respectively, so that a single titin molecule spans half the length of a sarcomere. Titin also contains binding sites for muscle-associated proteins so it serves as an adhesion template for the assembly of contractile machinery in muscle cells. It has also been identified as a structural protein for chromosomes. Considerable variability exists in the I-band, the M-line and the Z-disc regions of titin. Variability in the I-band region contributes to the differences in elasticity of different titin isoforms and, therefore, to the differences in elasticity of different muscle types. Of the many titin variants identified, five are described with complete transcript information available.

Mutations anywhere within the unusually long sequence of this gene can cause premature stop codons or other defects. Titin mutations are associated with hereditary myopathy with early respiratory failure, early-onset myopathy with fatal cardiomyopathy, core myopathy with heart disease, centronuclear myopathy, Limb-girdle muscular dystrophy type 2J, familial dilated cardiomyopathy 9, hypertrophic cardiomyopathy and tibial muscular dystrophy. Further research also suggests that no genetically linked form of any dystrophy or myopathy can be safely excluded from being caused by a mutation on the TTN gene. Truncating mutations in dilated cardiomyopathy patients are most commonly found in the A region; although truncations in the upstream I region might be expected to prevent translation of the A region entirely, alternative splicing creates some transcripts that do not encounter the premature stop codon, ameliorating its effect. Autoantibodies to titin are produced in patients with the autoimmune disease scleroderma.

**Congenital myopathy "central core disease" caused by mutation in ryanodine receptor (mutation with pseudoexons).** Central core disease (CCD), also known as central core myopathy, is an autosomal dominant congenital myopathy (inborn muscle disorder). It was first described by Shy and Magee in 1956. It is characterized by the appearance of the myofibril under the microscope.

The symptoms of CCD are variable, but usually involve hypotonia (decreased muscle tone) at birth, mild delay in child development (highly variable between cases), weakness of the facial muscles, and skeletal malformations such as scoliosis and hip dislocation.

Symptoms may be present at birth or may appear at any stage of life. There appears to be a growing number of people who do not become symptomatic until adulthood to middle age. While generally not progressive, again there appears to be a growing number of people who do experience a slow clinically significant progression of symptomatology. These cases may hypothetically be due to the large number of gene mutations of ryanodine receptor malfunction, and with continued research may in fact be found to be clinical variants.

The diagnosis is made on the combination of typical symptoms and the appearance on biopsy (tissue sample) from muscle. The name derives from the typical appearance of the biopsy on light microscopy, where the muscle cells have cores that are devoid of mitochondria and specific enzymes. Respiratory insufficiency develops in a small proportion of cases. Creatine kinase and electromyography (EMG) tend to be normal.

Central core disease is inherited in an autosomal dominant fashion. Most cases have demonstrable mutations in the ryanodine receptor type 1 (*RYR1*) gene, which are often *de novo* (newly developed). People with CCD are at risk for malignant hyperthermia (MH) when receiving general anesthesia.

There is no specific treatment but triggering anesthetics are avoided and relatives are screened for *RYR1* mutations as these may make them susceptible to MH.

**Myotonic Dystrophy.** Myotonic dystrophy is a long term genetic disorder that affects muscle function. Symptoms include gradually worsening muscle loss and weakness. Muscles often contract and are unable to relax. Other symptoms may include cataracts, intellectual disability, and heart conduction problems. In men there may be early balding and an inability to have children.

Myotonic dystrophy is an autosomal-dominant disorder which is typically inherited from a person's parents. There are two main types: type 1 (DM1) due to mutations in the DMPK gene and type 2 (DM2) due to mutations in the CNBP gene. The disorder generally worsens in each generation. A type of DM1 may be apparent at birth. DM2 is generally milder. They are types of muscular dystrophy. Diagnosis is confirmed by genetic testing.

There is no cure. Treatments may include braces or wheelchairs, pacemakers, and non-invasive positive pressure ventilation. The medications mexiletine or carbamazepine are occasionally helpful. Pain if it occurs may be treated with tricyclic antidepressants and nonsteroidal anti-inflammatory drugs (NSAIDs).

Myotonic dystrophy affects more than 1 in 8,000 people worldwide. While myotonic dystrophy can occur at any age, onset is typically in the 20s and 30s. It is the most common form of muscular dystrophy that begins in adulthood. It was first described in 1909 with the underlying cause of type 1 determined in 1992.

Presentation of symptoms and signs varies considerably by form (DM1/DM2), severity and even unusual DM2 phenotypes. DM1 symptoms for DM2 include problems with executive function (*e.g.,* organization, concentration, word-finding) and hypersomnia. Conduction abnormalities are more common in DM1 than DM2, but all people are advised to have an annual ECG. Both types are also associated with insulin resistance. Myotonic dystrophy may have a cortical cataract with a blue dot appearance, or a posterior subcapsular cataract.

DM2 is generally milder than DM1, with generally fewer DM2 people requiring assistive devices than DM1 people. In addition, the severe congenital form that affects babies in DM1 has not been found in DM2 and the early onset of symptoms is rarely noted to appear in younger people in the medical literature.

Symptoms may appear at any time from infancy to adulthood. DM causes general weakness, usually beginning in the muscles of the hands, feet, neck, or face. It slowly progresses to involve other muscle groups, including the heart. DM affects a wide variety of other organ systems as well.

Myotonic dystrophy is a genetic condition which is inherited in an autosomal dominant pattern and thus will be passed along to 50% of a carrier's offspring, on average. Myotonic dystrophy is one of several known trinucleotide repeat disorders. Certain areas of DNA have repeated sequences of two or three nucleotides.

Myotonic dystrophy (DM) is an inherited disease. A severe form of DM, congenital myotonic dystrophy, may appear in newborns of mothers who have DM. Congenital myotonic dystrophy can also be inherited via the paternal gene, although it is said to be relatively rare. Congenital means that the condition is present from birth.

In DM1, the affected gene is called *DMPK,* which codes for myotonic dystrophy protein kinase, a protein expressed predominantly in skeletal muscle. The gene is located on the long arm of chromosome 19.

In DM1, there is an expansion of the cytosine-thymine-guanine (CTG) triplet repeat in the *DMPK* gene. Between 5 and 37 repeats is considered normal, while individuals with between 38 and 49 repeats are considered to have a pre-mutation and are at risk of having children with further expanded repeats and, therefore, symptomatic disease. Individuals with greater than 50 repeats are almost invariably symptomatic, with some noted exceptions. Longer repeats are usually associated with earlier onset and more severe disease.

DMPK alleles with greater than 37 repeats are unstable and additional trinucleotide repeats may be inserted during cell division in mitosis and meiosis. Consequently, the children of individuals with premutations or mutations inherit DMPK alleles which are longer than their parents and therefore are more likely to be affected or display an earlier onset and greater severity of the condition, a phenomenon known as anticipation. Interestingly, paternal transmission of the condition is very uncommon, possibly due to selection pressures against sperm with expanded repeats, but anticipation tends to be less severe than in cases of maternal inheritance.

The RNA from the expanded trinucleotide repeat region forms intranucleoplasmic hairpin loops due to the extensive hydrogen bonding between C-G base pairs, and it has been demonstrated that these sequester the splicing regulator MBNL1 to form distinctive foci by labelling it with GFP and a probe oligonucleotide with the red-fluorescent dye Cyanine5 (Cy5).

DM2 is caused by a defect of the *ZNF9* gene on chromosome 3. The specific defect is a repeat of the cytosine-cytosine-thymine-guanosine (CCTG) tetranucleotide in the *ZNF9* gene. As it involves the repeat of four nucleotides, it is not a trinucleotide repeat disorder, but rather a tetranucleotide repeat disorder.

The repeat expansion for DM2 is much larger than for DM1, ranging from 75 to over 11,000 repeats. Unlike in DM1, the size of the repeated DNA expansion in DM2 does not appear to make a difference in the age of onset or disease severity. Anticipation appears to be less significant in DM2 and most current reviews only report mild anticipation as a feature of DM2.

**Friedreich's ataxia.** Friedreich's ataxia is an autosomal recessive inherited disease that causes progressive damage to the nervous system. It manifests in initial symptoms of poor coordination such as gait disturbance; it can also lead to scoliosis, heart disease and diabetes, but does not affect cognitive function. The disease progresses until a wheelchair is required for mobility. Its incidence in the general population is roughly 1 in 50,000.

The particular genetic mutation (expansion of an intronic GAA triplet repeat in the FXN gene) leads to reduced expression of the mitochondrial protein frataxin. Over time this deficiency causes the aforementioned damage, as well as frequent fatigue due to effects on cellular metabolism.

The ataxia of Friedreich's ataxia results from the degeneration of nervous tissue in the spinal cord, in particular sensory neurons essential (through connections with the cerebellum) for directing muscle movement of the arms and legs. The spinal cord becomes thinner and nerve cells lose some of their myelin sheath (the insulating covering on some nerve cells that helps conduct nerve impulses).

Symptoms typically begin sometime between the ages of 5 to 15 years, but in Late Onset FA may occur in the 20s or 30s. Symptoms include any combination, but not necessarily all, of muscle weakness in the arms and legs, loss of coordination, vision impairment, hearing impairment, slurred speech, curvature of the spine, high plantar arches (pes cavus deformity of the foot), diabetes (about 20% of people with Friedreich's ataxia develop carbohydrate intolerance and 10% develop diabetes mellitus) and heart disorders (*e.g.,* atrial fibrillation, and resultant tachycardia (fast heart rate) and hypertrophic cardiomyopathy).

It presents before 22 years of age with progressive staggering or stumbling gait and frequent falling. Lower extremities are more severely involved. The symptoms are slow and progressive. Long-term observation shows that many patients reach a plateau in symptoms in the patient's early adulthood. On average, after 10-15 years with the disease, patients are usually wheelchair bound and require assistance with all activities of daily living.

The following physical signs may be detected on physical examination: cerebellar signs such as nystagmus, fast saccadic eye movements, truncal ataxia, dysarthria, dysmetria; lower motor neuron lesions, such as absent deep tendon reflexes; pyramidal signs such as extensor plantar responses, and distal weakness are commonly found; dorsal column aspects, such as loss of vibratory and proprioceptive sensation occurs. Cardiac involvement occurs in 91% of patients, including cardiomegaly (up to dilated cardiomyopathy), symmetrical hypertrophy, heart murmurs, and conduction defects. Median age of death is 35 years, while females have better prognosis with a 20-year survival of 100% as compared to 63% in men. Twenty percent of cases are found in association with diabetes mellitus.

Friedreich's ataxia is an autosomal recessive disorder that occurs when the FXN gene contains amplified intronic GAA repeats (an example of trinucleotide repeat expansion). The FXN gene encodes the protein frataxin. GAA repeat expansion causes frataxin levels to be reduced. Frataxin is an iron-binding protein responsible for forming iron-sulphur clusters. One result of frataxin deficiency is mitochondrial iron overload which can cause damage to many proteins. The exact role of frataxin in normal physiology remains unclear. The gene is located on chromosome 9.

The mutant gene contains expanded GAA triplet repeats in the first intron; in a few pedigrees, point mutations have been detected. Because the defect is located in an intron (which is removed from the mRNA transcript between transcription and translation), this mutation does not result in the production of abnormal frataxin proteins. Instead, the mutation causes gene silencing (*i.e.,* the mutation decreases the transcription of the gene) through induction of a heterochromatin structure in a manner similar to position-effect variegation.

Besides reducing expression of frataxin, long tracts of GAA repeats induce chromosome breaks in *in vivo* yeast studies.

**Huntington's disease.** Huntington's disease (HD), also known as Huntington's chorea, is an inherited disorder that results in death of brain cells. The earliest symptoms are often subtle problems with mood or mental abilities. A general lack of coordination and an unsteady gait often follow. As the disease advances, uncoordinated, jerky body movements become more apparent. Physical abilities gradually worsen until coordinated movement becomes difficult and the person is unable to talk. Mental abilities generally decline into dementia. The specific symptoms vary somewhat between people. Symptoms usually begin between 30 and 50 years of age, but can start at any age. The disease may develop earlier in life in each successive generation. About 8% of cases start before the age of 20 years and typically present with symptoms more similar to Parkinson's disease. People with HD often underestimate the degree of their problems.

HD is typically inherited from a person's parents with 10% of cases due to a new mutation. The disease is caused by an autosomal dominant mutation in either of an individual's two copies of a gene called *Huntingtin.* This means a child of an affected person typically has a 50% chance of inheriting the disease. The *Huntingtin* gene provides the genetic information for a protein that is also called "huntingtin." Expansion of CAG (cytosine-adenine-guanine) triplet repeats in the gene coding for the Huntingtin protein results in an abnormal protein, which gradually damages cells in the brain, through mechanisms that are not fully understood. Diagnosis is by genetic testing, which can occur at any point in time regardless of whether or not symptoms are present. This fact raises several ethical debates: the age at which an individual is considered mature enough to choose testing; whether parents have the right to have their children tested; and managing confidentiality and disclosure of test results.

There is no cure for HD. Full-time care is required in the later stages of the disease. Treatments can relieve some symptoms and in some improve quality of life. The best evidence for treatment of the movement problems is with tetrabenazine. HD affects about 4 to 15 in 100,000 people of European descent. It is rare among Japanese and occurs at an unknown rate in Africa. The disease affects men and women equally. Complications such as pneumonia, heart disease, and physical injury from falls reduce life expectancy. Suicide is the cause of death in about 9% of cases. Death typically occurs fifteen to twenty years from when the disease was first detected.

Symptoms of Huntington's disease most commonly become noticeable between the ages of 35 and 44 years, but they can begin at any age from infancy to old age. In the early stages, there are subtle changes in personality, cognition, and physical skills. The physical symptoms are usually the first to be noticed, as cognitive and behavioral symptoms are generally not severe enough to be recognized on their own at the earlier stages. Almost everyone with Huntington's disease eventually exhibits similar physical symptoms, but the onset, progression and extent of cognitive and behavioral symptoms vary significantly between individuals.

The most characteristic initial physical symptoms are jerky, random, and uncontrollable movements called chorea. Chorea may be initially exhibited as general restlessness, small unintentionally initiated or uncompleted motions, lack of coordination, or slowed saccadic eye movements. These minor motor abnormalities usually precede more obvious signs of motor dysfunction by at least three years. The clear appearance of symptoms such as rigidity, writhing motions or abnormal posturing appear as the disorder progresses. These are signs that the system in the brain that is responsible for movement has been affected. Psychomotor functions become increasingly impaired, such that any action that requires muscle control is affected. Common consequences are physical instability, abnormal facial expression, and difficulties chewing, swallowing, and speaking. Eating difficulties commonly cause weight loss and may lead to malnutrition. Sleep disturbances are also associated symptoms. Juvenile HD differs from these symptoms in that it generally progresses faster and chorea is exhibited briefly, if at all, with rigidity being the dominant symptom. Seizures are also a common symptom of this form of HD.

Cognitive abilities are progressively impaired. Especially affected are executive functions which include planning, cognitive flexibility, abstract thinking, rule acquisition, initiation of appropriate actions, and inhibition of inappropriate actions. As the disease progresses, memory deficits tend to appear. Reported impairments range from short-term memory deficits to long-term memory difficulties, including deficits in episodic (memory of one's life), procedural (memory of the body of how to perform an activity) and working memory. Cognitive problems tend to worsen over time, ultimately leading to dementia. This pattern of deficits has been called a subcortical dementia syndrome to distinguish it from the typical effects of cortical dementias, e.g., Alzheimer's disease.

Reported neuropsychiatric manifestations are anxiety, depression, a reduced display of emotions (blunted affect), egocentrism, aggression, and compulsive behavior, the latter of which can cause or worsen addictions, including alcoholism, gambling, and hypersexuality. Difficulties in recognizing other people's negative expressions have also been observed. The prevalence of these symptoms is highly variable between studies, with estimated rates for lifetime prevalence of psychiatric disorders between 33% and 76%. For many sufferers and their families, these symptoms are among the most distressing aspects of the disease, often affecting daily functioning and constituting reason for institutionalization. Suicidal thoughts and suicide attempts are more common than in the general population. Often individuals have reduced awareness of chorea, cognitive and emotional impairments.

Mutant Huntingtin is expressed throughout the body and associated with abnormalities in peripheral tissues that are directly caused by such expression outside the brain. These abnormalities include muscle atrophy, cardiac failure, impaired glucose tolerance, weight loss, osteoporosis, and testicular atrophy.

All humans have two copies of the Huntingtin gene (*HTT*), which codes for the protein Huntingtin (HTT). The gene is also called *HD* and *IT15*, which stands for 'interesting transcript 15'. Part of this gene is a repeated section called a trinucleotide repeat, which varies in length between individuals and may change length between generations. If the repeat is present in a healthy gene, a dynamic mutation may increase the repeat count and result in a defective gene. When the length of this repeated section reaches a certain threshold, it produces an altered form of the protein, called mutant Huntingtin protein (mHTT). The differing functions of these proteins are the cause of pathological changes which in turn cause the disease symptoms. The Huntington's disease mutation is genetically dominant and almost fully penetrant: mutation of either of a person's *HTT* alleles causes the disease. It is not inherited according to sex, but the length of the repeated section of the gene and hence its severity can be influenced by the sex of the affected parent.

HD is one of several trinucleotide repeat disorders which are caused by the length of a repeated section of a gene exceeding a normal range. The *HTT* gene is located on the short arm of chromosome 4 at 4p16.3. *HTT* contains a sequence of three DNA bases-cytosine-adenine-guanine (CAG)-repeated multiple times (*i.e.,* ... CAGCAGCAG ...), known as a trinucleotide repeat. CAG is the 3-letter genetic code (codon) for the amino acid glutamine, so a series of them results in the production of a chain of glutamine known as a polyglutamine tract (or polyQ tract), and the repeated part of the gene, the *PolyQ region.*

Generally, people have fewer than 36 repeated glutamines in the polyQ region which results in production of the cytoplasmic protein Huntingtin. However, a sequence of 36 or more glutamines results in the production of a protein which has different characteristics. This altered form, called mutant huntingtin (mHTT), increases the decay rate of certain types of neurons. Regions of the brain have differing amounts and reliance on these types of neurons, and are affected accordingly. Generally, the number of CAG repeats is related to how much this process is affected, and accounts for about 60% of the variation of the age of the onset of symptoms. The remaining variation is attributed to environment and other genes that modify the mechanism of HD. 36-39 repeats result in a reduced-penetrance form of the disease, with a much later onset and slower progression of symptoms. In some cases the onset may be so late that symptoms are never noticed. With very large repeat counts, HD has full penetrance and can occur under the age of 20, when it is then referred to as juvenile HD, akinetic-rigid, or Westphal variant HD. This accounts for about 7% of HD carriers.

**Ataxia telangiectasia.** Ataxia-telangiectasia (AT or A-T), also referred to as ataxia-telangiectasia syndrome or Louis-Bar syndrome, is a rare, neurodegenerative, autosomal recessive disease causing severe disability. Ataxia refers to poor coordination and telangiectasia to small dilated blood vessels, both of which are hallmarks of the disease.

A-T affects many parts of the body. It impairs certain areas of the brain including the cerebellum, causing difficulty with movement and coordination. It weakens the immune system, causing a predisposition to infection. It prevents repair of broken DNA, increasing the risk of cancer.

Symptoms most often first appear in early childhood (the toddler stage) when children begin to walk. Though they usually start walking at a normal age, they wobble or sway when walking, standing still or sitting, and may appear almost as if they are drunk. In late pre-school and early school age, they develop difficulty moving their eyes in a natural manner from one place to the next (oculomotor apraxia). They develop slurred or distorted speech, and swallowing problems. Some have an increased number of respiratory tract infections (ear infections, sinusitis, bronchitis, and pneumonia). Because not all children develop in the same manner or at the same rate, it may be some years before A-T is properly diagnosed. Most children with A-T have stable neurologic symptoms for the first 4-5 years of life, but begin to show increasing problems in early school years.

A-T is caused by a defect in the ATM gene, which is responsible for managing the cell's response to multiple forms of stress including double-strand breaks in DNA. In simple terms, the protein produced by the ATM gene recognizes that there is a break in DNA, recruits other proteins to fix the break, and stops the cell from making new DNA until the repair is complete.

There is substantial variability in the severity of features of A-T among affected individuals, and at different ages. The following symptoms or problems are either common or important features of A-T:
Ataxia (difficulty with control of movement) that is apparent early but worsens in school to pre-teen years.
Oculomotor apraxia (difficulty with coordination of head and eye movement when shifting gaze from one place to the next).
Involuntary movements.
Telangiectasia (dilated blood vessels) over the white (sclera) of the eyes, making them appear bloodshot. These are not apparent in infancy and may first appear at age 5-8 years. Telangiectasia may also appear on sun-exposed areas of skin.
Problems with infections, especially of the ears, sinuses and lungs.
Increased incidence of cancer (primarily, but not exclusively, lymphomas and leukemias).
Delayed onset or incomplete pubertal development, and very early menopause.
Slowed rate of growth (weight and/or height).
Drooling particularly in young children when they are tired or concentrating on activities.
Dysarthria (slurred, slow, or distorted speech sounds).
Diabetes in adolescence or later.
Premature changes in hair and skin.
Many children are initially misdiagnosed as having ataxic cerebral palsy. The diagnosis of AT may not be made until the preschool years when the neurologic symptoms of impaired gait, hand coordination, speech and eye movement appear or worsen, and the telangiectasia first appear. Because A-T is so rare, doctors may not be familiar with the symptoms, or methods of making a diagnosis. The late appearance of telangiectasia may be a barrier to the diagnosis. It may take some time before doctors consider A-T as a possibility because of the early stability of symptoms and signs.

A-T is caused by mutations in the ATM (ATM serine/threonine kinase or ataxia-telangiectasia mutated) gene, which was cloned in 1995. ATM is located on human chromosome 11 (1 1q22.3) and is made up of 69 exons spread across 150kb of genomic DNA.

The mode of inheritance for A-T is autosomal recessive. Each parent is a carrier, meaning that they have one normal copy of the A-T gene (ATM) and one copy which is mutated. A-T occurs if a child inherits the mutated A-T gene from each parent, so in a family with two carrier parents, there is 1 chance in 4 that a child born to the parents will have the disorder. Prenatal diagnosis (and carrier detection) can be carried out in families if the errors (mutation) in an affected child's two ATM genes have been identified. The process of getting this done can be complicated and, as it requires time, should be arranged before conception.

Looking for mutations in the ATM gene of an unrelated person (for example, the spouse of a known A-T carrier) presents significant challenges. Genes often have variant spellings (polymorphisms) which do not affect function. In a gene as large as ATM, such variant spellings are likely to occur and doctors cannot always predict whether a specific variant will or will not cause disease. Genetic counseling can help family members of an A-T patient understand what can or cannot be tested, and how the test results should be interpreted.

Carriers of A-T, such as the parents of a person with A-T, have one mutated copy of the ATM gene and one normal copy. They are generally healthy, but there is an increased risk of breast cancer in women. This finding has been confirmed in a variety of different ways, and is the subject of current research. Standard surveillance (including monthly breast self-exams and mammography at the usual schedule for age) is recommended, unless additional tests are indicated because the individual has other risk factors (*e.g.,* family history of breast cancer).

**Neurofibromatosis type 1 and 2.** Neurofibromatosis (NF) is a group of three conditions in which tumors grow in the nervous system. The three types are neurofibromatosis type 1 (NF 1), neurofibromatosis type 2 (NF2), and schwannomatosis. In NF1 symptoms include light brown spots on the skin, freckles in the armpit and groin, small bumps within nerves, and scoliosis. In NF2 there may be hearing loss, cataracts at a young age, balance problems, flesh colored skin flaps, and muscle wasting. The tumors are generally non-cancerous.

The cause is a genetic mutation in certain genes. In half of cases these are inherited from a person's parents while in the rest they occur during early development. The tumors involve supporting cells in the nervous system rather than the neurons. In NF1 the tumors are neurofibromas (tumors of the peripheral nerves) while in NF2 and schwannomatosis tumors of Schwann cells are more common. Diagnosis is typically based on the signs and symptoms and occasionally supported by genetic testing.

There is no known prevention or cure. Surgery may be done to remove tumors that are causing problems or have become cancerous. Radiation and chemotherapy may also be used if cancer occurs. A cochlear implant or auditory brainstem implant may help some who have hearing loss.

In the United States about 1 in 3,500 people have NF 1 and 1 in 25,000 have NF2. Males and females are affected equally frequently. In NF1 symptom are often present at birth and otherwise develop before 10 years of age. While the condition typically worsens with time most people with NF1 have a normal life expectancy. In NF2 symptoms may not become apparent until early adulthood. NF2 increases the risk of early death. Descriptions of the condition occur as far back as the 1st century.

Neurofibromatosis (NF1) in early life may cause learning and behavior problems - about 60% of children who have NF 1 have a mild form of difficulty in school. In terms of signs the individual might have are the following: six or more light brown dermatological spots ("café-au-lait spots"), at least two neurofibromas, at least two growths on the eye's iris, and abnormal growth of the spine (scoliosis).

Neurofibromatosis is an autosomal dominant disorder, which means only one copy of the affected gene is needed for the disorder to develop. Therefore, if only one parent has neurofibromatosis, his or her children have a 50% chance of developing the condition as well. The affected child could have mild NF 1 even though inherited from a parent with a severe form of the disorder. There two types of neurofibromatosis. Neurofibromatosis type I is characterized by nerve tissue growth into tumors (neurofibromas) that may be benign and may cause serious damage by compressing nerves and other tissues. Neurofibromatosis type II exhibits bilateral acoustic neuromas (tumors of the vestibulocochlear nerve or cranial nerve 8 (CN VIII) also known as schwannoma), often leading to hearing loss.

Schwannomatosis, in which painful schwannomas develop on spinal and peripheral nerves.

**CEP290 mutation in Leber congenital amaurosis.** Centrosomal protein of 290 kDa is a protein that in humans is encoded by the *CEP290* gene. CEP290 is located on the Q arm of chromosome 12. The gene CEP290 is a centrosomal protein that plays an important role in centrosome and cilia development. This gene is vital in the formation of the primary cilium, a small antenna-like projections of the cell membrane that plays an important role in the photoreceptors at the back of the retina (which detect light and color) and in the kidney, brain, and many other organs of the body. Knocking down levels of the CEP290 gene transcript resulted in dramatic suppression of ciliogenesis in retinal pigment epithelial cells in culture, proving just how important CEP290 is to cilia formation.

On a molecular level, CEP290 has been shown to play a critical regulatory and structural role in primary cilium formation. Recent studies have implicated CEP290 as a microtubule and membrane binding protein that might serve as a structural link between the microtubule core of the cilium and the overlying ciliary membrane. Disruption of CEP290's microtubule binding domain in the rd16 mouse model of CEP290 disease has been shown to result in rapid and dramatic retinal degeneration, demonstrating the importance of CEP290 microtubule binding in disease. The role of CEP290 in promoting ciliogensis is inhibited both by auto-regulatory domains found at either end of the CEP290 protein and through CEP290's interaction with the inhibitory protein CP110.

The discovery of the CEP290 gene has led researchers to find another gene critical in retinal function, LCA5. Clinical trials involving gene replacement of these two genes have started in Philadelphia, where researchers are hopeful that Leber Congenital Amaurosis will one day be cured.

This gene encodes a protein with 13 putative coiled-coil domains, a region with homology to SMC chromosome segregation ATPases, six KID motifs, three tropomyosin homology domains and an ATP/GTP binding site motif A. The protein is localized to the centrosome and cilia and has sites for N-glycosylation, tyrosine sulfation, phosphorylation, N-myristoylation, and amidation.

Mutations in this gene have been associated with Joubert syndrome and nephronophthisis, and recently with a frequent form of Leber's Congenital Amaurosis, called LCA10. The presence of antibodies against this protein is associated with several forms of cancer.

A mutation in this gene leads to infant and child blindness, a disease known as Leber Congenital Amaurosis. As of today, 35 different mutations in CEP290 are responsible for causing LCA. Other mutations in CEP290 have also been identified in causing Meckel Syndrome and Joubert Syndrome, a few among many syndromes. A defective CEP290 gene is usually the cause of these disorders due to abnormal cilia. It is unknown how one mutation in a gene can cause so many different types of syndromes, particularly many of which affect the Central Nervous System.

### III. CRISPR Systems

### A. CRISPRs

CRISPRs (clustered regularly interspaced short palindromic repeats) are DNA loci containing short repetitions of base sequences. Each repetition is followed by short segments of "spacer DNA" from previous exposures to a virus. CRISPRs are found in approximately 40% of sequenced eubacteria genomes and 90% of sequenced archaea. CRISPRs are often associated with Cas genes that code for proteins related to CRISPRs. The CRISPR/Cas system is a prokaryotic immune system that confers resistance to foreign genetic elements such as plasmids and phages and provides a form of acquired immunity. CRISPR spacers recognize and silence these exogenous genetic elements like RNAi in eukaryotic organisms.

CRISPR repeats range in size from 24 to 48 base pairs. They usually show some dyad symmetry, implying the formation of a secondary structure such as a hairpin, but are not truly palindromic. Repeats are separated by spacers of similar length. Some CRISPR spacer sequences exactly match sequences from plasmids and phages, although some spacers match the prokaryote's genome (self-targeting spacers). New spacers can be added rapidly in response to phage infection.

### B. Cas Nucleases

CRISPR-associated (*cas*) genes are often associated with CRISPR repeat-spacer arrays. As of 2013, more than forty different Cas protein families had been described. Of these protein families, Cas1 appears to be ubiquitous among different CRISPR/Cas systems. Particular combinations of *cas* genes and repeat structures have been used to define 8 CRISPR subtypes (Ecoli, Ypest, Nmeni, Dvulg, Tneap, Hmari, Apern, and Mtube), some of which are associated with an additional gene module encoding repeat-associated mysterious proteins (RAMPs). More than one CRISPR subtype may occur in a single genome. The sporadic distribution of the CRISPR/Cas subtypes suggests that the system is subject to horizontal gene transfer during microbial evolution.

Exogenous DNA is apparently processed by proteins encoded by Cas genes into small elements (~30 base pairs in length), which are then somehow inserted into the CRISPR locus near the leader sequence. RNAs from the CRISPR loci are constitutively expressed and are processed by Cas proteins to small RNAs composed of individual, exogenously-derived sequence elements with a flanking repeat sequence. The RNAs guide other Cas proteins to silence exogenous genetic elements at the RNA or DNA level. Evidence suggests functional diversity among CRISPR subtypes. The Cse (Cas subtype *Ecoli*) proteins (called CasA-E in E. *coli*) form a functional complex, Cascade, that processes CRISPR RNA transcripts into spacer-repeat units that Cascade retains. In other prokaryotes, Cas6 processes the CRISPR transcripts. Interestingly, CRISPR-based phage inactivation in *E. coli* requires Cascade and Cas3, but not Cas1 and Cas2. The Cmr (Cas RAMP module) proteins found in *Pyrococcus furiosus* and other prokaryotes form a functional complex with small CRISPR RNAs that recognizes and cleaves complementary target RNAs. RNA-guided CRISPR enzymes are classified as type V restriction enzymes.

Cas9 is a nuclease, an enzyme specialized for cutting DNA, with two active cutting sites, one for each strand of the double helix. The team demonstrated that they could disable one or both sites while preserving Cas9's ability to locate its target DNA. Jinek *et al.* (2012) combined tracrRNA and spacer RNA into a "single-guide RNA" molecule that, mixed with Cas9, can find and cut the correct DNA targets and such synthetic guide RNAs are used for gene editing.

Cas9 proteins are highly enriched in pathogenic and commensal bacteria. CRISPR/Cas-mediated gene regulation may contribute to the regulation of endogenous bacterial genes, particularly during bacterial interaction with eukaryotic hosts. For example, Cas protein Cas9 of *Francisella novicida* uses a unique, small, CRISPR/Cas-associated RNA (scaRNA) to repress an endogenous transcript encoding a bacterial lipoprotein that is critical for *F. novicida* to dampen host response and promote virulence. Wang *et al.* (2013) showed that coinjection of Cas9 mRNA and sgRNAs into the germline (zygotes) generated mice with mutations. Delivery of Cas9 DNA sequences also is contemplated.

The systems CRISPR/Cas are separated into three classes. Class 1 uses several Cas proteins together with the CRISPR RNAs (crRNA) to build a functional endonuclease. Class 2 CRISPR systems use a single Cas protein with a crRNA. Cpfl has been recently identified as a Class II, Type V CRISPR/Cas systems containing a 1,300 amino acid protein. See also U.S. Patent Publication 2014/0068797.

In some embodiments, the compositions of the disclosure include a small version of a Cas9 from the bacterium *Staphylococcus aureus* (UniProt Accession No. J7RUA5). The small version of the Cas9 provides advantages over wildtype or full length Cas9. In some embodiments the Cas9 is a spCas9 (AddGene).

### C. Cpfl Nucleases

Clustered Regularly Interspaced Short Palindromic Repeats from *Prevotella* and *Francisella* 1 or CRISPR/Cpfl is a DNA-editing technology which shares some similarities with the CRISPR/Cas9 system. Cpfl is an RNA-guided endonuclease of a class II CRISPR/Cas system. This acquired immune mechanism is found in *Prevotella* and *Francisella* bacteria. It prevents genetic damage from viruses. Cpfl genes are associated with the CRISPR locus, coding for an endonuclease that use a guide RNA to find and cleave viral DNA. Cpfl is a smaller and simpler endonuclease than Cas9, overcoming some of the CRISPR/Cas9 system limitations.

Cpfl appears in many bacterial species. The ultimate Cpfl endonuclease that was developed into a tool for genome editing was taken from one of the first 16 species known to harbor it.

In embodiments, the Cpfl is a Cpfl enzyme from *Acidaminococcus* (species BV3L6, UniProt Accession No. U2UMQ6; SEQ ID NO: 870), having the sequence set forth below: 
1 MTQFEGFTNL YQVSKTLRFE LIPQGKTLKH IQEQGFIEED
KARNDHYKEL KPIIDRIYKT
 
61 YADQCLQLVQ LDWENLSAAI DSYRKEKTEE TRNALIEEQA TYRNAIHDYF
IGRTDNLTDA
 
121 INKRHAEIYK GLFKAELFNG KVLKQLGTVT TTEHENALLR SFDKFTTYFS
GFYENRKNVF
 
181 SAEDISTAIP HRIVQDNFPK FKENCHIFTR LITAVPSLRE HFENVKKAIG
IFVSTSIEEV
 
241 FSFPFYNQLL TQTQIDLYNQ LLGGISREAG TEKIKGLNEV LNLAIQKNDE
TAHIIASLPH
 
301 RFIPLFKQIL SDRNTLSFIL EEFKSDEEVI QSFCKYKTLL RNENVLETAE
ALFNELNSID
 
361 LTHIFISHKK LETISSALCD HWDTLRNALY ERRISELTGK ITKSAKEKVQ
RSLKHEDINL
 
421 QEIISAAGKE LSEAFKQKTS EILSHAHAAL DQPLPTTLKK QEEKEILKSQ
LDSLLGLYHL
 
481 LDWFAVDESN EVDPEFSARL TGIKLEMEPS LSFYNKARNY ATKKPYSVEK
FKLNFQMPTL
 
541 ASGWDVNKEK NNGAILFVKN GLYYLGIMPK QKGRYKALSF EPTEKTSEGF
DKMYYDYFPD
 
601 AAKMIPKCST QLKAVTAHFQ THTTPILLSN NFIEPLEITK EIYDLNNPEK
EPKKFQTAYA
 
661 KKTGDQKGYR EALCKWIDFT RDFLSKYTKT TSIDLSSLRP SSQYKDLGEY
YAELNPLLYH
 
721 ISFQRIAEKE IMDAVETGKL YLFQIYNKDF AKGHHGKPNL HTLYWTGLFS
PENLAKTSIK
 
781 LNGQAELFYR PKSRMKRMAH RLGEKMLNKK LKDQKTPIPD
TLYQELYDYV NHRLSHDLSD
 
841 EARALLPNVI TKEVSHEIIK DRRFTSDKFF FHVPITLNYQ AANSPSKFNQ
RVNAYLKEHP
 
901 ETPIIGIDRG ERNLIYITVI DSTGKILEQR SLNTIQQFDY QKKLDNREKE
RVAARQAWSV
 
961 VGTIKDLKQG YLSQVIHEIV DLMIHYQAVV VLENLNFGFK SKRTGIAEKA
VYQQFEKMLI
 
1021 DKLNCLVLKD YPAEKVGGVL NPYQLTDQFT SFAKMGTQSG
FLFYVPAPYT SKIDPLTGFV
 
1081 DPFVWKTIKN HESRKHFLEG FDFLHYDVKT GDFILHFKMN RNLSFQRGLP
GFMPAWDIVF
 
1141 EKNETQFDAK GTPFIAGKRI VPVIENHRFT GRYRDLYPAN ELIALLEEKG
IVFRDGSNIL
 
1201 PKLLENDDSH AIDTMVALIR SVLQMRNSNA ATGEDYINSP VRDLNGVCFD
SRFQNPEWPM
 1261 DADANGAYHI ALKGQLLLNH LKESKDLKLQ NGISNQDWLA YIQELRN

In some embodiments, the Cpfl is a Cpfl enzyme from *Lachnospiraceae* (species ND2006, UniProt Accession No. A0A182DWE3; SEQ ID NO: 871), having the sequence set forth below: 
1 AASKLEKFTN CYSLSKTLRF KAIPVGKTQE NIDNKRLLVE DEKRAEDYKG
VKKLLDRYYL
 
61 SFINDVLHSI KLKNLNNYIS LFRKKTRTEK ENKELENLEI NLRKEIAKAF
KGAAGYKSLF
 
121 KKDIIETILP EAADDKDEIA LVNSFNGFTT AFTGFFDNRE NMFSEEAKST
SIAFRCINEN
 
181 LTRYISNMDI FEKVDAIFDK HEVQEIKEKI LNSDYDVEDF FEGEFFNFVL
TQEGIDVYNA
 
241 IIGGFVTESG EKIKGLNEYI NLYNAKTKQA LPKFKPLYKQ VLSDRESLSF
YGEGYTSDEE
 
301 VLEVFRNTLN KNSEIFSSIK KLEKLFKNFD EYSSAGIFVK NGPAISTISK
DIFGEWNLIR
 
361 DKWNAEYDDI HLKKKAVVTE KYEDDRRKSF KKIGSFSLEQ LQEYADADLS
VVEKLKEIII
 
421 QKVDEIYKVY GSSEKLFDAD FVLEKSLKKN DAVVAIMKDL LDSVKSFENY
IKAFFGEGKE
 
481 TNRDESFYGD FVLAYDILLK VDHIYDAIRN YVTQKPYSKD KFKLYFQNPQ
FMGGWDKDKE
 
541 TDYRATILRY GSKYYLAIMD KKYAKCLQKI DKDDVNGNYE KINYKLLPGP
NKMLPKVFFS
 
601 KKWMAYYNPS EDIQKIYKNG TFKKGDMFNL NDCHKLIDFF KDSISRYPKW
SNAYDFNFSE
 
661 TEKYKDIAGF YREVEEQGYK VSFESASKKE VDKLVEEGKL YMFQIYNKDF
SDKSHGTPNL
 
721 HTMYFKLLFD ENNHGQIRLS GGAELFMRRA SLKKEELVVH PANSPIANKN
PDNPKKTTTL
 
781 SYDVYKDKRF SEDQYELHIP IAINKCPKNI FKINTEVRVL LKHDDNPYVI
GIDRGERNLL
 
841 YIVVVDGKGN IVEQYSLNEI INNFNGIRIK TDYHSLLDKK EKERFEARQN
WTSIENIKEL
 
901 KAGYISQVVH KICELVEKYD AVIALEDLNS GFKNSRVKVE KQVYQKFEKM
LIDKLNYMVD
 
961 KKSNPCATGG ALKGYQITNK FESFKSMSTQ NGFIFYIPAW LTSKIDPSTG
FVNLLKTKYT
 
1021 SIADSKKFIS SFDRIMYVPE EDLFEFALDY KNFSRTDADY IKKWKLYSYG
NRIRIFAAAK
 
1081 KNNVFAWEEV CLTSAYKELF NKYGINYQQG DIRALLCEQS DKAFYSSFMA
LMSLMLQMRN
 
1141 SITGRTDVDF LISPVKNSDG IFYDSRNYEA QENAILPKNA DANGAYNIAR
KVLWAIGQFK
 1201 KAEDEKLDKV KIAISNKEWL EYAQTSVK

In some embodiments, the Cpfl is codon optimized for expression in mammalian cells. In some embodiments, the Cpfl is codon optimized for expression in human cells or mouse cells.

The Cpfl locus contains a mixed alpha/beta domain, a RuvC-I followed by a helical region, a RuvC-II and a zinc finger-like domain. The Cpfl protein has a RuvC-like endonuclease domain that is similar to the RuvC domain of Cas9. Furthermore, Cpfl does not have a HNH endonuclease domain, and the N-terminal of Cpfl does not have the alpha-helical recognition lobe of Cas9.

Cpfl CRISPR-Cas domain architecture shows that Cpfl is functionally unique, being classified as Class 2, type V CRISPR system. The Cpfl loci encode Cas1, Cas2 and Cas4 proteins more similar to types I and III than from type II systems. Database searches suggest the abundance of Cpfl-family proteins in many bacterial species.

Functional Cpfl does not require a tracrRNA, therefore, only crRNA is required. This benefits genome editing because Cpfl is not only smaller than Cas9, but also it has a smaller sgRNA molecule (proximately half as many nucleotides as Cas9).

The Cpfl-crRNA complex cleaves target DNA or RNA by identification of a protospacer adjacent motif 5'-YTN-3' (where "Y" is a pyrimidine and "N" is any nucleobase) or 5'-TTN-3', in contrast to the G-rich PAM targeted by Cas9. After identification of PAM, Cpfl introduces a sticky-end-like DNA double- stranded break of 4 or 5 nucleotides overhang.

The CRISPR/Cpfl system consist of a Cpfl enzyme and a guide RNA that finds and positions the complex at the correct spot on the double helix to cleave target DNA. CRISPR/Cpfl systems activity has three stages:
Adaptation, during which Cas1 and Cas2 proteins facilitate the adaptation of small fragments of DNA into the CRISPR array;
Formation of crRNAs: processing of pre-cr-RNAs producing of mature crRNAs to guide the Cas protein; and
Interference, in which the Cpfl is bound to a crRNA to form a binary complex to identify and cleave a target DNA sequence.

### D. gRNA

As an RNA guided protein, Cas9 requires a short RNA to direct the recognition of DNA targets. Though Cas9 preferentially interrogates DNA sequences containing a PAM sequence NGG it can bind here without a protospacer target. However, the Cas9-gRNA complex requires a close match to the gRNA to create a double strand break. CRISPR sequences in bacteria are expressed in multiple RNAs and then processed to create guide strands for RNA. Because Eukaryotic systems lack some of the proteins required to process CRISPR RNAs the synthetic construct gRNA was created to combine the essential pieces of RNA for Cas9 targeting into a single RNA expressed with the RNA polymerase type III promoter U6. Synthetic gRNAs are slightly over 100bp at the minimum length and contain a portion which is targets the 20 protospacer nucleotides immediately preceding the PAM sequence NGG; gRNAs do not contain a PAM sequence.

In some embodiments, the gRNA targets a site within a wildtype dystrophin gene. In some embodiments, the gRNA targets a site within a mutant dystrophin gene. In some embodiments, the gRNA targets a dystrophin intron. In some embodiments, the gRNA targets a dystrophin exon. In some embodiments, the gRNA targets a site in a dystrophin exon that is expressed and is present in one or more of the dystrophin isoforms shown in Table 1. In embodiments, the gRNA targets a dystrophin splice site. In some embodiments, the gRNA targets a splice donor site on the dystrophin gene. In accordance with the present invention, the first, second and third gRNA each target a splice acceptor site on the dystrophin gene.

In embodiments, the guide RNA targets a mutant DMD exon. In some embodiments, the mutant exon is exon 23 or 51. In some embodiments, the guide RNA targets at least one of exons 1, 23, 41, 44, 46, 47, 48, 49, 50, 51, 52, 53, 54, or 55 of the dystrophin gene. In embodiments, the guide RNA targets at least one of introns 44, 45, 50, 51, 52, 53, 54, or 55 of the dystrophin gene. In preferred embodiments, the first, second and third guide RNAs are designed to induce skipping of exon 51 or exon 23. In embodiments, the first, second and third gRNAs are targeted to a splice acceptor site of exon 51 or exon 23.

In some embodiments, gRNAs of the disclosure comprise a sequence that is complementary to a target sequence within a coding sequence or a non-coding sequence corresponding to the *DMD* gene, and, therefore, hybridize to the target sequence. In some embodiments, gRNAs for Cpfl comprise a single crRNA containing a direct repeat scaffold sequence followed by 24 nucleotides of guide sequence. In some embodiments, a "guide" sequence of the crRNA comprises a sequence of the gRNA that is complementary to a target sequence. In some embodiments, crRNA of the disclosure comprises a sequence of the gRNA that is not complementary to a target sequence. "Scaffold" sequences of the disclosure link the gRNA to the Cpfl polypeptide. "Scaffold" sequences of the disclosure are not equivalent to a tracrRNA sequence of a gRNA-Cas9 construct.

In some embodiments, a nucleic acid may comprise three or more sequences encoding a DMD gRNA, wherein the first, second and third DMD gRNAs target a first, second and third genomic target sequence, respectively, wherein the first second and third genomic target sequence each comprise a dystrophin splice acceptor site and wherein the sequences encoding the first, second and third DMD gRNAs are identical. In some embodiments, a nucleic acid may comprise 3, 4, 5, 6, 7, 8, 9, 10, 11, 12, 13, 14, 15, 16, 17, 18, 19, 20 sequences encoding a gRNA. In some embodiments, all of the sequences encode the same gRNA. In some embodiments, the fourth and any subsequent gRNA sequences may encode different gRNAs to the first, second and third DMD gRNAs. In some embodiments, at least 3 of the sequences encode the same gRNA, for example at least 4, 5, 6, 7, 8, 9, 10, 11, 12, 13, 14, 15, 16, 17, 18, 19, or 20 of the sequences encode the same gRNA.

### E. Cas9 versus Cpfl

Cas9 requires two RNA molecules to cut DNA while Cpfl needs one. The proteins also cut DNA at different places, offering researchers more options when selecting an editing site. Cas9 cuts both strands in a DNA molecule at the same position, leaving behind 'blunt' ends. Cpfl leaves one strand longer than the other, creating 'sticky' ends that are easier to work with. Cpfl appears to be more able to insert new sequences at the cut site, compared to Cas9. Although the CRISPR/Cas9 system can efficiently disable genes, it is challenging to insert genes or generate a knock-in. Cpfl lacks tracrRNA, utilizes a T-rich PAM and cleaves DNA via a staggered DNA DSB.

In summary, important differences between Cpfl and Cas9 systems are that Cpfl recognizes different PAMs, enabling new targeting possibilities, creates 4-5 nt long sticky ends, instead of blunt ends produced by Cas9, enhancing the efficiency of genetic insertions and specificity during NHEJ or HDR, and cuts target DNA further away from PAM, further away from the Cas9 cutting site, enabling new possibilities for cleaving the DNA.

**Table 3: Differences between Cas9 and Cpfl**

| **Feature** | **Cas9** | **Cpf1** |
|---|---|---|
| **Structure** | Two RNA required (Or 1 fusion transcript (crRNA+tracrRNA=gRNA)) | One RNA required |
| **Cutting mechanism** | Blunt end cuts | Staggered end cuts |
| **Cutting site** | Proximal to recognition site | Distal from recognition site |
| **Target sites** | G-rich PAM | T-rich PAM |

### F. CRISPR -mediated gene editing

The first step in editing the DMD gene using CRISPR/Cpfl or CRISPR/Cas9 is to identify the genomic target sequence. The genomic target for the gRNAs of the disclosure can be any ~24 nucleotide DNA sequence, provided that the sequence is unique compared to the rest of the genome, although for the purposes of the claimed invention the first, second, and third genomic target sequence each comprise a dystrophin splice acceptor site, and the sequences encoding the first, second, and third DMD gRNAs are identical. In some embodiments, the genomic target sequence corresponds to a sequence within exon 51, exon 45, exon 44, exon 53, exon 46, exon 52, exon 50, exon 43, exon 6, exon 7, exon 8, and/or exon 55 of the human dystrophin gene. In some embodiments, the genomic target sequence is a 5' or 3' splice site of exon 51, exon 45, exon 44, exon 53, exon 46, exon 52, exon 50, exon 43, exon 6, exon 7, exon 8, and/or exon 55 of the human dystrophin gene. In some embodiments, the genomic target sequence corresponds to a sequence within an intron immediately upstream or downstream of exon 51, exon 45, exon 44, exon 53, exon 46, exon 52, exon 50, exon 43, exon 6, exon 7, exon 8, and/or exon 55 of the human dystrophin gene. Exemplary genomic target sequences can be found in Tables 6, 8, 10, 12, and 14.

The next step in editing the DMD gene is to identify all Protospacer Adjacent Motif (PAM) sequences within the genetic region to be targeted. The target sequence must be immediately upstream of a PAM. Once all possible PAM sequences and putative target sites have been identified, the next step is to choose which site is likely to result in the most efficient on-target cleavage. The gRNA targeting sequence needs to match the target sequence, and the gRNA targeting sequence must not match additional sites within the genome. In preferred embodiments, the gRNA targeting sequence has perfect homology to the target with no homology elsewhere in the genome. In some embodiments, a given gRNA targeting sequence will have additional sites throughout the genome where partial homology exists. These sites are called "off-targets" and should be considered when designing a gRNA. In general, off-target sites are not cleaved as efficiently when mismatches occur near the PAM sequence, so gRNAs with no homology or those with mismatches close to the PAM sequence will have the highest specificity. In addition to "off-target activity", factors that maximize cleavage of the desired target sequence ("on-target activity") must be considered. It is known to those of skill in the art that two gRNA targeting sequences, each having 100% homology to the target DNA may not result in equivalent cleavage efficiency. In fact, cleavage efficiency may increase or decrease depending upon the specific nucleotides within the selected target sequence. Close examination of predicted on-target and off-target activity of each potential gRNA targeting sequence is necessary to design the best gRNA. Several gRNA design programs have been developed that are capable of locating potential PAM and target sequences and ranking the associated gRNAs based on their predicted on-target and off-target activity (e.g. CRISPRdirect, available at www.crispr.dbcls.jp).

The next step is to synthesize and clone desired gRNAs. Targeting oligos can be synthesized, annealed, and inserted into plasmids containing the gRNA scaffold using standard restriction-ligation cloning. However, the exact cloning strategy will depend on the gRNA vector that is chosen. The gRNAs for Cpf1 are notably simpler than the gRNAs for Cas9, and only consist of a single crRNA containing direct repeat scaffold sequence followed by ~24 nucleotides of guide sequence.

Each gRNA should then be validated in one or more target cell lines. For example, after the Cas9 or Cpfl and the gRNA are delivered to the cell, the genomic target region may be amplified using PCR and sequenced according to methods known to those of skill in the art.

In some embodiments, gene editing may be performed in vitro or ex vivo. In some embodiments, cells are contacted in vitro or ex vivo with a Cas9 or a Cpfl and the first, second and third gRNAs that each targets a dystrophin splice acceptor site. In some embodiments, the cells are contacted with one or more nucleic acids encoding the Cas9 or Cpfl and the guide RNAs. In some embodiments, the one or more nucleic acids are introduced into the cells using, for example, lipofection or electroporation. Although not claimed, it is noted that gene editing may also be performed in zygotes. For example, zygotes may be injected with one or more nucleic acids encoding Cas9 or Cpfl and the gRNAs that targets a dystrophin splice acceptor site. The zygotes may subsequently be injected into a host.

In embodiments, the Cas9 or Cpfl is provided on a vector. In embodiments, the vector contains a Cas9 derived from S. *pyogenes* (SpCas9, SEQ ID NO. 872). In embodiments, the vector contains a Cas9 derived from S. *aureus* (SaCas9, SEQ ID NO. 873). In embodiments, the vector contains a Cpfl sequence derived from a *Lachnospiraceae* bacterium. See, for example, Uniprot Accession No. A0A182DWE3; SEQ ID NO. 871. In embodiments, the vector contains a Cpfl sequence derived from an *Acidaminococcus* bacterium. See, for example, Uniprot Accession No. U2UMQ6; SEQ ID NO. 870. In some embodiments, the Cas9 or Cpfl sequence is codon optimized for expression in human cells or mouse cells. In some embodiments, the vector further contains a sequence encoding a fluorescent protein, such as GFP, which allows Cas 9 or Cpfl-expressing cells to be sorted using fluorescence activated cell sorting (FACS). In some embodiments, the vector is a viral vector such as an adeno-associated viral vector.

In embodiments, the first, second and third gRNAs are provided on a vector. In some embodiments, the vector is a viral vector such as an adeno-associated viral vector. In embodiments, the Cas9 or Cpfl and the guide RNAs are provided on the same vector. In embodiments, the Cas9 or Cpfl and the guide RNAs are provided on different vectors.

In some embodiments, the cells are additionally contacted with a single-stranded DMD oligonucleotide to effect homology directed repair. In some embodiments, small INDELs restore the protein reading frame of dystrophin ("reframing" strategy). When the reframing strategy is used, the cells may be contacted with a single gRNA. In embodiments, a splice donor or splice acceptor site is disrupted, which results in exon skipping and restoration of the protein reading frame ("exon skipping" strategy). When the exon skipping strategy is used, the cells may be contacted with two or more gRNAs.

Efficiency of in vitro or ex vivo Cas9 or Cpfl-mediated DNA cleavage may be assessed using techniques known to those of skill in the art, such as the T7 E1 assay. Restoration of DMD expression may be confirmed using techniques known to those of skill in the art, such as RT-PCR, western blotting, and immunocytochemistry.

In some embodiments, the nucleic acids of the present invention may be useful for in vitro or ex vivo gene editing that is performed in a muscle or satellite cell. In some embodiments, gene editing is performed in iPSC or iCM cells. In embodiments, the iPSC cells are differentiated after gene editing. For example, the iPSC cells may be differentiated into a muscle cell or a satellite cell after editing. In embodiments, the iPSC cells are differentiated into cardiac muscle cells, skeletal muscle cells, or smooth muscle cells. In embodiments, the iPSC cells are differentiated into cardiomyocytes. iPSC cells may be induced to differentiate according to methods known to those of skill in the art.

In some embodiments, contacting the cell with the Cas9 or the Cpfl and the gRNAs restores dystrophin expression. In embodiments, cells which have been edited in vitro or ex vivo, or cells derived therefrom, show levels of dystrophin protein that is comparable to wildtype cells. In embodiments, the edited cells, or cells derived therefrom, express dystrophin at a level that is 50%, 60%, 70%, 80%, 90%, 95% or any percentage in between of wildtype dystrophin expression levels. In embodiments, the cells which have been edited in vitro or ex vivo, or cells derived therefrom, have a mitochondrial number that is comparable to that of wildtype cells. In embodiments the edited cells, or cells derived therefrom, have 50%, 60%, 70%, 80%, 90%, 95% or any percentage in between as many mitochondria as wildtype cells. In embodiments, the edited cells, or cells derived therefrom, show an increase in oxygen consumption rate (OCR) compared to non-edited cells at baseline.

### G. RNA Pol III and Pol III Promoters

In eukaryotes, RNA polymerase III (also called Pol III) transcribes DNA to synthesize ribosomal 5S rRNA, tRNA and other small RNAs. The genes transcribed by RNA Pol III fall in the category of "housekeeping" genes whose expression is required in all cell types and most environmental conditions. Therefore, the regulation of Pol III transcription is primarily tied to the regulation of cell growth and the cell cycle, thus requiring fewer regulatory proteins than RNA polymerase II. Under stress conditions however, the protein Maf1 represses Pol III activity.

In the process of transcription (by any polymerase) there are three main stages: (i) initiation, requiring construction of the RNA polymerase complex on the gene's promoter; (ii) elongation, the synthesis of the RNA transcript; and (iii) termination, the finishing of RNA transcription and disassembly of the RNA polymerase complex.

Promoters under the control of RNA Pol III include those for ribosomal 5S rRNA, tRNA and few other small RNAs such as U6 spliceosomal RNA, RNase P and RNase MRP RNA, 7SL RNA (the RNA component of the signal recognition particles), Vault RNAs, Y RNA, SINEs (short interspersed repetitive elements), 7SK RNA, two microRNAs, several small nucleolar RNAs and several few regulatory antisense RNAs

### IV. Nucleic Acid Delivery

As discussed above, in certain embodiments, expression cassettes are employed to express a transcription factor product, either for subsequent purification and delivery to a cell/subject, or for use directly in a genetic-based delivery approach. Provided herein are expression vectors which contain one or more nucleic acids encoding Cas9 or Cpfl and at least the first, second and third DMD guide RNAs that target a dystrophin splice acceptor site, wherein the sequences encoding the first, second and third DMD guide RNAs are identical. In some embodiments, a nucleic acid encoding Cas9 or Cpfl and a nucleic acid encoding at least the first, second and third guide RNAs are provided on the same vector. In further embodiments, a nucleic acid encoding Cas9 or Cpfl and a nucleic acid encoding least the first, second and third guide RNAs are provided on separate vectors.

Expression requires that appropriate signals be provided in the vectors, and include various regulatory elements such as enhancers/promoters from both viral and mammalian sources that drive expression of the genes of interest in cells. Elements designed to optimize messenger RNA stability and translatability in host cells also are defined. The conditions for the use of a number of dominant drug selection markers for establishing permanent, stable cell clones expressing the products are also provided, as is an element that links expression of the drug selection markers to expression of the polypeptide.

### A. Regulatory Elements

Throughout this application, the term "expression cassette" is meant to include any type of genetic construct containing a nucleic acid coding for a gene product in which part or all of the nucleic acid encoding sequence is capable of being transcribed and translated, i.e., is under the control of a promoter. A "promoter" refers to a DNA sequence recognized by the synthetic machinery of the cell, or introduced synthetic machinery, required to initiate the specific transcription of a gene. The phrase "under transcriptional control" means that the promoter is in the correct location and orientation in relation to the nucleic acid to control RNA polymerase initiation and expression of the gene. An "expression vector" is meant to include expression cassettes comprised in a genetic construct that is capable of replication, and thus including one or more of origins of replication, transcription termination signals, poly-A regions, selectable markers, and multipurpose cloning sites.

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (*tk*) and SV40 early transcription units. These studies, augmented by more recent work, have shown that promoters are composed of discrete functional modules, each consisting of approximately 7-20 bp of DNA, and containing one or more recognition sites for transcriptional activator or repressor proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

In some embodiments, the Cas9 or Cpfl constructs of the disclosure are expressed by a muscle-cell specific promoter. This muscle-cell specific promoter may be constitutively active or may be an inducible promoter.

Additional promoter elements regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either co-operatively or independently to activate transcription.

In certain embodiments, viral promotes such as the human cytomegalovirus (CMV) immediate early gene promoter, the SV40 early promoter, the Rous sarcoma virus long terminal repeat, rat insulin promoter and glyceraldehyde-3-phosphate dehydrogenase can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient for a given purpose. By employing a promoter with well-known properties, the level and pattern of expression of the protein of interest following transfection or transformation can be optimized. Further, selection of a promoter that is regulated in response to specific physiologic signals can permit inducible expression of the gene product.

Enhancers are genetic elements that increase transcription from a promoter located at a distant position on the same molecule of DNA. Enhancers are organized much like promoters. That is, they are composed of many individual elements, each of which binds to one or more transcriptional proteins. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have a very similar modular organization.

Below is a list of promoters/enhancers and inducible promoters/enhancers that could be used in combination with the nucleic acid encoding a gene of interest in an expression construct. Additionally, any promoter/enhancer combination (as per the Eukaryotic Promoter Data Base EPDB) could also be used to drive expression of the gene. Eukaryotic cells can support cytoplasmic transcription from certain bacterial promoters if the appropriate bacterial polymerase is provided, either as part of the delivery complex or as an additional genetic expression construct.

The promoter and/or enhancer may be, for example, immunoglobulin light chain, immunoglobulin heavy chain, T-cell receptor, HLA DQ a and/or DQ β, β-interferon, interleukin-2, interleukin-2 receptor, MHC class II 5, MHC class II HLA-Dra, β-Actin, muscle creatine kinase (MCK), prealbumin (transthyretin), elastase I, metallothionein (MTII), collagenase, albumin, α-fetoprotein, t-globin, β-globin, c-fos, c-HA-ras, insulin, neural cell adhesion molecule (NCAM), α₁-antitrypain, H2B (TH2B) histone, mouse and/or type I collagen, glucose-regulated proteins (GRP94 and GRP78), rat growth hormone, human serum amyloid A (SAA), troponin I (TN I), platelet-derived growth factor (PDGF), duchenne muscular dystrophy, SV40, polyoma, retroviruses, papilloma virus, hepatitis B virus, human immunodeficiency virus, cytomegalovirus (CMV), and gibbon ape leukemia virus.

In some embodiments, inducible elements may be used. In some embodiments, the inducible element is, for example, MTII, MMTV (mouse mammary tumor virus), β-interferon, adenovirus 5 E2, collagenase, stromelysin, SV40, murine MX gene, GRP78 gene, α-2-macroglobulin, vimentin, MHC class I gene H-2κb, HSP70, proliferin, tumor necrosis factor, and/or thyroid stimulating hormone α gene. In some embodiments, the inducer is phorbol ester (TFA), heavy metals, glucocorticoids, poly(rI)x, poly(rc), ElA, phorbol ester (TPA), interferon, Newcastle Disease Virus, A23187, IL-6, serum, interferon, SV40 large T antigen, PMA, and/or thyroid hormone. Any of the inducible elements described herein may be used with any of the inducers described herein.

Of particular interest are muscle specific promoters. These include the myosin light chain-2 promoter, the α-actin promoter, the troponin 1 promoter; the Na⁺/Ca²⁺ exchanger promoter, the dystrophin promoter, the α7 integrin promoter, the brain natriuretic peptide promoter and the αB-crystallin/small heat shock protein promoter, α-myosin heavy chain promoter and the ANF promoter. In some embodiments, the muscle specific promoter is the CK8 promoter. The CK8 promoter has the following sequence (SEQ ID NO. 874): 
1 CTAGACTAGC ATGCTGCCCA TGTAAGGAGG CAAGGCCTGG
GGACACCCGA GATGCCTGGT
 
61 TATAATTAAC CCAGACATGT GGCTGCCCCC CCCCCCCCAA
CACCTGCTGC CTCTAAAAAT
 
121 AACCCTGCAT GCCATGTTCC CGGCGAAGGG CCAGCTGTCC
CCCGCCAGCT AGACTCAGCA
 
181 CTTAGTTTAG GAACCAGTGA GCAAGTCAGC CCTTGGGGCA
GCCCATACAA GGCCATGGGG
 
241 CTGGGCAAGC TGCACGCCTG GGTCCGGGGT GGGCACGGTG
CCCGGGCAAC GAGCTGAAAG
 
301 CTCATCTGCT CTCAGGGGCC CCTCCCTGGG GACAGCCCCT
CCTGGCTAGT CACACCCTGT
 
361 AGGCTCCTCT ATATAACCCA GGGGCACAGG GGCTGCCCTC
ATTCTACCAC CACCTCCACA
 421 GCACAGACAG ACACTCAGGA GCCAGCCAGC

In some embodiments, the muscle-cell cell specific promoter is a variant of the CK8 promoter, called CK8e. The CK8e promoter has the following sequence (SEQ ID NO. 875): 
1 TGCCCATGTA AGGAGGCAAG GCCTGGGGAC ACCCGAGATG
CCTGGTTATA ATTAACCCAG
 
61 ACATGTGGCT GCCCCCCCCC CCCCAACACC TGCTGCCTCT
AAAAATAACC CTGCATGCCA
 
121 TGTTCCCGGC GAAGGGCCAG CTGTCCCCCG CCAGCTAGAC
TCAGCACTTA GTTTAGGAAC
 
181 CAGTGAGCAA GTCAGCCCTT GGGGCAGCCC ATACAAGGCC
ATGGGGCTGG GCAAGCTGCA
 
241 CGCCTGGGTC CGGGGTGGGC ACGGTGCCCG GGCAACGAGC
TGAAAGCTCA TCTGCTCTCA
 
301 GGGGCCCCTC CCTGGGGACA GCCCCTCCTG GCTAGTCACA
CCCTGTAGGC TCCTCTATAT
 
361 AACCCAGGGG CACAGGGGCT GCCCTCATTC TACCACCACC
TCCACAGCAC AGACAGACAC
 421 TCAGGAGCCA GCCAGC

Where a cDNA insert is employed, one will typically desire to include a polyadenylation signal to effect proper polyadenylation of the gene transcript. Any polyadenylation sequence may be employed such as human growth hormone and SV40 polyadenylation signals. Also contemplated as an element of the expression cassette is a terminator. These elements can serve to enhance message levels and to minimize read through from the cassette into other sequences.

### B. 2A Peptide

In some embodiments, a 2A-like self-cleaving domain from the insect virus *Thosea asigna* (TaV 2A peptide)(SEQ ID NO. 876, EGRGSLLTCGDVEENPGP) is used. These 2A-like domains have been shown to function across eukaryotes and cause cleavage of amino acids to occur co-translationally within the 2A-like peptide domain. Therefore, inclusion of TaV 2A peptide allows the expression of multiple proteins from a single mRNA transcript. Importantly, the domain of TaV when tested in eukaryotic systems has shown greater than 99% cleavage activity. Other acceptable 2A-like peptides include, but are not limited to, equine rhinitis A virus (ERAV) 2A peptide (SEQ ID NO. 877; QCTNYALLKLAGDVESNPGP), porcine teschovirus-1 (PTV1) 2A peptide (SEQ ID NO. 878; ATNFSLLKQAGDVEENPGP) and foot and mouth disease virus (FMDV) 2A peptide (SEQ ID NO. 879; PVKQLLNFDLLKLAGDVESNPGP) or modified versions thereof.

In some embodiments, the 2A peptide is used to express a reporter and a Cas9 or a Cpfl simultaneously. The reporter may be, for example, GFP.

Other self-cleaving peptides that may be used include, but are not limited to nuclear inclusion protein a (Nia) protease, a P1 protease, a 3C protease, a L protease, a 3C-like protease, or modified versions thereof.

### C. Delivery of Expression Vectors

There are a number of ways in which expression vectors may be introduced into cells. In certain embodiments, the expression construct comprises a virus or engineered construct derived from a viral genome. The ability of certain viruses to enter cells via receptor-mediated endocytosis, to integrate into host cell genome and express viral genes stably and efficiently have made them attractive candidates for the transfer of foreign genes into mammalian cells . These have a relatively low capacity for foreign DNA sequences and have a restricted host spectrum. Furthermore, their oncogenic potential and cytopathic effects in permissive cells raise safety concerns. They can accommodate only up to 8 kB of foreign genetic material but can be readily introduced in a variety of cell lines and laboratory animals.

One of the preferred methods for *in vivo* delivery involves the use of an adenovirus expression vector. "Adenovirus expression vector" is meant to include those constructs containing adenovirus sequences sufficient to (a) support packaging of the construct and (b) to express an antisense polynucleotide that has been cloned therein. In this context, expression does not require that the gene product be synthesized.

The expression vector comprises a genetically engineered form of adenovirus. Knowledge of the genetic organization of adenovirus, a 36 kB, linear, double-stranded DNA virus, allows substitution of large pieces of adenoviral DNA with foreign sequences up to 7 kB. In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

Adenovirus is particularly suitable for use as a gene transfer vector because of its mid-sized genome, ease of manipulation, high titer, wide target cell range and high infectivity. Both ends of the viral genome contain 100-200 base pair inverted repeats (ITRs), which are *cis* elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off. The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNAs issued from this promoter possess a 5 -tripartite leader (TPL) sequence which makes them preferred mRNAs for translation.In one system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the current adenovirus vectors, which are replication deficient, depend on a unique helper cell line, designated 293, which was transformed from human embryonic kidney cells by Ad5 DNA fragments and constitutively expresses E1 proteins. Since the E3 region is dispensable from the adenovirus genome, the current adenovirus vectors, with the help of 293 cells, carry foreign DNA in either the E1, the D3 or both regions. In nature, adenovirus can package approximately 105% of the wild-type genome, providing capacity for about 2 extra kb of DNA. Combined with the approximately 5.5 kb of DNA that is replaceable in the E1 and E3 regions, the maximum capacity of the current adenovirus vector is under 7.5 kb, or about 15% of the total length of the vector. More than 80% of the adenovirus viral genome remains in the vector backbone and is the source of vector-borne cytotoxicity. Also, the replication deficiency of the E1-deleted virus is incomplete.

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, e.g., Vero cells or other monkey embryonic mesenchymal or epithelial cells. As stated above, the preferred helper cell line is 293.

Improved methods for culturing 293 cells and propagating adenovirus are known in the art. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for 1 to 4 h. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 h.

The adenoviruses of the disclosure are replication defective, or at least conditionally replication defective. The adenovirus may be of any of the 42 different known serotypes or subgroups A-F. Adenovirus type 5 of subgroup C is the preferred starting material in order to obtain the conditional replication-defective adenovirus vector for use in the present disclosure.

As stated above, the typical vector according to the present disclosure is replication defective and will not have an adenovirus E1 region. Thus, it will be most convenient to introduce the polynucleotide encoding the gene of interest at the position from which the E1-coding sequences have been removed. However, the position of insertion of the construct within the adenovirus sequences is not critical. The polynucleotide encoding the gene of interest may also be inserted in lieu of the deleted E3 region in E3 replacement vectors, or in the E4 region where a helper cell line or helper virus complements the E4 defect.

Adenovirus is easy to grow and manipulate and exhibits broad host range *in vitro* and *in vivo.* This group of viruses can be obtained in high titers, *e.g.*, 10⁹-10¹² plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus, demonstrating their safety and therapeutic potential as *in vivo* gene transfer vectors.

Adenovirus vectors have been used in eukaryotic gene expression and vaccine development. Animal studies suggested that recombinant adenovirus could be used for gene therapy. Studies in administering recombinant adenovirus to different tissues include trachea instillation, muscle injection, peripheral intravenous injections and stereotactic inoculation into the brain.

The retroviruses are a group of single-stranded RNA viruses characterized by an ability to convert their RNA to double-stranded DNA in infected cells by a process of reverse-transcription. The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome.

In order to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. However, integration and stable expression require the division of host cells.

A novel approach designed to allow specific targeting of retrovirus vectors was recently developed based on the chemical modification of a retrovirus by the chemical addition of lactose residues to the viral envelope. This modification could permit the specific infection of hepatocytes via sialoglycoprotein receptors.

A different approach to targeting of recombinant retroviruses may be used, in which biotinylated antibodies against a retroviral envelope protein and against a specific cell receptor are used. The antibodies are coupled via the biotin components by using streptavidin. Using antibodies against major histocompatibility complex class I and class II antigens, it has been demonstrated the infection of a variety of human cells that bore those surface antigens with an ecotropic virus *in vitro* (Roux *et al.,* 1989).

There are certain limitations to the use of retrovirus vectors in all aspects of the present disclosure. For example, retrovirus vectors usually integrate into random sites in the cell genome. This can lead to insertional mutagenesis through the interruption of host genes or through the insertion of viral regulatory sequences that can interfere with the function of flanking genes. Another concern with the use of defective retrovirus vectors is the potential appearance of wild-type replication-competent virus in the packaging cells. This can result from recombination events in which the intact- sequence from the recombinant virus inserts upstream from the gag, pol, env sequence integrated in the host cell genome. However, new packaging cell lines are now available that should greatly decrease the likelihood of recombination (see, for example, Markowitz *et al.,* 1988; Hersdorffer *et al.,* 1990).

Other viral vectors may be employed as expression constructs in the present disclosure. Vectors derived from viruses such as vaccinia virus adeno-associated virus (AAV) and herpesviruses may be employed. They offer several attractive features for various mammalian cells.

In embodiments, the AAV vector is replication-defective or conditionally replication defective. In embodiments, the AAV vector is a recombinant AAV vector. In some embodiments, the AAV vector comprises a sequence isolated or derived from an AAV vector of serotype AAV1, AAV2, AAV3, AAV4, AAV5, AAV6, AAV7, AAV8, AAV9, AAV10, AAV11 or any combination thereof.

In some embodiments, a single viral vector is used to deliver a nucleic acid encoding a Cas9 or a Cpfl and a nucleic acid of the present invention comprising at least the first, second and third DMD gRNAs to a cell. In some embodiments, Cas9 or Cpfl is provided to a cell using a first viral vector and a nucleic acid of the present invention comprising at least the first, second and third DMD gRNAs is provided to the cell using a second viral vector.

In some embodiments, a single viral vector is used to deliver a nucleic acid encoding Cas9 or Cpfl and a nucleic acid of the present invention comprising at least the first, second and third DMD gRNAs to a cell. In some embodiments, Cas9 or Cpfl is provided to a cell using a first viral vector and a nucleic acid of the present invention comprising at least the first, second and third DMD gRNAs is provided to the cell using a second viral vector. In order to effect expression of sense or antisense gene constructs, the expression construct must be delivered into a cell. The cell may be a muscle cell, a satellite cell, a mesangioblast, a bone marrow derived cell, a stromal cell or a mesenchymal stem cell. In embodiments, the cell is a cardiac muscle cell, a skeletal muscle cell, or a smooth muscle cell. In embodiments, the cell is a cell in the tibialis anterior, quadriceps, soleus, diaphragm or heart. In some embodiments, the cell is an induced pluripotent stem cell (iPSC) or inner cell mass cell (iCM). In further embodiments, the cell is a human iPSC or a human iCM. In some embodiments, human iPSCs or human iCMs of the disclosure may be derived from a cultured stem cell line, an adult stem cell, a placental stem cell, or from another source of adult or embryonic stem cells that does not require the destruction of a human embryo. Delivery to a cell may be accomplished *in vitro,* as in laboratory procedures for transforming cells lines, or *in vivo* or *ex vivo*, as in the treatment of certain disease states. One mechanism for delivery is via viral infection where the expression construct is encapsidated in an infectious viral particle.

Several non-viral methods for the transfer of expression constructs into cultured mammalian cells also are contemplated by the present disclosure. These include calcium phosphate precipitation, DEAE-dextran, electroporation, direct microinjection, DNA-loaded liposomes and lipofectamine-DNA complexes, cell sonication, gene bombardment using high velocity microprojectiles, and receptor-mediated transfection. Some of these techniques may be successfully adapted for *in vivo* or *ex vivo* use.

Once the expression construct has been delivered into the cell the nucleic acid encoding the gene of interest may be positioned and expressed at different sites. In certain embodiments, the nucleic acid encoding the gene may be stably integrated into the genome of the cell. This integration may be in the cognate location and orientation via homologous recombination (gene replacement) or it may be integrated in a random, non-specific location (gene augmentation). In yet further embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression construct employed.

In yet another embodiment, the expression construct may simply consist of naked recombinant DNA or plasmids. Transfer of the construct may be performed by any of the methods mentioned above which physically or chemically permeabilize the cell membrane. This is particularly applicable for transfer *in vitro* but it may be applied to *in vivo* use as well. Dubensky *et al.* (1984) successfully injected polyomavirus DNA in the form of calcium phosphate precipitates into liver and spleen of adult and newborn mice demonstrating active viral replication and acute infection. Benvenisty and Neshif (1986) also demonstrated that direct intraperitoneal injection of calcium phosphate-precipitated plasmids results in expression of the transfected genes. DNA encoding a gene of interest may also be transferred in a similar manner *in vivo* and express the gene product.

In still another embodiment for transferring a naked DNA expression construct into cells may involve particle bombardment. This method depends on the ability to accelerate DNA-coated microprojectiles to a high velocity allowing them to pierce cell membranes and enter cells without killing them. Several devices for accelerating small particles have been developed. One such device relies on a high voltage discharge to generate an electrical current, which in turn provides the motive force. The microprojectiles used have consisted of biologically inert substances such as tungsten or gold beads.

In some embodiments, the expression construct is delivered directly to the liver, skin, and/or muscle tissue of a subject. This may require surgical exposure of the tissue or cells, to eliminate any intervening tissue between the gun and the target organ, *i.e.*, *ex vivo* treatment. Again, DNA encoding a particular gene may be delivered via this method and still be incorporated by the present disclosure.

In a further embodiment, the expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers. Also contemplated are lipofectamine-DNA complexes.

Liposome-mediated nucleic acid delivery and expression of foreign DNA *in vitro* has been very successful. A reagent known as Lipofectamine 2000^{™} is widely used and commercially available.

In certain embodiments, the liposome may be complexed with a hemagglutinating virus (HVJ) to facilitate fusion with the cell membrane and promote cell entry of liposome-encapsulated DNA. In other embodiments, the liposome may be complexed or employed in conjunction with nuclear non-histone chromosomal proteins (HMG-1). In yet further embodiments, the liposome may be complexed or employed in conjunction with both HVJ and HMG-1. In that such expression constructs have been successfully employed in transfer and expression of nucleic acid *in vitro* and *in vivo,* then they are applicable for the present disclosure. Where a bacterial promoter is employed in the DNA construct, it also will be desirable to include within the liposome an appropriate bacterial polymerase.

Other expression constructs which can be employed to deliver a nucleic acid encoding a particular gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific.

Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) and transferrin. A synthetic neoglycoprotein, which recognizes the same receptor as ASOR, has been used as a gene delivery vehicle and epidermal growth factor (EGF) has also been used to deliver genes to squamous carcinoma cells.

### D. AAV-Cas9 vectors

In some embodiments, a Cas9 may be packaged into an AAV vector. In some embodiments, the AAV vector is a wildtype AAV vector. In some embodiments, the AAV vector contains one or more mutations. In some embodiments, the AAV vector is isolated or derived from an AAV vector of serotype AAV1, AAV2, AAV3, AAV4, AAV5, AAV6, AAV7, AAV8, AAV9, AAV10, AAV1 1 or any combination thereof.

Exemplary AAV-Cas9 vectors contain two ITR (inverted terminal repeat) sequences which flank a central sequence region comprising the Cas9 sequence. In some embodiments, the ITRs are isolated or derived from an AAV vector of serotype AAV1, AAV2, AAV3, AAV4, AAV5, AAV6, AAV7, AAV8, AAV9, AAV10, AAV11 or any combination thereof. In some embodiments, the ITRs comprise or consist of full-length and/or wildtype sequences for an AAV serotype. In some embodiments, the ITRs comprise or consist of truncated sequences for an AAV serotype. In some embodiments, the ITRs comprise or consist of elongated sequences for an AAV serotype. In some embodiments, the ITRs comprise or consist of sequences comprising a sequence variation compared to a wildtype sequence for the same AAV serotype. In some embodiments, the sequence variation comprises one or more of a substitution, deletion, insertion, inversion, or transposition. In some embodiments, the ITRs comprise or consist of at least 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150 base pairs. In some embodiments, the ITRs comprise or consist of 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150 base pairs. In some embodiments, the ITRs have a length of 110 ± 10 base pairs. In some embodiments, the ITRs have a length of 120 ± 10 base pairs. In some embodiments, the ITRs have a length of 130 ± 10 base pairs. In some embodiments, the ITRs have a length of 140 ± 10 base pairs. In some embodiments, the ITRs have a length of 150 ± 10 base pairs. In some embodiments, the ITRs have a length of 115, 145, or 141 base pairs. In some embodiments, the ITRs have a sequence selected from SEQ. ID. NO: 880, SEQ ID NO: 881, SEQ ID NO; 882, SEQ ID NO: 883 and SEQ ID. NO: 946.

In some embodiments, the AAV-Cas9 vector may contain one or more nuclear localization signals (NLS). In some embodiments, the AAV-Cas9 vector contains 1, 2, 3, 4, or 5 nuclear localization signals. Exemplary NLS include the c-myc NLS (SEQ ID NO: 884), the SV40 NLS (SEQ ID NO: 885), the hnRNPAI M9 NLS (SEQ ID NO: 886), the nucleoplasmin NLS (SEQ ID NO: 887), the sequence RMRKFKNKGKDTAELRRRRVEVSVELRKAKKDEQILKRRNV (SEQ ID NO: 888) of the IBB domain from importin-alpha, the sequences VSRKRPRP (SEQ ID NO: 889) and PPKKARED (SEQ ID NO: 890) of the myoma T protein, the sequence PQPKKKPL (SEQ ID NO: 891) of human p53, the sequence SALIKKKKKMAP (SEQ ID NO: 892) of mouse c-abl IV, the sequences DRLRR (SEQ ID NO: 893) and PKQKKRK (SEQ ID NO: 894) of the influenza virus NS1, the sequence RKLKKKIKKL (SEQ ID NO: 895) of the Hepatitis virus delta antigen and the sequence REKKKFLKRR (SEQ ID NO: 896) of the mouse Mx1 protein. Further acceptable nuclear localization signals include bipartite nuclear localization sequences such as the sequence KRKGDEVDGVDEVAKKKSKK (SEQ ID NO: 897) of the human poly(ADP-ribose) polymerase or the sequence RKCLQAGMNLEARKTKK (SEQ ID NO: 898) of the steroid hormone receptors (human) glucocorticoid.

In some embodiments, the AAV-Cas9 vector may comprise additional elements to facilitate packaging of the vector and expression of the Cas9. In some embodiments, the AAV-Cas9 vector may comprise a polyA sequence. In some embodiments, the polyA sequence may be a mini-polyA sequence. In some embodiments, the AAV-CAs9 vector may comprise a transposable element. In some embodiments, the AAV-Cas9 vector may comprise a regulator element. In some embodiments, the regulator element is an activator or a repressor.

In some embodiments, the AAV-Cas9 may contain one or more promoters. In some embodiments, the one or more promoters drive expression of the Cas9. In some embodiments, the one or more promoters are muscle-specific promoters. Exemplary muscle-specific promoters include myosin light chain-2 promoter, the α-actin promoter, the troponin 1 promoter, the Na+/Ca2+ exchanger promoter, the dystrophin promoter, the α7 integrin promoter, the brain natriuretic peptide promoter, the αB-crystallin/small heat shock protein promoter, α-myosin heavy chain promoter, the ANF promoter, the CK8 promoter and the CK8e promoter.

In some embodiments, the AAV-Cas9 vector may be optimized for production in yeast, bacteria, insect cells, or mammalian cells. In some embodiments, the AAV-Cas9 vector may be optimized for expression in human cells. In some embodiments, the AAV-Cas9 vector may be optimized for expression in a bacculovirus expression system.

In some embodiments, the AAV-Cas9 vector comprises a sequence selected from SEQ ID NO: 899, SEQ ID NO: 900, SEQ ID NO: 901, or SEQ ID NO: 902, as shown in Table 4.

**Table 4: Exemplary gene editing constructs (from ITR to ITR for delivery via AAV vector)**

| SE Q ID NO: | Sequence |
|---|---|
| 899 | GGCCACTCCCTCTCTGCGCGCTCGCTCGCTCACTGAGGCCGGGCGACCA AAGGTCGCCCGACGCCCGGGCTTTGCCCGGGCGGCCTCAGTGAGCGAGC GAGCGCGCAGAGAGGGAGTGGCCAACTCCATCACTAGGGGTTCCTTGTA GTTAATGATTAACCCGCCATGCTACTTATCTACGTAGCCATGCTCTAGAT TAATTAATGCCCATGTAAGGAGGCAAGGCCTGGGGACACCCGAGATGCC TGGTTATAATTAACCCAGACATGTGGCTGCCCCCCCCCCCCCAACACCTG CTGCCTCTAAAAATAACCCTGCATGCCATGTTCCCGGCGAAGGGCCAGC TGTCCCCCGCCAGCTAGACTCAGCACTTAGTTTAGGAACCAGTGAGCAA |
| | GTCAGCCCTTGGGGCAGCCCATACAAGGCCATGGGGCTGGGCAAGCTGC ACGCCTGGGTCCGGGGTGGGCACGGTGCCCGGGCAACGAGCTGAAAGC TCATCTGCTCTCAGGGGCCCCTCCCTGGGGACAGCCCCTCCTGGCTAGTC ACACCCTGTAGGCTCCTCTATATAACCCAGGGGCACAGGGGCTGCCCTC ATTCTACCACCACCTCCACAGCACAGACAGACACTCAGGAGCCAGCCAG CGCTAGCGCCACCATGATGGCCCCAAAGAAGAAGCGGAAGGTCGGTAT CCACGGAGTCCCAGCAGCCGACAAGAAGTACAGCATCGGCCTGGACATC GGCACCAACTCTGTGGGCTGGGCCGTGATCACCGACGAGTACAAGGTGC CCAGCAAGAAATTCAAGGTGCTGGGCAACACCGACCGGCACAGCATCA AGAAGAACCTGATCGGAGCCCTGCTGTTCGACAGCGGCGAAACAGCCG AGGCCACCCGGCTGAAGAGAACCGCCAGAAGAAGATACACCAGACGGA AGAACCGGATCTGCTATCTGCAAGAGATCTTCAGCAACGAGATGGCCAA GGTGGACGACAGCTTCTTCCACAGACTGGAAGAGTCCTTCCTGGTGGAA GAGGATAAGAAGCACGAGCGGCACCCCATCTTCGGCAACATCGTGGAC GAGGTGGCCTACCACGAGAAGTACCCCACCATCTACCACCTGAGAAAGA AACTGGTGGACAGCACCGACAAGGCCGACCTGCGGCTGATCTATCTGGC CCTGGCCCACATGATCAAGTTCCGGGGCCACTTCCTGATCGAGGGCGAC CTGAACCCCGACAACAGCGACGTGGACAAGCTGTTCATCCAGCTGGTGC AGACCTACAACCAGCTGTTCGAGGAAAACCCCATCAACGCCAGCGGCGT GGACGCCAAGGCCATCCTGTCTGCCAGACTGAGCAAGAGCAGACGGCTG GAAAATCTGATCGCCCAGCTGCCCGGCGAGAAGAAGAATGGCCTGTTCG GCAACCTGATTGCCCTGAGCCTGGGCCTGACCCCCAACTTCAAGAGCAA CTTCGACCTGGCCGAGGATGCCAAACTGCAGCTGAGCAAGGACACCTAC GACGACGACCTGGACAACCTGCTGGCCCAGATCGGCGACCAGTACGCCG ACCTGTTTCTGGCCGCCAAGAACCTGTCCGACGCCATCCTGCTGAGCGA CATCCTGAGAGTGAACACCGAGATCACCAAGGCCCCCCTGAGCGCCTCT ATGATCAAGAGATACGACGAGCACCACCAGGACCTGACCCTGCTGAAA GCTCTCGTGCGGCAGCAGCTGCCTGAGAAGTACAAAGAGATTTTCTTCG ACCAGAGCAAGAACGGCTACGCCGGCTACATTGACGGCGGAGCCAGCC AGGAAGAGTTCTACAAGTTCATCAAGCCCATCCTGGAAAAGATGGACGG CACCGAGGAACTGCTCGTGAAGCTGAACAGAGAGGACCTGCTGCGGAA GCAGCGGACCTTCGACAACGGCAGCATCCCCCACCAGATCCACCTGGGA GAGCTGCACGCCATTCTGCGGCGGCAGGAAGATTTTTACCCATTCCTGA AGGACAACCGGGAAAAGATCGAGAAGATCCTGACCTTCCGCATCCCCTA CTACGTGGGCCCTCTGGCCAGGGGAAACAGCAGATTCGCCTGGATGACC AGAAAGAGCGAGGAAACCATCACCCCCTGGAACTTCGAGGAAGTGGTG GACAAGGGCGCTTCCGCCCAGAGCTTCATCGAGCGGATGACCAACTTCG ATAAGAACCTGCCCAACGAGAAGGTGCTGCCCAAGCACAGCCTGCTGTA CGAGTACTTCACCGTGTATAACGAGCTGACCAAAGTGAAATACGTGACC GAGGGAATGAGAAAGCCCGCCTTCCTGAGCGGCGAGCAGAAAAAGGCC ATCGTGGACCTGCTGTTCAAGACCAACCGGAAAGTGACCGTGAAGCAGC TGAAAGAGGACTACTTCAAGAAAATCGAGTGCTTCGACTCCGTGGAAAT CTCCGGCGTGGAAGATCGGTTCAACGCCTCCCTGGGCACATACCACGAT CTGCTGAAAATTATCAAGGACAAGGACTTCCTGGACAATGAGGAAAACG AGGACATTCTGGAAGATATCGTGCTGACCCTGACACTGTTTGAGGACAG AGAGATGATCGAGGAACGGCTGAAAACCTATGCCCACCTGTTCGACGAC AAAGTGATGAAGCAGCTGAAGCGGCGGAGATACACCGGCTGGGGCAGG CTGAGCCGGAAGCTGATCAACGGCATCCGGGACAAGCAGTCCGGCAAG ACAATCCTGGATTTCCTGAAGTCCGACGGCTTCGCCAACAGAAACTTCA TGCAGCTGATCCACGACGACAGCCTGACCTTTAAAGAGGACATCCAGAA |
| | AGCCCAGGTGTCCGGCCAGGGCGATAGCCTGCACGAGCACATTGCCAAT CTGGCCGGCAGCCCCGCCATTAAGAAGGGCATCCTGCAGACAGTGAAGG TGGTGGACGAGCTCGTGAAAGTGATGGGCCGGCACAAGCCCGAGAACA TCGTGATCGAAATGGCCAGAGAGAACCAGACCACCCAGAAGGGACAGA AGAACAGCCGCGAGAGAATGAAGCGGATCGAAGAGGGCATCAAAGAGC TGGGCAGCCAGATCCTGAAAGAACACCCCGTGGAAAACACCCAGCTGC AGAACGAGAAGCTGTACCTGTACTACCTGCAGAATGGGCGGGATATGTA CGTGGACCAGGAACTGGACATCAACCGGCTGTCCGACTACGATGTGGAC CATATCGTGCCTCAGAGCTTTCTGAAGGACGACTCCATCGACAACAAGG TGCTGACCAGAAGCGACAAGAACCGGGGCAAGAGCGACAACGTGCCCT CCGAAGAGGTCGTGAAGAAGATGAAGAACTACTGGCGGCAGCTGCTGA ACGCCAAGCTGATTACCCAGAGAAAGTTCGACAATCTGACCAAGGCCGA GAGAGGCGGCCTGAGCGAACTGGATAAGGCCGGCTTCATCAAGAGACA GCTGGTGGAAACCCGGCAGATCACAAAGCACGTGGCACAGATCCTGGA CTCCCGGATGAACACTAAGTACGACGAGAATGACAAGCTGATCCGGGA AGTGAAAGTGATCACCCTGAAGTCCAAGCTGGTGTCCGATTTCCGGAAG GATTTCCAGTTTTACAAAGTGCGCGAGATCAACAACTACCACCACGCCC ACGACGCCTACCTGAACGCCGTCGTGGGAACCGCCCTGATCAAAAAGTA CCCTAAGCTGGAAAGCGAGTTCGTGTACGGCGACTACAAGGTGTACGAC GTGCGGAAGATGATCGCCAAGAGCGAGCAGGAAATCGGCAAGGCTACC GCCAAGTACTTCTTCTACAGCAACATCATGAACTTTTTCAAGACCGAGAT TACCCTGGCCAACGGCGAGATCCGGAAGCGGCCTCTGATCGAGACAAAC GGCGAAACCGGGGAGATCGTGTGGGATAAGGGCCGGGATTTTGCCACC GTGCGGAAAGTGCTGAGCATGCCCCAAGTGAATATCGTGAAAAAGACC GAGGTGCAGACAGGCGGCTTCAGCAAAGAGTCTATCCTGCCCAAGAGG AACAGCGATAAGCTGATCGCCAGAAAGAAGGACTGGGACCCTAAGAAG TACGGCGGCTTCGACAGCCCCACCGTGGCCTATTCTGTGCTGGTGGTGGC CAAAGTGGAAAAGGGCAAGTCCAAGAAACTGAAGAGTGTGAAAGAGCT GCTGGGGATCACCATCATGGAAAGAAGCAGCTTCGAGAAGAATCCCATC GACTTTCTGGAAGCCAAGGGCTACAAAGAAGTGAAAAAGGACCTGATC ATCAAGCTGCCTAAGTACTCCCTGTTCGAGCTGGAAAACGGCCGGAAGA GAATGCTGGCCTCTGCCGGCGAACTGCAGAAGGGAAACGAACTGGCCCT GCCCTCCAAATATGTGAACTTCCTGTACCTGGCCAGCCACTATGAGAAG CTGAAGGGCTCCCCCGAGGATAATGAGCAGAAACAGCTGTTTGTGGAAC AGCACAAGCACTACCTGGACGAGATCATCGAGCAGATCAGCGAGTTCTC CAAGAGAGTGATCCTGGCCGACGCTAATCTGGACAAAGTGCTGTCCGCC TACAACAAGCACCGGGATAAGCCCATCAGAGAGCAGGCCGAGAATATC ATCCACCTGTTTACCCTGACCAATCTGGGAGCCCCTGCCGCCTTCAAGTA CTTTGACACCACCATCGACCGGAAGAGGTACACCAGCACCAAAGAGGTG CTGGACGCCACCCTGATCCACCAGAGCATCACCGGCCTGTACGAGACAC GGATCGACCTGTCTCAGCTGGGAGGCGACAAGCGTCCTGCTGCTACTAA GAAAGCTGGTCAAGCTAAGAAAAAGAAATGAATCGATTAGCAATAAAG GATCGTTTATTTTCATTGGAAGCGTGTGTTGGTTTTTTGATCAGGCGCGG GTACCAGGTCGCGGCCGCTCTAGAGCATGGCTACGTAGATAAGTAGCAT GGCGGGTTAATCATTAACTACAAGGAACCCCTAGTGATGGAGTTGGCCA CTCCCTCTCTGCGCGCTCGCTCGCTCACTGAGGCCGGGCGACCAAAGGT CGCCCGACGCCCGGGCTTTGCCCGGGCGGCCTCAGTGAGCGAGCGAGCG CGCAGAGAGGGA |
| | |
| 900 | CCTGCAGGCAGCTGCGCGCTCGCTCGCTCACTGAGGCCGCCCGGGCAAA GCCCGGGCGTCGGGCGACCTTTGGTCGCCCGGCCTCAGTGAGCGAGCGA GCGCGCAGAGAGGGAGTGGCCAACTCCATCACTAGGGGTTCCTGTGGCC AACTCCATCACTAGGGGTTCCTTGTAGTTAATGATTAACCCGCCATGCTA CTTATCTACGTAGCCATGCTCTAGATTAATTAATGCCCATGTAAGGAGGC AAGGCCTGGGGACACCCGAGATGCCTGGTTATAATTAACCCAGACATGT GGCTGCCCCCCCCCCCCCAACACCTGCTGCCTCTAAAAATAACCCTGCAT GCCATGTTCCCGGCGAAGGGCCAGCTGTCCCCCGCCAGCTAGACTCAGC ACTTAGTTTAGGAACCAGTGAGCAAGTCAGCCCTTGGGGCAGCCCATAC AAGGCCATGGGGCTGGGCAAGCTGCACGCCTGGGTCCGGGGTGGGCAC GGTGCCCGGGCAACGAGCTGAAAGCTCATCTGCTCTCAGGGGCCCCTCC CTGGGGACAGCCCCTCCTGGCTAGTCACACCCTGTAGGCTCCTCTATATA ACCCAGGGGCACAGGGGCTGCCCTCATTCTACCACCACCTCCACAGCAC AGACAGACACTCAGGAGCCAGCCAGCGCTAGCGCCACCATGATGGCCCC AAAGAAGAAGCGGAAGGTCGGTATCCACGGAGTCCCAGCAGCCGACAA GAAGTACAGCATCGGCCTGGACATCGGCACCAACTCTGTGGGCTGGGCC GTGATCACCGACGAGTACAAGGTGCCCAGCAAGAAATTCAAGGTGCTGG GCAACACCGACCGGCACAGCATCAAGAAGAACCTGATCGGAGCCCTGCT GTTCGACAGCGGCGAAACAGCCGAGGCCACCCGGCTGAAGAGAACCGC CAGAAGAAGATACACCAGACGGAAGAACCGGATCTGCTATCTGCAAGA GATCTTCAGCAACGAGATGGCCAAGGTGGACGACAGCTTCTTCCACAGA CTGGAAGAGTCCTTCCTGGTGGAAGAGGATAAGAAGCACGAGCGGCAC CCCATCTTCGGCAACATCGTGGACGAGGTGGCCTACCACGAGAAGTACC CCACCATCTACCACCTGAGAAAGAAACTGGTGGACAGCACCGACAAGG CCGACCTGCGGCTGATCTATCTGGCCCTGGCCCACATGATCAAGTTCCGG GGCCACTTCCTGATCGAGGGCGACCTGAACCCCGACAACAGCGACGTGG ACAAGCTGTTCATCCAGCTGGTGCAGACCTACAACCAGCTGTTCGAGGA AAACCCCATCAACGCCAGCGGCGTGGACGCCAAGGCCATCCTGTCTGCC AGACTGAGCAAGAGCAGACGGCTGGAAAATCTGATCGCCCAGCTGCCC GGCGAGAAGAAGAATGGCCTGTTCGGCAACCTGATTGCCCTGAGCCTGG GCCTGACCCCCAACTTCAAGAGCAACTTCGACCTGGCCGAGGATGCCAA ACTGCAGCTGAGCAAGGACACCTACGACGACGACCTGGACAACCTGCTG GCCCAGATCGGCGACCAGTACGCCGACCTGTTTCTGGCCGCCAAGAACC TGTCCGACGCCATCCTGCTGAGCGACATCCTGAGAGTGAACACCGAGAT CACCAAGGCCCCCCTGAGCGCCTCTATGATCAAGAGATACGACGAGCAC CACCAGGACCTGACCCTGCTGAAAGCTCTCGTGCGGCAGCAGCTGCCTG AGAAGTACAAAGAGATTTTCTTCGACCAGAGCAAGAACGGCTACGCCGG CTACATTGACGGCGGAGCCAGCCAGGAAGAGTTCTACAAGTTCATCAAG CCCATCCTGGAAAAGATGGACGGCACCGAGGAACTGCTCGTGAAGCTGA ACAGAGAGGACCTGCTGCGGAAGCAGCGGACCTTCGACAACGGCAGCA TCCCCCACCAGATCCACCTGGGAGAGCTGCACGCCATTCTGCGGCGGCA GGAAGATTTTTACCCATTCCTGAAGGACAACCGGGAAAAGATCGAGAAG ATCCTGACCTTCCGCATCCCCTACTACGTGGGCCCTCTGGCCAGGGGAA ACAGCAGATTCGCCTGGATGACCAGAAAGAGCGAGGAAACCATCACCC CCTGGAACTTCGAGGAAGTGGTGGACAAGGGCGCTTCCGCCCAGAGCTT CATCGAGCGGATGACCAACTTCGATAAGAACCTGCCCAACGAGAAGGTG CTGCCCAAGCACAGCCTGCTGTACGAGTACTTCACCGTGTATAACGAGC |
| | TGACCAAAGTGAAATACGTGACCGAGGGAATGAGAAAGCCCGCCTTCCT GAGCGGCGAGCAGAAAAAGGCCATCGTGGACCTGCTGTTCAAGACCAA CCGGAAAGTGACCGTGAAGCAGCTGAAAGAGGACTACTTCAAGAAAAT CGAGTGCTTCGACTCCGTGGAAATCTCCGGCGTGGAAGATCGGTTCAAC GCCTCCCTGGGCACATACCACGATCTGCTGAAAATTATCAAGGACAAGG ACTTCCTGGACAATGAGGAAAACGAGGACATTCTGGAAGATATCGTGCT GACCCTGACACTGTTTGAGGACAGAGAGATGATCGAGGAACGGCTGAA AACCTATGCCCACCTGTTCGACGACAAAGTGATGAAGCAGCTGAAGCGG CGGAGATACACCGGCTGGGGCAGGCTGAGCCGGAAGCTGATCAACGGC ATCCGGGACAAGCAGTCCGGCAAGACAATCCTGGATTTCCTGAAGTCCG ACGGCTTCGCCAACAGAAACTTCATGCAGCTGATCCACGACGACAGCCT GACCTTTAAAGAGGACATCCAGAAAGCCCAGGTGTCCGGCCAGGGCGAT AGCCTGCACGAGCACATTGCCAATCTGGCCGGCAGCCCCGCCATTAAGA AGGGCATCCTGCAGACAGTGAAGGTGGTGGACGAGCTCGTGAAAGTGA TGGGCCGGCACAAGCCCGAGAACATCGTGATCGAAATGGCCAGAGAGA ACCAGACCACCCAGAAGGGACAGAAGAACAGCCGCGAGAGAATGAAGC GGATCGAAGAGGGCATCAAAGAGCTGGGCAGCCAGATCCTGAAAGAAC ACCCCGTGGAAAACACCCAGCTGCAGAACGAGAAGCTGTACCTGTACTA CCTGCAGAATGGGCGGGATATGTACGTGGACCAGGAACTGGACATCAAC CGGCTGTCCGACTACGATGTGGACCATATCGTGCCTCAGAGCTTTCTGAA GGACGACTCCATCGACAACAAGGTGCTGACCAGAAGCGACAAGAACCG GGGCAAGAGCGACAACGTGCCCTCCGAAGAGGTCGTGAAGAAGATGAA GAACTACTGGCGGCAGCTGCTGAACGCCAAGCTGATTACCCAGAGAAAG TTCGACAATCTGACCAAGGCCGAGAGAGGCGGCCTGAGCGAACTGGAT AAGGCCGGCTTCATCAAGAGACAGCTGGTGGAAACCCGGCAGATCACA AAGCACGTGGCACAGATCCTGGACTCCCGGATGAACACTAAGTACGACG AGAATGACAAGCTGATCCGGGAAGTGAAAGTGATCACCCTGAAGTCCA AGCTGGTGTCCGATTTCCGGAAGGATTTCCAGTTTTACAAAGTGCGCGA GATCAACAACTACCACCACGCCCACGACGCCTACCTGAACGCCGTCGTG GGAACCGCCCTGATCAAAAAGTACCCTAAGCTGGAAAGCGAGTTCGTGT ACGGCGACTACAAGGTGTACGACGTGCGGAAGATGATCGCCAAGAGCG AGCAGGAAATCGGCAAGGCTACCGCCAAGTACTTCTTCTACAGCAACAT CATGAACTTTTTCAAGACCGAGATTACCCTGGCCAACGGCGAGATCCGG AAGCGGCCTCTGATCGAGACAAACGGCGAAACCGGGGAGATCGTGTGG GATAAGGGCCGGGATTTTGCCACCGTGCGGAAAGTGCTGAGCATGCCCC AAGTGAATATCGTGAAAAAGACCGAGGTGCAGACAGGCGGCTTCAGCA AAGAGTCTATCCTGCCCAAGAGGAACAGCGATAAGCTGATCGCCAGAA AGAAGGACTGGGACCCTAAGAAGTACGGCGGCTTCGACAGCCCCACCGT GGCCTATTCTGTGCTGGTGGTGGCCAAAGTGGAAAAGGGCAAGTCCAAG AAACTGAAGAGTGTGAAAGAGCTGCTGGGGATCACCATCATGGAAAGA AGCAGCTTCGAGAAGAATCCCATCGACTTTCTGGAAGCCAAGGGCTACA AAGAAGTGAAAAAGGACCTGATCATCAAGCTGCCTAAGTACTCCCTGTT CGAGCTGGAAAACGGCCGGAAGAGAATGCTGGCCTCTGCCGGCGAACT GCAGAAGGGAAACGAACTGGCCCTGCCCTCCAAATATGTGAACTTCCTG TACCTGGCCAGCCACTATGAGAAGCTGAAGGGCTCCCCCGAGGATAATG AGCAGAAACAGCTGTTTGTGGAACAGCACAAGCACTACCTGGACGAGAT CATCGAGCAGATCAGCGAGTTCTCCAAGAGAGTGATCCTGGCCGACGCT AATCTGGACAAAGTGCTGTCCGCCTACAACAAGCACCGGGATAAGCCCA TCAGAGAGCAGGCCGAGAATATCATCCACCTGTTTACCCTGACCAATCT GGGAGCCCCTGCCGCCTTCAAGTACTTTGACACCACCATCGACCGGAAG |
| | AGGTACACCAGCACCAAAGAGGTGCTGGACGCCACCCTGATCCACCAGA GCATCACCGGCCTGTACGAGACACGGATCGACCTGTCTCAGCTGGGAGG CGACAAGCGTCCTGCTGCTACTAAGAAAGCTGGTCAAGCTAAGAAAAAG AAATGAATCGATTAGCAATAAAGGATCGTTTATTTTCATTGGAAGCGTG TGTTGGTTTTTTGATCAGGCGCGGGTACCAGGTCGCGGCCGCTCTAGAGC ATGGCTACGTAGATAAGTAGCATGGCGGGTTAATCATTAACTACAAGGA ACCCCTAGTGATGGAGTTAGGAACCCCTAGTGATGGAGTTGGCCACTCC CTCTCTGCGCGCTCGCTCGCTCACTGAGGCCGGGCGACCAAAGGTCGCC CGACGCCCGGGCTTTGCCCGGGCGGCCTCAGTGAGCGAGCGAGCGCGCA GCTGCCTGCAGG |
| 901 | AAGATGACGGTTTGTCACATGGAGTTGGCAGGATGTTTGATTAAAAACA TAACAGGAAGAAAAATGCCCCGCTGTGGGCGGACAAAATAGTTGGGAA CTGGGAGGGGTGGAAATGGAGTTTTTAAGGATTATTTAGGGAAGAGTGA CAAAATAGATGGGAACTGGGTGTAGCGTCGTAAGCTAATACGAAAATTA AAAATGACAAAATAGTTTGGAACTAGATTTCACTTATCTGGTTCGGATCT CCTAGGCGATATCAGTGATCAcggatctcgaccaattgacattattgaagcaACTAGTATCG ATtttatcagggttattgtctcagaCCTGCAGGCAGCTGCGCGCTCGCTCGCTCACTGA GGCCGCCCGGGCAAAGCCCGGGCGTCGGGCGACCTTTGGTCGCCCGGCC TCAGTGAGCGAGCGAGCGCGCAGAGAGGGAGTGGCCAACTCCATCACT AGGGGTTCCTGTGGCCAACTCCATCACTAGGGGTTCCTTGTAGTTAATGA TTAACCCGCCATGCTACTTATCTACGTAGCCATGCTCTAGATTAATTAAT GCCCATGTAAGGAGGCAAGGCCTGGGGACACCCGAGATGCCTGGTTATA ATTAACCCAGACATGTGGCTGCCCCCCCCCCCCCAACACCTGCTGCCTCT AAAAATAACCCTGCATGCCATGTTCCCGGCGAAGGGCCAGCTGTCCCCC GCCAGCTAGACTCAGCACTTAGTTTAGGAACCAGTGAGCAAGTCAGCCC TTGGGGCAGCCCATACAAGGCCATGGGGCTGGGCAAGCTGCACGCCTGG GTCCGGGGTGGGCACGGTGCCCGGGCAACGAGCTGAAAGCTCATCTGCT CTCAGGGGCCCCTCCCTGGGGACAGCCCCTCCTGGCTAGTCACACCCTGT AGGCTCCTCTATATAACCCAGGGGCACAGGGGCTGCCCTCATTCTACCA CCACCTCCACAGCACAGACAGACACTCAGGAGCCAGCCAGCGCTAGCGC CACCATGATGGCCCCAAAGAAGAAGCGGAAGGTCGGTATCCACGGAGT CCCAGCAGCCGACAAGAAGTACAGCATCGGCCTGGACATCGGCACCAA CTCTGTGGGCTGGGCCGTGATCACCGACGAGTACAAGGTGCCCAGCAAG AAATTCAAGGTGCTGGGCAACACCGACCGGCACAGCATCAAGAAGAAC CTGATCGGAGCCCTGCTGTTCGACAGCGGCGAAACAGCCGAGGCCACCC GGCTGAAGAGAACCGCCAGAAGAAGATACACCAGACGGAAGAACCGGA TCTGCTATCTGCAAGAGATCTTCAGCAACGAGATGGCCAAGGTGGACGA CAGCTTCTTCCACAGACTGGAAGAGTCCTTCCTGGTGGAAGAGGATAAG AAGCACGAGCGGCACCCCATCTTCGGCAACATCGTGGACGAGGTGGCCT ACCACGAGAAGTACCCCACCATCTACCACCTGAGAAAGAAACTGGTGGA CAGCACCGACAAGGCCGACCTGCGGCTGATCTATCTGGCCCTGGCCCAC ATGATCAAGTTCCGGGGCCACTTCCTGATCGAGGGCGACCTGAACCCCG ACAACAGCGACGTGGACAAGCTGTTCATCCAGCTGGTGCAGACCTACAA CCAGCTGTTCGAGGAAAACCCCATCAACGCCAGCGGCGTGGACGCCAAG GCCATCCTGTCTGCCAGACTGAGCAAGAGCAGACGGCTGGAAAATCTGA TCGCCCAGCTGCCCGGCGAGAAGAAGAATGGCCTGTTCGGCAACCTGAT TGCCCTGAGCCTGGGCCTGACCCCCAACTTCAAGAGCAACTTCGACCTG |
| | GCCGAGGATGCCAAACTGCAGCTGAGCAAGGACACCTACGACGACGAC CTGGACAACCTGCTGGCCCAGATCGGCGACCAGTACGCCGACCTGTTTC TGGCCGCCAAGAACCTGTCCGACGCCATCCTGCTGAGCGACATCCTGAG AGTGAACACCGAGATCACCAAGGCCCCCCTGAGCGCCTCTATGATCAAG AGATACGACGAGCACCACCAGGACCTGACCCTGCTGAAAGCTCTCGTGC GGCAGCAGCTGCCTGAGAAGTACAAAGAGATTTTCTTCGACCAGAGCAA GAACGGCTACGCCGGCTACATTGACGGCGGAGCCAGCCAGGAAGAGTT CTACAAGTTCATCAAGCCCATCCTGGAAAAGATGGACGGCACCGAGGAA CTGCTCGTGAAGCTGAACAGAGAGGACCTGCTGCGGAAGCAGCGGACCT TCGACAACGGCAGCATCCCCCACCAGATCCACCTGGGAGAGCTGCACGC CATTCTGCGGCGGCAGGAAGATTTTTACCCATTCCTGAAGGACAACCGG GAAAAGATCGAGAAGATCCTGACCTTCCGCATCCCCTACTACGTGGGCC CTCTGGCCAGGGGAAACAGCAGATTCGCCTGGATGACCAGAAAGAGCG AGGAAACCATCACCCCCTGGAACTTCGAGGAAGTGGTGGACAAGGGCG CTTCCGCCCAGAGCTTCATCGAGCGGATGACCAACTTCGATAAGAACCT GCCCAACGAGAAGGTGCTGCCCAAGCACAGCCTGCTGTACGAGTACTTC ACCGTGTATAACGAGCTGACCAAAGTGAAATACGTGACCGAGGGAATG AGAAAGCCCGCCTTCCTGAGCGGCGAGCAGAAAAAGGCCATCGTGGAC CTGCTGTTCAAGACCAACCGGAAAGTGACCGTGAAGCAGCTGAAAGAG GACTACTTCAAGAAAATCGAGTGCTTCGACTCCGTGGAAATCTCCGGCG TGGAAGATCGGTTCAACGCCTCCCTGGGCACATACCACGATCTGCTGAA AATTATCAAGGACAAGGACTTCCTGGACAATGAGGAAAACGAGGACAT TCTGGAAGATATCGTGCTGACCCTGACACTGTTTGAGGACAGAGAGATG ATCGAGGAACGGCTGAAAACCTATGCCCACCTGTTCGACGACAAAGTGA TGAAGCAGCTGAAGCGGCGGAGATACACCGGCTGGGGCAGGCTGAGCC GGAAGCTGATCAACGGCATCCGGGACAAGCAGTCCGGCAAGACAATCC TGGATTTCCTGAAGTCCGACGGCTTCGCCAACAGAAACTTCATGCAGCT GATCCACGACGACAGCCTGACCTTTAAAGAGGACATCCAGAAAGCCCAG GTGTCCGGCCAGGGCGATAGCCTGCACGAGCACATTGCCAATCTGGCCG GCAGCCCCGCCATTAAGAAGGGCATCCTGCAGACAGTGAAGGTGGTGG ACGAGCTCGTGAAAGTGATGGGCCGGCACAAGCCCGAGAACATCGTGA TCGAAATGGCCAGAGAGAACCAGACCACCCAGAAGGGACAGAAGAACA GCCGCGAGAGAATGAAGCGGATCGAAGAGGGCATCAAAGAGCTGGGCA GCCAGATCCTGAAAGAACACCCCGTGGAAAACACCCAGCTGCAGAACG AGAAGCTGTACCTGTACTACCTGCAGAATGGGCGGGATATGTACGTGGA CCAGGAACTGGACATCAACCGGCTGTCCGACTACGATGTGGACCATATC GTGCCTCAGAGCTTTCTGAAGGACGACTCCATCGACAACAAGGTGCTGA CCAGAAGCGACAAGAACCGGGGCAAGAGCGACAACGTGCCCTCCGAAG AGGTCGTGAAGAAGATGAAGAACTACTGGCGGCAGCTGCTGAACGCCA AGCTGATTACCCAGAGAAAGTTCGACAATCTGACCAAGGCCGAGAGAG GCGGCCTGAGCGAACTGGATAAGGCCGGCTTCATCAAGAGACAGCTGGT GGAAACCCGGCAGATCACAAAGCACGTGGCACAGATCCTGGACTCCCG GATGAACACTAAGTACGACGAGAATGACAAGCTGATCCGGGAAGTGAA AGTGATCACCCTGAAGTCCAAGCTGGTGTCCGATTTCCGGAAGGATTTC CAGTTTTACAAAGTGCGCGAGATCAACAACTACCACCACGCCCACGACG CCTACCTGAACGCCGTCGTGGGAACCGCCCTGATCAAAAAGTACCCTAA GCTGGAAAGCGAGTTCGTGTACGGCGACTACAAGGTGTACGACGTGCGG AAGATGATCGCCAAGAGCGAGCAGGAAATCGGCAAGGCTACCGCCAAG TACTTCTTCTACAGCAACATCATGAACTTTTTCAAGACCGAGATTACCCT GGCCAACGGCGAGATCCGGAAGCGGCCTCTGATCGAGACAAACGGCGA |
| | AACCGGGGAGATCGTGTGGGATAAGGGCCGGGATTTTGCCACCGTGCGG AAAGTGCTGAGCATGCCCCAAGTGAATATCGTGAAAAAGACCGAGGTG CAGACAGGCGGCTTCAGCAAAGAGTCTATCCTGCCCAAGAGGAACAGC GATAAGCTGATCGCCAGAAAGAAGGACTGGGACCCTAAGAAGTACGGC GGCTTCGACAGCCCCACCGTGGCCTATTCTGTGCTGGTGGTGGCCAAAG TGGAAAAGGGCAAGTCCAAGAAACTGAAGAGTGTGAAAGAGCTGCTGG GGATCACCATCATGGAAAGAAGCAGCTTCGAGAAGAATCCCATCGACTT TCTGGAAGCCAAGGGCTACAAAGAAGTGAAAAAGGACCTGATCATCAA GCTGCCTAAGTACTCCCTGTTCGAGCTGGAAAACGGCCGGAAGAGAATG CTGGCCTCTGCCGGCGAACTGCAGAAGGGAAACGAACTGGCCCTGCCCT CCAAATATGTGAACTTCCTGTACCTGGCCAGCCACTATGAGAAGCTGAA GGGCTCCCCCGAGGATAATGAGCAGAAACAGCTGTTTGTGGAACAGCAC AAGCACTACCTGGACGAGATCATCGAGCAGATCAGCGAGTTCTCCAAGA GAGTGATCCTGGCCGACGCTAATCTGGACAAAGTGCTGTCCGCCTACAA CAAGCACCGGGATAAGCCCATCAGAGAGCAGGCCGAGAATATCATCCA CCTGTTTACCCTGACCAATCTGGGAGCCCCTGCCGCCTTCAAGTACTTTG ACACCACCATCGACCGGAAGAGGTACACCAGCACCAAAGAGGTGCTGG ACGCCACCCTGATCCACCAGAGCATCACCGGCCTGTACGAGACACGGAT CGACCTGTCTCAGCTGGGAGGCGACAAGCGTCCTGCTGCTACTAAGAAA GCTGGTCAAGCTAAGAAAAAGAAATGAATCGATTAGCAATAAAGGATC GTTTATTTTCATTGGAAGCGTGTGTTGGTTTTTTGATCAGGCGCGGGTAC CAGGTCGCGGCCGCTCTAGAGCATGGCTACGTAGATAAGTAGCATGGCG GGTTAATCATTAACTACAAGGAACCCCTAGTGATGGAGTTaggaacccctagtga tggagttggccactccctctctgcgcgctcgctcgctcactgaggccgggcgaccaaaggtcgcccgacgcccgggct ttgcccgggcggcctcagtgagcgagcgagcgcgcagctgcctgcaggCATGCAAGCTGTAGCCAA CCACTAGAACTATAGCTAGAGTCCTGGGCGAACAAACGATGCTCGCCTT CCAGAAAACCGAGGATGCGAACCACTTCATCCGGGGTCAGCACCACCGG CAAGCGCCGCGACGGCCGAGGTCTTCCGATCTCCTGAAGCCAGGGCAGA TCCGTGCACAGCACCTTGCCGTAGAAGAACAGCAAGGCCGCCAATGCCT GACGATGCGTGGAGACCGAAACCTTGCGCTCGTTCGCCAGCCAGGACAG AAATGCCTCGACTTCGCTGCTGCCCAAGGTTGCCGGGTGACGCACACCG TGGAAACGGATGAAGGCACGAACCCAGTTGACATAAGCCTGTTCGGTTC GTAAACTGTAATGCAAGTAGCGTATGCGCTCACGCAACTGGTCCAGAAC CTTGACCGAACGCAGCGGTGGTAACGGCGCAGTGGCGGTTTTCATGGCT TGTTATGACTGTTTTTTTGTACAGTCTATGCCTCGGGCATCCAAGCAGCA AGCGCGTTACGCCGTGGGTCGATGTTTGATGTTATGGAGCAGCAACGAT GTTACGCAGCAGCAACGATGTTACGCAGCAGGGCAGTCGCCCTAAAACA AAGTTAGGTGGCTCAAGTATGGGCATCATTCGCACATGTAGGCTCGGCC CTGACCAAGTCAAATCCATGCGGGCTGCTCTTGATCTTTTCGGTCGTGAG TTCGGAGACGTAGCCACCTACTCCCAACATCAGCCGGACTCCGATTACC TCGGGAACTTGCTCCGTAGTAAGACATTCATCGCGCTTGCTGCCTTCGAC CAAGAAGCGGTTGTTGGCGCTCTCGCGGCTTACGTTCTGCCCAAGTTTGA GCAGCCGCGTAGTGAGATCTATATCTATGATCTCGCAGTCTCCGGCGAG CACCGGAGGCAGGGCATTGCCACCGCGCTCATCAATCTCCTCAAGCATG AGGCCAACGCGCTTGGTGCTTATGTGATCTACGTGCAAGCAGATTACGG TGACGATCCCGCAGTGGCTCTCTATACAAAGTTGGGCATACGGGAAGAA GTGATGCACTTTGATATCGACCCAAGTACCGCCACCTAACAATTCGTTCA AGCCGAGATCGGCTTCCCGGCCGCGGAGTTGTTCGGTAAATTGTCACAA CGCCGCGAATATAGTCTTTACCATGCCCTTGGCCACGCCCCTCTTTAATA CGACGGGCAATTTGCACTTCAGAAAATGAAGAGTTTGCTTTAGCCATAA |
| | CAAAAGTCCAGTATGCTTTTTCACAGCATAACTGGACTGATTTCAGTTTA CAACTATTCTGTCTAGTTTAAGACTTTATTGTCATAGTTTAGATCTATTTT GTTCAGTTTAAGACTTTATTGTCCGCCCACACCCGCTTACGC |
| 902 | AAGATGACGGTTTGTCACATGGAGTTGGCAGGATGTTTGATTAAAAACA TAACAGGAAGAAAAATGCCCCGCTGTGGGCGGACAAAATAGTTGGGAA CTGGGAGGGGTGGAAATGGAGTTTTTAAGGATTATTTAGGGAAGAGTGA CAAAATAGATGGGAACTGGGTGTAGCGTCGTAAGCTAATACGAAAATTA AAAATGACAAAATAGTTTGGAACTAGATTTCACTTATCTGGTTCGGATCT CCTAGGCGATATCAGTGATCAcggatctcgaccaattgacattattgaagcaACTAGTATCG ATtttatcagggttattgtctcagaGGCCACTCCCTCTCTGCGCGCTCGCTCGCTCACTG AGGCCGGGCGACCAAAGGTCGCCCGACGCCCGGGCTTTGCCCGGGCGGC CTCAGTGAGCGAGCGAGCGCGCAGAGAGGGAGTGGCCAACTCCATCAC TAGGGGTTCCTTGTAGTTAATGATTAACCCGCCATGCTACTTATCTACGT AGCCATGCTCTAGATTAATTAATGCCCATGTAAGGAGGCAAGGCCTGGG GACACCCGAGATGCCTGGTTATAATTAACCCAGACATGTGGCTGCCCCC CCCCCCCCAACACCTGCTGCCTCTAAAAATAACCCTGCATGCCATGTTCC CGGCGAAGGGCCAGCTGTCCCCCGCCAGCTAGACTCAGCACTTAGTTTA GGAACCAGTGAGCAAGTCAGCCCTTGGGGCAGCCCATACAAGGCCATG GGGCTGGGCAAGCTGCACGCCTGGGTCCGGGGTGGGCACGGTGCCCGG GCAACGAGCTGAAAGCTCATCTGCTCTCAGGGGCCCCTCCCTGGGGACA GCCCCTCCTGGCTAGTCACACCCTGTAGGCTCCTCTATATAACCCAGGGG CACAGGGGCTGCCCTCATTCTACCACCACCTCCACAGCACAGACAGACA CTCAGGAGCCAGCCAGCGCTAGCGCCACCATGATGGCCCCAAAGAAGA AGCGGAAGGTCGGTATCCACGGAGTCCCAGCAGCCGACAAGAAGTACA GCATCGGCCTGGACATCGGCACCAACTCTGTGGGCTGGGCCGTGATCAC CGACGAGTACAAGGTGCCCAGCAAGAAATTCAAGGTGCTGGGCAACAC CGACCGGCACAGCATCAAGAAGAACCTGATCGGAGCCCTGCTGTTCGAC AGCGGCGAAACAGCCGAGGCCACCCGGCTGAAGAGAACCGCCAGAAGA AGATACACCAGACGGAAGAACCGGATCTGCTATCTGCAAGAGATCTTCA GCAACGAGATGGCCAAGGTGGACGACAGCTTCTTCCACAGACTGGAAG AGTCCTTCCTGGTGGAAGAGGATAAGAAGCACGAGCGGCACCCCATCTT CGGCAACATCGTGGACGAGGTGGCCTACCACGAGAAGTACCCCACCATC TACCACCTGAGAAAGAAACTGGTGGACAGCACCGACAAGGCCGACCTG CGGCTGATCTATCTGGCCCTGGCCCACATGATCAAGTTCCGGGGCCACTT CCTGATCGAGGGCGACCTGAACCCCGACAACAGCGACGTGGACAAGCT GTTCATCCAGCTGGTGCAGACCTACAACCAGCTGTTCGAGGAAAACCCC ATCAACGCCAGCGGCGTGGACGCCAAGGCCATCCTGTCTGCCAGACTGA GCAAGAGCAGACGGCTGGAAAATCTGATCGCCCAGCTGCCCGGCGAGA AGAAGAATGGCCTGTTCGGCAACCTGATTGCCCTGAGCCTGGGCCTGAC CCCCAACTTCAAGAGCAACTTCGACCTGGCCGAGGATGCCAAACTGCAG CTGAGCAAGGACACCTACGACGACGACCTGGACAACCTGCTGGCCCAGA TCGGCGACCAGTACGCCGACCTGTTTCTGGCCGCCAAGAACCTGTCCGA CGCCATCCTGCTGAGCGACATCCTGAGAGTGAACACCGAGATCACCAAG GCCCCCCTGAGCGCCTCTATGATCAAGAGATACGACGAGCACCACCAGG ACCTGACCCTGCTGAAAGCTCTCGTGCGGCAGCAGCTGCCTGAGAAGTA CAAAGAGATTTTCTTCGACCAGAGCAAGAACGGCTACGCCGGCTACATT GACGGCGGAGCCAGCCAGGAAGAGTTCTACAAGTTCATCAAGCCCATCC |
| | TGGAAAAGATGGACGGCACCGAGGAACTGCTCGTGAAGCTGAACAGAG AGGACCTGCTGCGGAAGCAGCGGACCTTCGACAACGGCAGCATCCCCCA CCAGATCCACCTGGGAGAGCTGCACGCCATTCTGCGGCGGCAGGAAGAT TTTTACCCATTCCTGAAGGACAACCGGGAAAAGATCGAGAAGATCCTGA CCTTCCGCATCCCCTACTACGTGGGCCCTCTGGCCAGGGGAAACAGCAG ATTCGCCTGGATGACCAGAAAGAGCGAGGAAACCATCACCCCCTGGAAC TTCGAGGAAGTGGTGGACAAGGGCGCTTCCGCCCAGAGCTTCATCGAGC GGATGACCAACTTCGATAAGAACCTGCCCAACGAGAAGGTGCTGCCCAA GCACAGCCTGCTGTACGAGTACTTCACCGTGTATAACGAGCTGACCAAA GTGAAATACGTGACCGAGGGAATGAGAAAGCCCGCCTTCCTGAGCGGC GAGCAGAAAAAGGCCATCGTGGACCTGCTGTTCAAGACCAACCGGAAA GTGACCGTGAAGCAGCTGAAAGAGGACTACTTCAAGAAAATCGAGTGCT TCGACTCCGTGGAAATCTCCGGCGTGGAAGATCGGTTCAACGCCTCCCT GGGCACATACCACGATCTGCTGAAAATTATCAAGGACAAGGACTTCCTG GACAATGAGGAAAACGAGGACATTCTGGAAGATATCGTGCTGACCCTGA CACTGTTTGAGGACAGAGAGATGATCGAGGAACGGCTGAAAACCTATGC CCACCTGTTCGACGACAAAGTGATGAAGCAGCTGAAGCGGCGGAGATA CACCGGCTGGGGCAGGCTGAGCCGGAAGCTGATCAACGGCATCCGGGA CAAGCAGTCCGGCAAGACAATCCTGGATTTCCTGAAGTCCGACGGCTTC GCCAACAGAAACTTCATGCAGCTGATCCACGACGACAGCCTGACCTTTA AAGAGGACATCCAGAAAGCCCAGGTGTCCGGCCAGGGCGATAGCCTGC ACGAGCACATTGCCAATCTGGCCGGCAGCCCCGCCATTAAGAAGGGCAT CCTGCAGACAGTGAAGGTGGTGGACGAGCTCGTGAAAGTGATGGGCCG GCACAAGCCCGAGAACATCGTGATCGAAATGGCCAGAGAGAACCAGAC CACCCAGAAGGGACAGAAGAACAGCCGCGAGAGAATGAAGCGGATCGA AGAGGGCATCAAAGAGCTGGGCAGCCAGATCCTGAAAGAACACCCCGT GGAAAACACCCAGCTGCAGAACGAGAAGCTGTACCTGTACTACCTGCAG AATGGGCGGGATATGTACGTGGACCAGGAACTGGACATCAACCGGCTGT CCGACTACGATGTGGACCATATCGTGCCTCAGAGCTTTCTGAAGGACGA CTCCATCGACAACAAGGTGCTGACCAGAAGCGACAAGAACCGGGGCAA GAGCGACAACGTGCCCTCCGAAGAGGTCGTGAAGAAGATGAAGAACTA CTGGCGGCAGCTGCTGAACGCCAAGCTGATTACCCAGAGAAAGTTCGAC AATCTGACCAAGGCCGAGAGAGGCGGCCTGAGCGAACTGGATAAGGCC GGCTTCATCAAGAGACAGCTGGTGGAAACCCGGCAGATCACAAAGCAC GTGGCACAGATCCTGGACTCCCGGATGAACACTAAGTACGACGAGAATG ACAAGCTGATCCGGGAAGTGAAAGTGATCACCCTGAAGTCCAAGCTGGT GTCCGATTTCCGGAAGGATTTCCAGTTTTACAAAGTGCGCGAGATCAAC AACTACCACCACGCCCACGACGCCTACCTGAACGCCGTCGTGGGAACCG CCCTGATCAAAAAGTACCCTAAGCTGGAAAGCGAGTTCGTGTACGGCGA CTACAAGGTGTACGACGTGCGGAAGATGATCGCCAAGAGCGAGCAGGA AATCGGCAAGGCTACCGCCAAGTACTTCTTCTACAGCAACATCATGAAC TTTTTCAAGACCGAGATTACCCTGGCCAACGGCGAGATCCGGAAGCGGC CTCTGATCGAGACAAACGGCGAAACCGGGGAGATCGTGTGGGATAAGG GCCGGGATTTTGCCACCGTGCGGAAAGTGCTGAGCATGCCCCAAGTGAA TATCGTGAAAAAGACCGAGGTGCAGACAGGCGGCTTCAGCAAAGAGTC TATCCTGCCCAAGAGGAACAGCGATAAGCTGATCGCCAGAAAGAAGGA CTGGGACCCTAAGAAGTACGGCGGCTTCGACAGCCCCACCGTGGCCTAT TCTGTGCTGGTGGTGGCCAAAGTGGAAAAGGGCAAGTCCAAGAAACTG AAGAGTGTGAAAGAGCTGCTGGGGATCACCATCATGGAAAGAAGCAGC TTCGAGAAGAATCCCATCGACTTTCTGGAAGCCAAGGGCTACAAAGAAG |
| | TGAAAAAGGACCTGATCATCAAGCTGCCTAAGTACTCCCTGTTCGAGCT GGAAAACGGCCGGAAGAGAATGCTGGCCTCTGCCGGCGAACTGCAGAA GGGAAACGAACTGGCCCTGCCCTCCAAATATGTGAACTTCCTGTACCTG GCCAGCCACTATGAGAAGCTGAAGGGCTCCCCCGAGGATAATGAGCAG AAACAGCTGTTTGTGGAACAGCACAAGCACTACCTGGACGAGATCATCG AGCAGATCAGCGAGTTCTCCAAGAGAGTGATCCTGGCCGACGCTAATCT GGACAAAGTGCTGTCCGCCTACAACAAGCACCGGGATAAGCCCATCAGA GAGCAGGCCGAGAATATCATCCACCTGTTTACCCTGACCAATCTGGGAG CCCCTGCCGCCTTCAAGTACTTTGACACCACCATCGACCGGAAGAGGTA CACCAGCACCAAAGAGGTGCTGGACGCCACCCTGATCCACCAGAGCATC ACCGGCCTGTACGAGACACGGATCGACCTGTCTCAGCTGGGAGGCGACA AGCGTCCTGCTGCTACTAAGAAAGCTGGTCAAGCTAAGAAAAAGAAATG AATCGATTAGCAATAAAGGATCGTTTATTTTCATTGGAAGCGTGTGTTGG TTTTTTGATCAGGCGCGGGTACCAGGTCGCGGCCGCTCTAGAGCATGGC TACGTAGATAAGTAGCATGGCGGGTTAATCATTAACTACAAGGAACCCC TAGTGATGGAGTTGGCCACTCCCTCTCTGCGCGCTCGCTCGCTCACTGAG GCCGGGCGACCAAAGGTCGCCCGACGCCCGGGCTTTGCCCGGGCGGCCT CAGTGAGCGAGCGAGCGCGCAGAGAGGGACATGCAAGCTGTAGCCAAC CACTAGAACTATAGCTAGAGTCCTGGGCGAACAAACGATGCTCGCCTTC CAGAAAACCGAGGATGCGAACCACTTCATCCGGGGTCAGCACCACCGGC AAGCGCCGCGACGGCCGAGGTCTTCCGATCTCCTGAAGCCAGGGCAGAT CCGTGCACAGCACCTTGCCGTAGAAGAACAGCAAGGCCGCCAATGCCTG ACGATGCGTGGAGACCGAAACCTTGCGCTCGTTCGCCAGCCAGGACAGA AATGCCTCGACTTCGCTGCTGCCCAAGGTTGCCGGGTGACGCACACCGT GGAAACGGATGAAGGCACGAACCCAGTTGACATAAGCCTGTTCGGTTCG TAAACTGTAATGCAAGTAGCGTATGCGCTCACGCAACTGGTCCAGAACC TTGACCGAACGCAGCGGTGGTAACGGCGCAGTGGCGGTTTTCATGGCTT GTTATGACTGTTTTTTTGTACAGTCTATGCCTCGGGCATCCAAGCAGCAA GCGCGTTACGCCGTGGGTCGATGTTTGATGTTATGGAGCAGCAACGATG TTACGCAGCAGCAACGATGTTACGCAGCAGGGCAGTCGCCCTAAAACAA AGTTAGGTGGCTCAAGTATGGGCATCATTCGCACATGTAGGCTCGGCCC TGACCAAGTCAAATCCATGCGGGCTGCTCTTGATCTTTTCGGTCGTGAGT TCGGAGACGTAGCCACCTACTCCCAACATCAGCCGGACTCCGATTACCT CGGGAACTTGCTCCGTAGTAAGACATTCATCGCGCTTGCTGCCTTCGACC AAGAAGCGGTTGTTGGCGCTCTCGCGGCTTACGTTCTGCCCAAGTTTGAG CAGCCGCGTAGTGAGATCTATATCTATGATCTCGCAGTCTCCGGCGAGC ACCGGAGGCAGGGCATTGCCACCGCGCTCATCAATCTCCTCAAGCATGA GGCCAACGCGCTTGGTGCTTATGTGATCTACGTGCAAGCAGATTACGGT GACGATCCCGCAGTGGCTCTCTATACAAAGTTGGGCATACGGGAAGAAG TGATGCACTTTGATATCGACCCAAGTACCGCCACCTAACAATTCGTTCAA GCCGAGATCGGCTTCCCGGCCGCGGAGTTGTTCGGTAAATTGTCACAAC GCCGCGAATATAGTCTTTACCATGCCCTTGGCCACGCCCCTCTTTAATAC GACGGGCAATTTGCACTTCAGAAAATGAAGAGTTTGCTTTAGCCATAAC AAAAGTCCAGTATGCTTTTTCACAGCATAACTGGACTGATTTCAGTTTAC AACTATTCTGTCTAGTTTAAGACTTTATTGTCATAGTTTAGATCTATTTTG TTCAGTTTAAGACTTTATTGTCCGCCCACACCCGCTTACGC |

In some embodiments of the gene editing constructs of the disclosure, including those embodiments encompassing SEQ ID NOs: 899-902, the construct comprises or consists of a promoter and a nuclease. In some embodiments, the construct comprises or consists of an CK8e promoter and a Cas9 nuclease. In some embodiments, the construct comprises or consists of an CK8e promoter and a Cas9 nuclease isolated or derived from *Staphylococcus pyogenes* ("SpCas9"). In some embodiments, the CK8e promoter comprises or consists of a nucleotide sequence of TGCCCATGTAAGGAGGCAAGGCCTGGGGACACCCGAGATGCCTGGTTATAATTA ACCCAGACATGTGGCTGCCCCCCCCCCCCCAACACCTGCTGCCTCTAAAAATAAC CCTGCATGCCATGTTCCCGGCGAAGGGCCAGCTGTCCCCCGCCAGCTAGACTCAG CACTTAGTTTAGGAACCAGTGAGCAAGTCAGCCCTTGGGGCAGCCCATACAAGG CCATGGGGCTGGGCAAGCTGCACGCCTGGGTCCGGGGTGGGCACGGTGCCCGGG CAACGAGCTGAAAGCTCATCTGCTCTCAGGGGCCCCTCCCTGGGGACAGCCCCTC CTGGCTAGTCACACCCTGTAGGCTCCTCTATATAACCCAGGGGCACAGGGGCTGC CCTCATTCTACCACCACCTCCACAGCACAGACAGACACTCAGGAGCCAGCCAGC (SEQ ID NO: 875). In some embodiments, the SpCas9 nuclease comprises or consists of a nucleotide sequence of GACAAGAAGTACAGCATCGGCCTGGACATCGGCACCAACTCTGTGGGCTGGGCC GTGATCACCGACGAGTACAAGGTGCCCAGCAAGAAATTCAAGGTGCTGGGCAAC ACCGACCGGCACAGCATCAAGAAGAACCTGATCGGAGCCCTGCTGTTCGACAGC GGCGAAACAGCCGAGGCCACCCGGCTGAAGAGAACCGCCAGAAGAAGATACAC CAGACGGAAGAACCGGATCTGCTATCTGCAAGAGATCTTCAGCAACGAGATGGC CAAGGTGGACGACAGCTTCTTCCACAGACTGGAAGAGTCCTTCCTGGTGGAAGA GGATAAGAAGCACGAGCGGCACCCCATCTTCGGCAACATCGTGGACGAGGTGGC CTACCACGAGAAGTACCCCACCATCTACCACCTGAGAAAGAAACTGGTGGACAG CACCGACAAGGCCGACCTGCGGCTGATCTATCTGGCCCTGGCCCACATGATCAAG TTCCGGGGCCACTTCCTGATCGAGGGCGACCTGAACCCCGACAACAGCGACGTG GACAAGCTGTTCATCCAGCTGGTGCAGACCTACAACCAGCTGTTCGAGGAAAAC CCCATCAACGCCAGCGGCGTGGACGCCAAGGCCATCCTGTCTGCCAGACTGAGC AAGAGCAGACGGCTGGAAAATCTGATCGCCCAGCTGCCCGGCGAGAAGAAGAAT GGCCTGTTCGGCAACCTGATTGCCCTGAGCCTGGGCCTGACCCCCAACTTCAAGA GCAACTTCGACCTGGCCGAGGATGCCAAACTGCAGCTGAGCAAGGACACCTACG ACGACGACCTGGACAACCTGCTGGCCCAGATCGGCGACCAGTACGCCGACCTGT TTCTGGCCGCCAAGAACCTGTCCGACGCCATCCTGCTGAGCGACATCCTGAGAGT GAACACCGAGATCACCAAGGCCCCCCTGAGCGCCTCTATGATCAAGAGATACGA CGAGCACCACCAGGACCTGACCCTGCTGAAAGCTCTCGTGCGGCAGCAGCTGCC TGAGAAGTACAAAGAGATTTTCTTCGACCAGAGCAAGAACGGCTACGCCGGCTA CATTGACGGCGGAGCCAGCCAGGAAGAGTTCTACAAGTTCATCAAGCCCATCCT GGAAAAGATGGACGGCACCGAGGAACTGCTCGTGAAGCTGAACAGAGAGGACC TGCTGCGGAAGCAGCGGACCTTCGACAACGGCAGCATCCCCCACCAGATCCACC TGGGAGAGCTGCACGCCATTCTGCGGCGGCAGGAAGATTTTTACCCATTCCTGAA GGACAACCGGGAAAAGATCGAGAAGATCCTGACCTTCCGCATCCCCTACTACGT GGGCCCTCTGGCCAGGGGAAACAGCAGATTCGCCTGGATGACCAGAAAGAGCGA GGAAACCATCACCCCCTGGAACTTCGAGGAAGTGGTGGACAAGGGCGCTTCCGC CCAGAGCTTCATCGAGCGGATGACCAACTTCGATAAGAACCTGCCCAACGAGAA GGTGCTGCCCAAGCACAGCCTGCTGTACGAGTACTTCACCGTGTATAACGAGCTG ACCAAAGTGAAATACGTGACCGAGGGAATGAGAAAGCCCGCCTTCCTGAGCGGC GAGCAGAAAAAGGCCATCGTGGACCTGCTGTTCAAGACCAACCGGAAAGTGACC GTGAAGCAGCTGAAAGAGGACTACTTCAAGAAAATCGAGTGCTTCGACTCCGTG GAAATCTCCGGCGTGGAAGATCGGTTCAACGCCTCCCTGGGCACATACCACGATC TGCTGAAAATTATCAAGGACAAGGACTTCCTGGACAATGAGGAAAACGAGGACA TTCTGGAAGATATCGTGCTGACCCTGACACTGTTTGAGGACAGAGAGATGATCGA GGAACGGCTGAAAACCTATGCCCACCTGTTCGACGACAAAGTGATGAAGCAGCT GAAGCGGCGGAGATACACCGGCTGGGGCAGGCTGAGCCGGAAGCTGATCAACG GCATCCGGGACAAGCAGTCCGGCAAGACAATCCTGGATTTCCTGAAGTCCGACG GCTTCGCCAACAGAAACTTCATGCAGCTGATCCACGACGACAGCCTGACCTTTAA AGAGGACATCCAGAAAGCCCAGGTGTCCGGCCAGGGCGATAGCCTGCACGAGCA CATTGCCAATCTGGCCGGCAGCCCCGCCATTAAGAAGGGCATCCTGCAGACAGT GAAGGTGGTGGACGAGCTCGTGAAAGTGATGGGCCGGCACAAGCCCGAGAACAT CGTGATCGAAATGGCCAGAGAGAACCAGACCACCCAGAAGGGACAGAAGAACA GCCGCGAGAGAATGAAGCGGATCGAAGAGGGCATCAAAGAGCTGGGCAGCCAG ATCCTGAAAGAACACCCCGTGGAAAACACCCAGCTGCAGAACGAGAAGCTGTAC CTGTACTACCTGCAGAATGGGCGGGATATGTACGTGGACCAGGAACTGGACATC AACCGGCTGTCCGACTACGATGTGGACCATATCGTGCCTCAGAGCTTTCTGAAGG ACGACTCCATCGACAACAAGGTGCTGACCAGAAGCGACAAGAACCGGGGCAAG AGCGACAACGTGCCCTCCGAAGAGGTCGTGAAGAAGATGAAGAACTACTGGCGG CAGCTGCTGAACGCCAAGCTGATTACCCAGAGAAAGTTCGACAATCTGACCAAG GCCGAGAGAGGCGGCCTGAGCGAACTGGATAAGGCCGGCTTCATCAAGAGACAG CTGGTGGAAACCCGGCAGATCACAAAGCACGTGGCACAGATCCTGGACTCCCGG ATGAACACTAAGTACGACGAGAATGACAAGCTGATCCGGGAAGTGAAAGTGATC ACCCTGAAGTCCAAGCTGGTGTCCGATTTCCGGAAGGATTTCCAGTTTTACAAAG TGCGCGAGATCAACAACTACCACCACGCCCACGACGCCTACCTGAACGCCGTCG TGGGAACCGCCCTGATCAAAAAGTACCCTAAGCTGGAAAGCGAGTTCGTGTACG GCGACTACAAGGTGTACGACGTGCGGAAGATGATCGCCAAGAGCGAGCAGGAA ATCGGCAAGGCTACCGCCAAGTACTTCTTCTACAGCAACATCATGAACTTTTTCA AGACCGAGATTACCCTGGCCAACGGCGAGATCCGGAAGCGGCCTCTGATCGAGA CAAACGGCGAAACCGGGGAGATCGTGTGGGATAAGGGCCGGGATTTTGCCACCG TGCGGAAAGTGCTGAGCATGCCCCAAGTGAATATCGTGAAAAAGACCGAGGTGC AGACAGGCGGCTTCAGCAAAGAGTCTATCCTGCCCAAGAGGAACAGCGATAAGC TGATCGCCAGAAAGAAGGACTGGGACCCTAAGAAGTACGGCGGCTTCGACAGCC CCACCGTGGCCTATTCTGTGCTGGTGGTGGCCAAAGTGGAAAAGGGCAAGTCCA AGAAACTGAAGAGTGTGAAAGAGCTGCTGGGGATCACCATCATGGAAAGAAGC AGCTTCGAGAAGAATCCCATCGACTTTCTGGAAGCCAAGGGCTACAAAGAAGTG AAAAAGGACCTGATCATCAAGCTGCCTAAGTACTCCCTGTTCGAGCTGGAAAAC GGCCGGAAGAGAATGCTGGCCTCTGCCGGCGAACTGCAGAAGGGAAACGAACTG GCCCTGCCCTCCAAATATGTGAACTTCCTGTACCTGGCCAGCCACTATGAGAAGC TGAAGGGCTCCCCCGAGGATAATGAGCAGAAACAGCTGTTTGTGGAACAGCACA AGCACTACCTGGACGAGATCATCGAGCAGATCAGCGAGTTCTCCAAGAGAGTGA TCCTGGCCGACGCTAATCTGGACAAAGTGCTGTCCGCCTACAACAAGCACCGGG ATAAGCCCATCAGAGAGCAGGCCGAGAATATCATCCACCTGTTTACCCTGACCA ATCTGGGAGCCCCTGCCGCCTTCAAGTACTTTGACACCACCATCGACCGGAAGAG GTACACCAGCACCAAAGAGGTGCTGGACGCCACCCTGATCCACCAGAGCATCAC CGGCCTGTACGAGACACGGATCGACCTGTCTCAGCTGGGAGGCGAC (SEQ ID NO:872). In some embodiments, the construct comprising a promoter and a nuclease further comprises at least two inverted terminal repeat (ITR) sequences. In some embodiments, the construct comprising a promoter and a nuclease further comprises at least two ITR sequences from isolated or derived from an AAV of serotype 2 (AAV2). In some embodiments, the construct comprising a promoter and a nuclease further comprises at least two ITR sequences each comprising or consisting of a nucleotide sequence of GGCCACTCCCTCTCTGCGCGCTCGCTCGCTCACTGAGGCCGGGCGACCAAAGGTC GCCCGACGCCCGGGCTTTGCCCGGGCGGCCTCAGTGAGCGAGCGAGCGCGCAGA GAGGGA (SEQ ID NO:880). In some embodiments, the construct comprising a promoter and a nuclease further comprises at least two ITR sequences, wherein the first ITR sequence comprises or consists of a nucleotide sequence of CCTGCAGGCAGCTGCGCGCTCGCTCGCTCACTGAGGCCGCCCGGGCAAAGCCCG GGCGTCGGGCGACCTTTGGTCGCCCGGCCTCAGTGAGCGAGCGAGCGCGCAGAG AGGGAGTGGCCAACTCCATCACTAGGGGTTCCT (SEQ ID NO: 881) and the second ITR sequence comprises or consist of a nucleotide sequence of AGGAACCCCTAGTGATGGAGTTGGCCACTCCCTCTCTGCGCGCTCGCTCGCTCAC TGAGGCCGGGCGACCAAAGGTCGCCCGACGCCCGGGCTTTGCCCGGGCGGCCTC AGTGAGCGAGCGAGCGCGCAGCTGCCTGCAGG (SEQ ID NO: 882). In some embodiments, the construct comprises or consists of, from 5' to 3' a first ITR, a sequence encoding a promoter, a sequence encoding a nuclease and a second ITR. In some embodiments, the construct comprises or consists of, from 5' to 3' a first AAV2 ITR, a sequence encoding an CK8e promoter, a sequence encoding a SpCas9 nuclease and a second AAV2 ITR. In some embodiments, the construct comprising or consisting of, from 5' to 3' a first ITR, a sequence encoding a promoter, a sequence encoding a nuclease and a second ITR, further comprises a poly A sequence. In some embodiments, the polyA sequence comprises or consists of a minipolyA sequence. Exemplary minipolyA sequences of the disclosure comprise or consist of a nucleotide sequence of TAGCAATAAAGGATCGTTTATTTTCATTGGAAGCGTGTGTTGGTTTTTTGATCAGG CGCG (SEQ ID NO: 903). In some embodiments, the construct comprises or consists of, from 5' to 3' a first ITR, a sequence encoding a promoter, a sequence encoding a nuclease, a poly A sequence and a second ITR. In some embodiments, the construct comprises or consists of, from 5' to 3' a first ITR, a sequence encoding a promoter, a sequence encoding a nuclease, a minipoly A sequence and a second ITR. In some embodiments, the construct comprises or consists of, from 5' to 3' a first AAV2 ITR, a sequence encoding an CK8e promoter, a sequence encoding a SpCas9 nuclease, a minipoly A sequence and a second AAV2 ITR. In some embodiments, the construct comprising, from 5' to 3' a first ITR, a sequence encoding a promoter, a sequence encoding a nuclease, a poly A sequence and a second ITR, further comprises at least one nuclear localization signal. In some embodiments, the construct comprising, from 5' to 3' a first ITR, a sequence encoding a promoter, a sequence encoding a nuclease, a poly A sequence and a second ITR, further comprises at least two nuclear localization signals. Exemplary nuclear localization signals of the disclosure comprise or consist of a nucleotide sequence of AAGCGTCCTGCTGCTACTAAGAAAGCTGGTCAAGCTAAGAAAAAGAAA (SEQ ID NO. 887), or a nucleotide sequence of ATGGCCCCAAAGAAGAAGCGGAAGGTCGGTATCCACGGAGTCCCAGCAGCC (SEQ ID NO. 885). In some embodiments, the construct comprises or consists of, from 5' to 3' a first ITR, a sequence encoding a promoter, a sequence encoding a first nuclear localization signal, a sequence encoding a nuclease, a poly A sequence and a second ITR. In some embodiments, the construct comprises or consists of, from 5' to 3' a first ITR, a sequence encoding a promoter, a sequence encoding a first nuclear localization signal, a sequence encoding a nuclease, a sequence encoding a second nuclear localization signal, a poly A sequence and a second ITR. In some embodiments, the construct comprises or consists of, from 5' to 3' a first AAV2 ITR, a sequence encoding a CK8e promoter, a nuclear localization signal having a sequence of SEQ ID NO: 885, a sequence encoding a SpCas9 nuclease, a minipoly A sequence and a second AAV2 ITR. In some embodiments, the construct comprises or consists of, from 5' to 3' a first AAV2 ITR, a sequence encoding a CK8e promoter, a sequence encoding a SpCas9 nuclease, a nuclear localization signal having a sequence of SEQ ID NO: 887, a minipoly A sequence and a second AAV2 ITR. In some embodiments, the construct comprises or consists of, from 5' to 3' a first AAV2 ITR, a sequence encoding a CK8e promoter, a nuclear localization signal having a sequence of SEQ ID NO. 885, a sequence encoding a SpCas9 nuclease, a nuclear localization signal having a sequence of SEQ ID NO: 887, a minipoly A sequence and a second AAV2 ITR. In some embodiments, the construct comprising, from 5' to 3' a first ITR, a sequence encoding a promoter, a sequence encoding a first nuclear localization signal, a sequence encoding a nuclease, a sequence encoding a second nuclear localization signal, a poly A sequence and a second ITR, further comprises a stop codon. The stop codon may have a sequence of TAG (SEQ ID NO. 904), TAA (SEQ ID NO. 905), or TGA (SEQ ID NO. 906). In some embodiments, the construct comprises or consists of, from 5' to 3' a first ITR, a sequence encoding a promoter, a sequence encoding a first nuclear localization signal, a sequence encoding a nuclease, a sequence encoding a second nuclear localization signal, a stop codon, a poly A sequence and a second ITR. In some embodiments, the construct comprises or consists of, from 5' to 3' a first AAV2 ITR, a sequence encoding a CK8e promoter, a nuclear localization signal having a sequence of SEQ ID NO: 885, a sequence encoding a SpCas9 nuclease, a stop codon, a minipoly A sequence and a second AAV2 ITR. In some embodiments, the construct comprises or consists of, from 5' to 3' a first AAV2 ITR, a sequence encoding an CK8e promoter, a sequence encoding a SpCas9 nuclease, a nuclear localization signal having a sequence of SEQ ID NO: 887, a stop codon, a minipoly A sequence and a second AAV2 ITR. In some embodiments, the construct comprises or consists of, from 5' to 3' a first AAV2 ITR, a sequence encoding an CK8e promoter, a nuclear localization signal having a sequence of SEQ ID NO. 885, a sequence encoding a SpCas9 nuclease, a nuclear localization signal having a sequence of SEQ ID NO: 887, a stop codon a minipoly A sequence and a second AAV2 ITR. In some embodiments, the construct comprising or consisting of, from 5' to 3' a first ITR, a sequence encoding a promoter, a sequence encoding a first nuclear localization signal, a sequence encoding a nuclease, a sequence encoding a second nuclear localization signal, a stop codon, a poly A sequence and a second ITR, further comprises transposable element inverted repeats. Exemplary transposable element inverted repeats of the disclosure comprise or consist of a nucleotide sequence of TGTGGGCGGACAAAATAGTTGGGAACTGGGAGGGGTGGAAATGGAGTTTTTAAG GATTATTTAGGGAAGAGTGACAAAATAGATGGGAACTGGGTGTAGCGTCGTAAG CTAATACGAAAATTAAAAATGACAAAATAGTTTGGAACTAGATTTCACTTATCTG GTT (SEQ ID NO. 907) and/or a nucleotide sequence of GAATATAGTCTTTACCATGCCCTTGGCCACGCCCCTCTTTAATACGACGGGCAAT TTGCACTTCAGAAAATGAAGAGTTTGCTTTAGCCATAACAAAAGTCCAGTATGCT TTTTCACAGCATAACTGGACTGATTTCAGTTTACAACTATTCTGTCTAGTTTAAGA CTTTATTGTCATAGTTTAGATCTATTTTGTTCAGTTTAAGACTTTATTGTCCGCCCA CA (SEQ ID NO. 908). In some embodiments, the construct comprises or consists of, from 5' to 3' a first transposable element inverted repeat, a first ITR, a sequence encoding a promoter, a sequence encoding a first nuclear localization signal, a sequence encoding a nuclease, a sequence encoding a second nuclear localization signal, a stop codon, a poly A sequence, a second ITR, and a second transposable element inverted repeat. In some embodiments, the construct comprises or consists of, from 5' to 3' a first transposable element inverted repeat having a sequence of SEQ ID NO. 907, a first AAV2 ITR, a sequence encoding a CK8e promoter, a nuclear localization signal having a sequence of SEQ ID NO. 885, a sequence encoding a SpCas9 nuclease, a nuclear localization signal having a sequence of SEQ ID NO: 887, a stop codon a minipoly A sequence, a second AAV2 ITR, and a second transposable element inverted repeat having a sequence of SEQ ID NO. 908. In some embodiments, the construct comprising or consisting of, from 5' to 3 ', a first transposable element inverted repeat, a first ITR, a sequence encoding a promoter, a sequence encoding a first nuclear localization signal, a sequence encoding a nuclease, a sequence encoding a second nuclear localization signal, a stop codon, a poly A sequence, a second ITR, and a second transposable element inverted repeat, further comprises a regulatory sequence. Exemplary regulatory sequences of the disclosure comprise or consist of a nucleotide sequence of CATGCAAGCTGTAGCCAACCACTAGAACTATAGCTAGAGTCCTGGGCGAACAAA CGATGCTCGCCTTCCAGAAAACCGAGGATGCGAACCACTTCATCCGGGGTCAGC ACCACCGGCAAGCGCCGCGACGGCCGAGGTCTTCCGATCTCCTGAAGCCAGGGC AGATCCGTGCACAGCACCTTGCCGTAGAAGAACAGCAAGGCCGCCAATGCCTGA CGATGCGTGGAGACCGAAACCTTGCGCTCGTTCGCCAGCCAGGACAGAAATGCC TCGACTTCGCTGCTGCCCAAGGTTGCCGGGTGACGCACACCGTGGAAACGGATG AAGGCACGAACCCAGTTGACATAAGCCTGTTCGGTTCGTAAACTGTAATGCAAGT AGCGTATGCGCTCACGCAACTGGTCCAGAACCTTGACCGAACGCAGCGGTGGTA ACGGCGCAGTGGCGGTTTTCATGGCTTGTTATGACTGTTTTTTTGTACAGTCTATG CCTCGGGCATCCAAGCAGCAAGCGCGTTACGCCGTGGGTCGATGTTTGATGTTAT GGAGCAGCAACGATGTTACGCAGCAGCAACGATGTTACGCAGCAGGGCAGTCGC CCTAAAACAAAGTTAGGTGGCTCAAGTATGGGCATCATTCGCACATGTAGGCTCG GCCCTGACCAAGTCAAATCCATGCGGGCTGCTCTTGATCTTTTCGGTCGTGAGTT CGGAGACGTAGCCACCTACTCCCAACATCAGCCGGACTCCGATTACCTCGGGAA CTTGCTCCGTAGTAAGACATTCATCGCGCTTGCTGCCTTCGACCAAGAAGCGGTT GTTGGCGCTCTCGCGGCTTACGTTCTGCCCAAGTTTGAGCAGCCGCGTAGTGAGA TCTATATCTATGATCTCGCAGTCTCCGGCGAGCACCGGAGGCAGGGCATTGCCAC CGCGCTCATCAATCTCCTCAAGCATGAGGCCAACGCGCTTGGTGCTTATGTGATC TACGTGCAAGCAGATTACGGTGACGATCCCGCAGTGGCTCTCTATACAAAGTTGG GCATACGGGAAGAAGTGATGCACTTTGATATCGACCCAAGTACCGCCACCTAAC AATTCGTTCAAGCCGAGATCGGCTTCCCGGCCGCGGAGTTGTTCGGTAAATTGTC ACAACGCCG (SEQ ID NO. 909). In some embodiments, the construct comprises or consists of, from 5' to 3' a first transposable element inverted repeat, a first ITR, a sequence encoding a promoter, a sequence encoding a first nuclear localization signal, a sequence encoding a nuclease, a sequence encoding a second nuclear localization signal, a stop codon, a poly A sequence, a second ITR, a regulatory sequence and a second transposable element inverted repeat. In some embodiments, the construct comprises or consists of, from 5' to 3' a first transposable element inverted repeat having a sequence of SEQ ID NO. 907, a first AAV2 ITR, a sequence encoding a CK8e promoter, a nuclear localization signal having a sequence of SEQ ID NO. 885, a sequence encoding a SpCas9 nuclease, a nuclear localization signal having a sequence of SEQ ID NO: 887, a stop codon a minipoly A sequence, a second AAV2 ITR, a regulatory sequence having a sequence of SEQ ID NO. 909, and a second transposable element inverted repeat having a sequence of SEQ ID NO. 908. In some embodiments, the construct may further comprise one or more spacer sequences. Exemplary spacer sequences of the disclosure have length from 1-1500 nucleotides, inclusive of all ranges therebetween. In some embodiments, the spacer sequences may be located either 5' to or 3' to an ITR, a promoter, a nuclear localization sequence, a nuclease, a stop codon, a polyA sequence, a transposable element inverted repeat, and/or a regulator element. In some embodiments, the construct may have a sequence comprising or consisting of SEQ ID NO: 899, SEQ ID NO: 900, SEQ ID NO: 901, or SEQ ID NO: 902.

### E. AAV-sgRNA vectors

In some embodiments, a nucleic acid of the present invention comprising sequences encoding at least the first, second and third DMD gRNAs may be packaged into an AAV vector. In some embodiments, at least a sequence encoding the first DMD gRNA, a sequence encoding the second DMD gRNA, a sequence encoding the third DMD gRNA, and a fourth sequence encoding a gRNA may be packaged into an AAV vector. In some embodiments, at least a sequence encoding the first DMD gRNA, a sequence encoding the second gRNA, a sequence encoding the third D gRNA, a fourth sequence encoding a gRNA, and a fifth sequence encoding a gRNA may be packaged into an AAV vector. In some embodiments, a plurality of sequences encoding a gRNA, including at least the first, second and third DMD gRNAs of the nucleic acid of the present invention, are packaged into an AAV vector. For example, 3, 4, 5, 6, 7, 8, 9, 10, 11, 12, 13, 14, 15, 16, 17, 18, 19, or 20 sequences encoding a gRNA, including at least the first, second and third DMD gRNAs of the nucleic acid of the present invention, may be packaged into an AAV vector. In some embodiments, in addition to at least the first, second and third DMD gRNAs of the nucleic acid of the present invention which are identical, each additional sequence encoding a gRNA is different. In some embodiments, at least 3, 4, 5, 6, 7, 8, 9, 10, 11, 12, 13, 14, 15, 16, 17, 18, 19, or 20 of the sequences encoding a gRNA are the same, and at least the first, second and third DMD gRNAs of the nucleic acid of the present invention are identical. In some embodiments, all of the sequence encoding a gRNA are the same.

In some embodiments, the AAV vector is a wildtype AAV vector. In some embodiments, the AAV vector contains one or more mutations. In some embodiments, the AAV vector is isolated or derived from an AAV vector of serotype AAV1, AAV2, AAV3, AAV4, AAV5, AAV6, AAV7, AAV8, AAV9, AAV10, AAV11 or any combination thereof.

Exemplary AAV-sgRNA vectors contain two ITR (inverted terminal repeat) sequences which flank a central sequence region comprising the sgRNA sequences. In some embodiments, the ITRs are isolated or derived from an AAV vector of serotype AAV1, AAV2, AAV3, AAV4, AAV5, AAV6, AAV7, AAV8, AAV9, AAV10, AAV11 or any combination thereof. In some embodiments, the ITRs are isolated or derived from an AAV vector of a first serotype and a sequence encoding a capsid protein of the AAV-sgRNA vector is isolated or derived from an AAV vector of a second serotype. In some embodiments, the first serotype and the second serotype are the same. In some embodiments, the first serotype and the second serotype are not the same. In some embodiments, the first serotype is AAV1, AAV2, AAV3, AAV4, AAV5, AAV6, AAV7, AAV8, AAV9, AAV10, or AAV11. In some embodiments, the second serotype is AAV1, AAV2, AAV3, AAV4, AAV5, AAV6, AAV7, AAV8, AAV9, AAV10, or AAV11. In some embodiments, the first serotype is AAV2 and the second serotype is AAV1, AAV2, AAV3, AAV4, AAV5, AAV6, AAV7, AAV8, AAV9, AAV10, or AAV11. In some embodiments, the first serotype is AAV2 and the second serotype is AAV9.

Exemplary AAV-sgRNA vectors contain two ITR (inverted terminal repeat) sequences which flank a central sequence region comprising the gRNA sequences. In some embodiments, the ITRs are isolated or derived from an AAV vector of serotype AAV1, AAV2, AAV3, AAV4, AAV5, AAV6, AAV7, AAV8, AAV9, AAV10, AAV11 or any combination thereof. In some embodiments, a first ITR is isolated or derived from an AAV vector of a first serotype, a second ITR is isolated or derived from an AAV vector of a second serotype and a sequence encoding a capsid protein of the AAV-sgRNA vector is isolated or derived from an AAV vector of a third serotype. In some embodiments, the first serotype and the second serotype are the same. In some embodiments, the first serotype and the second serotype are not the same. In some embodiments, the first serotype, the second serotype, and the third serotype are the same. In some embodiments, the first serotype, the second serotype, and the third serotype are not the same. In some embodiments, the first serotype is AAV1, AAV2, AAV3, AAV4, AAV5, AAV6, AAV7, AAV8, AAV9, AAV10, or AAV11. In some embodiments, the second serotype is AAV1, AAV2, AAV3, AAV4, AAV5, AAV6, AAV7, AAV8, AAV9, AAV10, or AAV11. In some embodiments, the third serotype is AAV1, AAV2, AAV3, AAV4, AAV5, AAV6, AAV7, AAV8, AAV9, AAV10, or AAV11. In some embodiments, the first serotype is AAV2, the second serotype is AAV4 and the third serotype is AAV1, AAV2, AAV3, AAV4, AAV5, AAV6, AAV7, AAV8, AAV9, AAV10, or AAV11. In some embodiments, the first serotype is AAV2, the second serotype is AAV4 and the third serotype is AAV9. Exemplary AAV-sgRNA vectors contain two ITR (inverted terminal repeat) sequences which flank a central sequence region comprising the sgRNA sequences. In some embodiments, the ITRs are isolated or derived from an AAV vector of serotype AAV1, AAV2, AAV3, AAV4, AAV5, AAV6, AAV7, AAV8, AAV9, AAV10, AAV11 or any combination thereof. In some embodiments, the ITRs comprise or consist of full-length and/or wildtype sequences for an AAV serotype. In some embodiments, the ITRs comprise or consist of truncated sequences for an AAV serotype. In some embodiments, the ITRs comprise or consist of elongated sequences for an AAV serotype. In some embodiments, the ITRs comprise or consist of sequences comprising a sequence variation compared to a wildtype sequence for the same AAV serotype. In some embodiments, the sequence variation comprises one or more of a substitution, deletion, insertion, inversion, or transposition. In some embodiments, the ITRs comprise or consist of at least 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150 base pairs. In some embodiments, the ITRs comprise or consist of 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150 base pairs. In some embodiments, the ITRs have a length of 110 ± 10 base pairs. In some embodiments, the ITRs have a length of 120 ± 10 base pairs. In some embodiments, the ITRs have a length of 130 ± 10 base pairs. In some embodiments, the ITRs have a length of 140 ± 10 base pairs. In some embodiments, the ITRs have a length of 150 ± 10 base pairs. In some embodiments, the ITRs have a length of 115, 145, or 141 base pairs. In some embodiments, the ITRs have a sequence selected from SEQ ID NO: 880, SEQ ID NO: 881, SEQ ID NO: 882, or SEQ ID NO: 883.

In some embodiments, the AAV-sgRNA vector may comprise additional elements to facilitate packaging of the vector and expression of the sgRNA. In some embodiments, the AAV-sgRNA vector may comprise a transposable element. In some embodiments, the AAV-sgRNA vector may comprise a regulatory element. In some embodiments, the regulatory element comprises an activator or a repressor. In some embodiments, the AAV-sgRNA sequence may comprise a non-functional or "stuffer" sequence. Exemplary stuffer sequences of the disclosure may have some (a non-zero percentage of) identity or homology to a genomic sequence of a mammal (including a human). Alternatively, exemplary stuffer sequences of the disclosure may have no identify or homology to a genomic sequence of a mammal (including a human). Exemplary stuffer sequences of the disclosure may comprise or consist of naturally occurring non-coding sequences or sequences that are neither transcribed nor translated following administration of the AAV vector to a subject.

In some embodiments, the AAV-sgRNA vector may be optimized for production in yeast, bacteria, insect cells, or mammalian cells. In some embodiments, the AAV-sgRNA vector may be optimized for expression in human cells. In some embodiments, the AAV-Cas9 vector may be optimized for expression in a bacculovirus expression system.

In some embodiments, the AAV-sgRNA vector comprises at least one promoter. In some embodiments, the AAV-sgRNA vector comprises at least two promoters. In some embodiments, the AAV-sgRNA vector comprises at least three promoters. In some embodiments, the AAV-sgRNA vector comprises at least four promoters. In some embodiments, the AAV-sgRNA vector comprises at least five promoters. Exemplary promoters include, for example, immunoglobulin light chain, immunoglobulin heavy chain, T-cell receptor, HLA DQ a and/or DQ β, β-interferon, interleukin-2, interleukin-2 receptor, MHC class II 5, MHC class II HLA-Dra, β-Actin, muscle creatine kinase (MCK), prealbumin (transthyretin), elastase I, metallothionein (MTII), collagenase, albumin, α-fetoprotein, t-globin, β-globin, c-fos, c-HA-ras, insulin, neural cell adhesion molecule (NCAM), α₁-antitrypain, H2B (TH2B) histone, mouse and/or type I collagen, glucose-regulated proteins (GRP94 and GRP78), rat growth hormone, human serum amyloid A (SAA), troponin I (TN I), platelet-derived growth factor (PDGF), duchenne muscular dystrophy, SV40, polyoma, retroviruses, papilloma virus, hepatitis B virus, human immunodeficiency virus, cytomegalovirus (CMV), and gibbon ape leukemia virus. Further exemplary promoters include the U6 promoter, the H1 promoter, and the 7SK promoter.

In some embodiments, the AAV vector comprises a nucleic acid of the present invention in which comprises a first sequence encoding a first DMD gRNA targeting a first genomic target sequence, a second sequence encoding a second DMD gRNA targeting a second genomic target sequence, a third sequence encoding a third DMD gRNA targeting a third genomic target sequence, a first promoter that drives expression of the first sequence encoding the first DMD gRNA, a second promoter that drives expression of the second sequence encoding the second DMD gRNA, and a third promoter that drives expression of the third sequence encoding the second DMD gRNA, wherein the first genomic target sequence, the second genomic target sequence and the third genomic sequence each comprise a dystrophin splice acceptor site, and wherein the sequence encoding the first DMD guide RNA, the sequence encoding the second DMD guide RNA genomic, and the sequence encoding the third DMD guide RNA target sequence are identical. In some embodiments, the first and second promoters are the same. In some embodiments, the first and second promoters are different. In some embodiments, the first and second promoters are selected from the H1 promoter, the U6 promoter, and the 7SK promoter.

In some embodiments, at least two of the first, second, and third promoters are the same. In some embodiments, each of the first, second, and third promoters are different. In some embodiments, the first, second, and third promoters are selected from the H1 promoter, the U6 promoter, and the 7SK promoter. In some embodiments, the first promoter is the U6 promoter. In some embodiments, the second promoter is the H1 promoter. In some embodiments, the third promoter is the 7SK promoter. In some embodiments, the first promoter is the U6 promoter, the second promoter is the H1 promoter, and the third promoter is the 7SK promoter.

In some embodiments, the AAV vector comprises a nucleic acid of the present invention in which comprises a first sequence encoding a first DMD gRNA targeting a first genomic target sequence, a second sequence encoding a second DMD gRNA targeting a second genomic target sequence, a third sequence encoding a third DMD gRNA targeting a third genomic target sequence, and a fourth sequence encoding a fourth gRNA, a first promoter that drives expression of the first sequence encoding athe first DMD gRNA, a second promoter that drives expression of the second sequence encoding the second DMD gRNA, a third promoter that drives expression of the third sequence encoding the third DMD gRNA, and a fourth promoter that drives expression of the fourth sequence encoding a gRNA, wherein the first genomic target sequence, the second genomic target sequence and the third genomic sequence each comprise a dystrophin splice acceptor site, and wherein the sequence encoding the first DMD guide RNA, the sequence encoding the second DMD guide RNA genomic, and the sequence encoding the third DMD guide RNA target sequence are identical. In some embodiments, at least two of the first, second, third, and fourth promoters are the same. In some embodiments, each of the first, second, third, and fourth promoters are different. In some embodiments, each of the first, second, third and fourth promoters are selected from the H1 promoter, the U6 promoter, and the 7SK promoter. In some embodiments, the first sequence encoding a gRNA, the second sequence encoding a gRNA, the third sequence encoding a gRNA, and the fourth sequence encoding a gRNA are identical. In some embodiments, the fourth sequence encoding the fourth gRNA is not identical to the first, second and third sequences encoding the first, second and third DMD gRNAs, respectively.

In some embodiments, the AAV vector comprises a nucleic acid of the present invention in which comprises a first sequence encoding the first DMD gRNA targeting a first genomic target sequence , a second sequence encoding the second DMD gRNA targeting a second genomic target sequence, a third sequence encoding the third DMD gRNA targeting a third genomic target sequence, a fourth sequence encoding a fourth gRNA, and a fifth sequence encoding a fifth gRNA, a first promoter that drives expression of the first sequence encoding the first DMD gRNA, a second promoter that drives expression of the second sequence encoding the second DMD gRNA, a third promoter drives expression of the third sequence encoding the third DMD gRNA, a fourth promoter drives expression of the fourth sequence encoding the fourth gRNA, and a fifth promoter drives expression of the fifth sequence encoding the fifth gRNA, wherein the first genomic target sequence, the second genomic target sequence and the third genomic sequence each comprise a dystrophin splice acceptor site, and wherein the sequence encoding the first DMD guide RNA, the sequence encoding the second DMD guide RNA genomic, and the sequence encoding the third DMD guide RNA target sequence are identical. In some embodiments, at least two of the first, second, third, fourth, and fifth promoters are the same. In some embodiments, each of the first, second, third, fourth, and fifth promoters are different. In some embodiments, each of the first, second, third, and fourth promoters are different. In some embodiments, each of the first, second, third, fourth and fifth promoters are selected from the H1 promoter, the U6 promoter, and the 7SK promoter. In some embodiments, the first sequence encoding the first DMD gRNA, the second sequence encoding the second DMD gRNA, the third sequence encoding the third DMD gRNA, the fourth sequence encoding the fourth gRNA, and the fifth sequence encoding the fifth gRNA are identical. In some embodiments, the fourth sequence encoding the fourth gRNA, and/or the fifth sequence encoding the fifth gRNA are not identical to the first sequence encoding the first DMD gRNA, the second sequence encoding the second DMD gRNA, and the third sequence encoding the third DMD gRNA.

In some embodiments, the AAV-sgRNA vector comprises a sequence selected from SEQ ID NO: 910, SEQ ID NO: 911, SEQ ID NO: 912, or SEQ ID NO: 913. In some embodiments, the AAV-sgRNA vector comprises a sequence selected from SEQ ID NO: 914, SEQ ID NO: 915, SEQ ID NO: 916, or SEQ ID NO: 917. In some embodiments, the AAV-sgRNA vector comprises a sequence selected from SEQ ID NO: 918, SEQ ID NO: 919, SEQ ID NO: 920, or SEQ ID NO: 921. Exemplary AAV-sgRNA vectors are provided in Table 5.

**Table 5. Exemplary AAV-sgRNA vectors.**

| SE Q ID NO. | Sequence |
|---|---|
| 910 | CCACTCCCTCTATGCGCGCTCGCTCACTCACTCGGCCCTGGAGACCAAAG GTCTCCAGACTGCCGGCCTCTGGCCGGCAGGGCCGAGTGAGTGAGCGAG CGCGCATAGAGGGAGTGGGTACCTCCATCATCTAGGTTTGCCAGATCTGA TATCGGCGCGCCCCTGGGCGCGCCCGAGTCCAACACCCGTGGGAATCCC ATGGGCACCATGGCCCCTCGCTCCAAAAATGCTTTCGCGTCGCGCAGACA CTGCTCGGTAGTTTCGGGGATCAGCGTTTGAGTAAGAGCCCGCGTCTGAA CCCTCCGCGCCGCCCCGGCCCCAGTGGAAAGACGCGCAGGCAAAACGCA CCACGTGACGGAGCGTGACCGCGCGCCGAGCGCGCGCCAAGGTCGGGCA GGAAGAGGGCCTATTTCCCATGATTCCTTCATATTTGCATATACGATACA AGGCTGTTAGAGAGATAATTAGAATTAATTTGACTGTAAACACAAAGAT ATTAGTACAAAATACGTGACGTAGAAAGTAATAATTTCTTGGGTAGTTTG CAGTTTTAAAATTATGTTTTAAAATGGACTATCATATGCTTACCGTAACTT GAAAGTATTTCGATTTCTTGGCTTTATATATCTTGTGGAAAGGACGAAAC ACCGCACTAGAGTAACAGTCTGACGTTTAAGAGCTATGCTGGAAACAGC ATAGCAAGTTTAAATAAGGCTAGTCCGTTATCAACTTGAAAAAGTGGCA CCGAGTCGGTGCTTTTTTTGCGGCCGCGCTCGGCGCGCCCATATTTGCAT GTCGCTATGTGTTCTGGGAAATCACCATAAACGTGAAATGTCTTTGGATT TGGGAATCTTATAAGTTCTGTATGAGACCACGGTACACCGCACTAGAGTA ACAGTCTGACGTTTAAGAGCTATGCTGGAAACAGCATAGCAAGTTTAAA TAAGGCTAGTCCGTTATCAACTTGAAAAAGTGGCACCGAGTCGGTGCTTT TTTTGCGGCCGCTGACGGCGCGCCCTGCAGTATTTAGCATGCCCCACCCA TCTGCAAGGCATTCTGGATAGTGTCAAAACAGCCGGAAATCAAGTCCGTT TATCTCAAACTTTAGCATTTTGGGAATAAATGATATTTGCTATGCTGGTTA AATTAGATTTTAGTTAAATTTCCTGCTGAAGCTCTAGTACGATAAGTAAC TTGACCTAAGTGTAAAGTTGAGATTTCCTTCAGGTTTATATAGCTTGTGC GCCGCCTGGGTACACCGCACTAGAGTAACAGTCTGACGTTTAAGAGCTAT GCTGGAAACAGCATAGCAAGTTTAAATAAGGCTAGTCCGTTATCAACTTG AAAAAGTGGCACCGAGTCGGTGCTTTTTTTGCGGCCGCGCTTGCGGCCGC CTCGAGTGATCAAAAAAACCAACACACGCTTCCAATGAAAATAAACGAT CCTTTATTGCTAGCCTTTACTTGTACAGCTCGTCCATGCCGAGAGTGATCC CGGCGGCGGTCACGAACTCCAGCAGGACCATGTGATCGCGCTTCTCGTTG |
| | GGGTCTTTGCTCAGGGCGGACTGGGTGCTCAGGTAGTGGTTGTCGGGCAG CAACACGGGGCCGTCGCCGATGGGGGTGTTCTGCTGGTAGTGGTCGGCG AGCTGCACGCTGCCGTCCTCGATGTTGTGGCGGATCTTGAAGTTCACCTT GATGCCGTTCTTCTGCTTGTCGGCCATGATATAGACGTTGTGGCTGTTGTA GTTGTACTCCAGCTTGTGCCCCAGGATGTTGCCGTCCTCCTTGAAGTCGA TGCCCTTCAGCTCGATGCGGTTCACCAGGGTGTCGCCCTCGAACTTCACC TCGGCGCGGGTCTTGTAGTTGCCGTCGTCCTTGAAGAAGATGGTGCGCTC CTGGACGTAGCCTTCGGGCATGGCGGACTTGAAGAAGTCGTGCTGCTTCA TGTGGTCGGGGTAGCGGCTGAAGCACTGCACGCCGTAGGTCAGGGTGGT CACGAGGGTGGGCCAGGGCACGGGCAGCTTGCCGGTGGTGCAGATGAAC TTCAGGGTCAGCTTGCCGTAGGTGGCATCGCCCTCGCCCTCGCCGGACAC GCTGAACTTGTGGCCGTTTACGTCGCCGTCCAGCTCGACCAGGATGGGCA CCACCCCGGTGAACAGCTCCTCGCCCTTGCTCACCATGGTGGCGACCGGT GGATCCCGGGCCCGCGGGGTGGCTTTACCAACAGTACCCGGAATGCCAA GCTTACTTAGATCGCAGTCTCGACGCTGGCTGGCTCCTGAGTGTCTGTCT GTGCTGTGGAGGTGGTGGTAGAATGAGGGCAGCCCCTGTGCCCCTGGGT TATATAGAGGAGCCTACAGGGTGTGACTAGCCAGGAGGGGCTGTCCCCA GGGAGGGGCCCCTGAGAGCAGATGAGCTTTCAGCTCGTTGCCCGGGCAC CGTGCCCACCCCGGACCCAGGCGTGCAGCTTGCCCAGCCCCATGGCCTTG TATGGGCTGCCCCAAGGGCTGACTTGCTCACTGGTTCCTAAACTAAGTGC TGAGTCTAGCTGGCGGGGGACAGCTGGCCCTTCGCCGGGAACATGGCAT GCAGGGTTATTTTTAGAGGCAGCAGGTGTTGGGGGGGGGGGGGCAGCCA CATGTCTGGGTTAATTATAACCAGGCATCTCGGGTGTCCCCAGGCCTTGC CTCCTTACATGGGCACGTCGACGATATCAGATCTAGGAACCCCTAGTGAT GGAGTTGGCCACTCCCTCTCTGCGCGCTCGCTCGCTCACTGAGGCCGCCC GGGCAAAGCCCGGGCGTCGGGCGACCTTTGGTCGCCCGGCCTCAGTGAG CGAGCGAGCGCGCAGAGAGGGAGTGG |
| 918 | CCACTCCCTCTATGCGCGCTCGCTCACTCACTCGGCCCTGGAGACCAAAG GTCTCCAGACTGCCGGCCTCTGGCCGGCAGGGCCGAGTGAGTGAGCGAG CGCGCATAGAGGGAGTGGGTACCTCCATCATCTAGGTTTGCCAGATCTGA TATCGGCGCGCCCCTGGGCGCGCCCGAGTCCAACACCCGTGGGAATCCC ATGGGCACCATGGCCCCTCGCTCCAAAAATGCTTTCGCGTCGCGCAGACA CTGCTCGGTAGTTTCGGGGATCAGCGTTTGAGTAAGAGCCCGCGTCTGAA CCCTCCGCGCCGCCCCGGCCCCAGTGGAAAGACGCGCAGGCAAAACGCA CCACGTGACGGAGCGTGACCGCGCGCCGAGCGCGCGCCAAGGTCGGGCA GGAAGAGGGCCTATTTCCCATGATTCCTTCATATTTGCATATACGATACA AGGCTGTTAGAGAGATAATTAGAATTAATTTGACTGTAAACACAAAGAT ATTAGTACAAAATACGTGACGTAGAAAGTAATAATTTCTTGGGTAGTTTG CAGTTTTAAAATTATGTTTTAAAATGGACTATCATATGCTTACCGTAACTT GAAAGTATTTCGATTTCTTGGCTTTATATATCTTGTGGAAAGGACGAAAC ACCGCACCAGAGTAACAGTCTGACGTTTAAGAGCTATGCTGGAAACAGC ATAGCAAGTTTAAATAAGGCTAGTCCGTTATCAACTTGAAAAAGTGGCA CCGAGTCGGTGCTTTTTTTGCGGCCGCGCTCGGCGCGCCCATATTTGCAT GTCGCTATGTGTTCTGGGAAATCACCATAAACGTGAAATGTCTTTGGATT TGGGAATCTTATAAGTTCTGTATGAGACCACGGTACACCGCACCAGAGTA ACAGTCTGACGTTTAAGAGCTATGCTGGAAACAGCATAGCAAGTTTAAA TAAGGCTAGTCCGTTATCAACTTGAAAAAGTGGCACCGAGTCGGTGCTTT |
| | TTTTGCGGCCGCTGACGGCGCGCCCTGCAGTATTTAGCATGCCCCACCCA TCTGCAAGGCATTCTGGATAGTGTCAAAACAGCCGGAAATCAAGTCCGTT TATCTCAAACTTTAGCATTTTGGGAATAAATGATATTTGCTATGCTGGTTA AATTAGATTTTAGTTAAATTTCCTGCTGAAGCTCTAGTACGATAAGTAAC TTGACCTAAGTGTAAAGTTGAGATTTCCTTCAGGTTTATATAGCTTGTGC GCCGCCTGGGTACACCGCACCAGAGTAACAGTCTGACGTTTAAGAGCTA TGCTGGAAACAGCATAGCAAGTTTAAATAAGGCTAGTCCGTTATCAACTT GAAAAAGTGGCACCGAGTCGGTGCTTTTTTTGCGGCCGCGCTGTTTAAAC CAACAAAATCAGCAGCTAATGAAGGCAAGTCAGCAGGTCACTCATCATT TTCCACTTCGGCAATGCAGTGGGATTATTCCAACAGAGGTTTTTCACAGC ATTCCTTCAGTTAACTGGAGATCGAATCTTGATTTTCACAGATATACTTG GCAAGGTCCGCCCTGTCATCAGCACATTCAAGCAGATCTCCATGGCAGCA TTCCGTGTGGACTTAGGTAAGATCTGTCACTAACTTGGAAACTTCTGCAA ACTCAGCTTAGGGAAATCTCTGGCTCAGGCGAGCTACTGCCTCAGCTTAG AAAGCTCTTTCTCCAAATTATTGGAGACTGGCACACTTAAGTCCCTGTTA GGCAGACGAAGCCTTCCCTTCATCCCGAAGTTCATCGAGCTTTGGCAACA GGCAGGCAGCTTTATCAGCAGCTTGGCAACATTCTGTAAAAGCAGCTTTA TACCTTTAAGCAAAGAAAAGGAGTTCCGGGGCATAAAAGTAAGGATGTC TTCTGGCAATTTATAATAAGTATTTTTTCAAAAATGTCTCTTCATTGTCAT GAAAAGCAGTGCATCACACATCAACCTCTGGTCTCACCAATCGGGGGAG GTTTGGGTTGTTTACTTAGTGTTGCAAGAATTATTTTATTCTCTCAGGTTC TTGTTTTGCACAGCAGTCAGCTCATTCACCATAGGTTTCACGAAGAGTTG CTGCGGCCGCCTCGATAATACGACTCACTATAGGGTCGACGATATCAGAT CTAGGAACCCCTAGTGATGGAGTTGGCCACTCCCTCTCTGCGCGCTCGCT CGCTCACTGAGGCCGCCCGGGCAAAGCCCGGGCGTCGGGCGACCTTTGG TCGCCCGGCCTCAGTGAGCGAGCGAGCGCGCAGAGAGGGAGTGG |
| 914 | CCACTCCCTCTATGCGCGCTCGCTCACTCACTCGGCCCTGGAGACCAAAG GTCTCCAGACTGCCGGCCTCTGGCCGGCAGGGCCGAGTGAGTGAGCGAG CGCGCATAGAGGGAGTGGGTACCTCCATCATCTAGGTTTGCCAGATCTGA TATCGGCGCGCCCCTGGGCGCGCCCGAGTCCAACACCCGTGGGAATCCC ATGGGCACCATGGCCCCTCGCTCCAAAAATGCTTTCGCGTCGCGCAGACA CTGCTCGGTAGTTTCGGGGATCAGCGTTTGAGTAAGAGCCCGCGTCTGAA CCCTCCGCGCCGCCCCGGCCCCAGTGGAAAGACGCGCAGGCAAAACGCA CCACGTGACGGAGCGTGACCGCGCGCCGAGCGCGCGCCAAGGTCGGGCA GGAAGAGGGCCTATTTCCCATGATTCCTTCATATTTGCATATACGATACA AGGCTGTTAGAGAGATAATTAGAATTAATTTGACTGTAAACACAAAGAT ATTAGTACAAAATACGTGACGTAGAAAGTAATAATTTCTTGGGTAGTTTG CAGTTTTAAAATTATGTTTTAAAATGGACTATCATATGCTTACCGTAACTT GAAAGTATTTCGATTTCTTGGCTTTATATATCTTGTGGAAAGGACGAAAC ACCGCACCAGAGTAACAGTCTGAGGTTTAAGAGCTATGCTGGAAACAGC ATAGCAAGTTTAAATAAGGCTAGTCCGTTATCAACTTGAAAAAGTGGCA CCGAGTCGGTGCTTTTTTTGCGGCCGCGCTCGGCGCGCCCATATTTGCAT GTCGCTATGTGTTCTGGGAAATCACCATAAACGTGAAATGTCTTTGGATT TGGGAATCTTATAAGTTCTGTATGAGACCACGGTACACCGCACCAGAGTA ACAGTCTGAGGTTTAAGAGCTATGCTGGAAACAGCATAGCAAGTTTAAA TAAGGCTAGTCCGTTATCAACTTGAAAAAGTGGCACCGAGTCGGTGCTTT TTTTGCGGCCGCTGACGGCGCGCCCTGCAGTATTTAGCATGCCCCACCCA |
| | TCTGCAAGGCATTCTGGATAGTGTCAAAACAGCCGGAAATCAAGTCCGTT TATCTCAAACTTTAGCATTTTGGGAATAAATGATATTTGCTATGCTGGTTA AATTAGATTTTAGTTAAATTTCCTGCTGAAGCTCTAGTACGATAAGTAAC TTGACCTAAGTGTAAAGTTGAGATTTCCTTCAGGTTTATATAGCTTGTGC GCCGCCTGGGTACACCGCACCAGAGTAACAGTCTGAGGTTTAAGAGCTA TGCTGGAAACAGCATAGCAAGTTTAAATAAGGCTAGTCCGTTATCAACTT GAAAAAGTGGCACCGAGTCGGTGCTTTTTTTGCGGCCGCGCTGTTTAAAC CAACAAAATCAGCAGCTAATGAAGGCAAGTCAGCAGGTCACTCATCATT TTCCACTTCGGCAATGCAGTGGGATTATTCCAACAGAGGTTTTTCACAGC ATTCCTTCAGTTAACTGGAGATCGAATCTTGATTTTCACAGATATACTTG GCAAGGTCCGCCCTGTCATCAGCACATTCAAGCAGATCTCCATGGCAGCA TTCCGTGTGGACTTAGGTAAGATCTGTCACTAACTTGGAAACTTCTGCAA ACTCAGCTTAGGGAAATCTCTGGCTCAGGCGAGCTACTGCCTCAGCTTAG AAAGCTCTTTCTCCAAATTATTGGAGACTGGCACACTTAAGTCCCTGTTA GGCAGACGAAGCCTTCCCTTCATCCCGAAGTTCATCGAGCTTTGGCAACA GGCAGGCAGCTTTATCAGCAGCTTGGCAACATTCTGTAAAAGCAGCTTTA TACCTTTAAGCAAAGAAAAGGAGTTCCGGGGCATAAAAGTAAGGATGTC TTCTGGCAATTTATAATAAGTATTTTTTCAAAAATGTCTCTTCATTGTCAT GAAAAGCAGTGCATCACACATCAACCTCTGGTCTCACCAATCGGGGGAG GTTTGGGTTGTTTACTTAGTGTTGCAAGAATTATTTTATTCTCTCAGGTTC TTGTTTTGCACAGCAGTCAGCTCATTCACCATAGGTTTCACGAAGAGTTG CTGCGGCCGCCTCGATAATACGACTCACTATAGGGTCGACGATATCAGAT CTAGGAACCCCTAGTGATGGAGTTGGCCACTCCCTCTCTGCGCGCTCGCT CGCTCACTGAGGCCGCCCGGGCAAAGCCCGGGCGTCGGGCGACCTTTGG TCGCCCGGCCTCAGTGAGCGAGCGAGCGCGCAGAGAGGGAGTGG |
| 911 | CCTGCAGGCAGCTGCGCGCTCGCTCGCTCACTGAGGCCGCCCGGGCAAA GCCCGGGCGTCGGGCGACCTTTGGTCGCCCGGCCTCAGTGAGCGAGCGA GCGCGCAGAGAGGGAGTGGCCAACTCCATCACTAGGGGTTCCTAGATCT GATATCGGCGCGCCCCTGGGCGCGCCCGAGTCCAACACCCGTGGGAATC CCATGGGCACCATGGCCCCTCGCTCCAAAAATGCTTTCGCGTCGCGCAGA CACTGCTCGGTAGTTTCGGGGATCAGCGTTTGAGTAAGAGCCCGCGTCTG AACCCTCCGCGCCGCCCCGGCCCCAGTGGAAAGACGCGCAGGCAAAACG CACCACGTGACGGAGCGTGACCGCGCGCCGAGCGCGCGCCAAGGTCGGG CAGGAAGAGGGCCTATTTCCCATGATTCCTTCATATTTGCATATACGATA CAAGGCTGTTAGAGAGATAATTAGAATTAATTTGACTGTAAACACAAAG ATATTAGTACAAAATACGTGACGTAGAAAGTAATAATTTCTTGGGTAGTT TGCAGTTTTAAAATTATGTTTTAAAATGGACTATCATATGCTTACCGTAA CTTGAAAGTATTTCGATTTCTTGGCTTTATATATCTTGTGGAAAGGACGA AACACCGCACTAGAGTAACAGTCTGACGTTTAAGAGCTATGCTGGAAAC AGCATAGCAAGTTTAAATAAGGCTAGTCCGTTATCAACTTGAAAAAGTG GCACCGAGTCGGTGCTTTTTTTGCGGCCGCGCTCGGCGCGCCCATATTTG CATGTCGCTATGTGTTCTGGGAAATCACCATAAACGTGAAATGTCTTTGG ATTTGGGAATCTTATAAGTTCTGTATGAGACCACGGTACACCGCACTAGA GTAACAGTCTGACGTTTAAGAGCTATGCTGGAAACAGCATAGCAAGTTT AAATAAGGCTAGTCCGTTATCAACTTGAAAAAGTGGCACCGAGTCGGTG CTTTTTTTGCGGCCGCTGACGGCGCGCCCTGCAGTATTTAGCATGCCCCA CCCATCTGCAAGGCATTCTGGATAGTGTCAAAACAGCCGGAAATCAAGT |
| | CCGTTTATCTCAAACTTTAGCATTTTGGGAATAAATGATATTTGCTATGCT GGTTAAATTAGATTTTAGTTAAATTTCCTGCTGAAGCTCTAGTACGATAA GTAACTTGACCTAAGTGTAAAGTTGAGATTTCCTTCAGGTTTATATAGCT TGTGCGCCGCCTGGGTACACCGCACTAGAGTAACAGTCTGACGTTTAAGA GCTATGCTGGAAACAGCATAGCAAGTTTAAATAAGGCTAGTCCGTTATCA ACTTGAAAAAGTGGCACCGAGTCGGTGCTTTTTTTGCGGCCGCGCTTGCG GCCGCCTCGAGTGATCAAAAAAACCAACACACGCTTCCAATGAAAATAA ACGATCCTTTATTGCTAGCCTTTACTTGTACAGCTCGTCCATGCCGAGAGT GATCCCGGCGGCGGTCACGAACTCCAGCAGGACCATGTGATCGCGCTTCT CGTTGGGGTCTTTGCTCAGGGCGGACTGGGTGCTCAGGTAGTGGTTGTCG GGCAGCAACACGGGGCCGTCGCCGATGGGGGTGTTCTGCTGGTAGTGGT CGGCGAGCTGCACGCTGCCGTCCTCGATGTTGTGGCGGATCTTGAAGTTC ACCTTGATGCCGTTCTTCTGCTTGTCGGCCATGATATAGACGTTGTGGCTG TTGTAGTTGTACTCCAGCTTGTGCCCCAGGATGTTGCCGTCCTCCTTGAAG TCGATGCCCTTCAGCTCGATGCGGTTCACCAGGGTGTCGCCCTCGAACTT CACCTCGGCGCGGGTCTTGTAGTTGCCGTCGTCCTTGAAGAAGATGGTGC GCTCCTGGACGTAGCCTTCGGGCATGGCGGACTTGAAGAAGTCGTGCTGC TTCATGTGGTCGGGGTAGCGGCTGAAGCACTGCACGCCGTAGGTCAGGG TGGTCACGAGGGTGGGCCAGGGCACGGGCAGCTTGCCGGTGGTGCAGAT GAACTTCAGGGTCAGCTTGCCGTAGGTGGCATCGCCCTCGCCCTCGCCGG ACACGCTGAACTTGTGGCCGTTTACGTCGCCGTCCAGCTCGACCAGGATG GGCACCACCCCGGTGAACAGCTCCTCGCCCTTGCTCACCATGGTGGCGAC CGGTGGATCCCGGGCCCGCGGGGTGGCTTTACCAACAGTACCCGGAATG CCAAGCTTACTTAGATCGCAGTCTCGACGCTGGCTGGCTCCTGAGTGTCT GTCTGTGCTGTGGAGGTGGTGGTAGAATGAGGGCAGCCCCTGTGCCCCTG GGTTATATAGAGGAGCCTACAGGGTGTGACTAGCCAGGAGGGGCTGTCC CCAGGGAGGGGCCCCTGAGAGCAGATGAGCTTTCAGCTCGTTGCCCGGG CACCGTGCCCACCCCGGACCCAGGCGTGCAGCTTGCCCAGCCCCATGGC CTTGTATGGGCTGCCCCAAGGGCTGACTTGCTCACTGGTTCCTAAACTAA GTGCTGAGTCTAGCTGGCGGGGGACAGCTGGCCCTTCGCCGGGAACATG GCATGCAGGGTTATTTTTAGAGGCAGCAGGTGTTGGGGGGGGGGGGGCA GCCACATGTCTGGGTTAATTATAACCAGGCATCTCGGGTGTCCCCAGGCC TTGCCTCCTTACATGGGCACGTCGACGATATCAGATCTAGGAACCCCTAG TGATGGAGTTGGCCACTCCCTCTCTGCGCGCTCGCTCGCTCACTGAGGCC GGGCGACCAAAGGTCGCCCGACGCCCGGGCTTTGCCCGGGCGGCCTCAG TGAGCGAGCGAGCGCGCAGCTGCCTGCAGG |
| 919 | CCTGCAGGCAGCTGCGCGCTCGCTCGCTCACTGAGGCCGCCCGGGCAAA GCCCGGGCGTCGGGCGACCTTTGGTCGCCCGGCCTCAGTGAGCGAGCGA GCGCGCAGAGAGGGAGTGGCCAACTCCATCACTAGGGGTTCCTAGATCT GATATCGGCGCGCCCCTGGGCGCGCCCGAGTCCAACACCCGTGGGAATC CCATGGGCACCATGGCCCCTCGCTCCAAAAATGCTTTCGCGTCGCGCAGA CACTGCTCGGTAGTTTCGGGGATCAGCGTTTGAGTAAGAGCCCGCGTCTG AACCCTCCGCGCCGCCCCGGCCCCAGTGGAAAGACGCGCAGGCAAAACG CACCACGTGACGGAGCGTGACCGCGCGCCGAGCGCGCGCCAAGGTCGGG CAGGAAGAGGGCCTATTTCCCATGATTCCTTCATATTTGCATATACGATA CAAGGCTGTTAGAGAGATAATTAGAATTAATTTGACTGTAAACACAAAG ATATTAGTACAAAATACGTGACGTAGAAAGTAATAATTTCTTGGGTAGTT |
| | TGCAGTTTTAAAATTATGTTTTAAAATGGACTATCATATGCTTACCGTAA CTTGAAAGTATTTCGATTTCTTGGCTTTATATATCTTGTGGAAAGGACGA AACACCGCACCAGAGTAACAGTCTGACGTTTAAGAGCTATGCTGGAAAC AGCATAGCAAGTTTAAATAAGGCTAGTCCGTTATCAACTTGAAAAAGTG GCACCGAGTCGGTGCTTTTTTTGCGGCCGCGCTCGGCGCGCCCATATTTG CATGTCGCTATGTGTTCTGGGAAATCACCATAAACGTGAAATGTCTTTGG ATTTGGGAATCTTATAAGTTCTGTATGAGACCACGGTACACCGCACCAGA GTAACAGTCTGACGTTTAAGAGCTATGCTGGAAACAGCATAGCAAGTTT AAATAAGGCTAGTCCGTTATCAACTTGAAAAAGTGGCACCGAGTCGGTG CTTTTTTTGCGGCCGCTGACGGCGCGCCCTGCAGTATTTAGCATGCCCCA CCCATCTGCAAGGCATTCTGGATAGTGTCAAAACAGCCGGAAATCAAGT CCGTTTATCTCAAACTTTAGCATTTTGGGAATAAATGATATTTGCTATGCT GGTTAAATTAGATTTTAGTTAAATTTCCTGCTGAAGCTCTAGTACGATAA GTAACTTGACCTAAGTGTAAAGTTGAGATTTCCTTCAGGTTTATATAGCT TGTGCGCCGCCTGGGTACACCGCACCAGAGTAACAGTCTGACGTTTAAG AGCTATGCTGGAAACAGCATAGCAAGTTTAAATAAGGCTAGTCCGTTATC AACTTGAAAAAGTGGCACCGAGTCGGTGCTTTTTTTGCGGCCGCGCTGTT TAAACCAACAAAATCAGCAGCTAATGAAGGCAAGTCAGCAGGTCACTCA TCATTTTCCACTTCGGCAATGCAGTGGGATTATTCCAACAGAGGTTTTTC ACAGCATTCCTTCAGTTAACTGGAGATCGAATCTTGATTTTCACAGATAT ACTTGGCAAGGTCCGCCCTGTCATCAGCACATTCAAGCAGATCTCCATGG CAGCATTCCGTGTGGACTTAGGTAAGATCTGTCACTAACTTGGAAACTTC TGCAAACTCAGCTTAGGGAAATCTCTGGCTCAGGCGAGCTACTGCCTCAG CTTAGAAAGCTCTTTCTCCAAATTATTGGAGACTGGCACACTTAAGTCCC TGTTAGGCAGACGAAGCCTTCCCTTCATCCCGAAGTTCATCGAGCTTTGG CAACAGGCAGGCAGCTTTATCAGCAGCTTGGCAACATTCTGTAAAAGCA GCTTTATACCTTTAAGCAAAGAAAAGGAGTTCCGGGGCATAAAAGTAAG GATGTCTTCTGGCAATTTATAATAAGTATTTTTTCAAAAATGTCTCTTCAT TGTCATGAAAAGCAGTGCATCACACATCAACCTCTGGTCTCACCAATCGG GGGAGGTTTGGGTTGTTTACTTAGTGTTGCAAGAATTATTTTATTCTCTCA GGTTCTTGTTTTGCACAGCAGTCAGCTCATTCACCATAGGTTTCACGAAG AGTTGCTGCGGCCGCCTCGATAATACGACTCACTATAGGGTCGACGATAT CAGATCTAGGAACCCCTAGTGATGGAGTTGGCCACTCCCTCTCTGCGCGC TCGCTCGCTCACTGAGGCCGGGCGACCAAAGGTCGCCCGACGCCCGGGC TTTGCCCGGGCGGCCTCAGTGAGCGAGCGAGCGCGCAGCTGCCTGCAGG |
| 915 | CCTGCAGGCAGCTGCGCGCTCGCTCGCTCACTGAGGCCGCCCGGGCAAA GCCCGGGCGTCGGGCGACCTTTGGTCGCCCGGCCTCAGTGAGCGAGCGA GCGCGCAGAGAGGGAGTGGCCAACTCCATCACTAGGGGTTCCTAGATCT GATATCGGCGCGCCCCTGGGCGCGCCCGAGTCCAACACCCGTGGGAATC CCATGGGCACCATGGCCCCTCGCTCCAAAAATGCTTTCGCGTCGCGCAGA CACTGCTCGGTAGTTTCGGGGATCAGCGTTTGAGTAAGAGCCCGCGTCTG AACCCTCCGCGCCGCCCCGGCCCCAGTGGAAAGACGCGCAGGCAAAACG CACCACGTGACGGAGCGTGACCGCGCGCCGAGCGCGCGCCAAGGTCGGG CAGGAAGAGGGCCTATTTCCCATGATTCCTTCATATTTGCATATACGATA CAAGGCTGTTAGAGAGATAATTAGAATTAATTTGACTGTAAACACAAAG ATATTAGTACAAAATACGTGACGTAGAAAGTAATAATTTCTTGGGTAGTT TGCAGTTTTAAAATTATGTTTTAAAATGGACTATCATATGCTTACCGTAA |
| | CTTGAAAGTATTTCGATTTCTTGGCTTTATATATCTTGTGGAAAGGACGA AACACCGCACCAGAGTAACAGTCTGAGGTTTAAGAGCTATGCTGGAAAC AGCATAGCAAGTTTAAATAAGGCTAGTCCGTTATCAACTTGAAAAAGTG GCACCGAGTCGGTGCTTTTTTTGCGGCCGCGCTCGGCGCGCCCATATTTG CATGTCGCTATGTGTTCTGGGAAATCACCATAAACGTGAAATGTCTTTGG ATTTGGGAATCTTATAAGTTCTGTATGAGACCACGGTACACCGCACCAGA GTAACAGTCTGAGGTTTAAGAGCTATGCTGGAAACAGCATAGCAAGTTT AAATAAGGCTAGTCCGTTATCAACTTGAAAAAGTGGCACCGAGTCGGTG CTTTTTTTGCGGCCGCTGACGGCGCGCCCTGCAGTATTTAGCATGCCCCA CCCATCTGCAAGGCATTCTGGATAGTGTCAAAACAGCCGGAAATCAAGT CCGTTTATCTCAAACTTTAGCATTTTGGGAATAAATGATATTTGCTATGCT GGTTAAATTAGATTTTAGTTAAATTTCCTGCTGAAGCTCTAGTACGATAA GTAACTTGACCTAAGTGTAAAGTTGAGATTTCCTTCAGGTTTATATAGCT TGTGCGCCGCCTGGGTACACCGCACCAGAGTAACAGTCTGAGGTTTAAG AGCTATGCTGGAAACAGCATAGCAAGTTTAAATAAGGCTAGTCCGTTATC AACTTGAAAAAGTGGCACCGAGTCGGTGCTTTTTTTGCGGCCGCGCTGTT TAAACCAACAAAATCAGCAGCTAATGAAGGCAAGTCAGCAGGTCACTCA TCATTTTCCACTTCGGCAATGCAGTGGGATTATTCCAACAGAGGTTTTTC ACAGCATTCCTTCAGTTAACTGGAGATCGAATCTTGATTTTCACAGATAT ACTTGGCAAGGTCCGCCCTGTCATCAGCACATTCAAGCAGATCTCCATGG CAGCATTCCGTGTGGACTTAGGTAAGATCTGTCACTAACTTGGAAACTTC TGCAAACTCAGCTTAGGGAAATCTCTGGCTCAGGCGAGCTACTGCCTCAG CTTAGAAAGCTCTTTCTCCAAATTATTGGAGACTGGCACACTTAAGTCCC TGTTAGGCAGACGAAGCCTTCCCTTCATCCCGAAGTTCATCGAGCTTTGG CAACAGGCAGGCAGCTTTATCAGCAGCTTGGCAACATTCTGTAAAAGCA GCTTTATACCTTTAAGCAAAGAAAAGGAGTTCCGGGGCATAAAAGTAAG GATGTCTTCTGGCAATTTATAATAAGTATTTTTTCAAAAATGTCTCTTCAT TGTCATGAAAAGCAGTGCATCACACATCAACCTCTGGTCTCACCAATCGG GGGAGGTTTGGGTTGTTTACTTAGTGTTGCAAGAATTATTTTATTCTCTCA GGTTCTTGTTTTGCACAGCAGTCAGCTCATTCACCATAGGTTTCACGAAG AGTTGCTGCGGCCGCCTCGATAATACGACTCACTATAGGGTCGACGATAT CAGATCTAGGAACCCCTAGTGATGGAGTTGGCCACTCCCTCTCTGCGCGC TCGCTCGCTCACTGAGGCCGGGCGACCAAAGGTCGCCCGACGCCCGGGC TTTGCCCGGGCGGCCTCAGTGAGCGAGCGAGCGCGCAGCTGCCTGCAGG |
| 912 | AAGATGACGGTTTGTCACATGGAGTTGGCAGGATGTTTGATTAAAAACAT AACAGGAAGAAAAATGCCCCGCTGTGGGCGGACAAAATAGTTGGGAACT GGGAGGGGTGGAAATGGAGTTTTTAAGGATTATTTAGGGAAGAGTGACA AAATAGATGGGAACTGGGTGTAGCGTCGTAAGCTAATACGAAAATTAAA AATGACAAAATAGTTTGGAACTAGATTTCACTTATCTGGTTCGGATCTCC TAGGCGATATCAGTGATCACGGATCTCGACCAATTGACATTATTGAAGCA ACTAGTATCGATTTTATCAGGGTTATTGTCTCAGACCTGCAGGCAGCTGC GCGCTCGCTCGCTCACTGAGGCCGCCCGGGCAAAGCCCGGGCGTCGGGC GACCTTTGGTCGCCCGGCCTCAGTGAGCGAGCGAGCGCGCAGAGAGGGA GTGGCCAACTCCATCACTAGGGGTTCCTAGATCTGATATCGGCGCGCCCC TGGGCGCGCCCGAGTCCAACACCCGTGGGAATCCCATGGGCACCATGGC CCCTCGCTCCAAAAATGCTTTCGCGTCGCGCAGACACTGCTCGGTAGTTT CGGGGATCAGCGTTTGAGTAAGAGCCCGCGTCTGAACCCTCCGCGCCGC |
| | CCCGGCCCCAGTGGAAAGACGCGCAGGCAAAACGCACCACGTGACGGA GCGTGACCGCGCGCCGAGCGCGCGCCAAGGTCGGGCAGGAAGAGGGCCT ATTTCCCATGATTCCTTCATATTTGCATATACGATACAAGGCTGTTAGAG AGATAATTAGAATTAATTTGACTGTAAACACAAAGATATTAGTACAAAA TACGTGACGTAGAAAGTAATAATTTCTTGGGTAGTTTGCAGTTTTAAAAT TATGTTTTAAAATGGACTATCATATGCTTACCGTAACTTGAAAGTATTTC GATTTCTTGGCTTTATATATCTTGTGGAAAGGACGAAACACCGCACTAGA GTAACAGTCTGACGTTTAAGAGCTATGCTGGAAACAGCATAGCAAGTTT AAATAAGGCTAGTCCGTTATCAACTTGAAAAAGTGGCACCGAGTCGGTG CTTTTTTTGCGGCCGCGCTTCGGCGCGCCCATATTTGCATGTCGCTATGTG TTCTGGGAAATCACCATAAACGTGAAATGTCTTTGGATTTGGGAATCTTA TAAGTTCTGTATGAGACCACGGTACACCGCACTAGAGTAACAGTCTGAC GTTTAAGAGCTATGCTGGAAACAGCATAGCAAGTTTAAATAAGGCTAGT CCGTTATCAACTTGAAAAAGTGGCACCGAGTCGGTGCTTTTTTTGCGGCC GCTGACGGCGCGCCCTGCAGTATTTAGCATGCCCCACCCATCTGCAAGGC ATTCTGGATAGTGTCAAAACAGCCGGAAATCAAGTCCGTTTATCTCAAAC TTTAGCATTTTGGGAATAAATGATATTTGCTATGCTGGTTAAATTAGATTT TAGTTAAATTTCCTGCTGAAGCTCTAGTACGATAAGTAACTTGACCTAAG TGTAAAGTTGAGATTTCCTTCAGGTTTATATAGCTTGTGCGCCGCCTGGG TACACCGCACTAGAGTAACAGTCTGACGTTTAAGAGCTATGCTGGAAAC AGCATAGCAAGTTTAAATAAGGCTAGTCCGTTATCAACTTGAAAAAGTG GCACCGAGTCGGTGCTTTTTTTGCGGCCGCGCTGCGGCCGCCTCGAGTGA TCAAAAAAACCAACACACGCTTCCAATGAAAATAAACGATCCTTTATTGC TAGCCTTTACTTGTACAGCTCGTCCATGCCGAGAGTGATCCCGGCGGCGG TCACGAACTCCAGCAGGACCATGTGATCGCGCTTCTCGTTGGGGTCTTTG CTCAGGGCGGACTGGGTGCTCAGGTAGTGGTTGTCGGGCAGCAACACGG GGCCGTCGCCGATGGGGGTGTTCTGCTGGTAGTGGTCGGCGAGCTGCAC GCTGCCGTCCTCGATGTTGTGGCGGATCTTGAAGTTCACCTTGATGCCGT TCTTCTGCTTGTCGGCCATGATATAGACGTTGTGGCTGTTGTAGTTGTACT CCAGCTTGTGCCCCAGGATGTTGCCGTCCTCCTTGAAGTCGATGCCCTTC AGCTCGATGCGGTTCACCAGGGTGTCGCCCTCGAACTTCACCTCGGCGCG GGTCTTGTAGTTGCCGTCGTCCTTGAAGAAGATGGTGCGCTCCTGGACGT AGCCTTCGGGCATGGCGGACTTGAAGAAGTCGTGCTGCTTCATGTGGTCG GGGTAGCGGCTGAAGCACTGCACGCCGTAGGTCAGGGTGGTCACGAGGG TGGGCCAGGGCACGGGCAGCTTGCCGGTGGTGCAGATGAACTTCAGGGT CAGCTTGCCGTAGGTGGCATCGCCCTCGCCCTCGCCGGACACGCTGAACT TGTGGCCGTTTACGTCGCCGTCCAGCTCGACCAGGATGGGCACCACCCCG GTGAACAGCTCCTCGCCCTTGCTCACCATGGTGGCGACCGGTGGATCCCG GGCCCGCGGGGTGGCTTTACCAACAGTACCCGGAATGCCAAGCTTACTTA GATCGCAGTCTCGACGCTGGCTGGCTCCTGAGTGTCTGTCTGTGCTGTGG AGGTGGTGGTAGAATGAGGGCAGCCCCTGTGCCCCTGGGTTATATAGAG GAGCCTACAGGGTGTGACTAGCCAGGAGGGGCTGTCCCCAGGGAGGGGC CCCTGAGAGCAGATGAGCTTTCAGCTCGTTGCCCGGGCACCGTGCCCACC CCGGACCCAGGCGTGCAGCTTGCCCAGCCCCATGGCCTTGTATGGGCTGC CCCAAGGGCTGACTTGCTCACTGGTTCCTAAACTAAGTGCTGAGTCTAGC TGGCGGGGGACAGCTGGCCCTTCGCCGGGAACATGGCATGCAGGGTTAT TTTTAGAGGCAGCAGGTGTTGGGGGGGGGGGGGCAGCCACATGTCTGGG TTAATTATAACCAGGCATCTCGGGTGTCCCCAGGCCTTGCCTCCTTACAT GGGCACGTCGACGATATCAGATCTAGGAACCCCTAGTGATGGAGTTGGC CACTCCCTCTCTGCGCGCTCGCTCGCTCACTGAGGCCGGGCGACCAAAGG |
| | TCGCCCGACGCCCGGGCTTTGCCCGGGCGGCCTCAGTGAGCGAGCGAGC GCGCAGCTGCCTGCAGGCATGCAAGCTGTAGCCAACCACTAGAACTATA GCTAGAGTCCTGGGCGAACAAACGATGCTCGCCTTCCAGAAAACCGAGG ATGCGAACCACTTCATCCGGGGTCAGCACCACCGGCAAGCGCCGCGACG GCCGAGGTCTTCCGATCTCCTGAAGCCAGGGCAGATCCGTGCACAGCAC CTTGCCGTAGAAGAACAGCAAGGCCGCCAATGCCTGACGATGCGTGGAG ACCGAAACCTTGCGCTCGTTCGCCAGCCAGGACAGAAATGCCTCGACTTC GCTGCTGCCCAAGGTTGCCGGGTGACGCACACCGTGGAAACGGATGAAG GCACGAACCCAGTTGACATAAGCCTGTTCGGTTCGTAAACTGTAATGCAA GTAGCGTATGCGCTCACGCAACTGGTCCAGAACCTTGACCGAACGCAGC GGTGGTAACGGCGCAGTGGCGGTTTTCATGGCTTGTTATGACTGTTTTTTT GTACAGTCTATGCCTCGGGCATCCAAGCAGCAAGCGCGTTACGCCGTGG GTCGATGTTTGATGTTATGGAGCAGCAACGATGTTACGCAGCAGCAACG ATGTTACGCAGCAGGGCAGTCGCCCTAAAACAAAGTTAGGTGGCTCAAG TATGGGCATCATTCGCACATGTAGGCTCGGCCCTGACCAAGTCAAATCCA TGCGGGCTGCTCTTGATCTTTTCGGTCGTGAGTTCGGAGACGTAGCCACC TACTCCCAACATCAGCCGGACTCCGATTACCTCGGGAACTTGCTCCGTAG TAAGACATTCATCGCGCTTGCTGCCTTCGACCAAGAAGCGGTTGTTGGCG CTCTCGCGGCTTACGTTCTGCCCAAGTTTGAGCAGCCGCGTAGTGAGATC TATATCTATGATCTCGCAGTCTCCGGCGAGCACCGGAGGCAGGGCATTGC CACCGCGCTCATCAATCTCCTCAAGCATGAGGCCAACGCGCTTGGTGCTT ATGTGATCTACGTGCAAGCAGATTACGGTGACGATCCCGCAGTGGCTCTC TATACAAAGTTGGGCATACGGGAAGAAGTGATGCACTTTGATATCGACC CAAGTACCGCCACCTAACAATTCGTTCAAGCCGAGATCGGCTTCCCGGCC GCGGAGTTGTTCGGTAAATTGTCACAACGCCGCGAATATAGTCTTTACCA TGCCCTTGGCCACGCCCCTCTTTAATACGACGGGCAATTTGCACTTCAGA AAATGAAGAGTTTGCTTTAGCCATAACAAAAGTCCAGTATGCTTTTTCAC AGCATAACTGGACTGATTTCAGTTTACAACTATTCTGTCTAGTTTAAGAC TTTATTGTCATAGTTTAGATCTATTTTGTTCAGTTTAAGACTTTATTGTCC GCCCACACCCGCTTACGC |
| 920 | AAGATGACGGTTTGTCACATGGAGTTGGCAGGATGTTTGATTAAAAACAT AACAGGAAGAAAAATGCCCCGCTGTGGGCGGACAAAATAGTTGGGAACT GGGAGGGGTGGAAATGGAGTTTTTAAGGATTATTTAGGGAAGAGTGACA AAATAGATGGGAACTGGGTGTAGCGTCGTAAGCTAATACGAAAATTAAA AATGACAAAATAGTTTGGAACTAGATTTCACTTATCTGGTTCGGATCTCC TAGGCGATATCAGTGATCACGGATCTCGACCAATTGACATTATTGAAGCA ACTAGTATCGATTTTATCAGGGTTATTGTCTCAGACCTGCAGGCAGCTGC GCGCTCGCTCGCTCACTGAGGCCGCCCGGGCAAAGCCCGGGCGTCGGGC GACCTTTGGTCGCCCGGCCTCAGTGAGCGAGCGAGCGCGCAGAGAGGGA GTGGCCAACTCCATCACTAGGGGTTCCTAGATCTGATATCGGCGCGCCCC TGGGCGCGCCCGAGTCCAACACCCGTGGGAATCCCATGGGCACCATGGC CCCTCGCTCCAAAAATGCTTTCGCGTCGCGCAGACACTGCTCGGTAGTTT CGGGGATCAGCGTTTGAGTAAGAGCCCGCGTCTGAACCCTCCGCGCCGC CCCGGCCCCAGTGGAAAGACGCGCAGGCAAAACGCACCACGTGACGGA GCGTGACCGCGCGCCGAGCGCGCGCCAAGGTCGGGCAGGAAGAGGGCCT ATTTCCCATGATTCCTTCATATTTGCATATACGATACAAGGCTGTTAGAG AGATAATTAGAATTAATTTGACTGTAAACACAAAGATATTAGTACAAAA |
| | TACGTGACGTAGAAAGTAATAATTTCTTGGGTAGTTTGCAGTTTTAAAAT TATGTTTTAAAATGGACTATCATATGCTTACCGTAACTTGAAAGTATTTC GATTTCTTGGCTTTATATATCTTGTGGAAAGGACGAAACACCGCACCAGA GTAACAGTCTGACGTTTAAGAGCTATGCTGGAAACAGCATAGCAAGTTT AAATAAGGCTAGTCCGTTATCAACTTGAAAAAGTGGCACCGAGTCGGTG CTTTTTTTGCGGCCGCGCTCGGCGCGCCCATATTTGCATGTCGCTATGTGT TCTGGGAAATCACCATAAACGTGAAATGTCTTTGGATTTGGGAATCTTAT AAGTTCTGTATGAGACCACGGTACACCGCACCAGAGTAACAGTCTGACG TTTAAGAGCTATGCTGGAAACAGCATAGCAAGTTTAAATAAGGCTAGTC CGTTATCAACTTGAAAAAGTGGCACCGAGTCGGTGCTTTTTTTGCGGCCG CTGACGGCGCGCCCTGCAGTATTTAGCATGCCCCACCCATCTGCAAGGCA TTCTGGATAGTGTCAAAACAGCCGGAAATCAAGTCCGTTTATCTCAAACT TTAGCATTTTGGGAATAAATGATATTTGCTATGCTGGTTAAATTAGATTTT AGTTAAATTTCCTGCTGAAGCTCTAGTACGATAAGTAACTTGACCTAAGT GTAAAGTTGAGATTTCCTTCAGGTTTATATAGCTTGTGCGCCGCCTGGGT ACACCGCACCAGAGTAACAGTCTGACGTTTAAGAGCTATGCTGGAAACA GCATAGCAAGTTTAAATAAGGCTAGTCCGTTATCAACTTGAAAAAGTGG CACCGAGTCGGTGCTTTTTTTGCGGCCGCGCTGTTTAAACCAACAAAATC AGCAGCTAATGAAGGCAAGTCAGCAGGTCACTCATCATTTTCCACTTCGG CAATGCAGTGGGATTATTCCAACAGAGGTTTTTCACAGCATTCCTTCAGT TAACTGGAGATCGAATCTTGATTTTCACAGATATACTTGGCAAGGTCCGC CCTGTCATCAGCACATTCAAGCAGATCTCCATGGCAGCATTCCGTGTGGA CTTAGGTAAGATCTGTCACTAACTTGGAAACTTCTGCAAACTCAGCTTAG GGAAATCTCTGGCTCAGGCGAGCTACTGCCTCAGCTTAGAAAGCTCTTTC TCCAAATTATTGGAGACTGGCACACTTAAGTCCCTGTTAGGCAGACGAAG CCTTCCCTTCATCCCGAAGTTCATCGAGCTTTGGCAACAGGCAGGCAGCT TTATCAGCAGCTTGGCAACATTCTGTAAAAGCAGCTTTATACCTTTAAGC AAAGAAAAGGAGTTCCGGGGCATAAAAGTAAGGATGTCTTCTGGCAATT TATAATAAGTATTTTTTCAAAAATGTCTCTTCATTGTCATGAAAAGCAGT GCATCACACATCAACCTCTGGTCTCACCAATCGGGGGAGGTTTGGGTTGT TTACTTAGTGTTGCAAGAATTATTTTATTCTCTCAGGTTCTTGTTTTGCAC AGCAGTCAGCTCATTCACCATAGGTTTCACGAAGAGTTGCTGCGGCCGCC TCGATAATACGACTCACTATAGGGTCGACGATATCAGATCTAGGAACCCC TAGTGATGGAGTTGGCCACTCCCTCTCTGCGCGCTCGCTCGCTCACTGAG GCCGGGCGACCAAAGGTCGCCCGACGCCCGGGCTTTGCCCGGGCGGCCT CAGTGAGCGAGCGAGCGCGCAGCTGCCTGCAGGCATGCAAGCTGTAGCC AACCACTAGAACTATAGCTAGAGTCCTGGGCGAACAAACGATGCTCGCC TTCCAGAAAACCGAGGATGCGAACCACTTCATCCGGGGTCAGCACCACC GGCAAGCGCCGCGACGGCCGAGGTCTTCCGATCTCCTGAAGCCAGGGCA GATCCGTGCACAGCACCTTGCCGTAGAAGAACAGCAAGGCCGCCAATGC CTGACGATGCGTGGAGACCGAAACCTTGCGCTCGTTCGCCAGCCAGGAC AGAAATGCCTCGACTTCGCTGCTGCCCAAGGTTGCCGGGTGACGCACACC GTGGAAACGGATGAAGGCACGAACCCAGTTGACATAAGCCTGTTCGGTT CGTAAACTGTAATGCAAGTAGCGTATGCGCTCACGCAACTGGTCCAGAA CCTTGACCGAACGCAGCGGTGGTAACGGCGCAGTGGCGGTTTTCATGGCT TGTTATGACTGTTTTTTTGTACAGTCTATGCCTCGGGCATCCAAGCAGCA AGCGCGTTACGCCGTGGGTCGATGTTTGATGTTATGGAGCAGCAACGATG TTACGCAGCAGCAACGATGTTACGCAGCAGGGCAGTCGCCCTAAAACAA AGTTAGGTGGCTCAAGTATGGGCATCATTCGCACATGTAGGCTCGGCCCT GACCAAGTCAAATCCATGCGGGCTGCTCTTGATCTTTTCGGTCGTGAGTT |
| | CGGAGACGTAGCCACCTACTCCCAACATCAGCCGGACTCCGATTACCTCG GGAACTTGCTCCGTAGTAAGACATTCATCGCGCTTGCTGCCTTCGACCAA GAAGCGGTTGTTGGCGCTCTCGCGGCTTACGTTCTGCCCAAGTTTGAGCA GCCGCGTAGTGAGATCTATATCTATGATCTCGCAGTCTCCGGCGAGCACC GGAGGCAGGGCATTGCCACCGCGCTCATCAATCTCCTCAAGCATGAGGC CAACGCGCTTGGTGCTTATGTGATCTACGTGCAAGCAGATTACGGTGACG ATCCCGCAGTGGCTCTCTATACAAAGTTGGGCATACGGGAAGAAGTGAT GCACTTTGATATCGACCCAAGTACCGCCACCTAACAATTCGTTCAAGCCG AGATCGGCTTCCCGGCCGCGGAGTTGTTCGGTAAATTGTCACAACGCCGC GAATATAGTCTTTACCATGCCCTTGGCCACGCCCCTCTTTAATACGACGG GCAATTTGCACTTCAGAAAATGAAGAGTTTGCTTTAGCCATAACAAAAGT CCAGTATGCTTTTTCACAGCATAACTGGACTGATTTCAGTTTACAACTATT CTGTCTAGTTTAAGACTTTATTGTCATAGTTTAGATCTATTTTGTTCAGTT TAAGACTTTATTGTCCGCCCACACCCGCTTACGC |
| 916 | AAGATGACGGTTTGTCACATGGAGTTGGCAGGATGTTTGATTAAAAACAT AACAGGAAGAAAAATGCCCCGCTGTGGGCGGACAAAATAGTTGGGAACT GGGAGGGGTGGAAATGGAGTTTTTAAGGATTATTTAGGGAAGAGTGACA AAATAGATGGGAACTGGGTGTAGCGTCGTAAGCTAATACGAAAATTAAA AATGACAAAATAGTTTGGAACTAGATTTCACTTATCTGGTTCGGATCTCC TAGGCGATATCAGTGATCACGGATCTCGACCAATTGACATTATTGAAGCA ACTAGTATCGATTTTATCAGGGTTATTGTCTCAGACCTGCAGGCAGCTGC GCGCTCGCTCGCTCACTGAGGCCGCCCGGGCAAAGCCCGGGCGTCGGGC GACCTTTGGTCGCCCGGCCTCAGTGAGCGAGCGAGCGCGCAGAGAGGGA GTGGCCAACTCCATCACTAGGGGTTCCTAGATCTGATATCGGCGCGCCCC TGGGCGCGCCCGAGTCCAACACCCGTGGGAATCCCATGGGCACCATGGC CCCTCGCTCCAAAAATGCTTTCGCGTCGCGCAGACACTGCTCGGTAGTTT CGGGGATCAGCGTTTGAGTAAGAGCCCGCGTCTGAACCCTCCGCGCCGC CCCGGCCCCAGTGGAAAGACGCGCAGGCAAAACGCACCACGTGACGGA GCGTGACCGCGCGCCGAGCGCGCGCCAAGGTCGGGCAGGAAGAGGGCCT ATTTCCCATGATTCCTTCATATTTGCATATACGATACAAGGCTGTTAGAG AGATAATTAGAATTAATTTGACTGTAAACACAAAGATATTAGTACAAAA TACGTGACGTAGAAAGTAATAATTTCTTGGGTAGTTTGCAGTTTTAAAAT TATGTTTTAAAATGGACTATCATATGCTTACCGTAACTTGAAAGTATTTC GATTTCTTGGCTTTATATATCTTGTGGAAAGGACGAAACACCGCACCAGA GTAACAGTCTGAGGTTTAAGAGCTATGCTGGAAACAGCATAGCAAGTTT AAATAAGGCTAGTCCGTTATCAACTTGAAAAAGTGGCACCGAGTCGGTG CTTTTTTTGCGGCCGCGCTCGGCGCGCCCATATTTGCATGTCGCTATGTGT TCTGGGAAATCACCATAAACGTGAAATGTCTTTGGATTTGGGAATCTTAT AAGTTCTGTATGAGACCACGGTACACCGCACCAGAGTAACAGTCTGAGG TTTAAGAGCTATGCTGGAAACAGCATAGCAAGTTTAAATAAGGCTAGTC CGTTATCAACTTGAAAAAGTGGCACCGAGTCGGTGCTTTTTTTGCGGCCG CTGACGGCGCGCCCTGCAGTATTTAGCATGCCCCACCCATCTGCAAGGCA TTCTGGATAGTGTCAAAACAGCCGGAAATCAAGTCCGTTTATCTCAAACT TTAGCATTTTGGGAATAAATGATATTTGCTATGCTGGTTAAATTAGATTTT AGTTAAATTTCCTGCTGAAGCTCTAGTACGATAAGTAACTTGACCTAAGT GTAAAGTTGAGATTTCCTTCAGGTTTATATAGCTTGTGCGCCGCCTGGGT ACACCGCACCAGAGTAACAGTCTGAGGTTTAAGAGCTATGCTGGAAACA |
| | GCATAGCAAGTTTAAATAAGGCTAGTCCGTTATCAACTTGAAAAAGTGG CACCGAGTCGGTGCTTTTTTTGCGGCCGCGCTGTTTAAACCAACAAAATC AGCAGCTAATGAAGGCAAGTCAGCAGGTCACTCATCATTTTCCACTTCGG CAATGCAGTGGGATTATTCCAACAGAGGTTTTTCACAGCATTCCTTCAGT TAACTGGAGATCGAATCTTGATTTTCACAGATATACTTGGCAAGGTCCGC CCTGTCATCAGCACATTCAAGCAGATCTCCATGGCAGCATTCCGTGTGGA CTTAGGTAAGATCTGTCACTAACTTGGAAACTTCTGCAAACTCAGCTTAG GGAAATCTCTGGCTCAGGCGAGCTACTGCCTCAGCTTAGAAAGCTCTTTC TCCAAATTATTGGAGACTGGCACACTTAAGTCCCTGTTAGGCAGACGAAG CCTTCCCTTCATCCCGAAGTTCATCGAGCTTTGGCAACAGGCAGGCAGCT TTATCAGCAGCTTGGCAACATTCTGTAAAAGCAGCTTTATACCTTTAAGC AAAGAAAAGGAGTTCCGGGGCATAAAAGTAAGGATGTCTTCTGGCAATT TATAATAAGTATTTTTTCAAAAATGTCTCTTCATTGTCATGAAAAGCAGT GCATCACACATCAACCTCTGGTCTCACCAATCGGGGGAGGTTTGGGTTGT TTACTTAGTGTTGCAAGAATTATTTTATTCTCTCAGGTTCTTGTTTTGCAC AGCAGTCAGCTCATTCACCATAGGTTTCACGAAGAGTTGCTGCGGCCGCC TCGATAATACGACTCACTATAGGGTCGACGATATCAGATCTAGGAACCCC TAGTGATGGAGTTGGCCACTCCCTCTCTGCGCGCTCGCTCGCTCACTGAG GCCGGGCGACCAAAGGTCGCCCGACGCCCGGGCTTTGCCCGGGCGGCCT CAGTGAGCGAGCGAGCGCGCAGCTGCCTGCAGGCATGCAAGCTGTAGCC AACCACTAGAACTATAGCTAGAGTCCTGGGCGAACAAACGATGCTCGCC TTCCAGAAAACCGAGGATGCGAACCACTTCATCCGGGGTCAGCACCACC GGCAAGCGCCGCGACGGCCGAGGTCTTCCGATCTCCTGAAGCCAGGGCA GATCCGTGCACAGCACCTTGCCGTAGAAGAACAGCAAGGCCGCCAATGC CTGACGATGCGTGGAGACCGAAACCTTGCGCTCGTTCGCCAGCCAGGAC AGAAATGCCTCGACTTCGCTGCTGCCCAAGGTTGCCGGGTGACGCACACC GTGGAAACGGATGAAGGCACGAACCCAGTTGACATAAGCCTGTTCGGTT CGTAAACTGTAATGCAAGTAGCGTATGCGCTCACGCAACTGGTCCAGAA CCTTGACCGAACGCAGCGGTGGTAACGGCGCAGTGGCGGTTTTCATGGCT TGTTATGACTGTTTTTTTGTACAGTCTATGCCTCGGGCATCCAAGCAGCA AGCGCGTTACGCCGTGGGTCGATGTTTGATGTTATGGAGCAGCAACGATG TTACGCAGCAGCAACGATGTTACGCAGCAGGGCAGTCGCCCTAAAACAA AGTTAGGTGGCTCAAGTATGGGCATCATTCGCACATGTAGGCTCGGCCCT GACCAAGTCAAATCCATGCGGGCTGCTCTTGATCTTTTCGGTCGTGAGTT CGGAGACGTAGCCACCTACTCCCAACATCAGCCGGACTCCGATTACCTCG GGAACTTGCTCCGTAGTAAGACATTCATCGCGCTTGCTGCCTTCGACCAA GAAGCGGTTGTTGGCGCTCTCGCGGCTTACGTTCTGCCCAAGTTTGAGCA GCCGCGTAGTGAGATCTATATCTATGATCTCGCAGTCTCCGGCGAGCACC GGAGGCAGGGCATTGCCACCGCGCTCATCAATCTCCTCAAGCATGAGGC CAACGCGCTTGGTGCTTATGTGATCTACGTGCAAGCAGATTACGGTGACG ATCCCGCAGTGGCTCTCTATACAAAGTTGGGCATACGGGAAGAAGTGAT GCACTTTGATATCGACCCAAGTACCGCCACCTAACAATTCGTTCAAGCCG AGATCGGCTTCCCGGCCGCGGAGTTGTTCGGTAAATTGTCACAACGCCGC GAATATAGTCTTTACCATGCCCTTGGCCACGCCCCTCTTTAATACGACGG GCAATTTGCACTTCAGAAAATGAAGAGTTTGCTTTAGCCATAACAAAAGT CCAGTATGCTTTTTCACAGCATAACTGGACTGATTTCAGTTTACAACTATT CTGTCTAGTTTAAGACTTTATTGTCATAGTTTAGATCTATTTTGTTCAGTT TAAGACTTTATTGTCCGCCCACACCCGCTTACGC |
| | |
| 913 | AAGATGACGGTTTGTCACATGGAGTTGGCAGGATGTTTGATTAAAAACAT AACAGGAAGAAAAATGCCCCGCTGTGGGCGGACAAAATAGTTGGGAACT GGGAGGGGTGGAAATGGAGTTTTTAAGGATTATTTAGGGAAGAGTGACA AAATAGATGGGAACTGGGTGTAGCGTCGTAAGCTAATACGAAAATTAAA AATGACAAAATAGTTTGGAACTAGATTTCACTTATCTGGTTCGGATCTCC TAGGCGATATCAGTGATCACGGATCTCGACCAATTGACATTATTGAAGCA ACTAGTATCGATTTTATCAGGGTTATTGTCTCAGACCACTCCCTCTATGCG CGCTCGCTCACTCACTCGGCCCTGGAGACCAAAGGTCTCCAGACTGCCGG CCTCTGGCCGGCAGGGCCGAGTGAGTGAGCGAGCGCGCATAGAGGGAGT GGGTACCTCCATCATCTAGGTTTGCCAGATCTGATATCGGCGCGCCCCTG GGCGCGCCCGAGTCCAACACCCGTGGGAATCCCATGGGCACCATGGCCC CTCGCTCCAAAAATGCTTTCGCGTCGCGCAGACACTGCTCGGTAGTTTCG GGGATCAGCGTTTGAGTAAGAGCCCGCGTCTGAACCCTCCGCGCCGCCCC GGCCCCAGTGGAAAGACGCGCAGGCAAAACGCACCACGTGACGGAGCG TGACCGCGCGCCGAGCGCGCGCCAAGGTCGGGCAGGAAGAGGGCCTATT TCCCATGATTCCTTCATATTTGCATATACGATACAAGGCTGTTAGAGAGA TAATTAGAATTAATTTGACTGTAAACACAAAGATATTAGTACAAAATACG TGACGTAGAAAGTAATAATTTCTTGGGTAGTTTGCAGTTTTAAAATTATG TTTTAAAATGGACTATCATATGCTTACCGTAACTTGAAAGTATTTCGATTT CTTGGCTTTATATATCTTGTGGAAAGGACGAAACACCGCACTAGAGTAAC AGTCTGACGTTTAAGAGCTATGCTGGAAACAGCATAGCAAGTTTAAATA AGGCTAGTCCGTTATCAACTTGAAAAAGTGGCACCGAGTCGGTGCTTTTT TTGCGGCCGCGCTTCGGCGCGCCCATATTTGCATGTCGCTATGTGTTCTG GGAAATCACCATAAACGTGAAATGTCTTTGGATTTGGGAATCTTATAAGT TCTGTATGAGACCACGGTACACCGCACTAGAGTAACAGTCTGACGTTTAA GAGCTATGCTGGAAACAGCATAGCAAGTTTAAATAAGGCTAGTCCGTTA TCAACTTGAAAAAGTGGCACCGAGTCGGTGCTTTTTTTGCGGCCGCTGAC GGCGCGCCCTGCAGTATTTAGCATGCCCCACCCATCTGCAAGGCATTCTG GATAGTGTCAAAACAGCCGGAAATCAAGTCCGTTTATCTCAAACTTTAGC ATTTTGGGAATAAATGATATTTGCTATGCTGGTTAAATTAGATTTTAGTTA AATTTCCTGCTGAAGCTCTAGTACGATAAGTAACTTGACCTAAGTGTAAA GTTGAGATTTCCTTCAGGTTTATATAGCTTGTGCGCCGCCTGGGTACACC GCACTAGAGTAACAGTCTGACGTTTAAGAGCTATGCTGGAAACAGCATA GCAAGTTTAAATAAGGCTAGTCCGTTATCAACTTGAAAAAGTGGCACCG AGTCGGTGCTTTTTTTGCGGCCGCGCTGCGGCCGCCTCGAGTGATCAAAA AAACCAACACACGCTTCCAATGAAAATAAACGATCCTTTATTGCTAGCCT TTACTTGTACAGCTCGTCCATGCCGAGAGTGATCCCGGCGGCGGTCACGA ACTCCAGCAGGACCATGTGATCGCGCTTCTCGTTGGGGTCTTTGCTCAGG GCGGACTGGGTGCTCAGGTAGTGGTTGTCGGGCAGCAACACGGGGCCGT CGCCGATGGGGGTGTTCTGCTGGTAGTGGTCGGCGAGCTGCACGCTGCCG TCCTCGATGTTGTGGCGGATCTTGAAGTTCACCTTGATGCCGTTCTTCTGC TTGTCGGCCATGATATAGACGTTGTGGCTGTTGTAGTTGTACTCCAGCTT GTGCCCCAGGATGTTGCCGTCCTCCTTGAAGTCGATGCCCTTCAGCTCGA TGCGGTTCACCAGGGTGTCGCCCTCGAACTTCACCTCGGCGCGGGTCTTG TAGTTGCCGTCGTCCTTGAAGAAGATGGTGCGCTCCTGGACGTAGCCTTC GGGCATGGCGGACTTGAAGAAGTCGTGCTGCTTCATGTGGTCGGGGTAG CGGCTGAAGCACTGCACGCCGTAGGTCAGGGTGGTCACGAGGGTGGGCC |
| | AGGGCACGGGCAGCTTGCCGGTGGTGCAGATGAACTTCAGGGTCAGCTT GCCGTAGGTGGCATCGCCCTCGCCCTCGCCGGACACGCTGAACTTGTGGC CGTTTACGTCGCCGTCCAGCTCGACCAGGATGGGCACCACCCCGGTGAAC AGCTCCTCGCCCTTGCTCACCATGGTGGCGACCGGTGGATCCCGGGCCCG CGGGGTGGCTTTACCAACAGTACCCGGAATGCCAAGCTTACTTAGATCGC AGTCTCGACGCTGGCTGGCTCCTGAGTGTCTGTCTGTGCTGTGGAGGTGG TGGTAGAATGAGGGCAGCCCCTGTGCCCCTGGGTTATATAGAGGAGCCT ACAGGGTGTGACTAGCCAGGAGGGGCTGTCCCCAGGGAGGGGCCCCTGA GAGCAGATGAGCTTTCAGCTCGTTGCCCGGGCACCGTGCCCACCCCGGAC CCAGGCGTGCAGCTTGCCCAGCCCCATGGCCTTGTATGGGCTGCCCCAAG GGCTGACTTGCTCACTGGTTCCTAAACTAAGTGCTGAGTCTAGCTGGCGG GGGACAGCTGGCCCTTCGCCGGGAACATGGCATGCAGGGTTATTTTTAGA GGCAGCAGGTGTTGGGGGGGGGGGGGCAGCCACATGTCTGGGTTAATTA TAACCAGGCATCTCGGGTGTCCCCAGGCCTTGCCTCCTTACATGGGCACG TCGACGATATCAGATCTAGGAACCCCTAGTGATGGAGTTGGCCACTCCCT CTCTGCGCGCTCGCTCGCTCACTGAGGCCGCCCGGGCAAAGCCCGGGCGT CGGGCGACCTTTGGTCGCCCGGCCTCAGTGAGCGAGCGAGCGCGCAGAG AGGGAGTGGCATGCAAGCTGTAGCCAACCACTAGAACTATAGCTAGAGT CCTGGGCGAACAAACGATGCTCGCCTTCCAGAAAACCGAGGATGCGAAC CACTTCATCCGGGGTCAGCACCACCGGCAAGCGCCGCGACGGCCGAGGT CTTCCGATCTCCTGAAGCCAGGGCAGATCCGTGCACAGCACCTTGCCGTA GAAGAACAGCAAGGCCGCCAATGCCTGACGATGCGTGGAGACCGAAACC TTGCGCTCGTTCGCCAGCCAGGACAGAAATGCCTCGACTTCGCTGCTGCC CAAGGTTGCCGGGTGACGCACACCGTGGAAACGGATGAAGGCACGAACC CAGTTGACATAAGCCTGTTCGGTTCGTAAACTGTAATGCAAGTAGCGTAT GCGCTCACGCAACTGGTCCAGAACCTTGACCGAACGCAGCGGTGGTAAC GGCGCAGTGGCGGTTTTCATGGCTTGTTATGACTGTTTTTTTGTACAGTCT ATGCCTCGGGCATCCAAGCAGCAAGCGCGTTACGCCGTGGGTCGATGTTT GATGTTATGGAGCAGCAACGATGTTACGCAGCAGCAACGATGTTACGCA GCAGGGCAGTCGCCCTAAAACAAAGTTAGGTGGCTCAAGTATGGGCATC ATTCGCACATGTAGGCTCGGCCCTGACCAAGTCAAATCCATGCGGGCTGC TCTTGATCTTTTCGGTCGTGAGTTCGGAGACGTAGCCACCTACTCCCAAC ATCAGCCGGACTCCGATTACCTCGGGAACTTGCTCCGTAGTAAGACATTC ATCGCGCTTGCTGCCTTCGACCAAGAAGCGGTTGTTGGCGCTCTCGCGGC TTACGTTCTGCCCAAGTTTGAGCAGCCGCGTAGTGAGATCTATATCTATG ATCTCGCAGTCTCCGGCGAGCACCGGAGGCAGGGCATTGCCACCGCGCT CATCAATCTCCTCAAGCATGAGGCCAACGCGCTTGGTGCTTATGTGATCT ACGTGCAAGCAGATTACGGTGACGATCCCGCAGTGGCTCTCTATACAAA GTTGGGCATACGGGAAGAAGTGATGCACTTTGATATCGACCCAAGTACC GCCACCTAACAATTCGTTCAAGCCGAGATCGGCTTCCCGGCCGCGGAGTT GTTCGGTAAATTGTCACAACGCCGCGAATATAGTCTTTACCATGCCCTTG GCCACGCCCCTCTTTAATACGACGGGCAATTTGCACTTCAGAAAATGAAG AGTTTGCTTTAGCCATAACAAAAGTCCAGTATGCTTTTTCACAGCATAAC TGGACTGATTTCAGTTTACAACTATTCTGTCTAGTTTAAGACTTTATTGTC ATAGTTTAGATCTATTTTGTTCAGTTTAAGACTTTATTGTCCGCCCACACC CGCTTACGC |
| 921 | AAGATGACGGTTTGTCACATGGAGTTGGCAGGATGTTTGATTAAAAACAT AACAGGAAGAAAAATGCCCCGCTGTGGGCGGACAAAATAGTTGGGAACT GGGAGGGGTGGAAATGGAGTTTTTAAGGATTATTTAGGGAAGAGTGACA AAATAGATGGGAACTGGGTGTAGCGTCGTAAGCTAATACGAAAATTAAA AATGACAAAATAGTTTGGAACTAGATTTCACTTATCTGGTTCGGATCTCC TAGGCGATATCAGTGATCACGGATCTCGACCAATTGACATTATTGAAGCA ACTAGTATCGATTTTATCAGGGTTATTGTCTCAGACCACTCCCTCTATGCG CGCTCGCTCACTCACTCGGCCCTGGAGACCAAAGGTCTCCAGACTGCCGG CCTCTGGCCGGCAGGGCCGAGTGAGTGAGCGAGCGCGCATAGAGGGAGT GGGTACCTCCATCATCTAGGTTTGCCAGATCTGATATCGGCGCGCCCCTG GGCGCGCCCGAGTCCAACACCCGTGGGAATCCCATGGGCACCATGGCCC CTCGCTCCAAAAATGCTTTCGCGTCGCGCAGACACTGCTCGGTAGTTTCG GGGATCAGCGTTTGAGTAAGAGCCCGCGTCTGAACCCTCCGCGCCGCCCC GGCCCCAGTGGAAAGACGCGCAGGCAAAACGCACCACGTGACGGAGCG TGACCGCGCGCCGAGCGCGCGCCAAGGTCGGGCAGGAAGAGGGCCTATT TCCCATGATTCCTTCATATTTGCATATACGATACAAGGCTGTTAGAGAGA TAATTAGAATTAATTTGACTGTAAACACAAAGATATTAGTACAAAATACG TGACGTAGAAAGTAATAATTTCTTGGGTAGTTTGCAGTTTTAAAATTATG TTTTAAAATGGACTATCATATGCTTACCGTAACTTGAAAGTATTTCGATTT CTTGGCTTTATATATCTTGTGGAAAGGACGAAACACCGCACCAGAGTAAC AGTCTGACGTTTAAGAGCTATGCTGGAAACAGCATAGCAAGTTTAAATA AGGCTAGTCCGTTATCAACTTGAAAAAGTGGCACCGAGTCGGTGCTTTTT TTGCGGCCGCGCTCGGCGCGCCCATATTTGCATGTCGCTATGTGTTCTGG GAAATCACCATAAACGTGAAATGTCTTTGGATTTGGGAATCTTATAAGTT CTGTATGAGACCACGGTACACCGCACCAGAGTAACAGTCTGACGTTTAA GAGCTATGCTGGAAACAGCATAGCAAGTTTAAATAAGGCTAGTCCGTTA TCAACTTGAAAAAGTGGCACCGAGTCGGTGCTTTTTTTGCGGCCGCTGAC GGCGCGCCCTGCAGTATTTAGCATGCCCCACCCATCTGCAAGGCATTCTG GATAGTGTCAAAACAGCCGGAAATCAAGTCCGTTTATCTCAAACTTTAGC ATTTTGGGAATAAATGATATTTGCTATGCTGGTTAAATTAGATTTTAGTTA AATTTCCTGCTGAAGCTCTAGTACGATAAGTAACTTGACCTAAGTGTAAA GTTGAGATTTCCTTCAGGTTTATATAGCTTGTGCGCCGCCTGGGTACACC GCACCAGAGTAACAGTCTGACGTTTAAGAGCTATGCTGGAAACAGCATA GCAAGTTTAAATAAGGCTAGTCCGTTATCAACTTGAAAAAGTGGCACCG AGTCGGTGCTTTTTTTGCGGCCGCGCTGTTTAAACCAACAAAATCAGCAG CTAATGAAGGCAAGTCAGCAGGTCACTCATCATTTTCCACTTCGGCAATG CAGTGGGATTATTCCAACAGAGGTTTTTCACAGCATTCCTTCAGTTAACT GGAGATCGAATCTTGATTTTCACAGATATACTTGGCAAGGTCCGCCCTGT CATCAGCACATTCAAGCAGATCTCCATGGCAGCATTCCGTGTGGACTTAG GTAAGATCTGTCACTAACTTGGAAACTTCTGCAAACTCAGCTTAGGGAAA TCTCTGGCTCAGGCGAGCTACTGCCTCAGCTTAGAAAGCTCTTTCTCCAA ATTATTGGAGACTGGCACACTTAAGTCCCTGTTAGGCAGACGAAGCCTTC CCTTCATCCCGAAGTTCATCGAGCTTTGGCAACAGGCAGGCAGCTTTATC AGCAGCTTGGCAACATTCTGTAAAAGCAGCTTTATACCTTTAAGCAAAGA AAAGGAGTTCCGGGGCATAAAAGTAAGGATGTCTTCTGGCAATTTATAA TAAGTATTTTTTCAAAAATGTCTCTTCATTGTCATGAAAAGCAGTGCATC ACACATCAACCTCTGGTCTCACCAATCGGGGGAGGTTTGGGTTGTTTACT TAGTGTTGCAAGAATTATTTTATTCTCTCAGGTTCTTGTTTTGCACAGCAG TCAGCTCATTCACCATAGGTTTCACGAAGAGTTGCTGCGGCCGCCTCGAT AATACGACTCACTATAGGGTCGACGATATCAGATCTAGGAACCCCTAGT |
| | GATGGAGTTGGCCACTCCCTCTCTGCGCGCTCGCTCGCTCACTGAGGCCG CCCGGGCAAAGCCCGGGCGTCGGGCGACCTTTGGTCGCCCGGCCTCAGT GAGCGAGCGAGCGCGCAGAGAGGGAGTGGCATGCAAGCTGTAGCCAAC CACTAGAACTATAGCTAGAGTCCTGGGCGAACAAACGATGCTCGCCTTCC AGAAAACCGAGGATGCGAACCACTTCATCCGGGGTCAGCACCACCGGCA AGCGCCGCGACGGCCGAGGTCTTCCGATCTCCTGAAGCCAGGGCAGATC CGTGCACAGCACCTTGCCGTAGAAGAACAGCAAGGCCGCCAATGCCTGA CGATGCGTGGAGACCGAAACCTTGCGCTCGTTCGCCAGCCAGGACAGAA ATGCCTCGACTTCGCTGCTGCCCAAGGTTGCCGGGTGACGCACACCGTGG AAACGGATGAAGGCACGAACCCAGTTGACATAAGCCTGTTCGGTTCGTA AACTGTAATGCAAGTAGCGTATGCGCTCACGCAACTGGTCCAGAACCTTG ACCGAACGCAGCGGTGGTAACGGCGCAGTGGCGGTTTTCATGGCTTGTTA TGACTGTTTTTTTGTACAGTCTATGCCTCGGGCATCCAAGCAGCAAGCGC GTTACGCCGTGGGTCGATGTTTGATGTTATGGAGCAGCAACGATGTTACG CAGCAGCAACGATGTTACGCAGCAGGGCAGTCGCCCTAAAACAAAGTTA GGTGGCTCAAGTATGGGCATCATTCGCACATGTAGGCTCGGCCCTGACCA AGTCAAATCCATGCGGGCTGCTCTTGATCTTTTCGGTCGTGAGTTCGGAG ACGTAGCCACCTACTCCCAACATCAGCCGGACTCCGATTACCTCGGGAAC TTGCTCCGTAGTAAGACATTCATCGCGCTTGCTGCCTTCGACCAAGAAGC GGTTGTTGGCGCTCTCGCGGCTTACGTTCTGCCCAAGTTTGAGCAGCCGC GTAGTGAGATCTATATCTATGATCTCGCAGTCTCCGGCGAGCACCGGAGG CAGGGCATTGCCACCGCGCTCATCAATCTCCTCAAGCATGAGGCCAACGC GCTTGGTGCTTATGTGATCTACGTGCAAGCAGATTACGGTGACGATCCCG CAGTGGCTCTCTATACAAAGTTGGGCATACGGGAAGAAGTGATGCACTTT GATATCGACCCAAGTACCGCCACCTAACAATTCGTTCAAGCCGAGATCG GCTTCCCGGCCGCGGAGTTGTTCGGTAAATTGTCACAACGCCGCGAATAT AGTCTTTACCATGCCCTTGGCCACGCCCCTCTTTAATACGACGGGCAATT TGCACTTCAGAAAATGAAGAGTTTGCTTTAGCCATAACAAAAGTCCAGTA TGCTTTTTCACAGCATAACTGGACTGATTTCAGTTTACAACTATTCTGTCT AGTTTAAGACTTTATTGTCATAGTTTAGATCTATTTTGTTCAGTTTAAGAC TTTATTGTCCGCCCACACCCGCTTACGC |
| 917 | AAGATGACGGTTTGTCACATGGAGTTGGCAGGATGTTTGATTAAAAACAT AACAGGAAGAAAAATGCCCCGCTGTGGGCGGACAAAATAGTTGGGAACT GGGAGGGGTGGAAATGGAGTTTTTAAGGATTATTTAGGGAAGAGTGACA AAATAGATGGGAACTGGGTGTAGCGTCGTAAGCTAATACGAAAATTAAA AATGACAAAATAGTTTGGAACTAGATTTCACTTATCTGGTTCGGATCTCC TAGGCGATATCAGTGATCACGGATCTCGACCAATTGACATTATTGAAGCA ACTAGTATCGATTTTATCAGGGTTATTGTCTCAGACCACTCCCTCTATGCG CGCTCGCTCACTCACTCGGCCCTGGAGACCAAAGGTCTCCAGACTGCCGG CCTCTGGCCGGCAGGGCCGAGTGAGTGAGCGAGCGCGCATAGAGGGAGT GGGTACCTCCATCATCTAGGTTTGCCAGATCTGATATCGGCGCGCCCCTG GGCGCGCCCGAGTCCAACACCCGTGGGAATCCCATGGGCACCATGGCCC CTCGCTCCAAAAATGCTTTCGCGTCGCGCAGACACTGCTCGGTAGTTTCG GGGATCAGCGTTTGAGTAAGAGCCCGCGTCTGAACCCTCCGCGCCGCCCC GGCCCCAGTGGAAAGACGCGCAGGCAAAACGCACCACGTGACGGAGCG TGACCGCGCGCCGAGCGCGCGCCAAGGTCGGGCAGGAAGAGGGCCTATT TCCCATGATTCCTTCATATTTGCATATACGATACAAGGCTGTTAGAGAGA |
| | TAATTAGAATTAATTTGACTGTAAACACAAAGATATTAGTACAAAATACG TGACGTAGAAAGTAATAATTTCTTGGGTAGTTTGCAGTTTTAAAATTATG TTTTAAAATGGACTATCATATGCTTACCGTAACTTGAAAGTATTTCGATTT CTTGGCTTTATATATCTTGTGGAAAGGACGAAACACCGCACCAGAGTAAC AGTCTGAGGTTTAAGAGCTATGCTGGAAACAGCATAGCAAGTTTAAATA AGGCTAGTCCGTTATCAACTTGAAAAAGTGGCACCGAGTCGGTGCTTTTT TTGCGGCCGCGCTCGGCGCGCCCATATTTGCATGTCGCTATGTGTTCTGG GAAATCACCATAAACGTGAAATGTCTTTGGATTTGGGAATCTTATAAGTT CTGTATGAGACCACGGTACACCGCACCAGAGTAACAGTCTGAGGTTTAA GAGCTATGCTGGAAACAGCATAGCAAGTTTAAATAAGGCTAGTCCGTTA TCAACTTGAAAAAGTGGCACCGAGTCGGTGCTTTTTTTGCGGCCGCTGAC GGCGCGCCCTGCAGTATTTAGCATGCCCCACCCATCTGCAAGGCATTCTG GATAGTGTCAAAACAGCCGGAAATCAAGTCCGTTTATCTCAAACTTTAGC ATTTTGGGAATAAATGATATTTGCTATGCTGGTTAAATTAGATTTTAGTTA AATTTCCTGCTGAAGCTCTAGTACGATAAGTAACTTGACCTAAGTGTAAA GTTGAGATTTCCTTCAGGTTTATATAGCTTGTGCGCCGCCTGGGTACACC GCACCAGAGTAACAGTCTGAGGTTTAAGAGCTATGCTGGAAACAGCATA GCAAGTTTAAATAAGGCTAGTCCGTTATCAACTTGAAAAAGTGGCACCG AGTCGGTGCTTTTTTTGCGGCCGCGCTGTTTAAACCAACAAAATCAGCAG CTAATGAAGGCAAGTCAGCAGGTCACTCATCATTTTCCACTTCGGCAATG CAGTGGGATTATTCCAACAGAGGTTTTTCACAGCATTCCTTCAGTTAACT GGAGATCGAATCTTGATTTTCACAGATATACTTGGCAAGGTCCGCCCTGT CATCAGCACATTCAAGCAGATCTCCATGGCAGCATTCCGTGTGGACTTAG GTAAGATCTGTCACTAACTTGGAAACTTCTGCAAACTCAGCTTAGGGAAA TCTCTGGCTCAGGCGAGCTACTGCCTCAGCTTAGAAAGCTCTTTCTCCAA ATTATTGGAGACTGGCACACTTAAGTCCCTGTTAGGCAGACGAAGCCTTC CCTTCATCCCGAAGTTCATCGAGCTTTGGCAACAGGCAGGCAGCTTTATC AGCAGCTTGGCAACATTCTGTAAAAGCAGCTTTATACCTTTAAGCAAAGA AAAGGAGTTCCGGGGCATAAAAGTAAGGATGTCTTCTGGCAATTTATAA TAAGTATTTTTTCAAAAATGTCTCTTCATTGTCATGAAAAGCAGTGCATC ACACATCAACCTCTGGTCTCACCAATCGGGGGAGGTTTGGGTTGTTTACT TAGTGTTGCAAGAATTATTTTATTCTCTCAGGTTCTTGTTTTGCACAGCAG TCAGCTCATTCACCATAGGTTTCACGAAGAGTTGCTGCGGCCGCCTCGAT AATACGACTCACTATAGGGTCGACGATATCAGATCTAGGAACCCCTAGT GATGGAGTTGGCCACTCCCTCTCTGCGCGCTCGCTCGCTCACTGAGGCCG CCCGGGCAAAGCCCGGGCGTCGGGCGACCTTTGGTCGCCCGGCCTCAGT GAGCGAGCGAGCGCGCAGAGAGGGAGTGGCATGCAAGCTGTAGCCAAC CACTAGAACTATAGCTAGAGTCCTGGGCGAACAAACGATGCTCGCCTTCC AGAAAACCGAGGATGCGAACCACTTCATCCGGGGTCAGCACCACCGGCA AGCGCCGCGACGGCCGAGGTCTTCCGATCTCCTGAAGCCAGGGCAGATC CGTGCACAGCACCTTGCCGTAGAAGAACAGCAAGGCCGCCAATGCCTGA CGATGCGTGGAGACCGAAACCTTGCGCTCGTTCGCCAGCCAGGACAGAA ATGCCTCGACTTCGCTGCTGCCCAAGGTTGCCGGGTGACGCACACCGTGG AAACGGATGAAGGCACGAACCCAGTTGACATAAGCCTGTTCGGTTCGTA AACTGTAATGCAAGTAGCGTATGCGCTCACGCAACTGGTCCAGAACCTTG ACCGAACGCAGCGGTGGTAACGGCGCAGTGGCGGTTTTCATGGCTTGTTA TGACTGTTTTTTTGTACAGTCTATGCCTCGGGCATCCAAGCAGCAAGCGC GTTACGCCGTGGGTCGATGTTTGATGTTATGGAGCAGCAACGATGTTACG CAGCAGCAACGATGTTACGCAGCAGGGCAGTCGCCCTAAAACAAAGTTA GGTGGCTCAAGTATGGGCATCATTCGCACATGTAGGCTCGGCCCTGACCA |
| | AGTCAAATCCATGCGGGCTGCTCTTGATCTTTTCGGTCGTGAGTTCGGAG ACGTAGCCACCTACTCCCAACATCAGCCGGACTCCGATTACCTCGGGAAC TTGCTCCGTAGTAAGACATTCATCGCGCTTGCTGCCTTCGACCAAGAAGC GGTTGTTGGCGCTCTCGCGGCTTACGTTCTGCCCAAGTTTGAGCAGCCGC GTAGTGAGATCTATATCTATGATCTCGCAGTCTCCGGCGAGCACCGGAGG CAGGGCATTGCCACCGCGCTCATCAATCTCCTCAAGCATGAGGCCAACGC GCTTGGTGCTTATGTGATCTACGTGCAAGCAGATTACGGTGACGATCCCG CAGTGGCTCTCTATACAAAGTTGGGCATACGGGAAGAAGTGATGCACTTT GATATCGACCCAAGTACCGCCACCTAACAATTCGTTCAAGCCGAGATCG GCTTCCCGGCCGCGGAGTTGTTCGGTAAATTGTCACAACGCCGCGAATAT AGTCTTTACCATGCCCTTGGCCACGCCCCTCTTTAATACGACGGGCAATT TGCACTTCAGAAAATGAAGAGTTTGCTTTAGCCATAACAAAAGTCCAGTA TGCTTTTTCACAGCATAACTGGACTGATTTCAGTTTACAACTATTCTGTCT AGTTTAAGACTTTATTGTCATAGTTTAGATCTATTTTGTTCAGTTTAAGAC TTTATTGTCCGCCCACACCCGCTTACGC |

In some embodiments of the gene editing constructs of the disclosure, including those embodiments encompassing SEQ ID NOs: 910 to 921, the construct comprises or consists of a first promoter, a first sequence encoding a gRNA, a second promoter, and a second sequence encoding a gRNA, a third promoter, and a third sequence encoding a gRNA. In some embodiments, the sequence encoding the gRNA is CACTAGAGTAACAGTCTGAC (SEQ ID NO. 708). In some embodiments, the sequence encoding the gRNA is CACCAGAGTAACAGTCTGAG (SEQ ID NO. 714). In some embodiments, the construct comprises, from 5' to 3', a first promoter, the sequence encoding a gRNA of SEQ ID NO. 708, a second promoter, a second sequence encoding a gRNA of SEQ ID NO. 708, a third promoter, and a third sequence encoding a gRNA of SEQ ID NO. 708. In some embodiments, the construct comprises, from 5' to 3', a first promoter, the sequence encoding a gRNA of SEQ ID NO. 714, a second promoter, a second sequence encoding a gRNA of SEQ ID NO. 714, a third promoter, and a third sequence encoding a gRNA of SEQ ID NO. 714. Exemplary promoters of the disclosure include the U6 promoter having a sequence of CGAGTCCAACACCCGTGGGAATCCCATGGGCACCATGGCCCCTCGCTCCAAAAA TGCTTTCGCGTCGCGCAGACACTGCTCGGTAGTTTCGGGGATCAGCGTTTGAGTA AGAGCCCGCGTCTGAACCCTCCGCGCCGCCCCGGCCCCAGTGGAAAGACGCGCA GGCAAAACGCACCACGTGACGGAGCGTGACCGCGCGCCGAGCGCGCGCCAAGGT CGGGCAGGAAGAGGGCCTATTTCCCATGATTCCTTCATATTTGCATATACGATAC AAGGCTGTTAGAGAGATAATTAGAATTAATTTGACTGTAAACACAAAGATATTA GTACAAAATACGTGACGTAGAAAGTAATAATTTCTTGGGTAGTTTGCAGTTTTAA AATTATGTTTTAAAATGGACTATCATATGCTTACCGTAACTTGAAAGTATTTCGAT TTCTTGGCTTTATATATCTTGTGGAAAGGACGAAA (SEQ ID NO: 922), the H1 promoter having a sequence of GCTCGGCGCGCCCATATTTGCATGTCGCTATGTGTTCTGGGAAATCACCATAAAC GTGAAATGTCTTTGGATTTGGGAATCTTATAAGTTCTGTATGAGACCACGGTA (SEQ ID. No923), and the 7SK promoter having a sequence of TGACGGCGCGCCCTGCAGTATTTAGCATGCCCCACCCATCTGCAAGGCATTCTGG ATAGTGTCAAAACAGCCGGAAATCAAGTCCGTTTATCTCAAACTTTAGCATTTTG GGAATAAATGATATTTGCTATGCTGGTTAAATTAGATTTTAGTTAAATTTCCTGCT GAAGCTCTAGTACGATAAGTAACTTGACCTAAGTGTAAAGTTGAGATTTCCTTCA GGTTTATATAGCTTGTGCGCCGCCTGGGTA (SEQ ID NO. 924). In some embodiments, the first, second, and third promoter are each individually selected from the U6 promoter (SEQ ID NO: 922), the H1 promoter (SEQ ID NO: 923), and the 7SK promoter (SEQ ID NO: 924). In some embodiments, the first, second, and third promoter are each individually selected from the U6 promoter (SEQ ID NO: 922), and the H1 promoter (SEQ ID NO: 923). In some embodiments, the construct comprises, from 5' to 3', a U6 promoter, a first sequence encoding a gRNA, a H1 promoter, a second sequence encoding a gRNA, a 7SK promoter, and a third sequence encoding a gRNA. In some embodiments, the construct comprises, from 5' to 3', the U6 promoter, the sequence encoding a gRNA of SEQ ID NO: 708, the H1 promoter, a second sequence encoding a gRNA of SEQ ID NO: 708, the 7SK promoter, and a third sequence encoding a gRNA of SEQ ID NO: 708. In some embodiments, the construct comprises, from 5' to 3', the U6 promoter, the sequence encoding a gRNA of SEQ ID NO: 714, the H1 promoter, a second sequence encoding a gRNA of SEQ ID NO: 714, the 7SK promoter, and a third sequence encoding a gRNA of SEQ ID NO: 714. In some embodiments, the construct comprising a first promoter, a first sequence encoding a gRNA, a second promoter, and a second sequence encoding a gRNA, a third promoter, and a third sequence encoding a gRNA further comprises at least two inverted terminal repeat (ITR) sequences. In some embodiments, the construct comprising a first promoter, a first sequence encoding a gRNA, a second promoter, and a second sequence encoding a gRNA, a third promoter, and a third sequence encoding a gRNA further comprises at least two ITR sequences isolated or derived from an AAV of serotype 2 (AAV2). In some embodiments, the construct comprising a first promoter, a first sequence encoding a gRNA, a second promoter, and a second sequence encoding a gRNA, a third promoter, and a third sequence encoding a gRNA further comprises at least two ITR sequences, wherein the first ITR sequence is isolated or derived from an AAV of serotype 4 (AAV4) and the second ITR sequence is isolated or derived from an AAV of serotype 2 (AAV2). Exemplary ITR sequences are CCTGCAGGCAGCTGCGCGCTCGCTCGCTCACTGAGGCCGCCCGGGCAAAGCCCG GGCGTCGGGCGACCTTTGGTCGCCCGGCCTCAGTGAGCGAGCGAGCGCGCAGAG AGGGAGTGGCCAACTCCATCACTAGGGGTTCCT (SEQ ID NO: 881), CCACTCCCTCTATGCGCGCTCGCTCACTCACTCGGCCCTGGAGACCAAAGGTCTC CAGACTGCCGGCCTCTGGCCGGCAGGGCCGAGTGAGTGAGCGAGCGCGCATAGA GGGAGTGGGTACCTCCATCATCTAGGTTTGCC (SEQ ID NO. 883), AGGAACCCCTAGTGATGGAGTTGGCCACTCCCTCTCTGCGCGCTCGCTCGCTCAC TGAGGCCGCCCGGGCAAAGCCCGGGCGTCGGGCGACCTTTGGTCGCCCGGCCTC AGTGAGCGAGCGAGCGCGCAGAGAGGGAGTGG (SEQ ID NO. 946), and AGGAACCCCTAGTGATGGAGTTGGCCACTCCCTCTCTGCGCGCTCGCTCGCTCAC TGAGGCCGGGCGACCAAAGGTCGCCCGACGCCCGGGCTTTGCCCGGGCGGCCTC AGTGAGCGAGCGAGCGCGCAGCTGCCTGCAGG (SEQ ID 882). In some embodiments, the construct comprises or consists of, from 5' to 3', a first ITR, a first promoter, a first sequence encoding a gRNA, a second promoter, and a second sequence encoding a gRNA, a third promoter, a third sequence encoding a gRNA, and a second ITR. In some embodiments, the construct comprises or consists of, from 5' to 3', a first ITR, a U6 promoter, a first sequence encoding a gRNA, a H1 promoter, and a second sequence encoding a gRNA, a 7SK promoter, a third sequence encoding a gRNA, and a second ITR. In some embodiments, the construct comprises or consists of, from 5' to 3', a first ITR, a first promoter, the sequence encoding a gRNA of SEQ ID NO: 708, a second promoter, and the sequence encoding a gRNA of SEQ ID NO: 708, a third promoter, the sequence encoding a gRNA of SEQ ID NO: 708, and a second ITR. In some embodiments, the construct comprises or consists of, from 5' to 3', a first ITR, a first promoter, the sequence encoding a gRNA of SEQ ID NO: 714, a second promoter, and the sequence encoding a gRNA of SEQ ID NO: 714, a third promoter, the sequence encoding a gRNA of SEQ ID NO: 714, and a second ITR. In some embodiments, the construct comprises or consists of, from 5' to 3', a first ITR, a U6, the sequence encoding a gRNA of SEQ ID NO: 708, a H1 promoter, and the sequence encoding a gRNA of SEQ ID NO: 708, a 7SK promoter, the sequence encoding a gRNA of SEQ ID NO: 708, and a second ITR. In some embodiments, the construct comprises or consists of, from 5' to 3', a first ITR, a U6, the sequence encoding a gRNA of SEQ ID NO: 714, a H1 promoter, and the sequence encoding a gRNA of SEQ ID NO: 714, a 7SK promoter, the sequence encoding a gRNA of SEQ ID NO: 714, and a second ITR. In some embodiments, the construct comprising, from 5' to 3' a first ITR, a first promoter, a first sequence encoding a gRNA, a second promoter, and a second sequence encoding a gRNA, a third promoter, a third sequence encoding a gRNA, and a second ITR, further comprises a poly A sequence. In some embodiments, the polyA sequence comprises or consists of a minipolyA sequence. Exemplary minipolyA sequences of the disclosure comprise or consist of a nucleotide sequence of TAGCAATAAAGGATCGTTTATTTTCATTGGAAGCGTGTGTTGGTTTTTTGATCAGG CGCG (SEQ ID NO: 903). In some embodiments, the construct comprises or consists of, from 5' to 3', a first ITR, a first promoter, a first sequence encoding a gRNA, a second promoter, and a second sequence encoding s gRNA, a third promoter, a third sequence encoding a gRNA, a minipolyA sequence, and a second ITR. In some embodiments, the construct comprises or consists of, from 5' to 3' a first ITR, the U6 promoter, a first sequence encoding a gRNA, the H1 promoter, a second sequence encoding a gRNA, the 7SK promoter, a third sequence encoding a gRNA, a minipolyA sequence, and a second ITR. In some embodiments, the construct comprises or consists of, from 5' to 3' a first ITR, a first promoter, the sequence encoding a gRNA of SEQ ID NO: 708, a second promoter, the sequence encoding a gRNA of SEQ ID NO: 708, a third promoter, the sequence encoding a gRNA of SEQ ID NO: 708, a minipolyA sequence, and a second ITR. In some embodiments, the construct comprises or consists of, from 5' to 3' a first ITR, a first promoter, the sequence encoding a gRNA of SEQ ID NO: 714, a second promoter, the sequence encoding a gRNA of SEQ ID NO. 714, a third promoter, the sequence encoding a gRNA of SEQ ID NO: 714, a minipolyA sequence, and a second ITR. In some embodiments, the construct comprises or consists of, from 5' to 3' a first ITR, the U6 promoter, the sequence encoding a gRNA of SEQ ID NO: 708, the H1 promoter, the sequence encoding a gRNA of SEQ ID NO: 708, the 7SK promoter, the sequence encoding a gRNA of SEQ ID NO: 708, a minipolyA sequence, and a second ITR. In some embodiments, the construct comprises or consists of, from 5' to 3' a first ITR, the U6 promoter, the sequence encoding a gRNA of SEQ ID NO: 714, the H1 promoter, the sequence encoding a gRNA of SEQ ID NO: 714, the 7SK promoter, the sequence encoding a gRNA of SEQ ID NO: 714, a minipolyA sequence, and a second ITR. In some embodiments, the construct comprising, from 5' to 3' a first ITR, a first promoter, a first sequence encoding a gRNA, a second promoter, and a second sequence encoding a gRNA, a third promoter, a third sequence encoding a gRNA, a minipolyA sequence, and a second ITR further comprises transposable element inverted repeats. Exemplary transposable element inverted repeats of the disclosure comprise or consist of a nucleotide sequence of TGTGGGCGGACAAAATAGTTGGGAACTGGGAGGGGTGGAAATGGAGTTTTTAAG GATTATTTAGGGAAGAGTGACAAAATAGATGGGAACTGGGTGTAGCGTCGTAAG CTAATACGAAAATTAAAAATGACAAAATAGTTTGGAACTAGATTTCACTTATCTG GTT (SEQ ID NO: 907) and/or a nucleotide sequence of GAATATAGTCTTTACCATGCCCTTGGCCACGCCCCTCTTTAATACGACGGGCAAT TTGCACTTCAGAAAATGAAGAGTTTGCTTTAGCCATAACAAAAGTCCAGTATGCT TTTTCACAGCATAACTGGACTGATTTCAGTTTACAACTATTCTGTCTAGTTTAAGA CTTTATTGTCATAGTTTAGATCTATTTTGTTCAGTTTAAGACTTTATTGTCCGCCCA CA (SEQ ID NO: 908). In some embodiments, the construct comprises or consists of, from 5' to 3', a first transposable element inverted repeat, a first ITR, a first promoter, a first sequence encoding a gRNA, a second promoter, a second sequence encoding a gRNA, a third promoter, a third sequence encoding a gRNA, a minipolyA sequence, a second ITR, and a second transposable element inverted repeat. In some embodiments, the construct comprises or consists of, from 5' to 3', a first transposable element inverted repeat, a first ITR, the U6 promoter, a first sequence encoding a gRNA, the H1 promoter, a second sequence encoding a gRNA, the 7SK promoter, a third sequence encoding a gRNA, a minipolyA sequence, a second ITR, and a second transposable element inverted repeat. In some embodiments, the construct comprises or consists of, from 5' to 3', a first transposable element inverted repeat, a first ITR, a first promoter, the sequence encoding a gRNA of SEQ ID NO: 708, a second promoter, the sequence encoding a gRNA of SEQ ID NO: 708, a third promoter, the sequence encoding a gRNA of SEQ ID NO: 708, a minipolyA sequence, a second ITR, and a second transposable element inverted repeat. In some embodiments, the construct comprises or consists of, from 5' to 3', a first transposable element inverted repeat, a first ITR, a first promoter, the sequence encoding a gRNA of SEQ ID NO: 714, a second promoter, the sequence encoding a gRNA of SEQ ID NO: 714, a third promoter, the sequence encoding a gRNA of SEQ ID NO: 714, a minipolyA sequence, a second ITR, and a second transposable element inverted repeat. In some embodiments, the construct comprises or consists of, from 5' to 3', a first transposable element inverted repeat, a first ITR, the U6 promoter, the sequence encoding a gRNA of SEQ ID NO: 708, the H1 promoter, the sequence encoding a gRNA of SEQ ID NO: 708, the 7SK promoter, the sequence encoding a gRNA of SEQ ID NO: 708, a minipolyA sequence, a second ITR, and a second transposable element inverted repeat. In some embodiments, the construct comprises or consists of, from 5' to 3', a first transposable element inverted repeat, a first ITR, the U6 promoter, the sequence encoding a gRNA of SEQ ID NO: 714, the H1 promoter, the sequence encoding a gRNA of SEQ ID NO: 714, the 7SK promoter, the sequence encoding a gRNA of SEQ ID NO: 714, a minipolyA sequence, a second ITR, and a second transposable element inverted repeat. In some embodiments, the construct comprising a first transposable element inverted repeat, a first ITR, a first promoter, a first sequence encoding a gRNA, a second promoter, a second sequence encoding a gRNA, a third promoter, a third sequence encoding a gRNA, a minipolyA sequence, a second ITR, a second transposable element inverted repeat, further comprises a regulatory sequence. Exemplary regulatory sequences of the disclosure comprise or consist of a nucleotide sequence of CATGCAAGCTGTAGCCAACCACTAGAACTATAGCTAGAGTCCTGGGCGAACAAA CGATGCTCGCCTTCCAGAAAACCGAGGATGCGAACCACTTCATCCGGGGTCAGC ACCACCGGCAAGCGCCGCGACGGCCGAGGTCTTCCGATCTCCTGAAGCCAGGGC AGATCCGTGCACAGCACCTTGCCGTAGAAGAACAGCAAGGCCGCCAATGCCTGA CGATGCGTGGAGACCGAAACCTTGCGCTCGTTCGCCAGCCAGGACAGAAATGCC TCGACTTCGCTGCTGCCCAAGGTTGCCGGGTGACGCACACCGTGGAAACGGATG AAGGCACGAACCCAGTTGACATAAGCCTGTTCGGTTCGTAAACTGTAATGCAAGT AGCGTATGCGCTCACGCAACTGGTCCAGAACCTTGACCGAACGCAGCGGTGGTA ACGGCGCAGTGGCGGTTTTCATGGCTTGTTATGACTGTTTTTTTGTACAGTCTATG CCTCGGGCATCCAAGCAGCAAGCGCGTTACGCCGTGGGTCGATGTTTGATGTTAT GGAGCAGCAACGATGTTACGCAGCAGCAACGATGTTACGCAGCAGGGCAGTCGC CCTAAAACAAAGTTAGGTGGCTCAAGTATGGGCATCATTCGCACATGTAGGCTCG GCCCTGACCAAGTCAAATCCATGCGGGCTGCTCTTGATCTTTTCGGTCGTGAGTT CGGAGACGTAGCCACCTACTCCCAACATCAGCCGGACTCCGATTACCTCGGGAA CTTGCTCCGTAGTAAGACATTCATCGCGCTTGCTGCCTTCGACCAAGAAGCGGTT GTTGGCGCTCTCGCGGCTTACGTTCTGCCCAAGTTTGAGCAGCCGCGTAGTGAGA TCTATATCTATGATCTCGCAGTCTCCGGCGAGCACCGGAGGCAGGGCATTGCCAC CGCGCTCATCAATCTCCTCAAGCATGAGGCCAACGCGCTTGGTGCTTATGTGATC TACGTGCAAGCAGATTACGGTGACGATCCCGCAGTGGCTCTCTATACAAAGTTGG GCATACGGGAAGAAGTGATGCACTTTGATATCGACCCAAGTACCGCCACCTAAC AATTCGTTCAAGCCGAGATCGGCTTCCCGGCCGCGGAGTTGTTCGGTAAATTGTC ACAACGCCG (SEQ ID NO: 909). In some embodiments, the construct comprises or consists of, from 5' to 3' a first transposable element inverted repeat, a first ITR, a first promoter, a first sequence encoding a gRNA, a second promoter, a second sequence encoding a gRNA, a third promoter, a third sequence encoding a gRNA, a minipolyA sequence, a second ITR, a regulatory sequence, and a second transposable element inverted repeat. In some embodiments, the construct comprises or consists of, from 5' to 3' a first transposable element inverted repeat, a first ITR, the U6 promoter, a first sequence encoding a gRNA, the H1 promoter, a second sequence encoding a gRNA, the 7SK promoter, a third sequence encoding a gRNA, a minipolyA sequence, a second ITR, a regulatory sequence, and a second transposable element inverted repeat. In some embodiments, the construct comprises or consists of, from 5' to 3' a first transposable element inverted repeat, a first ITR, a first promoter, the sequence encoding a gRNA of SEQ ID NO: 708, a second promoter, the sequence encoding a gRNA of SEQ ID NO: 708, a third promoter, the sequence encoding a gRNA of SEQ ID NO: 708, a minipolyA sequence, a second ITR, a regulatory sequence, and a second transposable element inverted repeat. In some embodiments, the construct comprises or consists of, from 5' to 3' a first transposable element inverted repeat, a first ITR, a first promoter, the sequence encoding a gRNA of SEQ ID NO: 714, a second promoter, the sequence encoding a gRNA of SEQ ID NO: 714, a third promoter, the sequence encoding a gRNA of SEQ ID NO: 714, a minipolyA sequence, a second ITR, a regulatory sequence, and a second transposable element inverted repeat. In some embodiments, the construct comprises or consists of, from 5' to 3' a first transposable element inverted repeat, a first ITR, the U6 promoter, the sequence encoding a gRNA of SEQ ID NO: 708, the H1 promoter, the sequence encoding a gRNA of SEQ ID NO. 708, the 7SK promoter, the sequence encoding a gRNA of SEQ ID NO: 708, a minipolyA sequence, a second ITR, a regulatory sequence, and a second transposable element inverted repeat. In some embodiments, the construct comprises or consists of, from 5' to 3' a first transposable element inverted repeat, a first ITR, the U6 promoter, a sequence encoding a gRNA of SEQ ID NO: 714, the H1 promoter, a sequence encoding a gRNA of SEQ ID NO: 714, the 7SK promoter, a sequence encoding a gRNA of SEQ ID NO: 714, a minipolyA sequence, a second ITR, a regulatory sequence, and a second transposable element inverted repeat In some embodiments, the construct comprising a first ITR, a first promoter, a first sequence encoding a gRNA, a second promoter, and a second sequence encoding a gRNA, a third promoter, a third sequence encoding a gRNA, a minipolyA sequence, and a second ITR, further comprises a stuffer sequence. In some embodiments, the construct comprises or consists of, from 5' to 3' a first ITR, the U6 promoter, a first sequence encoding a gRNA, the H1 promoter, a second sequence encoding a gRNA, the 7SK promoter, a third sequence encoding a gRNA, a stuffer sequence, a minipolyA sequence, and a second ITR. In some embodiments, the construct comprises or consists of, from 5' to 3' a first ITR, a first promoter, the sequence encoding a gRNA of SEQ ID NO: 708, a second promoter, the sequence encoding a gRNA of SEQ ID NO: 708, a third promoter, the sequence encoding a gRNA of SEQ ID NO: 708, a stuffer sequence a minipolyA sequence, and a second ITR. In some embodiments, the construct comprises or consists of, from 5' to 3' a first ITR, a first promoter, the sequence encoding a gRNA of SEQ ID NO: 714, a second promoter, the sequence encoding a gRNA of SEQ ID NO: 714, a third promoter, the sequence encoding a gRNA of SEQ ID NO. 714, a stuffer sequence, a minipolyA sequence, and a second ITR. In some embodiments, the construct comprises or consists of, from 5' to 3' a first ITR, the U6 promoter, the sequence encoding a gRNA of SEQ ID NO: 708, the H1 promoter, the sequence encoding a gRNA of SEQ ID NO: 708, the 7SK promoter, the sequence encoding a gRNA of SEQ ID NO: 708, a stuffer sequence, a minipolyA sequence, and a second ITR. In some embodiments, the construct comprises or consists of, from 5' to 3' a first ITR, the U6 promoter, the sequence encoding a gRNA of SEQ ID NO: 714, the H1 promoter, the sequence encoding a gRNA of SEQ ID NO: 714, the 7SK promoter, the sequence encoding a gRNA of SEQ ID NO: 714, a stuffer sequence, a minipolyA sequence, and a second ITR In some embodiments, the construct comprises or consists of, from 5' to 3' a first transposable element inverted repeat, a first ITR, a first promoter, a first sequence encoding a gRNA, a second promoter, a second sequence encoding a gRNA, a third promoter, a third sequence encoding a gRNA, a stuffer sequence, a minipolyA sequence, a second ITR, a regulatory sequence, and a second transposable element inverted repeat. In some embodiments, the construct comprises or consists of, from 5' to 3' a first transposable element inverted repeat, a first ITR, the U6 promoter, a first sequence encoding a gRNA, the H1 promoter, a second sequence encoding a gRNA, the 7SK promoter, a third sequence encoding a gRNA, a stuffer sequence, a minipolyA sequence, a second ITR, a regulatory sequence, and a second transposable element inverted repeat. In some embodiments, the construct comprises or consists of, from 5' to 3' a first transposable element inverted repeat, a first ITR, a first promoter, the sequence encoding a gRNA of SEQ ID NO: 708, a second promoter, the sequence encoding a gRNA of SEQ ID NO: 708, a third promoter, the sequence encoding a gRNA of SEQ ID NO: 708, a stuffer sequence, a minipolyA sequence, a second ITR, a regulatory sequence, and a second transposable element inverted repeat. In some embodiments, the construct comprises or consists of, from 5' to 3' a first transposable element inverted repeat, a first ITR, a first promoter, the sequence encoding a gRNA of SEQ ID NO: 714, a second promoter, the sequence encoding a gRNA of SEQ ID NO: 714, a third promoter, the sequence encoding a gRNA of SEQ ID NO: 714, a stuffer sequence, a minipolyA sequence, a second ITR, a regulatory sequence, and a second transposable element inverted repeat. In some embodiments, the construct comprises or consists of, from 5' to 3' a first transposable element inverted repeat, a first ITR, the U6 promoter, the sequence encoding a gRNA of SEQ ID NO: 708, the H1 promoter, the sequence encoding a gRNA of SEQ ID NO: 708, the 7SK promoter, the sequence encoding a gRNA of SEQ ID NO: 708, a stuffer sequence, a minipolyA sequence, a second ITR, a regulatory sequence, and a second transposable element inverted repeat. In some embodiments, the construct comprises or consists of, from 5' to 3' a first transposable element inverted repeat, a first ITR, the U6 promoter, a sequence encoding a gRNA of SEQ ID NO: 714, the H1 promoter, a sequence encoding a gRNA of SEQ ID NO: 714, the 7SK promoter, a sequence encoding a gRNA of SEQ ID NO: 714, a stuffer sequence, a minipolyA sequence, a second ITR, a regulatory sequence, and a second transposable element inverted repeat. In some embodiments, the construct may further comprise one or more spacer sequences. Exemplary spacer sequences of the disclosure have length from 1-1500 nucleotides, inclusive of all ranges therebetween. In some embodiments, the spacer sequences may be located at a position that is 5' to or 3' to an ITR, a promoter, a sequence encoding a gRNA, a polyA sequence, a transposable element inverted repeat, a stuffer sequence, and/or a regulator element.

### V. Pharmaceutical Compositions and Delivery Methods

For clinical applications, pharmaceutical compositions are prepared in a form appropriate for the intended application. Generally, this entails preparing compositions that are essentially free of pyrogens, as well as other impurities that could be harmful to humans or animals.

Appropriate salts and buffers are used to render drugs, proteins or delivery vectors stable and allow for uptake by target cells. Aqueous compositions of the present disclosure comprise an effective amount of the drug, vector or proteins, dissolved or dispersed in a pharmaceutically acceptable carrier or aqueous medium. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Any conventional media or agent that is not incompatible with the active ingredients of the present disclosure, its use in therapeutic compositions may be used. Supplementary active ingredients also can be incorporated into the compositions, provided they do not inactivate the vectors or cells of the compositions.

In some embodiments, the active compositions of the present disclosure may include classic pharmaceutical preparations. Administration of these compositions according to the present disclosure may be via any common route so long as the target tissue is available via that route, but generally including systemic administration. This includes oral, nasal, or buccal. Alternatively, administration may be by intradermal, subcutaneous, intramuscular, intraperitoneal or intravenous injection, or by direct injection into muscle tissue. Such compositions would normally be administered as pharmaceutically acceptable compositions, as described *supra.*

The active compounds may also be administered parenterally or intraperitoneally. By way of illustration, solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations generally contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. Generally, these preparations are sterile and fluid to the extent that easy injectability exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions may be prepared by incorporating the active compounds in an appropriate amount into a solvent along with any other ingredients (for example as enumerated above) as desired, followed by filtered sterilization. Generally, dispersions are prepared by incorporating the various sterilized active ingredients into a sterile vehicle which contains the basic dispersion medium and the desired other ingredients, e.g., as enumerated above. In the case of sterile powders for the preparation of sterile injectable solutions, the preferred methods of preparation include vacuum-drying and freeze-drying techniques which yield a powder of the active ingredient(s) plus any additional desired ingredient from a previously sterile-filtered solution thereof.

In some embodiments, the compositions of the present disclosure are formulated in a neutral or salt form. Pharmaceutically-acceptable salts include, for example, acid addition salts (formed with the free amino groups of the protein) derived from inorganic acids (*e.g.*, hydrochloric or phosphoric acids, or from organic acids (*e.g.*, acetic, oxalic, tartaric, mandelic, and the like. Salts formed with the free carboxyl groups of the protein can also be derived from inorganic bases (e.g., sodium, potassium, ammonium, calcium, or ferric hydroxides) or from organic bases (e.g., isopropylamine, trimethylamine, histidine, procaine) and the like.

Upon formulation, solutions are preferably administered in a manner compatible with the dosage formulation and in such amount as is therapeutically effective. The formulations may easily be administered in a variety of dosage forms such as injectable solutions, drug release capsules and the like. For parenteral administration in an aqueous solution, for example, the solution generally is suitably buffered and the liquid diluent first rendered isotonic for example with sufficient saline or glucose. Such aqueous solutions may be used, for example, for intravenous, intramuscular, subcutaneous and intraperitoneal administration. Preferably, sterile aqueous media are employed as is known to those of skill in the art, particularly in light of the present disclosure. By way of illustration, a single dose may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

In some embodiments, the Cas9 or Cpf1 and gRNAs described herein may be delivered to the patient using adoptive cell transfer (ACT). In adoptive cell transfer, one or more expression constructs are provided ex vivo to cells which have originated from the patient (autologous) or from one or more individual(s) other than the patient (allogeneic). The cells are subsequently introduced or reintroduced into the patient. Thus, in some embodiments, one or more nucleic acids encoding Cas9 or Cpf1 and a guide RNA that targets a dystrophin splice site are provided to a cell ex vivo before the cell is introduced or reintroduced to a patient.

The following tables provide exemplary primer and genomic targeting sequences for use in connection with the compositions and methods disclosed herein.

**TABLE 6 - Genomic Target Sequences**

| **Targeted gRNA Exon** | **Guide #** | **Strand** | **Genomic Target Sequence*** | **PAM** | **SEQ ID NO.** |
|---|---|---|---|---|---|
| Human-Exon 51 | 4 | 1 | tctttttcttcttttttccttttt | tttt | 60 |
| Human-Exon 51 | 5 | 1 | ctttttcttcttttttcctttttG | tttt | 61 |
| Human-Exon 51 | 6 | 1 | tttttcttcttttttcctttttGC | tttc | 62 |
| Human-Exon 51 | 7 | 1 | tcttcttttttcctttttGCAAAA | tttt | 63 |
| Human-Exon 51 | 8 | 1 | cttcttttttcctttttGCAAAAA | tttt | 64 |
| Human-Exon 51 | 9 | 1 | ttcttttttcctttttGCAAAAAC | tttc | 65 |
| Human-Exon 51 | 10 | 1 | ttcctttttGCAAAAACCCAAAAT | tttt | 66 |
| Human-Exon 51 | 11 | 1 | tcctttttGCAAAAACCCAAAATA | tttt | 67 |
| Human-Exon 51 | 12 | 1 | cctttttGCAAAAACCCAAAATAT | tttt | 68 |
| Human-Exon 51 | 13 | 1 | ctttttGCAAAAACCCAAAATATT | tttc | 69 |
| Human-Exon 51 | 14 | 1 | tGCAAAAACCCAAAATATTTTAGC | tttt | 70 |
| Human-Exon 51 | 15 | 1 | GCAAAAACCCAAAATATTTTAGCT | tttt | 71 |
| Human-Exon 51 | 16 | 1 | CAAAAACCCAAAATATTTTAGCTC | tttG | 72 |
| Human-Exon 51 | 17 | 1 | AGCTCCTACTCAGACTGTTACTCT | TTTT | 73 |
| Human-Exon 51 | 18 | 1 | GCTCCTACTCAGACTGTTACTCTG | TTTA | 74 |
| Human-Exon 51 | 19 | -1 | CTTAGTAACCACAGGTTGTGTCAC | TTTC | 75 |
| Human-Exon 51 | 20 | -1 | GAGATGGCAGTTTCCTTAGTAACC | TTTG | 76 |
| Human-Exon 51 | 21 | -1 | TAGTTTGGAGATGGCAGTTTCCTT | TTTC | 77 |
| Human-Exon 51 | 22 | -1 | TTCTCATACCTTCTGCTTGATGAT | TTTT | 78 |
| Human-Exon 51 | 23 | -1 | TCATTTTTTCTCATACCTTCTGCT | TTTA | 79 |
| Human-Exon 51 | 24 | -1 | ATCATTTTTTCTCATACCTTCTGC | TTTT | 80 |
| Human-Exon 51 | 25 | -1 | AAGAAAAACTTCTGCCAACTTTTA | TTTA | 81 |
| Human-Exon 51 | 26 | -1 | AAAGAAAAACTTCTGCCAACTTTT | TTTT | 82 |
| Human-Exon 51 | 27 | 1 | TCTTTAAAATGAAGATTTTCCACC | TTTT | 83 |
| Human-Exon 51 | 28 | 1 | CTTTAAAATGAAGATTTTCCACCA | TTTT | 84 |
| Human-Exon 51 | 29 | 1 | TTTAAAATGAAGATTTTCCACCAA | TTTC | 85 |
| Human-Exon 51 | 30 | 1 | AAATGAAGATTTTCCACCAATCAC | TTTA | 86 |
| Human-Exon 51 | 31 | 1 | CCACCAATCACTTTACTCTCCTAG | TTTT | 87 |
| Human-Exon 51 | 32 | 1 | CACCAATCACTTTACTCTCCTAGA | TTTC | 88 |
| Human-Exon 51 | 33 | 1 | CTCTCCTAGACCATTTCCCACCAG | TTTA | 89 |
| Human-Exon 45 | 1 | -1 | agaaaagattaaacagtgtgctac | tttg | 90 |
| Human-Exon 45 | 2 | -1 | tttgagaaaagattaaacagtgtg | TTTa | 91 |
| Human-Exon 45 | 3 | -1 | atttgagaaaagattaaacagtgt | TTTT | 92 |
| Human-Exon 45 | 4 | -1 | Tatttgagaaaagattaaacagtg | TTTT | 93 |
| Human-Exon 45 | 5 | 1 | atcttttctcaaatAAAAAGACAT | ttta | 94 |
| Human-Exon 45 | 6 | 1 | ctcaaatAAAAAGACATGGGGCTT | tttt | 95 |
| Human-Exon 45 | 7 | 1 | tcaaatAAAAAGACATGGGGCTTC | tttc | 96 |
| Human-Exon 45 | 8 | 1 | TGTTTTGCCTTTTTGGTATCTTAC | TTTT | 97 |
| Human-Exon 45 | 9 | 1 | GTTTTGCCTTTTTGGTATCTTACA | TTTT | 98 |
| Human-Exon 45 | 10 | 1 | TTTTGCCTTTTTGGTATCTTACAG | TTTG | 99 |
| Human-Exon 45 | 11 | 1 | GCCTTTTTGGTATCTTACAGGAAC | TTTT | 100 |
| Human-Exon 45 | 12 | 1 | CCTTTTTGGTATCTTACAGGAACT | TTTG | 101 |
| Human-Exon 45 | 13 | 1 | TGGTATCTTACAGGAACTCCAGGA | TTTT | 102 |
| Human-Exon 45 | 14 | 1 | GGTATCTTACAGGAACTCCAGGAT | TTTT | 103 |
| Human-Exon 45 | 15 | -1 | AGGATTGCTGAATTATTTCTTCCC | TTTG | 104 |
| Human-Exon 45 | 16 | -1 | GAGGATTGCTGAATTATTTCTTCC | TTTT | 105 |
| Human-Exon 45 | 17 | -1 | TGAGGATTGCTGAATTATTTCTTC | TTTT | 106 |
| Human-Exon 45 | 18 | -1 | CTGTAGAATACTGGCATCTGTTTT | TTTC | 107 |
| Human-Exon 45 | 19 | -1 | CCTGTAGAATACTGGCATCTGTTT | TTTT | 108 |
| Human-Exon 45 | 20 | -1 | TCCTGTAGAATACTGGCATCTGTT | TTTT | 109 |
| Human-Exon 45 | 21 | -1 | CAGACCTCCTGCCACCGCAGATTC | TTTG | 110 |
| Human-Exon 45 | 22 | -1 | TGTCTGACAGCTGTTTGCAGACCT | TTTC | 111 |
| Human-Exon 45 | 23 | -1 | CTGTCTGACAGCTGTTTGCAGACC | TTTT | 112 |
| Human-Exon 45 | 24 | -1 | TCTGTCTGACAGCTGTTTGCAGAC | TTTT | 113 |
| Human-Exon 45 | 25 | -1 | TTCTGTCTGACAGCTGTTTGCAGA | TTTT | 114 |
| Human-Exon 45 | 26 | -1 | ATTCCTATTAGATCTGTCGCCCTA | TTTC | 115 |
| Human-Exon 45 | 27 | -1 | CATTCCTATTAGATCTGTCGCCCT | TTTT | 116 |
| Human-Exon 45 | 28 | 1 | AGCAGACTTTTTAAGCTTTCTTTA | TTTT | 117 |
| Human-Exon 45 | 29 | 1 | GCAGACTTTTTAAGCTTTCTTTAG | TTTA | 118 |
| Human-Exon 45 | 30 | 1 | TAAGCTTTCTTTAGAAGAATATTT | TTTT | 119 |
| Human-Exon 45 | 31 | 1 | AAGCTTTCTTTAGAAGAATATTTC | TTTT | 120 |
| Human-Exon 45 | 32 | 1 | AGCTTTCTTTAGAAGAATATTTCA | TTTA | 121 |
| Human-Exon 45 | 33 | 1 | TTTAGAAGAATATTTCATGAGAGA | TTTC | 122 |
| Human-Exon 45 | 34 | 1 | GAAGAATATTTCATGAGAGATTAT | TTTA | 123 |
| Human-Exon 44 | 1 | 1 | TCAGTATAACCAAAAAATATACGC | TTTG | 124 |
| Human-Exon 44 | 2 | 1 | acataatccatctatttttcttga | tttt | 125 |
| Human-Exon 44 | 3 | 1 | cataatccatctatttttcttgat | ttta | 126 |
| Human-Exon 44 | 4 | 1 | tcttgatccatatgcttttACCTG | tttt | 127 |
| Human-Exon 44 | 5 | 1 | cttgatccatatgcttttACCTGC | tttt | 128 |
| Human-Exon 44 | 6 | 1 | ttgatccatatgcttttACCTGCA | tttc | 129 |
| Human-Exon 44 | 7 | -1 | TCAACAGATCTGTCAAATCGCCTG | TTTC | 130 |
| Human-Exon 44 | 8 | 1 | ACCTGCAGGCGATTTGACAGATCT | tttt | 131 |
| Human-Exon 44 | 9 | 1 | CCTGCAGGCGATTTGACAGATCTG | ttta | 132 |
| Human-Exon 44 | 10 | 1 | ACAGATCTGTTGAGAAATGGCGGC | TTTG | 133 |
| Human-Exon 44 | 11 | -1 | TATCATAATGAAAACGCCGCCATT | TTTA | 134 |
| Human-Exon 44 | 12 | 1 | CATTATGATATAAAGATATTTAAT | TTTT | 135 |
| Human-Exon 44 | 13 | -1 | TATTTAGCATGTTCCCAATTCTCA | TTTG | 136 |
| Human-Exon 44 | 14 | -1 | GAAAAAACAAATCAAAGACTTACC | TTTC | 137 |
| Human-Exon 44 | 15 | 1 | ATTTGTTTTTTCGAAATTGTATTT | TTTG | 138 |
| Human-Exon 44 | 16 | 1 | TTTTTTCGAAATTGTATTTATCTT | TTTG | 139 |
| Human-Exon 44 | 17 | 1 | TTCGAAATTGTATTTATCTTCAGC | TTTT | 140 |
| Human-Exon 44 | 18 | 1 | TCGAAATTGTATTTATCTTCAGCA | TTTT | 141 |
| Human-Exon 44 | 19 | 1 | CGAAATTGTATTTATCTTCAGCAC | TTTT | 142 |
| Human-Exon 44 | 20 | 1 | GAAATTGTATTTATCTTCAGCACA | TTTC | 143 |
| Human-Exon 44 | 21 | -1 | AGAAGTTAAAGAGTCCAGATGTGC | TTTA | 144 |
| Human-Exon 44 | 22 | 1 | TCTTCAGCACATCTGGACTCTTTA | TTTA | 145 |
| Human-Exon 44 | 23 | -1 | CATCACCCTTCAGAACCTGATCTT | TTTC | 146 |
| Human-Exon 44 | 24 | 1 | ACTTCTTAAAGATCAGGTTCTGAA | TTTA | 147 |
| Human-Exon 44 | 25 | 1 | GACTGTTGTTGTCATCATTATATT | TTTT | 148 |
| Human-Exon 44 | 26 | 1 | ACTGTTGTTGTCATCATTATATTA | TTTG | 149 |
| Human-Exon 53 | 1 | -1 | AACTAGAATAAAAGGAAAAATAAA | TTTC | 150 |
| Human-Exon 53 | 2 | 1 | CTACTATATATTTATTTTTCCTTT | TTTA | 151 |
| Human-Exon 53 | 3 | 1 | TTTTTCCTTTTATTCTAGTTGAAA | TTTA | 152 |
| Human-Exon 53 | 4 | 1 | TCCTTTTATTCTAGTTGAAAGAAT | TTTT | 153 |
| Human-Exon 53 | 5 | 1 | CCTTTTATTCTAGTTGAAAGAATT | TTTT | 154 |
| Human-Exon 53 | 6 | 1 | CTTTTATTCTAGTTGAAAGAATTC | TTTC | 155 |
| Human-Exon 53 | 7 | 1 | ATTCTAGTTGAAAGAATTCAGAAT | TTTT | 156 |
| Human-Exon 53 | 8 | 1 | TTCTAGTTGAAAGAATTCAGAATC | TTTA | 157 |
| Human-Exon 53 | 9 | -1 | ATTCAACTGTTGCCTCCGGTTCTG | TTTC | 158 |
| Human-Exon 53 | 10 | -1 | ACATTTCATTCAACTGTTGCCTCC | TTTA | 159 |
| Human-Exon 53 | 11 | -1 | CTTTTGGATTGCATCTACTGTATA | TTTT | 160 |
| Human-Exon 53 | 12 | -1 | TGTGATTTTCTTTTGGATTGCATC | TTTC | 161 |
| Human-Exon 53 | 13 | -1 | ATACTAACCTTGGTTTCTGTGATT | TTTG | 162 |
| Human-Exon 53 | 14 | -1 | AAAAGGTATCTTTGATACTAACCT | TTTA | 163 |
| Human-Exon 53 | 15 | -1 | AAAAAGGTATCTTTGATACTAACC | TTTT | 164 |
| Human-Exon 53 | 16 | -1 | TTTTAAAAAGGTATCTTTGATACT | TTTA | 165 |
| Human-Exon 53 | 17 | -1 | ATTTTAAAAAGGTATCTTTGATAC | TTTT | 166 |
| Human-Exon 46 | 1 | -1 | TTAATGCAAACTGGGACACAAACA | TTTG | 167 |
| Human-Exon 46 | 2 | 1 | TAAATTGCCATGTTTGTGTCCCAG | TTTT | 168 |
| Human-Exon 46 | 3 | 1 | AAATTGCCATGTTTGTGTCCCAGT | TTTT | 169 |
| Human-Exon 46 | 4 | 1 | AATTGCCATGTTTGTGTCCCAGTT | TTTA | 170 |
| Human-Exon 46 | 5 | 1 | TGTCCCAGTTTGCATTAACAAATA | TTTG | 171 |
| Human-Exon 46 | 6 | -1 | CAACATAGTTCTCAAACTATTTGT | tttc | 172 |
| Human-Exon 46 | 7 | -1 | CCAACATAGTTCTCAAACTATTTG | tttt | 173 |
| Human-Exon 46 | 8 | -1 | tCCAACATAGTTCTCAAACTATTT | tttt | 174 |
| Human-Exon 46 | 9 | -1 | tttCCAACATAGTTCTCAAACTAT | tttt | 175 |
| Human-Exon 46 | 10 | -1 | ttttCCAACATAGTTCTCAAACTA | tttt | 176 |
| Human-Exon 46 | 11 | -1 | tttttCCAACATAGTTCTCAAACT | tttt | 177 |
| Human-Exon 46 | 12 | 1 | CATTAACAAATAGTTTGAGAACTA | TTTG | 178 |
| Human-Exon 46 | 13 | 1 | AGAACTATGTTGGaaaaaaaaaTA | TTTG | 179 |
| Human-Exon 46 | 14 | -1 | GTTCTTCTAGCCTGGAGAAAGAAG | TTTT | 180 |
| Human-Exon 46 | 15 | 1 | ATTCTTCTTTCTCCAGGCTAGAAG | TTTT | 181 |
| Human-Exon 46 | 16 | 1 | TTCTTCTTTCTCCAGGCTAGAAGA | TTTA | 182 |
| Human-Exon 46 | 17 | 1 | TCCAGGCTAGAAGAACAAAAGAAT | TTTC | 183 |
| Human-Exon 46 | 18 | -1 | AAATTCTGACAAGATATTCTTTTG | TTTG | 184 |
| Human-Exon 46 | 19 | -1 | CTTTTAGTTGCTGCTCTTTTCCAG | TTTT | 185 |
| Human-Exon 46 | 20 | -1 | AGAAAATAAAATTACCTTGACTTG | TTTG | 186 |
| Human-Exon 46 | 21 | -1 | TGCAAGCAGGCCCTGGGGGATTTG | TTTA | 187 |
| Human-Exon 46 | 22 | 1 | ATTTTCTCAAATCCCCCAGGGCCT | TTTT | 188 |
| Human-Exon 46 | 23 | 1 | TTTTCTCAAATCCCCCAGGGCCTG | TTTA | 189 |
| Human-Exon 46 | 24 | 1 | CTCAAATCCCCCAGGGCCTGCTTG | TTTT | 190 |
| Human-Exon 46 | 25 | 1 | TCAAATCCCCCAGGGCCTGCTTGC | TTTC | 191 |
| Human-Exon 46 | 26 | 1 | TTAATTCAATCATTGGTTTTCTGC | TTTT | 192 |
| Human-Exon 46 | 27 | 1 | TAATTCAATCATTGGTTTTCTGCC | TTTT | 193 |
| Human-Exon 46 | 28 | 1 | AATTCAATCATTGGTTTTCTGCCC | TTTT | 194 |
| Human-Exon 46 | 29 | 1 | ATTCAATCATTGGTTTTCTGCCCA | TTTA | 195 |
| Human-Exon 46 | 30 | -1 | GCAAGGAACTATGAATAACCTAAT | TTTA | 196 |
| Human-Exon 46 | 31 | 1 | CTGCCCATTAGGTTATTCATAGTT | TTTT | 197 |
| Human-Exon 46 | 32 | 1 | TGCCCATTAGGTTATTCATAGTTC | TTTC | 198 |
| Human-Exon 52 | 1 | -1 | TAGAAAACAATTTAACAGGAAATA | TTTA | 199 |
| Human-Exon 52 | 2 | 1 | CTGTTAAATTGTTTTCTATAAACC | TTTC | 200 |
| Human-Exon 52 | 3 | -1 | GAAATAAAAAAGATGTTACTGTAT | TTTA | 201 |
| Human-Exon 52 | 4 | -1 | AGAAATAAAAAAGATGTTACTGTA | TTTT | 202 |
| Human-Exon 52 | 5 | 1 | CTATAAACCCTTATACAGTAACAT | TTTT | 203 |
| Human-Exon 52 | 6 | 1 | TATAAACCCTTATACAGTAACATC | TTTC | 204 |
| Human-Exon 52 | 7 | 1 | TTATTTCTAAAAGTGTTTTGGCTG | TTTT | 205 |
| Human-Exon 52 | 8 | 1 | TATTTCTAAAAGTGTTTTGGCTGG | TTTT | 206 |
| Human-Exon 52 | 9 | 1 | ATTTCTAAAAGTGTTTTGGCTGGT | TTTT | 207 |
| Human-Exon 52 | 10 | 1 | TTTCTAAAAGTGTTTTGGCTGGTC | TTTA | 208 |
| Human-Exon 52 | 11 | 1 | TAAAAGTGTTTTGGCTGGTCTCAC | TTTC | 209 |
| Human-Exon 52 | 12 | -1 | CATAATACAAAGTAAAGTACAATT | TTTA | 210 |
| Human-Exon 52 | 13 | -1 | ACATAATACAAAGTAAAGTACAAT | TTTT | 211 |
| Human-Exon 52 | 14 | 1 | GGCTGGTCTCACAATTGTACTTTA | TTTT | 212 |
| Human-Exon 52 | 15 | 1 | GCTGGTCTCACAATTGTACTTTAC | TTTG | 213 |
| Human-Exon 52 | 16 | 1 | CTTTGTATTATGTAAAAGGAATAC | TTTA | 214 |
| Human-Exon 52 | 17 | 1 | TATTATGTAAAAGGAATACACAAC | TTTG | 215 |
| Human-Exon 52 | 18 | 1 | TTCTTACAGGCAACAATGCAGGAT | TTTG | 216 |
| Human-Exon 52 | 19 | 1 | GAACAGAGGCGTCCCCAGTTGGAA | TTTG | 217 |
| Human-Exon 52 | 20 | -1 | GGCAGCGGTAATGAGTTCTTCCAA | TTTG | 218 |
| Human-Exon 52 | 21 | -1 | TCAAATTTTGGGCAGCGGTAATGA | TTTT | 219 |
| Human-Exon 52 | 22 | 1 | AAAAACAAGACCAGCAATCAAGAG | TTTG | 220 |
| Human-Exon 52 | 23 | -1 | TGTGTCCCATGCTTGTTAAAAAAC | TTTG | 221 |
| Human-Exon 52 | 24 | 1 | TTAACAAGCATGGGACACACAAAG | TTTT | 222 |
| Human-Exon 52 | 25 | 1 | TAACAAGCATGGGACACACAAAGC | TTTT | 223 |
| Human-Exon 52 | 26 | 1 | AACAAGCATGGGACACACAAAGCA | TTTT | 224 |
| Human-Exon 52 | 27 | 1 | ACAAGCATGGGACACACAAAGCAA | TTTA | 225 |
| Human-Exon 52 | 28 | -1 | TTGAAACTTGTCATGCATCTTGCT | TTTA | 226 |
| Human-Exon 52 | 29 | -1 | ATTGAAACTTGTCATGCATCTTGC | TTTT | 227 |
| Human-Exon 52 | 30 | -1 | TATTGAAACTTGTCATGCATCTTG | TTTT | 228 |
| Human-Exon 52 | 31 | 1 | AATAAAAACTTAAGTTCATATATC | TTTC | 229 |
| Human-Exon 50 | 1 | -1 | GTGAATATATTATTGGATTTCTAT | TTTG | 230 |
| Human-Exon 50 | 2 | -1 | AAGATAATTCATGAACATCTTAAT | TTTG | 231 |
| Human-Exon 50 | 3 | -1 | ACAGAAAAGCATACACATTACTTA | TTTA | 232 |
| Human-Exon 50 | 4 | 1 | CTGTTAAAGAGGAAGTTAGAAGAT | TTTT | 233 |
| Human-Exon 50 | 5 | 1 | TGTTAAAGAGGAAGTTAGAAGATC | TTTC | 234 |
| Human-Exon 50 | 6 | -1 | CCGCCTTCCACTCAGAGCTCAGAT | TTTA | 235 |
| Human-Exon 50 | 7 | -1 | CCCTCAGCTCTTGAAGTAAACGGT | TTTG | 236 |
| Human-Exon 50 | 8 | 1 | CTTCAAGAGCTGAGGGCAAAGCAG | TTTA | 237 |
| Human-Exon 50 | 9 | -1 | AACAAATAGCTAGAGCCAAAGAGA | TTTG | 238 |
| Human-Exon 50 | 10 | -1 | GAACAAATAGCTAGAGCCAAAGAG | TTTT | 239 |
| Human-Exon 50 | 11 | 1 | GCTCTAGCTATTTGTTCAAAAGTG | TTTG | 240 |
| Human-Exon 50 | 12 | 1 | TTCAAAAGTGCAACTATGAAGTGA | TTTG | 241 |
| Human-Exon 50 | 13 | -1 | TCTCTCACCCAGTCATCACTTCAT | TTTC | 242 |
| Human-Exon 50 | 14 | -1 | CTCTCTCACCCAGTCATCACTTCA | TTTT | 243 |
| Human-Exon 43 | 1 | 1 | tatatatatatatatTTTTCTCTT | TTTG | 244 |
| Human-Exon 43 | 2 | 1 | TCTCTTTCTATAGACAGCTAATTC | tTTT | 245 |
| Human-Exon 43 | 3 | 1 | CTCTTTCTATAGACAGCTAATTCA | TTTT | 246 |
| Human-Exon 43 | 4 | -1 | AAACAGTAAAAAAATGAATTAGCT | TTTA | 247 |
| Human-Exon 43 | 5 | 1 | TCTTTCTATAGACAGCTAATTCAT | TTTC | 248 |
| Human-Exon 43 | 6 | -1 | AAAACAGTAAAAAAATGAATTAGC | TTTT | 249 |
| Human-Exon 43 | 7 | 1 | TATAGACAGCTAATTCATTTTTTT | TTTC | 250 |
| Human-Exon 43 | 8 | -1 | TATTCTGTAATATAAAAATTTTAA | TTTA | 251 |
| Human-Exon 43 | 9 | -1 | ATATTCTGTAATATAAAAATTTTA | TTTT | 252 |
| Human-Exon 43 | 10 | 1 | TTTACTGTTTTAAAATTTTTATAT | TTTT | 253 |
| Human-Exon 43 | 11 | 1 | TTACTGTTTTAAAATTTTTATATT | TTTT | 254 |
| Human-Exon 43 | 12 | 1 | TACTGTTTTAAAATTTTTATATTA | TTTT | 255 |
| Human-Exon 43 | 13 | 1 | ACTGTTTTAAAATTTTTATATTAC | TTTT | 256 |
| Human-Exon 43 | 14 | 1 | CTGTTTTAAAATTTTTATATTACA | TTTA | 257 |
| Human-Exon 43 | 15 | 1 | AAAATTTTTATATTACAGAATATA | TTTT | 258 |
| Human-Exon 43 | 16 | 1 | AAATTTTTATATTACAGAATATAA | TTTA | 259 |
| Human-Exon 43 | 17 | -1 | TTGTAGACTATCTTTTATATTCTG | TTTG | 260 |
| Human-Exon 43 | 18 | 1 | TATATTACAGAATATAAAAGATAG | TTTT | 261 |
| Human-Exon 43 | 19 | 1 | ATATTACAGAATATAAAAGATAGT | TTTT | 262 |
| Human-Exon 43 | 20 | 1 | TATTACAGAATATAAAAGATAGTC | TTTA | 263 |
| Human-Exon 43 | 21 | -1 | CAATGCTGCTGTCTTCTTGCTATG | TTTG | 264 |
| Human-Exon 43 | 22 | 1 | CAATGGGAAAAAGTTAACAAAATG | TTTC | 265 |
| Human-Exon 43 | 23 | -1 | TGCAAGTATCAAGAAAAATATATG | TTTC | 266 |
| Human-Exon 43 | 24 | 1 | TCTTGATACTTGCAGAAATGATTT | TTTT | 267 |
| Human-Exon 43 | 25 | 1 | CTTGATACTTGCAGAAATGATTTG | TTTT | 268 |
| Human-Exon 43 | 26 | 1 | TTGATACTTGCAGAAATGATTTGT | TTTC | 269 |
| Human-Exon 43 | 27 | 1 | TTTTCAGGGAACTGTAGAATTTAT | TTTG | 270 |
| Human-Exon 43 | 28 | -1 | CATGGAGGGTACTGAAATAAATTC | TTTC | 271 |
| Human-Exon 43 | 29 | -1 | CCATGGAGGGTACTGAAATAAATT | TTTT | 272 |
| Human-Exon 43 | 30 | 1 | CAGGGAACTGTAGAATTTATTTCA | TTTT | 273 |
| Human-Exon 43 | 31 | -1 | TCCATGGAGGGTACTGAAATAAAT | TTTT | 274 |
| Human-Exon 43 | 32 | 1 | AGGGAACTGTAGAATTTATTTCAG | TTTC | 275 |
| Human-Exon 43 | 33 | -1 | TTCCATGGAGGGTACTGAAATAAA | TTTT | 276 |
| Human-Exon 43 | 34 | -1 | CCTGTCTTTTTTCCATGGAGGGTA | TTTC | 277 |
| Human-Exon 43 | 35 | -1 | CCCTGTCTTTTTTCCATGGAGGGT | TTTT | 278 |
| Human-Exon 43 | 36 | -1 | TCCCTGTCTTTTTTCCATGGAGGG | TTTT | 279 |
| Human-Exon 43 | 37 | 1 | TTTCAGTACCCTCCATGGAAAAAA | TTTA | 280 |
| Human-Exon 43 | 38 | 1 | AGTACCCTCCATGGAAAAAAGACA | TTTC | 281 |
| Human-Exon 6 | 1 | 1 | AGTTTGCATGGTTCTTGCTCAAGG | TTTA | 282 |
| Human-Exon 6 | 2 | -1 | ATAAGAAAATGCATTCCTTGAGCA | TTTC | 283 |
| Human-Exon 6 | 3 | -1 | CATAAGAAAATGCATTCCTTGAGC | TTTT | 284 |
| Human-Exon 6 | 4 | 1 | CATGGTTCTTGCTCAAGGAATGCA | TTTG | 285 |
| Human-Exon 6 | 5 | -1 | ACCTACATGTGGAAATAAATTTTC | TTTG | 286 |
| Human-Exon 6 | 6 | -1 | GACCTACATGTGGAAATAAATTTT | TTTT | 287 |
| Human-Exon 6 | 7 | -1 | TGACCTACATGTGGAAATAAATTT | TTTT | 288 |
| Human-Exon 6 | 8 | 1 | CTTATGAAAATTTATTTCCACATG | TTTT | 289 |
| Human-Exon 6 | 9 | 1 | TTATGAAAATTTATTTCCACATGT | TTTC | 290 |
| Human-Exon 6 | 10 | -1 | ATTACATTTTTGACCTACATGTGG | TTTC | 291 |
| Human-Exon 6 | 11 | -1 | CATTACATTTTTGACCTACATGTG | TTTT | 292 |
| Human-Exon 6 | 12 | -1 | TCATTACATTTTTGACCTACATGT | TTTT | 293 |
| Human-Exon 6 | 13 | 1 | TTTCCACATGTAGGTCAAAAATGT | TTTA | 294 |
| Human-Exon 6 | 14 | 1 | CACATGTAGGTCAAAAATGTAATG | TTTC | 295 |
| Human-Exon 6 | 15 | -1 | TTGCAATCCAGCCATGATATTTTT | TTTG | 296 |
| Human-Exon 6 | 16 | -1 | ACTGTTGGTTTGTTGCAATCCAGC | TTTC | 297 |
| Human-Exon 6 | 17 | -1 | CACTGTTGGTTTGTTGCAATCCAG | TTTT | 298 |
| Human-Exon 6 | 18 | 1 | AATGCTCTCATCCATAGTCATAGG | TTTG | 299 |
| Human-Exon 6 | 19 | -1 | ATGTCTCAGTAATCTTCTTACCTA | TTTA | 300 |
| Human-Exon 6 | 20 | -1 | CAAGTTATTTAATGTCTCAGTAAT | TTTA | 301 |
| Human-Exon 6 | 21 | -1 | ACAAGTTATTTAATGTCTCAGTAA | TTTT | 302 |
| Human-Exon 6 | 22 | 1 | GACTCTGATGACATATTTTTCCCC | TTTA | 303 |
| Human-Exon 6 | 23 | 1 | TCCCCAGTATGGTTCCAGATCATG | TTTT | 304 |
| Human-Exon 6 | 24 | 1 | CCCCAGTATGGTTCCAGATCATGT | TTTT | 305 |
| Human-Exon 6 | 25 | 1 | CCCAGTATGGTTCCAGATCATGTC | TTTC | 306 |
| Human-Exon 7 | 1 | 1 | TATTTGTCTTtgtgtatgtgtgta | TTTA | 307 |
| Human-Exon 7 | 2 | 1 | TCTTtgtgtatgtgtgtatgtgta | TTTG | 308 |
| Human-Exon 7 | 3 | 1 | tgtatgtgtgtatgtgtatgtgtt | TTtg | 309 |
| Human-Exon 7 | 4 | 1 | AGGCCAGACCTATTTGACTGGAAT | ttTT | 310 |
| Human-Exon 7 | 5 | 1 | GGCCAGACCTATTTGACTGGAATA | tTTA | 311 |
| Human-Exon 7 | 6 | 1 | ACTGGAATAGTGTGGTTTGCCAGC | TTTG | 312 |
| Human-Exon 7 | 7 | 1 | CCAGCAGTCAGCCACACAACGACT | TTTG | 313 |
| Human-Exon 7 | 8 | -1 | TCTATGCCTAATTGATATCTGGCG | TTTC | 314 |
| Human-Exon 7 | 9 | -1 | CCAACCTTCAGGATCGAGTAGTTT | TTTA | 315 |
| Human-Exon 7 | 10 | 1 | TGGACTACCACTGCTTTTAGTATG | TTTC | 316 |
| Human-Exon 7 | 11 | 1 | AGTATGGTAGAGTTTAATGTTTTC | TTTT | 317 |
| Human-Exon 7 | 12 | 1 | GTATGGTAGAGTTTAATGTTTTCA | TTTA | 318 |
| Human-Exon 8 | 1 | -1 | AGACTCTAAAAGGATAATGAACAA | TTTG | 319 |
| Human-Exon 8 | 2 | 1 | ACTTTGATTTGTTCATTATCCTTT | TTTA | 320 |
| Human-Exon 8 | 3 | -1 | TATATTTGAGACTCTAAAAGGATA | TTTC | 321 |
| Human-Exon 8 | 4 | 1 | ATTTGTTCATTATCCTTTTAGAGT | TTTG | 322 |
| Human-Exon 8 | 5 | -1 | GTTTCTATATTTGAGACTCTAAAA | TTTG | 323 |
| Human-Exon 8 | 6 | -1 | GGTTTCTATATTTGAGACTCTAAA | TTTT | 324 |
| Human-Exon 8 | 7 | -1 | TGGTTTCTATATTTGAGACTCTAA | TTTT | 325 |
| Human-Exon 8 | 8 | 1 | TTCATTATCCTTTTAGAGTCTCAA | TTTG | 326 |
| Human-Exon 8 | 9 | 1 | AGAGTCTCAAATATAGAAACCAAA | TTTT | 327 |
| Human-Exon 8 | 10 | 1 | GAGTCTCAAATATAGAAACCAAAA | TTTA | 328 |
| Human-Exon 8 | 11 | -1 | CACTTCCTGGATGGCTTCAATGCT | TTTC | 329 |
| Human-Exon 8 | 12 | 1 | GCCTCAACAAGTGAGCATTGAAGC | TTTT | 330 |
| Human-Exon 8 | 13 | 1 | CCTCAACAAGTGAGCATTGAAGCC | TTTG | 331 |
| Human-Exon 8 | 14 | -1 | GGTGGCCTTGGCAACATTTCCACT | TTTA | 332 |
| Human-Exon 8 | 15 | -1 | GTCACTTTAGGTGGCCTTGGCAAC | TTTA | 333 |
| Human-Exon 8 | 16 | -1 | ATGATGTAACTGAAAATGTTCTTC | TTTG | 334 |
| Human-Exon 8 | 17 | -1 | CCTGTTGAGAATAGTGCATTTGAT | TTTA | 335 |
| Human-Exon 8 | 18 | 1 | CAGTTACATCATCAAATGCACTAT | TTTT | 336 |
| Human-Exon 8 | 19 | 1 | AGTTACATCATCAAATGCACTATT | TTTC | 337 |
| Human-Exon 8 | 20 | -1 | CACACTTTACCTGTTGAGAATAGT | TTTA | 338 |
| Human-Exon 8 | 21 | 1 | CTGTTTTATATGCATTTTTAGGTA | TTTT | 339 |
| Human-Exon 8 | 22 | 1 | TGTTTTATATGCATTTTTAGGTAT | TTTC | 340 |
| Human-Exon 8 | 23 | 1 | ATATGCATTTTTAGGTATTACGTG | TTTT | 341 |
| Human-Exon 8 | 24 | 1 | TATGCATTTTTAGGTATTACGTGC | TTTA | 342 |
| Human-Exon 8 | 25 | 1 | TAGGTATTACGTGCACatatatat | TTTT | 343 |
| Human-Exon 8 | 26 | 1 | AGGTATTACGTGCACatatatata | TTTT | 344 |
| Human-Exon 8 | 27 | 1 | GGTATTACGTGCACatatatatat | TTTA | 345 |
| Human-Exon 55 | 1 | -1 | AGCAACAACTATAATATTGTGCAG | TTTA | 346 |
| Human-Exon 55 | 2 | 1 | GTTCCTCCATCTTTCTCTTTTTAT | TTTA | 347 |
| Human-Exon 55 | 3 | 1 | TCTTTTTATGGAGTTCACTAGGTG | TTTC | 348 |
| Human-Exon 55 | 4 | 1 | TATGGAGTTCACTAGGTGCACCAT | TTTT | 349 |
| Human-Exon 55 | 5 | 1 | ATGGAGTTCACTAGGTGCACCATT | TTTT | 350 |
| Human-Exon 55 | 6 | 1 | TGGAGTTCACTAGGTGCACCATTC | TTTA | 351 |
| Human-Exon 55 | 7 | 1 | ATAATTGCATCTGAACATTTGGTC | TTTA | 352 |
| Human-Exon 55 | 8 | 1 | GTCCTTTGCAGGGTGAGTGAGCGA | TTTG | 353 |
| Human-Exon 55 | 9 | -1 | TTCCAAAGCAGCCTCTCGCTCACT | TTTC | 354 |
| Human-Exon 55 | 10 | 1 | CAGGGTGAGTGAGCGAGAGGCTGC | TTTG | 355 |
| Human-Exon 55 | 11 | 1 | GAAGAAACTCATAGATTACTGCAA | TTTG | 356 |
| Human-Exon 55 | 12 | -1 | CAGGTCCAGGGGGAACTGTTGCAG | TTTC | 357 |
| Human-Exon 55 | 13 | -1 | CCAGGTCCAGGGGGAACTGTTGCA | TTTT | 358 |
| Human-Exon 55 | 14 | -1 | AGCTTCTGTAAGCCAGGCAAGAAA | TTTC | 359 |
| Human-Exon 55 | 15 | 1 | TTGCCTGGCTTACAGAAGCTGAAA | TTTC | 360 |
| Human-Exon 55 | 16 | -1 | CTTACGGGTAGCATCCTGTAGGAC | TTTC | 361 |
| Human-Exon 55 | 17 | -1 | CTCCCTTGGAGTCTTCTAGGAGCC | TTTA | 362 |
| Human-Exon 55 | 18 | -1 | ACTCCCTTGGAGTCTTCTAGGAGC | TTTT | 363 |
| Human-Exon 55 | 19 | -1 | ATCAGCTCTTTTACTCCCTTGGAG | TTTC | 364 |
| Human-Exon 55 | 20 | 1 | CGCTTTAGCACTCTTGTGGATCCA | TTTC | 365 |
| Human-Exon 55 | 21 | 1 | GCACTCTTGTGGATCCAATTGAAC | TTTA | 366 |
| Human-Exon 55 | 22 | -1 | TCCCTGGCTTGTCAGTTACAAGTA | TTTG | 367 |
| Human-Exon 55 | 23 | -1 | GTCCCTGGCTTGTCAGTTACAAGT | TTTT | 368 |
| Human-Exon 55 | 24 | -1 | TTTTGTCCCTGGCTTGTCAGTTAC | TTTG | 369 |
| Human-Exon 55 | 25 | -1 | GTTTTGTCCCTGGCTTGTCAGTTA | TTTT | 370 |
| Human-Exon 55 | 26 | 1 | TACTTGTAACTGACAAGCCAGGGA | TTTG | 371 |
| Human-G1-exon51 | | 1 | gCTCCTACTCAGACTGTTACTCTG | TTTA | 372 |
| Human-G2-exon51 | | 1 | taccatgtattgctaaacaaagta | TTTC | 373 |
| Human-G3-exon51 | | -1 | attgaagagtaacaatttgagcca | TTTA | 374 |
| mouse-Exon23-G1 | | 1 | aggctctgcaaagttctTTGAAAG | TTTG | 375 |
| mouse-Exon23-G2 | | 1 | AAAGAGCAACAAAATGGCttcaac | TTTG | 376 |
| mouse-Exon23-G3 | | 1 | AAAGAGCAATAAAATGGCttcaac | TTTG | 377 |
| mouse-Exon23-G4 | | -1 | AAAGAACTTTGCAGAGCctcaaaa | TTTC | 378 |
| mouse-Exon23-G5 | | -1 | ctgaatatctatgcattaataact | TTTA | 379 |
| mouse-Exon23-G6 | | -1 | tattatattacagggcatattata | TTTC | 380 |
| mouse-Exon23-G7 | | 1 | Aggtaagccgaggtttggccttta | TTTC | 381 |
| mouse-Exon23-G8 | | 1 | cccagagtccttcaaagatattga | TTTA | 382 |

| | | | | | |
|---|---|---|---|---|---|
| * In this table, upper case letters represent nucleotides that align to the exon sequence of the gene. Lower case letters represent nucleotides that align to the intron sequence of the gene. | | | | | |

**TABLE 7 - gRNA sequences**

| **Targeted gRNA Exon** | **Guide #** | **Strand** | **gRNA sequence*** | **PAM** | **SEQ ID NO.** |
|---|---|---|---|---|---|
| Human-Exon 51 | 4 | 1 | aaaaaggaaaaaagaagaaaaaga | tttt | 383 |
| Human-Exon 51 | 5 | 1 | Caaaaaggaaaaaagaagaaaaag | tttt | 384 |
| Human-Exon 51 | 6 | 1 | GCaaaaaggaaaaaagaagaaaaa | tttc | 385 |
| Human-Exon 51 | 7 | 1 | LTUUUGCaaaaaggaaaaaagaaga | tttt | 386 |
| Human-Exon 51 | 8 | 1 | UUUUUGCaaaaaggaaaaaagaag | tttt | 387 |
| Human-Exon 51 | 9 | 1 | GUUUUUGCaaaaaggaaaaaagaa | tttc | 388 |
| Human-Exon 51 | 10 | 1 | AUUUUGGGUUUUUGCaaaaaggaa | tttt | 389 |
| Human-Exon 51 | 11 | 1 | UAUUUUGGGUUUUUGCaaaaagga | tttt | 390 |
| Human-Exon 51 | 12 | 1 | AUAUUUUGGGUUUUUGCaaaaagg | tttt | 391 |
| Human-Exon 51 | 13 | 1 | AAUAUUUUGGGUUUUUGCaaaaag | tttc | 392 |
| Human-Exon 51 | 14 | 1 | GCUAAAAUAUUUUGGGUUUUUGCa | tttt | 393 |
| Human-Exon 51 | 15 | 1 | AGCUAAAAUAUUUUGGGUUUUUGC | tttt | 394 |
| Human-Exon 51 | 16 | 1 | GAGCUAAAAUAUUUUGGGUUUUUG | tttG | 395 |
| Human-Exon 51 | 17 | 1 | AGAGUAACAGUCUGAGUAGGAGCU | TTTT | 396 |
| Human-Exon 51 | 18 | 1 | CAGAGUAACAGUCUGAGUAGGAGC | TTTA | 397 |
| Human-Exon 51 | 19 | -1 | GUGACACAACCUGUGGUUACUAAG | TTTC | 398 |
| Human-Exon 51 | 20 | -1 | GGUUACUAAGGAAACUGCCAUCU | TTTG | 399 |
| Human-Exon 51 | 21 | -1 | AAGGAAACUGCCAUCUCCAAACUA | TTTC | 400 |
| Human-Exon 51 | 22 | -1 | AUCAUCAAGCAGAAGGUAUGAGAA | TTTT | 401 |
| Human-Exon 51 | 23 | -1 | AGCAGAAGGUAUGAGAAAAAAUGA | TTTA | 402 |
| Human-Exon 51 | 24 | -1 | GCAGAAGGUAUGAGAAAAAAUGAU | TTTT | 403 |
| Human-Exon 51 | 25 | -1 | UAAAAGUUGGCAGAAGUUUUUCUU | TTTA | 404 |
| Human-Exon 51 | 26 | -1 | AAAAGUUGGCAGAAGUUUUUCUUU | TTTT | 405 |
| Human-Exon 51 | 27 | 1 | GGUGGAAAAUCUUCAUUUUAAAGA | TTTT | 406 |
| Human-Exon 51 | 28 | 1 | UGGUGGAAAAUCUUCAUUUUAAAG | TTTT | 407 |
| Human-Exon 51 | 29 | 1 | UUGGUGGAAAAUCUUCAUUUUAAA | TTTC | 408 |
| Human-Exon 51 | 30 | 1 | GUGAUUGGUGGAAAAUCUUCAUUU | TTTA | 409 |
| Human-Exon 51 | 31 | 1 | CUAGGAGAGUAAAGUGAUUGGUGG | TTTT | 410 |
| Human-Exon 51 | 32 | 1 | UCUAGGAGAGUAAAGUGAUUGGUG | TTTC | 411 |
| Human-Exon 51 | 33 | 1 | CUGGUGGGAAAUGGUCUAGGAGA | TTTA | 412 |
| Human-Exon 45 | 1 | -1 | guagcacacuguuuaaucuuuucu | tttg | 413 |
| Human-Exon 45 | 2 | -1 | cacacuguuuaaucuuuucucaaa | TTTa | 414 |
| Human-Exon 45 | 3 | -1 | acacuguuuaaucuuuucucaaau | TTTT | 415 |
| Human-Exon 45 | 4 | -1 | cacuguuuaaucuuuucucaaauA | TTTT | 416 |
| Human-Exon 45 | 5 | 1 | AU GU CUUUUU auuugagaaaagau | ttta | 417 |
| Human-Exon 45 | 6 | 1 | AAGCCCCAUGUCUUUUUauuugag | tttt | 418 |
| Human-Exon 45 | 7 | 1 | GAAGCCCCAUGUCUUUUUauuuga | tttc | 419 |
| Human-Exon 45 | 8 | 1 | GUAAGAUACCAAAAAGGCAAAACA | TTTT | 420 |
| Human-Exon 45 | 9 | 1 | UGUAAGAUACCAAAAAGGCAAAAC | TTTT | 421 |
| Human-Exon 45 | 10 | 1 | CUGUAAGAUACCAAAAAGGCAAAA | TTTG | 422 |
| Human-Exon 45 | 11 | 1 | GUUCCUGUAAGAUACCAAAAAGGC | TTTT | 423 |
| Human-Exon 45 | 12 | 1 | AGUUCCUGUAAGAUACCAAAAAGG | TTTG | 424 |
| Human-Exon 45 | 13 | 1 | UCCUGGAGUUCCUGUAAGAUACCA | TTTT | 425 |
| Human-Exon 45 | 14 | 1 | AUCCUGGAGUUCCUGUAAGAUACC | TTTT | 426 |
| Human-Exon 45 | 15 | -1 | GGGAAGAAAUAAUUCAGCAAUCCU | TTTG | 427 |
| Human-Exon 45 | 16 | -1 | GGAAGAAAUAAUUCAGCAAUCCUC | TTTT | 428 |
| Human-Exon 45 | 17 | -1 | GAAGAAAUAAUUCAGCAAUCCUCA | TTTT | 429 |
| Human-Exon 45 | 18 | -1 | AAAACAGAUGCCAGUAUUCUACAG | TTTC | 430 |
| Human-Exon 45 | 19 | -1 | AAACAGAUGCCAGUAUUCUACAGG | TTTT | 431 |
| Human-Exon 45 | 20 | -1 | AACAGAUGCCAGUAUUCUACAGGA | TTTT | 432 |
| Human-Exon 45 | 21 | -1 | GAAUCUGCGGUGGCAGGAGGUCUG | TTTG | 433 |
| Human-Exon 45 | 22 | -1 | AGGUCUGCAAACAGCUGUCAGACA | TTTC | 434 |
| Human-Exon 45 | 23 | -1 | GGUCUGCAAACAGCUGUCAGACAG | TTTT | 435 |
| Human-Exon 45 | 24 | -1 | GUCUGCAAACAGCUGUCAGACAGA | TTTT | 436 |
| Human-Exon 45 | 25 | -1 | UCUGCAAACAGCUGUCAGACAGAA | TTTT | 437 |
| Human-Exon 45 | 26 | -1 | UAGGGCGACAGAUCUAAUAGGAAU | TTTC | 438 |
| Human-Exon 45 | 27 | -1 | AGGGCGACAGAUCUAAUAGGAAUG | TTTT | 439 |
| Human-Exon 45 | 28 | 1 | UAAAGAAAGCUUAAAAAGUCUGCU | TTTT | 440 |
| Human-Exon 45 | 29 | 1 | CUAAAGAAAGCUUAAAAAGUCUGC | TTTA | 441 |
| Human-Exon 45 | 30 | 1 | AAAUAUUCUUCUAAAGAAAGCUUA | TTTT | 442 |
| Human-Exon 45 | 31 | 1 | GAAAUAUUCUUCUAAAGAAAGCUU | TTTT | 443 |
| Human-Exon 45 | 32 | 1 | UGAAAUAUUCUUCUAAAGAAAGCU | TTTA | 444 |
| Human-Exon 45 | 33 | 1 | UCUCUCAUGAAAUAUUCUUCUAAA | TTTC | 445 |
| Human-Exon 45 | 34 | 1 | AUAAUCUCUCAUGAAAUAUUCUUC | TTTA | 446 |
| Human-Exon 44 | 1 | 1 | GCGUAUAUUUUUUGGUUAUACUGA | TTTG | 447 |
| Human-Exon 44 | 2 | 1 | ucaagaaaaauagauggauuaugu | tttt | 448 |
| Human-Exon 44 | 3 | 1 | aucaagaaaaauagauggauuaug | ttta | 449 |
| Human-Exon 44 | 4 | 1 | CAGGUaaaagcauauggaucaaga | tttt | 450 |
| Human-Exon 44 | 5 | 1 | GCAGGUaaaagcauauggaucaag | tttt | 451 |
| Human-Exon 44 | 6 | 1 | UGCAGGUaaaagcauauggaucaa | tttc | 452 |
| Human-Exon 44 | 7 | -1 | CAGGCGAUUUGACAGAUCUGUUGA | TTTC | 453 |
| Human-Exon 44 | 8 | 1 | AGAUCUGUCAAAUCGCCUGCAGGU | tttt | 454 |
| Human-Exon 44 | 9 | 1 | CAGAUCUGUCAAAUCGCCUGCAGG | ttta | 455 |
| Human-Exon 44 | 10 | 1 | GCCGCCAUUUCUCAACAGAUCUGU | TTTG | 456 |
| Human-Exon 44 | 11 | -1 | AAUGGCGGCGUUUUCAUUAUGAUA | TTTA | 457 |
| Human-Exon 44 | 12 | 1 | AUUAAAUAUCUUUAUAUCAUAAUG | TTTT | 458 |
| Human-Exon 44 | 13 | -1 | UGAGAAUUGGGAACAUGCUAAAUA | TTTG | 459 |
| Human-Exon 44 | 14 | -1 | GGUAAGUCUUUGAUUUGUUUUUUC | TTTC | 460 |
| Human-Exon 44 | 15 | 1 | AAAUACAAUUUCGAAAAAACAAAU | TTTG | 461 |
| Human-Exon 44 | 16 | 1 | AAGAUAAAUACAAUUUCGAAAAAA | TTTG | 462 |
| Human-Exon 44 | 17 | 1 | GCUGAAGAUAAAUACAAUUUCGAA | TTTT | 463 |
| Human-Exon 44 | 18 | 1 | UGCUGAAGAUAAAUACAAUUUCGA | TTTT | 464 |
| Human-Exon 44 | 19 | 1 | GUGCUGAAGAUAAAUACAAUUUCG | TTTT | 465 |
| Human-Exon 44 | 20 | 1 | UGUGCUGAAGAUAAAUACAAUUUC | TTTC | 466 |
| Human-Exon 44 | 21 | -1 | GCACAUCUGGACUCUUUAACUUCU | TTTA | 467 |
| Human-Exon 44 | 22 | 1 | UAAAGAGUCCAGAUGUGCUGAAGA | TTTA | 468 |
| Human-Exon 44 | 23 | -1 | AAGAUCAGGUUCUGAAGGGUGAUG | TTTC | 469 |
| Human-Exon 44 | 24 | 1 | UUCAGAACCUGAUCUUUAAGAAGU | TTTA | 470 |
| Human-Exon 44 | 25 | 1 | AAUAUAAUGAUGACAACAACAGUC | TTTT | 471 |
| Human-Exon 44 | 26 | 1 | UAAUAUAAUGAUGACAACAACAGU | TTTG | 472 |
| Human-Exon 53 | 1 | -1 | UUUAUUUUUCCUUUUAUUCUAGUU | TTTC | 473 |
| Human-Exon 53 | 2 | 1 | AAAGGAAAAAUAAAUAUAUAGUAG | TTTA | 474 |
| Human-Exon 53 | 3 | 1 | UUUCAACUAGAAUAAAAGGAAAAA | TTTA | 475 |
| Human-Exon 53 | 4 | 1 | AUUCUUUCAACUAGAAUAAAAGGA | TTTT | 476 |
| Human-Exon 53 | 5 | 1 | AAUUCUUUCAACUAGAAUAAAAGG | TTTT | 477 |
| Human-Exon 53 | 6 | 1 | GAAUUCUUUCAACUAGAAUAAAAG | TTTC | 478 |
| Human-Exon 53 | 7 | 1 | AUUCUGAAUUCUUUCAACUAGAAU | TTTT | 479 |
| Human-Exon 53 | 8 | 1 | GAUUCUGAAUUCUUUCAACUAGAA | TTTA | 480 |
| Human-Exon 53 | 9 | -1 | CAGAACCGGAGGCAACAGUUGAAU | TTTC | 481 |
| Human-Exon 53 | 10 | -1 | GGAGGCAACAGUUGAAUGAAAUGU | TTTA | 482 |
| Human-Exon 53 | 11 | -1 | UAUACAGUAGAUGCAAUCCAAAAG | TTTT | 483 |
| Human-Exon 53 | 12 | -1 | GAUGCAAUCCAAAAGAAAAUCACA | TTTC | 484 |
| Human-Exon 53 | 13 | -1 | AAUCACAGAAACCAAGGUUAGUAU | TTTG | 485 |
| Human-Exon 53 | 14 | -1 | AGGUUAGUAUCAAAGAUACCUUU | TTTA | 486 |
| Human-Exon 53 | 15 | -1 | GGUUAGUAUCAAAGAUACCUUUUU | TTTT | 487 |
| Human-Exon 53 | 16 | -1 | AGUAUCAAAGAUACCUUUUUAAAA | TTTA | 488 |
| Human-Exon 53 | 17 | -1 | GUAUCAAAGAUACCUUUUUAAAAU | TTTT | 489 |
| Human-Exon 46 | 1 | -1 | UGUUUGUGUCCCAGUUUGCAUUAA | TTTG | 490 |
| Human-Exon 46 | 2 | 1 | CUGGGACACAAACAUGGCAAUUUA | TTTT | 491 |
| Human-Exon 46 | 3 | 1 | ACUGGGACACAAACAUGGCAAUUU | TTTT | 492 |
| Human-Exon 46 | 4 | 1 | AACUGGGACACAAACAUGGCAAUU | TTTA | 493 |
| Human-Exon 46 | 5 | 1 | UAUUUGUUAAUGCAAACUGGGACA | TTTG | 494 |
| Human-Exon 46 | 6 | -1 | ACAAAUAGUUUGAGAACUAUGUUG | tttC | 495 |
| Human-Exon 46 | 7 | -1 | CAAAUAGUUUGAGAACUAUGUUGG | tttt | 496 |
| Human-Exon 46 | 8 | -1 | AAAUAGUUUGAGAACUAUGUUGGa | tttt | 497 |
| Human-Exon 46 | 9 | -1 | AUAGUUUGAGAACUAUGUUGGaaa | tttt | 498 |
| Human-Exon 46 | 10 | -1 | UAGUUUGAGAACUAUGUUGGaaaa | tttt | 499 |
| Human-Exon 46 | 11 | -1 | AGUUUGAGAACUAUGUUGGaaaaa | tttt | 500 |
| Human-Exon 46 | 12 | 1 | UAGUUCUCAAACUAUUUGUUAAUG | TTTG | 501 |
| Human-Exon 46 | 13 | 1 | UAuuuuuuuuuCCAACAUAGUUCU | TTTG | 502 |
| Human-Exon 46 | 14 | -1 | CUUCUUUCUCCAGGCUAGAAGAAC | TTTT | 503 |
| Human-Exon 46 | 15 | 1 | CUUCUAGCCUGGAGAAAGAAGAAU | TTTT | 504 |
| Human-Exon 46 | 16 | 1 | UCUUCUAGCCUGGAGAAAGAAGAA | TTTA | 505 |
| Human-Exon 46 | 17 | 1 | AUUCUUUUGUUCUUCUAGCCUGGA | TTTC | 506 |
| Human-Exon 46 | 18 | -1 | CAAAAGAAUAUCUUGUCAGAAUUU | TTTG | 507 |
| Human-Exon 46 | 19 | -1 | CUGGAAAAGAGCAGCAACUAAAAG | TTTT | 508 |
| Human-Exon 46 | 20 | -1 | CAAGUCAAGGUAAUUUUAUUUUCU | TTTG | 509 |
| Human-Exon 46 | 21 | -1 | CAAAUCCCCCAGGGCCUGCUUGCA | TTTA | 510 |
| Human-Exon 46 | 22 | 1 | AGGCCCUGGGGGAUUUGAGAAAAU | TTTT | 511 |
| Human-Exon 46 | 23 | 1 | CAGGCCCUGGGGGAUUUGAGAAAA | TTTA | 512 |
| Human-Exon 46 | 24 | 1 | CAAGCAGGCCCUGGGGGAUUUGAG | TTTT | 513 |
| Human-Exon 46 | 25 | 1 | GCAAGCAGGCCCUGGGGGAUUUGA | TTTC | 514 |
| Human-Exon 46 | 26 | 1 | GCAGAAAACCAAUGAUUGAAUUAA | TTTT | 515 |
| Human-Exon 46 | 27 | 1 | GGCAGAAAACCAAUGAUUGAAUUA | TTTT | 516 |
| Human-Exon 46 | 28 | 1 | GGGCAGAAAACCAAUGAUUGAAUU | TTTT | 517 |
| Human-Exon 46 | 29 | 1 | UGGGCAGAAAACCAAUGAUUGAAU | TTTA | 518 |
| Human-Exon 46 | 30 | -1 | AUUAGGUUAUUCAUAGUUCCUUGC | TTTA | 519 |
| Human-Exon 46 | 31 | 1 | AACUAUGAAUAACCUAAUGGGCAG | TTTT | 520 |
| Human-Exon 46 | 32 | 1 | GAACUAUGAAUAACCUAAUGGGCA | TTTC | 521 |
| Human-Exon 52 | 1 | -1 | UAUUUCCUGUUAAAUUGUUUUCUA | TTTA | 522 |
| Human-Exon 52 | 2 | 1 | GGUUUAUAGAAAACAAUUUAACAG | TTTC | 523 |
| Human-Exon 52 | 3 | -1 | AUACAGUAACAUCUUUUUUAUUUC | TTTA | 524 |
| Human-Exon 52 | 4 | -1 | UACAGUAACAUCUUUUUUAUUUCU | TTTT | 525 |
| Human-Exon 52 | 5 | 1 | AUGUUACUGUAUAAGGGUUUAUAG | TTTT | 526 |
| Human-Exon 52 | 6 | 1 | GAUGUUACUGUAUAAGGGUUUAUA | TTTC | 527 |
| Human-Exon 52 | 7 | 1 | CAGCCAAAACACUUUUAGAAAUAA | TTTT | 528 |
| Human-Exon 52 | 8 | 1 | CCAGCCAAAACACUUUUAGAAAUA | TTTT | 529 |
| Human-Exon 52 | 9 | 1 | ACCAGCCAAAACACUUUUAGAAAU | TTTT | 530 |
| Human-Exon 52 | 10 | 1 | GACCAGCCAAAACACUUUUAGAAA | TTTA | 531 |
| Human-Exon 52 | 11 | 1 | GUGAGACCAGCCAAAACACUUUUA | TTTC | 532 |
| Human-Exon 52 | 12 | -1 | AAUUGUACUUUACUUUGUAUUAUG | TTTA | 533 |
| Human-Exon 52 | 13 | -1 | AUUGUACUUUACUUUGUAUUAUGU | TTTT | 534 |
| Human-Exon 52 | 14 | 1 | UAAAGUACAAUUGUGAGACCAGCC | TTTT | 535 |
| Human-Exon 52 | 15 | 1 | GUAAAGUACAAUUGUGAGACCAGC | TTTG | 536 |
| Human-Exon 52 | 16 | 1 | GUAUUCCUUUUACAUAAUACAAAG | TTTA | 537 |
| Human-Exon 52 | 17 | 1 | GUUGUGUAUUCCUUUUACAUAAUA | TTTG | 538 |
| Human-Exon 52 | 18 | 1 | AUCCUGCAUUGUUGCCUGUAAGAA | TTTG | 539 |
| Human-Exon 52 | 19 | 1 | UUCCAACUGGGGACGCCUCUGUUC | TTTG | 540 |
| Human-Exon 52 | 20 | -1 | UUGGAAGAACUCAUUACCGCUGCC | TTTG | 541 |
| Human-Exon 52 | 21 | -1 | UCAUUACCGCUGCCCAAAAUUUGA | TTTT | 542 |
| Human-Exon 52 | 22 | 1 | CUCUUGAUUGCUGGUCUUGUUUUU | TTTG | 543 |
| Human-Exon 52 | 23 | -1 | GUUUUUUAACAAGCAUGGGACACA | TTTG | 544 |
| Human-Exon 52 | 24 | 1 | CUUUGUGUGUCCCAUGCUUGUUAA | TTTT | 545 |
| Human-Exon 52 | 25 | 1 | GCUUUGUGUGUCCCAUGCUUGUUA | TTTT | 546 |
| Human-Exon 52 | 26 | 1 | UGCUUUGUGUGUCCCAUGCUUGUU | TTTT | 547 |
| Human-Exon 52 | 27 | 1 | UUGCUUUGUGUGUCCCAUGCUUGU | TTTA | 548 |
| Human-Exon 52 | 28 | -1 | AGCAAGAUGCAUGACAAGUUUCAA | TTTA | 549 |
| Human-Exon 52 | 29 | -1 | GCAAGAUGCAUGACAAGUUUCAAU | TTTT | 550 |
| Human-Exon 52 | 30 | -1 | CAAGAUGCAUGACAAGUUUCAAUA | TTTT | 551 |
| Human-Exon 52 | 31 | 1 | GAUAUAUGAACUUAAGUUUUUAUU | TTTC | 552 |
| Human-Exon 50 | 1 | -1 | AUAGAAAUCCAAUAAUAUAUUCAC | TTTG | 553 |
| Human-Exon 50 | 2 | -1 | AUUAAGAUGUUCAUGAAUUAUCUU | TTTG | 554 |
| Human-Exon 50 | 3 | -1 | UAAGUAAUGUGUAUGCUUUUCUGU | TTTA | 555 |
| Human-Exon 50 | 4 | 1 | AUCUUCUAACUUCCUCUUUAACAG | TTTT | 556 |
| Human-Exon 50 | 5 | 1 | GAUCUUCUAACUUCCUCUUUAACA | TTTC | 557 |
| Human-Exon 50 | 6 | -1 | AUCUGAGCUCUGAGUGGAAGGCGG | TTTA | 558 |
| Human-Exon 50 | 7 | -1 | ACCGUUUACUUCAAGAGCUGAGGG | TTTG | 559 |
| Human-Exon 50 | 8 | 1 | CUGCUUUGCCCUCAGCUCUUGAAG | TTTA | 560 |
| Human-Exon 50 | 9 | -1 | UCUCUUUGGCUCUAGCUAUUUGUU | TTTG | 561 |
| Human-Exon 50 | 10 | -1 | CUCUUUGGCUCUAGCUAUUUGUUC | TTTT | 562 |
| Human-Exon 50 | 11 | 1 | CACUUUUGAACAAAUAGCUAGAGC | TTTG | 563 |
| Human-Exon 50 | 12 | 1 | UCACUUCAUAGUUGCACUUUUGAA | TTTG | 564 |
| Human-Exon 50 | 13 | -1 | AUGAAGUGAUGACUGGGUGAGAGA | TTTC | 565 |
| Human-Exon 50 | 14 | -1 | UGAAGUGAUGACUGGGUGAGAGAG | TTTT | 566 |
| Human-Exon 43 | 1 | 1 | AAGAGAAAAauauauauauauaua | TTTG | 567 |
| Human-Exon 43 | 2 | 1 | GAAUUAGCUGUCUAUAGAAAGAGA | tTTT | 568 |
| Human-Exon 43 | 3 | 1 | UGAAUUAGCUGUCUAUAGAAAGAG | TTTT | 569 |
| Human-Exon 43 | 4 | -1 | AGCUAAUUCAUUUUUUUACUGUUU | TTTA | 570 |
| Human-Exon 43 | 5 | 1 | AUGAAUUAGCUGUCUAUAGAAAGA | TTTC | 571 |
| Human-Exon 43 | 6 | -1 | GCUAAUUCAUUUUUUUACUGUUUU | TTTT | 572 |
| Human-Exon 43 | 7 | 1 | AAAAAAAUGAAUUAGCUGUCUAUA | TTTC | 573 |
| Human-Exon 43 | 8 | -1 | UUAAAAUUUUUAUAUUACAGAAUA | TTTA | 574 |
| Human-Exon 43 | 9 | -1 | UAAAAUUUUUAUAUUACAGAAUAU | TTTT | 575 |
| Human-Exon 43 | 10 | 1 | AUAUAAAAAUUUUAAAACAGUAAA | TTTT | 576 |
| Human-Exon 43 | 11 | 1 | AAUAUAAAAAUUUUAAAACAGUAA | TTTT | 577 |
| Human-Exon 43 | 12 | 1 | UAAUAUAAAAAUUUUAAAACAGUA | TTTT | 578 |
| Human-Exon 43 | 13 | 1 | GUAAUAUAAAAAUUUUAAAACAGU | TTTT | 579 |
| Human-Exon 43 | 14 | 1 | UGUAAUAUAAAAAUUUUAAAACAG | TTTA | 580 |
| Human-Exon 43 | 15 | 1 | UAUAUUCUGUAAUAUAAAAAUUUU | TTTT | 581 |
| Human-Exon 43 | 16 | 1 | UUAUAUUCUGUAAUAUAAAAAUUU | TTTA | 582 |
| Human-Exon 43 | 17 | -1 | CAGAAUAUAAAAGAUAGUCUACAA | TTTG | 583 |
| Human-Exon 43 | 18 | 1 | CUAUCUUUUAUAUUCUGUAAUAUA | TTTT | 584 |
| Human-Exon 43 | 19 | 1 | ACUAUCUUUUAUAUUCUGUAAUAU | TTTT | 585 |
| Human-Exon 43 | 20 | 1 | GACUAUCUUUUAUAUUCUGUAAUA | TTTA | 586 |
| Human-Exon 43 | 21 | -1 | CAUAGCAAGAAGACAGCAGCAUUG | TTTG | 587 |
| Human-Exon 43 | 22 | 1 | CAUUUUGUUAACUUUUUCCCAUUG | TTTC | 588 |
| Human-Exon 43 | 23 | -1 | CAUAUAUUUUUCUUGAUACUUGCA | TTTC | 589 |
| Human-Exon 43 | 24 | 1 | AAAUCAUUUCUGCAAGUAUCAAGA | TTTT | 590 |
| Human-Exon 43 | 25 | 1 | CAAAUCAUUUCUGCAAGUAUCAAG | TTTT | 591 |
| Human-Exon 43 | 26 | 1 | ACAAAUCAUUUCUGCAAGUAUCAA | TTTC | 592 |
| Human-Exon 43 | 27 | 1 | AUAAAUUCUACAGUUCCCUGAAAA | TTTG | 593 |
| Human-Exon 43 | 28 | -1 | GAAUUUAUUUCAGUACCCUCCAUG | TTTC | 594 |
| Human-Exon 43 | 29 | -1 | AAUUUAUUUCAGUACCCUCCAUGG | TTTT | 595 |
| Human-Exon 43 | 30 | 1 | UGAAAUAAAUUCUACAGUUCCCUG | TTTT | 596 |
| Human-Exon 43 | 31 | -1 | AUUUAUUUCAGUACCCUCCAUGGA | TTTT | 597 |
| Human-Exon 43 | 32 | 1 | CUGAAAUAAAUUCUACAGUUCCCU | TTTC | 598 |
| Human-Exon 43 | 33 | -1 | UUUAUUUCAGUACCCUCCAUGGAA | TTTT | 599 |
| Human-Exon 43 | 34 | -1 | UACCCUCCAUGGAAAAAAGACAGG | TTTC | 600 |
| Human-Exon 43 | 35 | -1 | ACCCUCCAUGGAAAAAAGACAGGG | TTTT | 601 |
| Human-Exon 43 | 36 | -1 | CCCUCCAUGGAAAAAAGACAGGGA | TTTT | 602 |
| Human-Exon 43 | 37 | 1 | UUUUUUCCAUGGAGGGUACUGAAA | TTTA | 603 |
| Human-Exon 43 | 38 | 1 | UGUCUUUUUUCCAUGGAGGGUACU | TTTC | 604 |
| Human-Exon 6 | 1 | 1 | CCUUGAGCAAGAACCAUGCAAACU | TTTA | 605 |
| Human-Exon 6 | 2 | -1 | UGCUCAAGGAAUGCAUUUUCUUAU | TTTC | 606 |
| Human-Exon 6 | 3 | -1 | GCUCAAGGAAUGCAUUUUCUUAUG | TTTT | 607 |
| Human-Exon 6 | 4 | 1 | UGCAUUCCUUGAGCAAGAACCAUG | TTTG | 608 |
| Human-Exon 6 | 5 | -1 | GAAAAUUUAUUUCCACAUGUAGGU | TTTG | 609 |
| Human-Exon 6 | 6 | -1 | AAAAUUUAUUUCCACAUGUAGGUC | TTTT | 610 |
| Human-Exon 6 | 7 | -1 | AAAUUUAUUUCCACAUGUAGGUCA | TTTT | 611 |
| Human-Exon 6 | 8 | 1 | CAUGUGGAAAUAAAUUUUCAUAAG | TTTT | 612 |
| Human-Exon 6 | 9 | 1 | ACAUGUGGAAAUAAAUUUUCAUAA | TTTC | 613 |
| Human-Exon 6 | 10 | -1 | CCACAUGUAGGUCAAAAAUGUAAU | TTTC | 614 |
| Human-Exon 6 | 11 | -1 | CACAUGUAGGUCAAAAAUGUAAUG | TTTT | 615 |
| Human-Exon 6 | 12 | -1 | ACAUGUAGGUCAAAAAUGUAAUGA | TTTT | 616 |
| Human-Exon 6 | 13 | 1 | ACAUUUUUGACCUACAUGUGGAAA | TTTA | 617 |
| Human-Exon 6 | 14 | 1 | CAUUACAUUUUUGACCUACAUGUG | TTTC | 618 |
| Human-Exon 6 | 15 | -1 | AAAAAUAUCAUGGCUGGAUUGCAA | TTTG | 619 |
| Human-Exon 6 | 16 | -1 | GCUGGAUUGCAACAAACCAACAGU | TTTC | 620 |
| Human-Exon 6 | 17 | -1 | CUGGAUUGCAACAAACCAACAGUG | TTTT | 621 |
| Human-Exon 6 | 18 | 1 | CCUAUGACUAUGGAUGAGAGCAUU | TTTG | 622 |
| Human-Exon 6 | 19 | -1 | UAGGUAAGAAGAUUACUGAGACAU | TTTA | 623 |
| Human-Exon 6 | 20 | -1 | AUUACUGAGACAUUAAAUAACUUG | TTTA | 624 |
| Human-Exon 6 | 21 | -1 | UUACUGAGACAUUAAAUAACUUGU | TTTT | 625 |
| Human-Exon 6 | 22 | 1 | GGGGAAAAAUAUGUCAUCAGAGUC | TTTA | 626 |
| Human-Exon 6 | 23 | 1 | CAUGAUCUGGAACCAUACUGGGGA | TTTT | 627 |
| Human-Exon 6 | 24 | 1 | ACAUGAUCUGGAACCAUACUGGGG | TTTT | 628 |
| Human-Exon 6 | 25 | 1 | GACAUGAUCUGGAACCAUACUGGG | TTTC | 629 |
| Human-Exon 7 | 1 | 1 | uacacacauacacaAAGACAAAUA | TTTA | 630 |
| Human-Exon 7 | 2 | 1 | uacacauacacacauacacaAAGA | TTTG | 631 |
| Human-Exon 7 | 3 | 1 | aacacauacacauacacacauaca | TTtg | 632 |
| Human-Exon 7 | 4 | 1 | AUUCCAGUCAAAUAGGUCUGGCCU | ttTT | 633 |
| Human-Exon 7 | 5 | 1 | UAUUCCAGUCAAAUAGGUCUGGCC | tTTA | 634 |
| Human-Exon 7 | 6 | 1 | GCUGGCAAACCACACUAUUCCAGU | TTTG | 635 |
| Human-Exon 7 | 7 | 1 | AGUCGUUGUGUGGCUGACUGCUGG | TTTG | 636 |
| Human-Exon 7 | 8 | -1 | CGCCAGAUAUCAAUUAGGCAUAGA | TTTC | 637 |
| Human-Exon 7 | 9 | -1 | AAACUACUCGAUCCUGAAGGUUGG | TTTA | 638 |
| Human-Exon 7 | 10 | 1 | CAUACUAAAAGCAGUGGUAGUCCA | TTTC | 639 |
| Human-Exon 7 | 11 | 1 | GAAAACAUUAAACUCUACCAUACU | TTTT | 640 |
| Human-Exon 7 | 12 | 1 | UGAAAACAUUAAACUCUACCAUAC | TTTA | 641 |
| Human-Exon 8 | 1 | -1 | UUGUUCAUUAUCCUUUUAGAGUCU | TTTG | 642 |
| Human-Exon 8 | 2 | 1 | AAAGGAUAAUGAACAAAUCAAAGU | TTTA | 643 |
| Human-Exon 8 | 3 | -1 | UAUCCUUUUAGAGUCUCAAAUAUA | TTTC | 644 |
| Human-Exon 8 | 4 | 1 | ACUCUAAAAGGAUAAUGAACAAAU | TTTG | 645 |
| Human-Exon 8 | 5 | -1 | UUUUAGAGUCUCAAAUAUAGAAAC | TTTG | 646 |
| Human-Exon 8 | 6 | -1 | UUUAGAGUCUCAAAUAUAGAAACC | TTTT | 647 |
| Human-Exon 8 | 7 | -1 | UUAGAGUCUCAAAUAUAGAAACCA | TTTT | 648 |
| Human-Exon 8 | 8 | 1 | UUGAGACUCUAAAAGGAUAAUGAA | TTTG | 649 |
| Human-Exon 8 | 9 | 1 | UUUGGUUUCUAUAUUUGAGACUCU | TTTT | 650 |
| Human-Exon 8 | 10 | 1 | UUUUGGUUUCUAUAUUUGAGACUC | TTTA | 651 |
| Human-Exon 8 | 11 | -1 | AGCAUUGAAGCCAUCCAGGAAGUG | TTTC | 652 |
| Human-Exon 8 | 12 | 1 | GCUUCAAUGCUCACUUGUUGAGGC | TTTT | 653 |
| Human-Exon 8 | 13 | 1 | GGCUUCAAUGCUCACUUGUUGAGG | TTTG | 654 |
| Human-Exon 8 | 14 | -1 | AGUGGAAAUGUUGCCAAGGCCACC | TTTA | 655 |
| Human-Exon 8 | 15 | -1 | GUUGCCAAGGCCACCUAAAGUGAC | TTTA | 656 |
| Human-Exon 8 | 16 | -1 | GAAGAACAUUUUCAGUUACAUCAU | TTTG | 657 |
| Human-Exon 8 | 17 | -1 | AUCAAAUGCACUAUUCUCAACAGG | TTTA | 658 |
| Human-Exon 8 | 18 | 1 | AUAGUGCAUUUGAUGAUGUAACUG | TTTT | 659 |
| Human-Exon 8 | 19 | 1 | AAUAGUGCAUUUGAUGAUGUAACU | TTTC | 660 |
| Human-Exon 8 | 20 | -1 | ACUAUUCUCAACAGGUAAAGUGUG | TTTA | 661 |
| Human-Exon 8 | 21 | 1 | UACCUAAAAAUGCAUAUAAAACAG | TTTT | 662 |
| Human-Exon 8 | 22 | 1 | AUACCUAAAAAUGCAUAUAAAACA | TTTC | 663 |
| Human-Exon 8 | 23 | 1 | CACGUAAUACCUAAAAAUGCAUAU | TTTT | 664 |
| Human-Exon 8 | 24 | 1 | GCACGUAAUACCUAAAAAUGCAUA | TTTA | 665 |
| Human-Exon 8 | 25 | 1 | auauauauGUGCACGUAAUACCUA | TTTT | 666 |
| Human-Exon 8 | 26 | 1 | uauauauauGUGCACGUAAUACCU | TTTT | 667 |
| Human-Exon 8 | 27 | 1 | auauauauauGUGCACGUAAUACC | TTTA | 668 |
| Human-Exon 55 | 1 | -1 | CUGCACAAUAUUAUAGUUGUUGCU | TTTA | 669 |
| Human-Exon 55 | 2 | 1 | AUAAAAAGAGAAAGAUGGAGGAAC | TTTA | 670 |
| Human-Exon 55 | 3 | 1 | CACCUAGUGAACUCCAUAAAAAGA | TTTC | 671 |
| Human-Exon 55 | 4 | 1 | AUGGUGCACCUAGUGAACUCCAUA | TTTT | 672 |
| Human-Exon 55 | 5 | 1 | AAUGGUGCACCUAGUGAACUCCAU | TTTT | 673 |
| Human-Exon 55 | 6 | 1 | GAAUGGUGCACCUAGUGAACUCCA | TTTA | 674 |
| Human-Exon 55 | 7 | 1 | GACCAAAUGUUCAGAUGCAAUUAU | TTTA | 675 |
| Human-Exon 55 | 8 | 1 | UCGCUCACUCACCCUGCAAAGGAC | TTTG | 676 |
| Human-Exon 55 | 9 | -1 | AGUGAGCGAGAGGCUGCUUUGGAA | TTTC | 677 |
| Human-Exon 55 | 10 | 1 | GCAGCCUCUCGCUCACUCACCCUG | TTTG | 678 |
| Human-Exon 55 | 11 | 1 | UUGCAGUAAUCUAUGAGUUUCUUC | TTTG | 679 |
| Human-Exon 55 | 12 | -1 | CUGCAACAGUUCCCCCUGGACCUG | TTTC | 680 |
| Human-Exon 55 | 13 | -1 | UGCAACAGUUCCCCCUGGACCUGG | TTTT | 681 |
| Human-Exon 55 | 14 | -1 | UUUCUUGCCUGGCUUACAGAAGCU | TTTC | 682 |
| Human-Exon 55 | 15 | 1 | UUUCAGCUUCUGUAAGCCAGGCAA | TTTC | 683 |
| Human-Exon 55 | 16 | -1 | GUCCUACAGGAUGCUACCCGUAAG | TTTC | 684 |
| Human-Exon 55 | 17 | -1 | GGCUCCUAGAAGACUCCAAGGGAG | TTTA | 685 |
| Human-Exon 55 | 18 | -1 | GCUCCUAGAAGACUCCAAGGGAGU | TTTT | 686 |
| Human-Exon 55 | 19 | -1 | CUCCAAGGGAGUAAAAGAGCUGAU | TTTC | 687 |
| Human-Exon 55 | 20 | 1 | UGGAUCCACAAGAGUGCUAAAGCG | TTTC | 688 |
| Human-Exon 55 | 21 | 1 | GUUCAAUUGGAUCCACAAGAGUGC | TTTA | 689 |
| Human-Exon 55 | 22 | -1 | UACUUGUAACUGACAAGCCAGGGA | TTTG | 690 |
| Human-Exon 55 | 23 | -1 | ACUUGUAACUGACAAGCCAGGGAC | TTTT | 691 |
| Human-Exon 55 | 24 | -1 | GUAACUGACAAGCCAGGGACAAAA | TTTG | 692 |
| Human-Exon 55 | 25 | -1 | UAACUGACAAGCCAGGGACAAAAC | TTTT | 693 |
| Human-Exon 55 | 26 | 1 | UCCCUGGCUUGUCAGUUACAAGUA | TTTG | 694 |
| Human-G1-exon51 | | 1 | CAGAGUAACAGUCUGAGUAGGAGc | TTTA | 695 |
| Human-G2-exon51 | | 1 | uacuuuguuuagcaauacauggua | TTTC | 696 |
| Human-G3-exon51 | | -1 | uggcucaaauuguuacucuucaau | TTTA | 697 |
| mouse-Exon23-G1 | | 1 | CUUUCAAagaacuuugcagagccu | TTTG | 698 |
| mouse-Exon23-G2 | | 1 | guugaaGCCAUUUUGUUGCUCUUU | TTTG | 699 |
| mouse-Exon23 -G3 | | 1 | guugaaGCCAUUUUAUUGCUCUUU | TTTG | 700 |
| mouse-Exon23 -G4 | | -1 | uuuugagGCUCUGCAAAGUUCUUU | TTTC | 701 |
| mouse-Exon23 -G5 | | -1 | aguuauuaaugcauagauauucag | TTTA | 702 |
| mouse-Exon23 -G6 | | -1 | uauaauaugcccuguaauauaaua | TTTC | 703 |
| mouse-Exon23 -G7 | | 1 | uaaaggccaaaccucggcuuaccU | TTTC | 704 |
| mouse-Exon23 -G8 | | 1 | ucaauaucuuugaaggacucuggg | TTTA | 705 |

| | | | | | |
|---|---|---|---|---|---|
| * In this table, upper case letters represent sgRNA nucleotides that align to the exon sequence of the gene. Lower case letters represent sgRNA nucleotides that align to the intron sequence of the gene. | | | | | |

### VI. Sequence Tables

**Table 8: Genomic target sites for sgRNA in mouse Dmd Exon 51**

| **ID sgRNA** | **Strand** | **Target site** | **SEQ ID NO:** | **PAM** |
|---|---|---|---|---|
| Ex51-SA1 | 3' | AGAGTAACAGTCTGACTGG | 706 | CAG |
| Ex51-SD | 5' | GAAATGATCATCAAACAGA | 707 | AGG |
| Ex51-SA-2 | 3' | CACTAGAGTAACAGTCTGAC | 708 | TGG |

**Table 9: gRNA sequences targeting mouse Dmd Exon 51**

| **ID sgRNA** | **Strand** | **Target site** | **SEQ ID NO:** | **PAM** |
|---|---|---|---|---|
| Ex51-SA1 | 3' | CCAGUCAGACUGUUACUCU | 709 | CAG |
| Ex51-SD | 5' | UCUGUUUGAUGAUCAUUUC | 710 | AGG |
| Ex51-SA-2 | 3' | GUCAGACUGUUACUCUAGUG | 711 | TGG |

**Table 10: Genomic target sequences for sgRNAs targeting human Dmd Exon 51**

| **ID sgRNA** | **Strand** | **Target site** | **SEQ ID NO:** | **PAM** |
|---|---|---|---|---|
| Ex51-SA | 3' | AGAGTAACAGTCTGAGTAG | 712 | GAG |
| Ex51-SD | 5' | GAGATGATCATCAAGCAGA | 713 | AGG |
| Ex51-SA-2 | 3' | CACCAGAGTAACAGTCTGAG | 714 | TAG |

**Table 11: sgRNA sequences targeting human Dmd Exon 51**

| **ID sgRNA** | **Strand** | **Target site** | **SEQ ID NO:** | **PAM** |
|---|---|---|---|---|
| Ex51-SA | 3' | CUACUCAGACUGUUACUCU | 715 | GAG |
| Ex51-SD | 5' | UCUGCUUGAUGAUCAUCUC | 716 | AGG |
| Ex51-SA-2 | 3' | CUCAGACUGUUACUCUGGUG | 717 | TAG |

**Table 12: Genomic target sequences for sgRNAs targeting sites in various human Dmd Exons**

| **ID sgRNA** | **Strand** | **Target site** | **SEQ ID NO:** | **PAM** |
|---|---|---|---|---|
| Exon51-#1 | 3' | CAGAGTAACAGTCTGAGTAG | 947 | GAG |
| Exon51-#2 | 3' | CACCAGAGTAACAGTCTGAG | 718 | TAG |
| Exon51-#3 | 3' | TATTTTGGGTTTTTGCAAAA | 719 | AGG |
| Exon51-#4 | 3' | AGTAGGAGCTAAAATATTTT | 720 | GGG |
| Exon51-#5 | 3' | GAGTAGGAGCTAAAATATTT | 721 | TGG |
| Exon51-#6 | 3' | ACCAGAGTAACAGTCTGAGT | 722 | AGG |
| Exon51-#7 | 5' | TCCTACTCAGACTGTTACTC | 723 | TGG |
| Exon51-#8 | 5' | TACTCTGGTGACACAACCTG | 724 | TGG |
| Exon51-#9 | 3' | GCAGTTTCCTTAGTAACCAC | 725 | AGG |
| Exon51-#10 | 5' | GACACAACCTGTGGTTACTA | 726 | AGG |
| Exon51-#11 | 3' | TGTCACCAGAGTAACAGTCT | 727 | GAG |
| Exon51-#12 | 3' | AGGTTGTGTCACCAGAGTAA | 728 | CAG |
| Exon51-#13 | 3' | AACCACAGGTTGTGTCACCA | 729 | GAG |
| Exon51-#14 | 3' | GTAACCACAGGTTGTGTCAC | 730 | CAG |
| | | | | |
| Exon53-#1 | 5' | ATTTATTTTTCCTTTTATTC | 731 | TAG |
| Exon53-#2 | 5' | TTTCCTTTTATTCTAGTTGA | 732 | AAG |
| Exon53-#3 | 3' | TGATTCTGAATTCTTTCAAC | 733 | TAG |
| Exon53-#4 | 3' | AATTCTTTCAACTAGAATAA | 734 | AAG |
| Exon53-#6 | 5' | TTATTCTAGTTGAAAGAATT | 735 | CAG |
| Exon53-#7 | 5' | TAGTTGAAAGAATTCAGAAT | 736 | CAG |
| Exon53-#8 | 5' | AATTCAGAATCAGTGGGATG | 737 | AAG |
| Exon53-#9 | 3' | ATTCTTTCAACTAGAATAAA | 738 | AGG |
| Exon53-#10 | 5' | TTGAAAGAATTCAGAATCAG | 739 | TGG |
| Exon53-#11 | 5' | TGAAAGAATTCAGAATCAGT | 740 | GGG |
| Exon53-#12 | 3' | ACTGTTGCCTCCGGTTCTGA | 741 | AGG |
| | | | | |
| Exon44-#1 | 3' | CAGATCTGTCAAATCGCCTG | 742 | CAG |
| Exon44-#2 | 3' | AAAACGCCGCCATTTCTCAA | 743 | CAG |
| Exon44-#3 | 3' | AGATCTGTCAAATCGCCTGC | 744 | AGG |
| Exon44-#4 | 3' | TATGGATCAAGAAAAATAGA | 745 | TGG |
| Exon44-#5 | 3' | CGCCTGCAGGTAAAAGCATA | 746 | TGG |
| Exon44-#6 | 5' | ATCCATATGCTTTTACCTGC | 747 | AGG |
| Exon44-#8 | 5' | TTGACAGATCTGTTGAGAAA | 748 | TGG |
| Exon44-#9 | 5' | ACAGATCTGTTGAGAAATGG | 749 | CGG |
| Exon44-#11 | 5' | GGCGATTTGACAGATCTGTT | 750 | GAG |
| Exon44-#13 | 5' | GGCGTTTTCATTATGATATA | 751 | AAG |
| Exon44-#14 | 5' | ATGATATAAAGATATTTAAT | 752 | CAG |
| Exon44-#15 | 5' | GATATTTAATCAGTGGCTAA | 753 | CAG |
| Exon44-#16 | 5' | ATTTAATCAGTGGCTAACAG | 754 | AAG |
| Exon44-# 17 | 3' | AGAAACTGTTCAGCTTCTGT | 755 | TAG |
| | | | | |
| Exon43-#1 | 5' | GTTTTAAAATTTTTATATTA | 756 | CAG |
| Exon43-#2 | 5' | TTTTATATTACAGAATATAA | 757 | AAG |
| Exon43-#3 | 5' | ATATTACAGAATATAAAAGA | 758 | TAG |
| | | | | |
| Exon45-#1 | 3' | GTTCCTGTAAGATACCAAAA | 759 | AGG |
| Exon45-#2 | 5' | TTGCCTTTTTGGTATCTTAC | 760 | AGG |
| Exon45-#3 | 5' | TGGTATCTTACAGGAACTCC | 761 | AGG |
| Exon45-#4 | 5' | ATCTTACAGGAACTCCAGGA | 762 | TGG |
| Exon45-#5 | 3' | GCCGCTGCCCAATGCCATCC | 763 | TGG |
| Exon45-#6 | 5' | CAGGAACTCCAGGATGGCAT | 764 | TGG |
| Exon45-#7 | 5' | AGGAACTCCAGGATGGCATT | 765 | GGG |
| Exon45-#8 | 5' | TCCAGGATGGCATTGGGCAG | 766 | CGG |
| Exon45-#9 | 5' | GTCAGAACATTGAATGCAAC | 767 | TGG |
| Exon45-#10 | 3' | AGTTCCTGTAAGATACCAAA | 768 | AAG |
| Exon45-#11 | 3' | TGCCATCCTGGAGTTCCTGT | 769 | AAG |
| Exon45-#12 | 5' | TTGGTATCTTACAGGAACTC | 770 | CAG |
| Exon45-#13 | 3' | CGCTGCCCAATGCCATCCTG | 771 | GAG |
| Exon45-#14 | 5' | AACTCCAGGATGGCATTGGG | 772 | CAG |
| Exon45-#15 | 5' | GGGCAGCGGCAAACTGTTGT | 773 | CAG |
| | | | | |
| Exon52-#1 | 3' | AGATCTGTCAAATCGCCTGC | 774 | AGG |
| Exon52-#2 | 3' | AATCCTGCATTGTTGCCTGT | 775 | AAG |
| Exon52-#3 | 5' | CGCTGAAGAACCCTGATACT | 776 | AAG |
| Exon52-#4 | 3' | GAACAAATATCCCTTAGTAT | 777 | CAG |
| Exon52-#5 | 3' | CTGTAAGAACAAATATCCCT | 778 | TAG |
| Exon52-#6 | 5' | CTAAGGGATATTTGTTCTTA | 779 | CAG |
| Exon52-#8 | 5' | TGTTCTTACAGGCAACAATG | 780 | CAG |
| Exon52-#9 | 5' | CAACAATGCAGGATTTGGAA | 781 | CAG |
| Exon52-#10 | 5' | ACAATGCAGGATTTGGAACA | 782 | GAG |
| Exon52-#11 | 5' | ATTTGGAACAGAGGCGTCCC | 783 | CAG |
| Exon52-#12 | 5' | ACAGAGGCGTCCCCAGTTGG | 784 | AAG |
| | | | | |
| Exon2-#1 | 5' | TATTTTTTTATTTTGCATTT | 785 | TAG |
| Exon2-#2 | 5' | TTATTTTGCATTTTAGATGA | 786 | AAG |
| Exon2-#3 | 5' | ATTTTGCATTTTAGATGAAA | 787 | GAG |
| Exon2-#4 | 5' | TTGCATTTTAGATGAAAGAG | 788 | AAG |
| Exon2-#5 | 5' | ATGAAAGAGAAGATGTTCAA | 789 | AAG |

**Table 13: gRNA sequences for targeting sites in various human Dmd Exons**

| **ID sgRNA** | **Strand** | **Target site** | **SEQ ID NO:** | **PAM** |
|---|---|---|---|---|
| Exon51-#1 | 3' | CUACUCAGACUGUUACUCUG | 790 | GAG |
| Exon51-#2 | 3' | CUCAGACUGUUACUCUGGUG | 791 | TAG |
| Exon51-#3 | 3' | UUUUGCAAAAACCCAAAAUA | 792 | AGG |
| Exon51-#4 | 3' | AAAAUAUUUUAGCUCCUACU | 793 | GGG |
| Exon51-#5 | 3' | AAAUAUUUUAGCUCCUACUC | 794 | TGG |
| Exon51-#6 | 3' | ACUCAGACUGUUACUCUGGU | 795 | AGG |
| Exon51-#7 | 5' | GAGUAACAGUCUGAGUAGGA | 796 | TGG |
| Exon51-#8 | 5' | CAGGUUGUGUCACCAGAGUA | 797 | TGG |
| Exon51-#9 | 3' | GUGGUUACUAAGGAAACUGC | 798 | AGG |
| Exon51-#10 | 5' | UAGUAACCACAGGUUGUGUC | 799 | AGG |
| Exon51-#11 | 3' | AGACUGUUACUCUGGUGACA | 800 | GAG |
| Exon51-#12 | 3' | UUACUCUGGUGACACAACCU | 801 | CAG |
| Exon51-#13 | 3' | UGGUGACACAACCUGUGGUU | 802 | GAG |
| Exon51-#14 | 3' | GUGACACAACCUGUGGUUAC | 803 | CAG |
| | | | | |
| Exon53-#1 | 5' | GAAUAAAAGGAAAAAUAAAU | 804 | TAG |
| Exon53-#2 | 5' | UCAACUAGAAUAAAAGGAAA | 805 | AAG |
| Exon53-#3 | 3' | GUUGAAAGAAUUCAGAAUCA | 806 | TAG |
| Exon53-#4 | 3' | UUAUUCUAGUUGAAAGAAUU | 807 | AAG |
| Exon53-#6 | 5' | AAUUCUUUCAACUAGAAUAA | 808 | CAG |
| Exon53-#7 | 5' | AUUCUGAAUUCUUUCAACUA | 809 | CAG |
| Exon53-#8 | 5' | CAUCCCACUGAUUCUGAAUU | 810 | AAG |
| Exon53-#9 | 3' | UUUAUUCUAGUUGAAAGAAU | 811 | AGG |
| Exon53-#10 | 5' | CUGAUUCUGAAUUCUUUCAA | 812 | TGG |
| Exon53-#11 | 5' | ACUGAUUCUGAAUUCUUUCA | 813 | GGG |
| Exon53-#12 | 3' | UCAGAACCGGAGGCAACAGU | 814 | AGG |
| | | | | |
| Exon44-#1 | 3' | CAGGCGAUUUGACAGAUCUG | 815 | CAG |
| Exon44-#2 | 3' | UUGAGAAAUGGCGGCGUUUU | 816 | CAG |
| Exon44-#3 | 3' | GCAGGCGAUUUGACAGAUCU | 817 | AGG |
| Exon44-#4 | 3' | UCUAUUUUUCUUGAUCCAUA | 818 | TGG |
| Exon44-#5 | 3' | UAUGCUUUUACCUGCAGGCG | 819 | TGG |
| Exon44-#6 | 5' | GCAGGUAAAAGCAUAUGGAU | 820 | AGG |
| Exon44-#8 | 5' | UUUCUCAACAGAUCUGUCAA | 821 | TGG |
| Exon44-#9 | 5' | CCAUUUCUCAACAGAUCUGU | 822 | CGG |
| Exon44-#11 | 5' | AACAGAUCUGUCAAAUCGCC | 823 | GAG |
| Exon44-#13 | 5' | UAUAUCAUAAUGAAAACGCC | 824 | AAG |
| Exon44-#14 | 5' | AUUAAAUAUCUUUAUAUCAU | 825 | CAG |
| Exon44-#15 | 5' | UUAGCCACUGAUUAAAUAUC | 826 | CAG |
| Exon44-#16 | 5' | CUGUUAGCCACUGAUUAAAU | 827 | AAG |
| Exon44-#17 | 3' | ACAGAAGCUGAACAGUUUCU | 828 | TAG |
| | | | | |
| Exon43-#1 | 5' | UAAUAUAAAAAUUUUAAAAC | 829 | CAG |
| Exon43-#2 | 5' | UUAUAUUCUGUAAUAUAAAA | 830 | AAG |
| Exon43-#3 | 5' | UCUUUUAUAUUCUGUAAUAU | 831 | TAG |
| | | | | |
| Exon45-#1 | 3' | UUUUGGUAUCUUACAGGAAC | 832 | AGG |
| Exon45-#2 | 5' | GUAAGAUACCAAAAAGGCAA | 833 | AGG |
| Exon45-#3 | 5' | GGAGUUCCUGUAAGAUACCA | 834 | AGG |
| Exon45-#4 | 5' | UCCUGGAGUUCCUGUAAGAU | 835 | TGG |
| Exon45-#5 | 3' | GGAUGGCAUUGGGCAGCGGC | 836 | TGG |
| Exon45-#6 | 5' | AUGCCAUCCUGGAGUUCCUG | 837 | TGG |
| Exon45-#7 | 5' | AAUGCCAUCCUGGAGUUCCU | 838 | GGG |
| Exon45-#8 | 5' | CUGCCCAAUGCCAUCCUGGA | 839 | CGG |
| Exon45-#9 | 5' | GUUGCAUUCAAUGUUCUGAC | 840 | TGG |
| Exon45-#10 | 3' | UUUGGUAUCUUACAGGAACU | 841 | AAG |
| Exon45-#11 | 3' | ACAGGAACUCCAGGAUGGCA | 842 | AAG |
| Exon45-#12 | 5' | GAGUUCCUGUAAGAUACCAA | 843 | CAG |
| Exon45-#13 | 3' | CAGGAUGGCAUUGGGCAGCG | 844 | GAG |
| Exon45-#14 | 5' | CCCAAUGCCAUCCUGGAGUU | 845 | CAG |
| Exon45-#15 | 5' | ACAACAGUUUGCCGCUGCCC | 846 | CAG |
| | | | | |
| Exon52-#1 | 3' | GCAGGCGAUUUGACAGAUCU | 847 | AGG |
| Exon52-#2 | 3' | ACAGGCAACAAUGCAGGAUU | 848 | AAG |
| Exon52-#3 | 5' | AGUAUCAGGGUUCUUCAGCG | 849 | AAG |
| Exon52-#4 | 3' | AUACUAAGGGAUAUUUGUUC | 850 | CAG |
| Exon52-#5 | 3' | AGGGAUAUUUGUUCUUACAG | 851 | TAG |
| Exon52-#6 | 5' | UAAGAACAAAUAUCCCUUAG | 852 | CAG |
| Exon52-#8 | 5' | CAUUGUUGCCUGUAAGAACA | 853 | CAG |
| Exon52-#9 | 5' | UUCCAAAUCCUGCAUUGUUG | 854 | CAG |
| Exon52-#10 | 5' | UGUUCCAAAUCCUGCAUUGU | 855 | GAG |
| Exon52-#11 | 5' | GGGACGCCUCUGUUCCAAAU | 856 | CAG |
| Exon52-#12 | 5' | CCAACUGGGGACGCCUCUGU | 857 | AAG |
| | | | | |
| Exon2-#1 | 5' | ACAGAGGCGUCCCCAGUUGG | 858 | TAG |
| Exon2-#2 | 5' | UCAUCUAAAAUGCAAAAUAA | 859 | AAG |
| Exon2-#3 | 5' | UUUCAUCUAAAAUGCAAAAU | 860 | GAG |
| Exon2-#4 | 5' | CUCUUUCAUCUAAAAUGCAA | 861 | AAG |
| Exon2-#5 | 5' | UUGAACAUCUUCUCUUUCAU | 862 | AAG |

**Table 14: Genomic targeting sequence for sgRNAs targeting dog Dmd Exon 51**

| **ID sgRNA** | **Strand** | **Target site** | **SEQ ID NO:** | **PAM** |
|---|---|---|---|---|
| Ex51-SA-2 | 3' | CACCAGAGTAACAGTCTGAC | 863 | TGG |

**Table 15: gRNA sequence for targeting dog Dmd Exon 51**

| | | | | 07-Jun-2024 |
|---|---|---|---|---|
| **ID sgRNA** | **Strand** | **Target site** | **SEQ ID NO:** | **PAM** |
| Ex51-SA-2 | 3' | GUCAGACUGUUACUCUGGUG | 864 | TGG |

### VII. Examples

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice.

### EXAMPLE 1 - Materials and Methods

**Study Approval.** All experimental procedures involving animals in this study were reviewed and approved by the University of Texas Southwestern Medical Center's Institutional Animal Care and Use Committee.

**CRISPR/Cas9-mediated exon 50 deletion in mice.** Two single-guide RNA (sgRNA) specific intronic region surrounding exon 50 sequence of the mouse *Dmd* locus were cloned into vector px330 using the following primers: *Dmd* exon 50_F1: 5'-CACCGAAATGATGAGTGAAGTTATAT-3' (SEQ ID NO: 926); *Dmd* exon 50_R1: 5'-AAACATATAACTTCACTCATCATTTC-3' (SEQ ID NO: 927); *Dmd* exon 50_F2: 5' CACCGGTTTGTTCAAAAGCGTGGCT-3' (SEQ ID NO: 928); *Dmd* exon 50_R2: 5'-AAACAGCCACGCTTTTGAACAAAC-3' (SEQ ID NO: 929).

For the in vitro transcription of sgRNA, T7 promoter sequence was added to the sgRNA template by PCR using the following primers: *Dmd* exon 50_T7-F1:
GAATTGTAATACGACTCACTATAGGAATGATGAGTGAAGTTATAT (SEQ ID NO: 930); *Dmd* exon 50_T7-F2:
GAATTGTAATACGACTCACTATAGGGTTTGTTCAAAAGCGTGGCT (SEQ ID NO: 931); *Dmd* exon 50_T7-Rv: AAAAGCACCGACTCGGTGCCAC (SEQ ID NO: 932).

The gel purified PCR products were used as template for in vitro transcription using the MEGAshortscript T7 Kit (Life Technologies). sgRNA were purified by MEGAclear kit (Life Technologies) and eluted with nuclease-free water (Ambion). The concentration of guide RNA was measured by a NanoDrop instrument (Thermo Scientific).

**Genotyping of ΔEx50 Mice.** ΔEx50 mice were genotyped using primers encompassing the targeted region: Geno50-F: 5'-GGATTGACTGAAATGATGGCCAAGG-3' (SEQ ID NO: 937); Geno50-R: 5'-CTGCCACGATTACTCTGCTTCCAG-3' (SEQ ID NO: 938). Tail biopsies were digested in 100 µL of 25-mM NaOH, 0.2-mM EDTA (pH 12) for 20 min at 95 °C. Tails were briefly centrifuged followed by addition of 100 µL of 40-mM Tris•HCl (pH 5) and mixed to homogenize. Two microliters of this reaction was used for subsequent PCR reactions with the primers below, followed by gel electrophoresis.

**Plasmids.** The pSpCas9(BB)-2A-GFP (PX458) plasmid containing the human codon optimized SpCas9 gene with 2A-EGFP and the backbone of sgRNA was purchased from Addgene (Plasmid #48138). AAV TRIPSR plasmids were obtained from Dr. Dirk Grimm (Heidelberg University Hospital). Cloning of sgRNA was done using a BbsI site. pGL3-CK8e plasmid was obtained from Dr. Stephen Hauschka (Department of Biochemistry, University of Washington, Seattle, USA). AAV-miniCMV-Cas9-shortPolyA plasmid was obtained from Dr. Dirk Grimm (Heidelberg University Hospital). To generate the final AAV9-CK8-CRISPR/Cas9 vector used in this manuscript, AAV-miniCMV-Cas9-short-PolyA was digested with PacI and NheI enzyme to remove the miniCMV promoter. CK8 promoter was amplified from pGL3-CK8e plasmid using primers containing PacI and NheI site sequence and cloned into digested vector to generate AAV-CK8-Cas9-shortPolyA plasmid.

**sgRNA Identification and Cloning for skipping exon 51.** *Dmd* exon 51 guide RNAs were defined using crispr.mit.edu. Guide sequences were cloned into Addgene plasmid #42230 (6), a gift from Feng Zhang, using the following primers: *Dmd* exon 51_F1: 5'-CACCGAGAGTAACAGTCTGACTGG -3' (SEQ ID NO: 942); *Dmd* exon 51_R1: 5'-AAACGTCAGACTGTTACTCTAGTGC-3' (SEQ ID NO: 943); *Dmd* exon 51_F2: 5'-CACCGCACTAGAGTAACAGTCTGAC -3' (SEQ ID NO: 944); *Dmd* exon 51_R2: 5'-AAACCCAGTCAGACTGTTACTCTC -3' (SEQ ID NO: 945). Guide sequences were tested in culture using 10T1/2 cells before cloning into the AAV backbone.

**Triplicate sgRNA assembly in AAV backbone using Golden Gate system.** The assembly of the AAV TRISPR backbone cloning system relies on two consecutive steps of Golden Gate Assembly. First step assembly of gRNA into donor plasmid. Annealing of oligonucleotides is performed by heating a reaction containing 2.5 µl of each oligo (0.5 µM), 5 µl NEBuffer 2 (NEB) and 40 µE ddH₂O to 95 °C for 5 minutes using heating block. For the assembly reaction into donor plasmid mix 40 fmol (~100 ng) destination backbone, 1 µL annealed, diluted oligos, 0.75 µL of Esp3I, 1 µL buffer tango (both Thermo Scientific), 1 µL of T4 DNA Ligase (400 U/µL) (NEB) as well as ATP and DTT at a final concentration of 1 mM in 10 µL total volume. Using a thermocycler, conduct 25 to 50 cycles of 37°C/3 min followed by 20 °C/5 minutes. Denature restriction enzyme and ligase by heating to 80 °C for 20 minutes. Use 3 µL of this reaction for transformation of chemo-competent bacteria, recover in SOC (37 °C, 800 rpm, 40 min) and spread on LB-Agar plates containing chloramphenicol (25 µg/mL). Annealed oligonucleotides encoding for the sgRNA are cloned into donor plasmids that carry the negative selection marker *ccdB* (to reduce background during cloning) as well as the chloramphenicol resistance gene. To test the correct assembly the plasmid are sequenced using the primer Dono-R-5'-GTATGTTGTGTGGAATTGTGAG-3' (SEQ ID NO: 948). Second step is that three of these donor plasmids driving expression of one sgRNA under transcriptional control of U6, H1 or 7SK promoter are pooled in a second Golden Gate assembly along with a recipient plasmid that carries AAV ITRs. The assembly reaction will contain all four plasmids: donor plasmid-#1-U6-sgRNA, donor plasmid-#2-H1-sgRNA, donor plasmid-#3-7SK-sgRNA and recipient plasmid containing the ITR. Digest with BbsI will generate unique overhangs for each fragment (U6, H1, 7SK, recipient backbone). During the ligation procedure, these overhangs anneal a circularized plasmid is only obtained, when the three cassettes match each other.

**Serum creatine kinase (CK) measurement.** Mouse serum CK was measured by the Metabolic Phenotyping Core at UT Southwestern Medical Center. Blood was collected from the submandibular vein and serum CK level was measured by VITROS Chemistry 7 Products CK Slides to quantitatively measure CK activity using VITROS 250 Chemistry System.

***In vivo* canine studies.** Intra-muscular (IM) injections were performed in 1 month old ΔE50 MD dogs under ventilated general anaesthesia. One single injection was performed at 4 different sites of the left cranial tibialis muscle. All dogs received premedication of an opiate (buprenorphine), perioperative antibiosis and 4 days of postoperative analgesia (carprofen). Two affected dogs received rAAV-mixed with myoediting components, AAV-Cas9 and AAV-sgRNA-ex51, which were prepared in lactated Ringer's solution to a final concentration of 1E13vg per injected muscle.

### EXAMPLE 2 - Results

**A humanized model of DMD.** The most common hot spot mutation region in DMD patients is the region between exon 45 to 51, and skipping of exon 51 could be used to treat the largest group (13-14%) of patients. To investigate CRISPR/Cas9-mediated exon 51 skipping *in vivo,* the inventors generated a mouse model that mimics the human "hot spot" region by deleting exon 50 using the CRISPR/Cas9 system directed by 2 sgRNAs (FIG. 1A). The deletion of exon 50 was confirmed by DNA sequencing (FIG. 1B). Deletion of exon 50 placed the dystrophin gene out of frame leading to the absence of dystrophin protein in skeletal muscle and heart (FIGS. 1C-E). Mice lacking exon 50 showed pronounced dystrophic muscle changes by 2 months of age (FIG. 1E). Serum analysis of delta-exon 50 mice showed a significant increase in creatine kinase (CK) levels, indicative of muscle damage (FIG. 1F). Taken together, dystrophin protein expression, muscle histology and serum CK levels validated the dystrophic phenotype of the ΔEx50 mouse model.

**Restoration of dystrophin expression using a single cut strategy to skip exon 51.** *S. pyogenes* Cas9 requires NAG/NGG as a PAM sequence to generate a double-strand DNA break. Interestingly, the universal splice acceptor and donor sites of exons contain NAG or NGG (FIG. 3B). Therefore, to correct the reading frame and dystrophin expression in the ΔEx50 mouse model, the inventors generated sgRNA that targeted splice acceptor and donor sites of exon 51 to delete it, thereby recreating the in-frame dystrophin protein. To test whether the sgRNA guides were able to efficiently cut, the inventors first evaluated their effectiveness in mouse and human cell lines (FIG. 5). To determine the most efficient way to correct the DMD reading frame, the inventors compared 2 different strategies: (1) double-guide strategy in which one copy of a first sgRNA targeting splice acceptor site (sgRNA-SA) and one copy of a second sgRNA targeting donor acceptor site (sgRNA-SD), were cloned into the rAAV9-sgRNA vector; (2) triplicate strategy in which the inventors cloned 3 copies of the same sgRNA (sgRNA-SA) into the rAAV9-sgRNA vector (FIG. 3C). Expression of each copy of sgRNA-SA was driven by a different RNA promoter (U6, H1 and 7SK). The inventors generated AAV-Cas9 using an AAV-Cas9 vector (CK8-Cas9-shortPolyA), which employs a CK8 promoter to drive expression of the humanized SpCas9 specifically in skeletal muscle and heart tissues. Following intra-muscular (IM) injection of P12 mice with AAVs, muscle tissues were analyzed. RT-PCR of RNA from ΔEx50 mice injected with AAV-Tri-SA and AAV-SA+SD showed that deletion of exon 51 (ΔEx50-51) allowed splicing from exon 49 to 52 (FIG. 2A, lower band). Sequencing of RT-PCR products of the ΔEx50-51 band confirmed that exon 49 spliced to exon 52 (FIG. 2B). Unexpectedly, the RT-PCR analysis showed that a single cut strategy using a triplicate version of sgRNA-SA (AAV-Tri-SA) is as efficient as using two sgRNAs - sgRNA-SA and sgRNA-SD (AAV-SA+SD).

To further assess the efficiency of the AAV-Tri-SA editing strategy, the inventors performed histological analysis of injected muscle to evaluate the number of fibers that express dystrophin throughout entire muscle sections. Interestingly, dystrophin immunostaining of muscle cryosections from ΔEx50 mice injected with AAV-Tri-SA revealed significantly higher numbers of dystrophin-positive fibers (average of 43 ± 0.9%) compared to the muscle from ΔEx50 mice injected with AAV-SA+SD (average of 31 ± 0.1%) (FIGS. 3D-E, FIG. 6). Western blot analysis confirmed the restoration of dystrophin expression in skeletal muscle. (FIGS. 3F-G). Hematoxylin and eosin (H&E) staining of muscle showed that histopathologic hallmarks of muscular dystrophy, such as necrotic myofibers, were diminished in TA muscle at 3-weeks post-AAV delivery (FIG. 4A). Quantitative analysis of the distribution of myofiber areas showed a clear increase in fiber size for both AAV-Tri-SA and AAV-SA-SD treated muscles compared to ΔEx50 muscles (FIG. 4B). However, AAV-Tri-SA treated muscles revealed a higher decrease in the frequency of small fibers (<500 µm) compared to AAV-SA+SD treated muscles. Together, these results demonstrate that targeting the splice acceptor site of exon 51 with one single cut using AAV-Tri-SA is highly efficient in restoring dystrophin expression in DMD. This approach has usefulness for many disorders that can be corrected by exon skipping.

**Tailoring of the single DNA cut genome editing strategy.** *S. pyogenes* Cas9 guided by sgRNAs binds to the targeted genomic locus next to a PAM and generates a double-strand DNA break (DSB) 3 nucleotides preceding the PAM sequence. To further assess the efficiency of the method by targeting the splice acceptor site, the inventors designed a second sgRNA triple guide construct (sgRNA-ex51-SA2), targeting a region adjacent to the exon 51 splice acceptor site. This gRNA uses a PAM sequence 3 nucleotides further into the exon in order to generate the DSB close to the splice acceptor site for exon 51 (FIG. 7A-FIG. 7B). Cutting in the vicinity of the splice acceptor region and within the exon sequence resulted in reframing events and exon skipping events. Moreover, designing the sgRNA in the exon sequence that shows higher conservation than intron sequence across species facilitates translation of the sgRNA to other species.

The DNA cutting activity of Cas9 coupled with sgRNA-ex51-SA2 was evaluated in 10T1/2 mouse fibroblasts using the mismatch-specific T7 endonuclease I (T7E1) assay (FIG. 8A). To investigate the type of mutations generated by Cas9 coupled with sgRNA-51-SA2, genomic deep-sequencing analysis was performed. The sequencing analysis revealed that 9.3% of mutations contained a single adenosine (A) insertion located 3 nucleotides 3' of the PAM sequence. In addition, 7.3% of mutations contained deletions covering the splice acceptor site and a highly-predicted exonic splicing enhancer site for exon 51 (FIG. 8B). The sgRNA-ex51-SA2 corresponds to a highly conserved region of the *Dmd* gene (FIGS. 8C-D), and the inventors tested the ability of Cas9 and human sgRNA-51 to cut the human *Dmd* locus in 293 T cells. The T7E1 assay revealed clear cleavage at the predicted site (FIG. 8E). Similarly, sequence analysis revealed that Cas9 coupled with human sgRNA-ex51-SA2 generated the same adenosine (A) insertion and a different range of deletions around the cleavage site (FIG. 8F).

For the *in vivo* delivery of Cas9 and sgRNA-ex51-SA2 to skeletal muscle and heart tissue, adeno-associated virus 9 (AAV9) was used, which displays preferential tropism for these tissues. To further enhance muscle-specific expression, an AAV9-Cas9 vector (CK8e-Cas9-shortPolyA) was employed, which contains a muscle-specific CK regulatory cassette, referred to as the CK8e promoter, which is highly specific for expression in muscle and heart (FIG. 9A). Together, this 436 bp muscle-specific cassette and the 4101 bp Cas9 cDNA are within the packaging limit of AAV9. Expression of each sgRNA was driven by one of three RNA polymerase III promoters (U6, H1 and 7SK) (FIG. 9B).

**Correction of the dystrophin reading frame in ΔEx50 mice by a single DNA cut.** The sgRNA-ex51-SA2 was delivered to mice in triple copy (AAV-Tri-SA2), along with a Cas9 (AAV-Cas9), by intra-muscular (IM) injection. Following the injection, muscle tissues were analyzed. *In vivo* targeting efficiency was estimated by RT-PCR with primers for sequences in exons 48 and 53 and the T7E1 assay for the targeted genomic regions. To investigate whether efficient target cleavage was achieved, the inventors amplified a 771 bp region spanning the target site and analyzed it using the T7EI assay (FIG. 10A). The activity of SpCas9 with the corresponding sgRNA was analyzed on the target site. T7EI assays revealed mutagenesis of the *Dmd* locus after delivery of AAV-Cas9 and AAV9-sgRNA-51-SA2 (FIG. 10A). To investigate the type of mutations generated in ΔEx50 mice injected with Cas9 and sgRNA-expressing AAV9s, genomic PCR amplification products spanning the target site were analyzed by amplicon deep-sequencing analysis. Deep sequencing of the targeted region indicated that 27.9% of total reads contained changes at the targeted genomic site (FIG. 10B). On average, 15% of the identified mutations contained the same A insertion seen in mouse 10T1/2 and human 293 cells in vitro. The deletions identified using this method encompassed a highly-predicted exonic splicing enhancer site for exon 51 (FIG. 10B).

RT-PCR products of RNA from muscle of ΔEx50 mice injected intramuscularly with AAV9-Cas9 and AAV9-sgRNA-51 showed that deletion of exon 51 (ΔEx50-51) allowed splicing from exon 49 to 52 (FIG. 11A, lower band). By sequencing RT-PCR products of the ΔEx50-51 band, it was confirmed that exon 49 was spliced to exon 52. To further define the mutations introduced by our gene editing strategy, RT-PCR amplification products from 4 samples were directly subjected to topoisomerase-based thymidine to adenosine (TOPO-TA) cloning without gel purification, then sequenced. Surprisingly, sequence analysis of 40 clones from each sample showed that in addition to exon 51-skipped cDNA products (ΔEx50-51) identified in 15% of sequenced clones, ΔEx50 mice injected with AAV9-Cas9 and AAV9-sgRNA-51 showed a high frequency of reframing events. Of sequenced clones, 63% contained a single nucleotide insertion in the sequence of exon 51 (FIGS. 11 B-C). The most dominant insertion mutation seen was an A insertion.

On gels, the A insertion was indistinguishable in size from non-edited cDNA products, so deep-sequencing analysis was performed to determine the abundance of this insertion compared to other small insertions. Deep-sequencing of the upper band containing the non-edited cDNA product and reframed cDNA products indicated that 69.22% of total reads contained reframed cDNA products with an A insertion, 17.71% contained non-edited cDNA product, and the rest contained small deletions and insertions (FIG. 11D). The deep-sequencing analysis of uninjected ΔEx50 mice confirmed that the A insertion is a result of Cas9-generated editing. These amplicon deep-sequencing results confirmed the results from TOPO-TA cloning and sequencing. Taken together, RT-PCR analysis revealed that ΔEx50 mice injected with AAV9-Cas9 and AAV9-sgRNA-51-SA2 showed a high frequency of reframing events with cDNA products containing an A insertion in the sequence of exon 51 in addition to exon 51 skipping events resulting from deletion in a highly conserved exonic splicing enhancer region.

**Restoration of dystrophin expression after intramuscular AAV9 delivery of Cas9 and sgRNA-51-SA2.** Remarkably, dystrophin immunostaining of muscle cryosections from ΔEx50 mice injected with AAV-Tri-SA2 revealed significantly higher numbers of dystrophin-positive fibers with an average of 99% restoration of normal fibers (FIG. 12A, FIG. 13). Western blot analysis confirmed the restoration of dystrophin expression in skeletal muscle. (FIG. 12C, FIG. 12D). Hematoxylin and eosin (H&E) staining of muscle showed that histopathologic hallmarks of muscular dystrophy, such as necrotic myofibers, were corrected in TA muscle at 3-weeks post-AAV delivery (FIG. 12B and FIG. 14). This method, using a distinct sgRNA design, represents a major advance in efficiency of DMD correction with direct applicability to the patients with the most common dystrophin mutations.

**Rescue of dystrophin expression following intramuscular injections of AAV9-Cas9 combined with different AAV9s expressing single copy or triple copy of sgRNA.** To evaluate the benefit of triple promoter expression of sgRNA-ex51-SA2 in vivo, different constructs were investigated, where sgRNA expression was driven by a single RNA polymerase III promoter (U6 or H1 or 7SK) and, separately, by three RNA polymerase III promoters (U6, H1 and 7SK) (FIG. 15A). The inventors delivered the sgRNA-ex51-SA2 in single copy driven separately by the U6 promoter (AAV9-U6-sgRNA-51-SA2), the H1 promoter (AAV9-H1-sgRNA-51-SA2), the 7SK promoter (AAV9-7SK-sgRNA-51-SA2) and triple copy (AAV9-Triple-sgRNA-51-SA2). Following intra-muscular (IM) injection of P12 mice with AAV9s, muscle tissues were analyzed. Unexpectedly, dystrophin immunostaining of muscle cryosections from ΔEx50 mice injected with AAV9-Triple-sgRNA-51-SA2 revealed significantly higher numbers of dystrophin-positive fibers with an average of 95% restoration of normal fibers compared to ΔEx50 mice injected with AAV9-U6-sgRNA-51-SA2, AAV-H1-sgRNA-51-SA2 and AAV-7SK-sgRNA-51-SA2 with an average of 70%; 40% and 50% restoration of normal fibers respectively (FIGS. 15B).

**Rescue of muscle structure and function following systemic delivery of AAV9-Cas9 and AAV9-sgRNA-51-SA2.** Systemic delivery of AAV9-Cas9 and AAV9-sgRNA-51-SA2 to P4 ΔEx50 mice yielded widespread dystrophin expression in the heart, triceps, tibialis anterior (TA) muscle, and diaphragm in gene-edited ΔEx50 mice at 4 and 8 weeks postinjection (FIG. 16A and FIG. 17A). Western blot analysis confirmed the restoration of dystrophin expression in skeletal and heart muscles (FIG. 16B and FIG. 17B). Grip strength testing also showed a significant increase in muscle strength of ΔEx50 mice at 4 weeks postintraperitoneal AAV9 injection compared to ΔEx50 control mice (wildtype control 92.6±1.63; ΔEx50 control 50.5±1.85; ΔEx50-AAV9-sgRNA-51 79.7±2.63) (FIG. 18A). Consistently, AAV9-sgRNA-51-SA2 gene-edited ΔEx50 mice also showed significant reductions in serum CK concentrations compared to ΔEx50 control mice (FIG. 18B).

**Correction of dystrophin expression in a dog model of Duchenne muscular dystrophy.** To further assess the efficiency and therapeutic potential of this new approach, the inventors investigated the correction the disease-causing mutation in a dog model of DMD, the ΔE50-MD dog, which harbors a missense mutation in the 5' donor splice site of exon 50 that results in deletion of exon 50 ((Walmsley *et al.,* 2010). The ΔE50-MD dog is an ideal canine model for the investigation of gene-editing as an approach to permanently correct the most common DMD mutations in humans.

The inventors used sgRNA (sgRNA-ex51-SA2) targeting the same genomic locus of the splice acceptor region. The sgRNA-ex51-SA2 sequence is highly conserved. The target sequences of the mouse sgRNA-ex51-SA2 and the dog sgRNA-ex51-SA2 (sgRNA-D-ex51-SA2) differ only by one single nucleotide (Table 4). The inventors delivered the sgRNA-Dex51-SA2 in triple copy (AAV-D-Tri-SA2). Following intra-muscular (IM) injection of 1 month old dogs with AAVs, muscle tissues were analyzed. Remarkably, dystrophin immunostaining of muscle cryosections from ΔEx50 mice injected with AAV-Tri-SA2 revealed significantly higher numbers of dystrophin-positive fibers (FIG. 19). Western blot analysis confirmed the restoration of dystrophin expression in skeletal muscle (FIG. 20). Hematoxylin and eosin (H&E) staining of muscle showed that histopathologic hallmarks of muscular dystrophy, such as necrotic myofibers, were diminished in cranial tibialis muscle at 6-weeks post-AAV delivery (FIG. 21). This method, using a distinct sgRNA design, represents a major advance in efficiency of DMD correction with direct applicability to the patients with the most common dystrophin mutations.

All of the compositions and/or methods disclosed herein can be made and executed without undue experimentation in light of the present disclosure. While the compositions and methods of this disclosure have been described in terms of preferred embodiments, it will be apparent to those of skill in the art that variations may be applied to the compositions and/or methods and in the steps or in the sequence of steps of the method described herein. More specifically, it will be apparent that certain agents which are both chemically and physiologically related may be substituted for the agents described herein while the same or similar results would be achieved.

### VII. References

Angel et al., Mol. Cell. Biol., 7:2256, 1987a.
Angel et al., Cell, 49:729, 1987b.
Aartsma-Rus et al., Hum. Mutat. 30, 293-299, 2009.
Baichwal and Sugden, In: Gene Transfer, Kucherlapati (Ed), NY, Plenum Press, 117-148, 1986.
Banerji et al., Cell, 27(2 Pt 1):299-308, 1981.
Banerji et al., Cell, 33(3):729-740, 1983.
Barnes et al., J. Biol. Chem., 272(17):11510-7, 1997.
Baskin et al., EMBO Mol Med 6, 1610-1621, 2014.
Benvenisty and Neshif, Proc. Natl. Acad. Sci. USA, 83:9551-9555, 1986.
Berkhout et al., Cell, 59:273-282, 1989.
Bhavsar et al., Genomics, 35(1):11-23, 1996.
Bikard et al., Nucleic Acids Res. 41(15): 7429-7437, 2013.
Blanar et al., EMBO J., 8:1139, 1989.
Bodine and Ley, EMBO J., 6:2997, 1987.
Boshart et al., Cell, 41:521, 1985.
Bostick et al., Mol Ther 19, 1826-1832, 2011.
Bosze et al., EMBO J., 5(7):1615-1623, 1986.
Braddock et al., Cell, 58:269, 1989.
Brinster et al., Proc. Natl. Acad. Sci. USA, 82(13):4438-4442, 1985.
Bulla and Siddiqui, J. Virol., 62:1437, 1986.
Burridge et al., Nat. Methods 11, 855-860, 2014.
Bushby et al., Lancet Neurol., 9(1): 77-93 (2010).
Bushby et al., Lancet Neurol., 9(2): 177-198 (2010).
Campbell & Kahl, Nature 338, 259-262, 1989.
Campbell and Villarreal, Mol. Cell. Biol., 8:1993, 1988.
Campere and Tilghman, Genes and Dev., 3:537, 1989.
Campo et al., Nature, 303:77, 1983.
Celander and Haseltine, J. Virology, 61:269, 1987.
Celander et al., J. Virology, 62:1314, 1988.
Chandler et al., Cell, 33:489, 1983.
Chang et al., Mol. Cell. Biol., 9:2153, 1989.
Chang et al., Stem Cells., 27:1042-1049, 2009.
Chatterjee et al., Proc. Natl. Acad. Sci. USA, 86:9114, 1989.
Chen and Okayama, Mol. Cell Biol., 7:2745-2752, 1987.
Cho et al., Nat. Biotechnol. 31(3): 230-232, 2013.
Choi et al., Cell, 53:519, 1988.
Cirak et al., The Lancet 378, 595-605, 2011.
Coffin, In: Virology, Fields et al. (Eds.), Raven Press, NY, 1437-1500, 1990.
Cohen et al., J. Cell. Physiol., 5:75, 1987.
Costa et al., Mol. Cell. Biol., 8:81, 1988.
Couch et al., Am. Rev. Resp. Dis., 88:394-403, 1963.
Coupar et al., Gene, 68: 1-10, 1988.
Cripe et al., EMBO J., 6:3745, 1987.
Culotta and Hamer, Mol. Cell. Biol., 9:1376, 1989.
Dandolo et al., J. Virology, 47:55-64, 1983.
De Villiers et al., Nature, 312(5991):242-246, 1984.
Deschamps et al., Science, 230:1174-1177, 1985.
DeWitt et al., Sci Transl Med 8, 360ral34-360ral34, 2016.
Donnelly et al., J. Gen. Virol. 82, 1027-1041, 2001.
Dubensky et al., Proc. Natl. Acad. Sci. USA, 81:7529-7533, 1984.
Edbrooke et al., Mol. Cell. Biol., 9: 1908, 1989.
Edlund et al., Science, 230:912-916, 1985.
EP 0273085
Fechheimer et al., Proc Natl. Acad. Sci. USA, 84:8463-8467, 1987.
Feng and Holland, Nature, 334:6178, 1988.
Ferkol et al., FASEB J., 7:1081-1091, 1993.
Firak et al., Mol. Cell. Biol., 6:3667, 1986.
Foecking et al., Gene, 45(1):101-105, 1986.
Fonfara et al., Nature 532, 517-521, 2016.
Fraley et al., Proc Natl. Acad. Sci. USA, 76:3348-3352, 1979
Franz etal., Cardoscience, 5(4):235-43, 1994.
Friedmann, Science, 244:1275-1281, 1989.
Fujita et al., Cell, 49:357, 1987.
Ghosh and Bachhawat, In: Liver Diseases, Targeted Diagnosis and Therapy Using Specific Receptors and Ligands, Wu et al. (Eds.), Marcel Dekker, NY, 87-104, 1991.
Ghosh-Choudhury et al., EMBO J., 6:1733-1739, 1987.
Gilles et al., Cell, 33:717, 1983.
Gloss et al., EMBO J., 6:3735, 1987.
Godbout et al.,Mol. Cell. Biol., 8:1169, 1988.
Gomez-Foix et al., J. Biol. Chem., 267:25129-25134, 1992.
Goncalves et al., Mol Ther, 19(7): 1331-1341 (2011).
Goodbourn and Maniatis, Proc. Natl. Acad. Sci. USA, 85:1447, 1988.
Goodbourn et al., Cell, 45:601, 1986.
Gopal, Mol. Cell Biol., 5:1188-1190, 1985.
Gopal-Srivastava et al., J. Mol. Cell. Biol., 15(12):7081-90, 1995.
Graham and Prevec, In: Methods in Molecular Biology: Gene Transfer and Expression Protocol, Murray (Ed.), Humana Press, Clifton, NJ, 7:109-128, 1991.
Graham and van der Eb, Virology, 52:456-467, 1973.
Graham et al., J. Gen. Virol., 36:59-72, 1977.
Greene et al., Immunology Today, 10:272, 1989
Grosschedl and Baltimore, Cell, 41:885, 1985.
Grunhaus and Horwitz, Seminar in Virology, 3:237-252, 1992.
Harland and Weintraub, J. Cell Biol., 101:1094-1099, 1985.
Haslinger and Karin, Proc. Natl. Acad. Sci. USA, 82:8572, 1985.
Hauber and Cullen, J. Virology, 62:673, 1988.
Hen et al., Nature, 321:249, 1986.
Hensel et al., Lymphokine Res., 8:347, 1989.
Hermonat and Muzycska, Proc. Nat'l Acad. Sci. USA, 81:6466-6470, 1984.
Herr and Clarke, Cell, 45:461, 1986.
Hersdorffer et al., DNA Cell Biol., 9:713-723, 1990.
Herz and Gerard, Proc. Nat'l. Acad. Sci. USA 90:2812-2816, 1993.
Hirochika et al., J. Virol., 61:2599, 1987.
Holbrook et al., Virology, 157:211, 1987.
Hollinger & Chamberlain, Current Opinion in Neurology 28, 522-527, 2015.
Horlick and Benfield, Mol. Cell. Biol., 9:2396, 1989.
Horwich et al., J. Virol., 64:642-650, 1990.
Hsu et al., Natl Biotechnol. 31:827-832, 2013
Huang et al., Cell, 27:245, 1981*.*
Hug et al., Mol. Cell. Biol., 8:3065, 1988.
Hwang et al., Mol. Cell. Biol., 10:585, 1990.
Imagawa et al., Cell, 51:251, 1987.
Imbra and Karin, Nature, 323:555, 1986.
Imler et al., Mol. Cell. Biol., 7:2558, 1987.
Imperiale and Nevins, Mol. Cell. Biol., 4:875, 1984.
Jakobovits et al., Mol. Cell. Biol., 8:2555, 1988.
Jameel and Siddiqui, Mol. Cell. Biol., 6:710, 1986.
Jaynes et al., Mol. Cell. Biol., 8:62, 1988.
Jinek et al., Science 337, 816-821, 2012.
Johnson et al., Mol. Cell. Biol., 9:3393, 1989.
Jones and Shenk, Cell, 13:181-188, 1978.
Kadesch and Berg, Mol. Cell. Biol., 6:2593, 1986.
Kaneda et al., Science, 243:375-378, 1989.
Karin et al., Mol. Cell. Biol., 7:606, 1987.
Karlsson et al., EMBO J., 5:2377-2385, 1986.
Katinka et al., Cell, 20:393, 1980.
Kato et al., J Biol Chem., 266(6):3361-3364, 1991.
Kawamoto et al., Mol. Cell. Biol., 8:267, 1988.
Kelly et al., J. Cell Biol., 129(2):383-96, 1995.
Kiledjian et al., Mol. Cell. Biol., 8:145, 1988.
Kim et al., Nature Biotechnology, 1-2, 2016.
Kim et al., Nature Biotechnology 34, 876-881, 2016.
Kimura et al., Dev. Growth Differ., 39(3):257-65, 1997.
Klamut et al., Mol. Cell. Biol., 10:193, 1990.
Klein et al., Nature, 327:70-73, 1987.
Koch et al., Mol. Cell. Biol., 9:303, 1989.
Kriegler and Botchan, In: Eukaryotic Viral Vectors, Gluzman (Ed.), Cold Spring Harbor: Cold Spring Harbor Laboratory, NY, 1982.
Kriegler and Botchan, Mol. Cell. Biol., 3:325, 1983.
Kriegler et al., Cell, 38:483, 1984.
Kriegler et al., Cell, 53:45, 1988.
Kuhl et al., Cell, 50:1057, 1987.
Kunz et al., Nucl. Acids Res., 17:1121, 1989.
LaPointe et al., Hypertension, 27(3):715-22, 1996
LaPointe et al., J. Biol. Chem., 263(19):9075-8, 1988.
Larsen et al., Proc. Natl. Acad. Sci. USA., 83 :8283, 1986.
Laspia et al., Cell, 59:283, 1989.
Latimer et al., Mol. Cell. Biol., 10:760, 1990.
Le Gal La Salle et al., Science, 259:988-990, 1993.
Lee et al., Nature, 294:228, 1981.
Levinson et al., Nature, 295:79, 1982.
Levrero et al., Gene, 101:195-202, 1991.
Lin et al., Mol. Cell. Biol., 10:850, 1990.
Long et al., Science 345: 1184-1188, 2014.
Long et al., Science 351, 400-403, 2016.
Pinello et al., Nature Biotechnol. 34, 695-697, 2016.
Long et al., JAMA Neurol., 3388, 2016.
Long et al., Science 351, 400-403, 2016.
Luria et al., EMBO J., 6:3307, 1987.
Lusky and Botchan, Proc. Natl. Acad. Sci. USA, 83:3609, 1986.
Lusky et al., Mol. Cell. Biol., 3:1108, 1983.
Majors and Varmus, Proc. Natl. Acad. Sci. USA, 80:5866, 1983.
Mali et al., Science 339, 823-826, 2013a.
Mali et al., Nat Methods 10, 957-963, 2013b.
Mali et al., Nat. Biotechnol. 31:833-838, 2013c.
Mann et al., Cell, 33:153-159, 1983.
Maresca et al., Genome Research 23, 539-546, 2013.
Markowitz et al., J. Virol., 62:1120-1124, 1988.
McNeall et al., Gene, 76:81, 1989.
Miksicek et al., Cell, 46:203, 1986.
Millay et al., Nat. Med. 14, 442-447, 2008.
Mojica et al., J. Mol. Evol. 60, 174-182, 2005.
Mordacq and Linzer, Genes and Dev., 3:760, 1989.
Moreau et al., Nucl. Acids Res., 9:6047, 1981.
Moss et al., J. Gen. Physiol., 108(6):473-84, 1996.
Muesing et al., Cell, 48:691, 1987.
Nelson et al., Science 351, 403-407, 2016.
Nicolas and Rubinstein, In: Vectors: A survey of molecular cloning vectors and their uses, Rodriguez and Denhardt (Eds.), Stoneham: Butterworth, 494-513, 1988.
Nicolau and Sene, Biochim. Biophys. Acta, 721:185-190, 1982.
Nicolau et al., Methods Enzymol., 149:157-176, 1987.
Ondek et al., EMBO J., 6:1017, 1987.
Ornitz et al., Mol. Cell. Biol., 7:3466, 1987.
Padgett, R.A., Trends Genet. 28, 147-154, 2012.
Palmiter et al., Cell, 29:701, 1982a.
Palmiter et al., Nature, 300:611, 1982b.
Paskind et al., Virology, 67:242-248, 1975.
Pech et al., Mol. Cell. Biol., 9:396, 1989.
Perales et al., Proc. Natl. Acad. Sci. USA, 91(9):4086-4090, 1994.
Perez-Stable and Constantini, Mol. Cell. Biol., 10:1116, 1990.
Picard et al., Nature, 307:83, 1984.
Pinkert et al., Genes and Dev., 1:268, 1987.
Ponta et al., Proc. Natl. Acad. Sci. USA, 82:1020, 1985.
Porton et al., Mol. Cell. Biol., 10: 1076, 1990.
Potter et al., Proc. Natl. Acad. Sci. USA, 81:7161-7165, 1984.
Queen and Baltimore, Cell, 35:741, 1983.
Quinn et al., Mol. Cell. Biol., 9:4713, 1989.
Racher et al., Biotech. Techniques, 9:169-174, 1995.
Ragot et al., Nature, 361:647-650, 1993.
Ran et al., Nature 520, 186-191, 2015.
Redondo et al., Science, 247:1225, 1990.
Reisman and Rotter, Mol. Cell. Biol., 9:3571, 1989.
Renan, Radiother. Oncol., 19:197-218, 1990.
Resendez Jr. et al., Mol. Cell. Biol., 8:4579, 1988.
Rich et al., Hum. Gene Ther., 4:461-476, 1993.
Ridgeway, In: Vectors: A survey of molecular cloning vectors and their uses, Stoneham: Butterworth, 467-492, 1988.
Ripe et al., Mol. Cell. Biol., 9:2224, 1989.
Rippe et al., Mol. Cell Biol., 10:689-695, 1990.
Rittling et al., Nuc. Acids Res., 17:1619, 1989.
Rosen et al., Cell, 41:813, 1988.
Rosenfeld et al., Cell, 68:143-155,1992.
Rosenfeld et al., Science, 252:431-434,1991.
Roux et al., Proc. Natl. Acad. Sci. USA, 86:9079-9083, 1989.
Sambrook and Russell, Molecular Cloning: A Laboratory Manual 3rd Ed., Cold Spring Harbor Laboratory Press, 2001.
Sakai et al., Genes and Dev., 2:1144, 1988.
Satake et al., J. Virology, 62:970, 1988.
Schaffner et al., J. Mol. Biol., 201:81, 1988.
Searle et al., Mol. Cell. Biol., 5:1480, 1985.
Sharp et al., Cell, 59:229, 1989.
Shaul and Ben-Levy, EMBO J., 6:1913, 1987.
Sherman et al., Mol. Cell. Biol., 9:50, 1989.
Sleigh et al., J. EMBO, 4:3831, 1985.
Shimizu-Motohashi et al., Am J Transl Res 8, 2471-89, 2016.
Spalholz et al., Cell, 42:183, 1985.
Spandau and Lee, J. Virology, 62:427, 1988.
Spandidos and Wilkie, EMBO J., 2:1193, 1983.
Stephens and Hentschel, Biochem. J., 248:1, 1987.
Stratford-Perricaudet and Perricaudet, In: Human Gene Transfer, Cohen-Haguenauer and Boiron (Eds.), John Libbey Eurotext, France, 51-61, 1991.
Stratford-Perricaudet et al., Hum. Gene. Ther., 1:241-256, 1990.
Stuart et al., Nature, 317:828, 1985.
Sullivan and Peterlin, Mol. Cell. Biol., 7:3315, 1987.
Swartzendruber and Lehman, J. Cell. Physiology, 85:179, 1975.
Tabebordbar et al., Science 351, 407-411, 2016.
Takebe et al., Mol. Cell. Biol., 8:466, 1988.
Tavernier et al., Nature, 301:634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:15160, 1989.
Temin, In: Gene Transfer, Kucherlapati (Ed.), NY, Plenum Press, 149-188, 1986.
Thiesen et al., J. Virology, 62:614, 1988.
Top et al., J. Infect. Dis., 124:155-160, 1971.
Tóth et al., Biology Direct, 1-14, 2016.
Tronche et al., Mol. Biol. Med., 7:173, 1990.
Trudel and Constantini, Genes and Dev. 6:954, 1987.
Tsai et al., Nature Biotechnology 34, 882-887, 2016.
Tur-Kaspa et al., Mol. Cell Biol., 6:716-718, 1986.
Tyndell et al., Nuc. Acids. Res., 9:6231, 1981.
Vannice and Levinson, J. Virology, 62:1305, 1988.
Varmus et al., Cell, 25:23-36, 1981.
Vasseur et al., Proc Natl. Acad. Sci. U.S.A., 77:1068, 1980.
Wagner et al., Proc. Natl. Acad. Sci. USA, 87(9):3410-3414, 1990.
Wang and Calame, Cell, 47:241, 1986.
Wang et al., Cell, 153:910-910, 2013.
Weber et al., Cell, 36:983, 1984.
Weinberger et al. Mol. Cell. Biol., 8:988, 1984.
Winoto et al., Cell, 59:649, 1989.
Wong et al., Gene, 10:87-94, 1980.
Wu and Wu, Adv. Drug Delivery Rev., 12:159-167, 1993.
Wu and Wu, Biochemistry, 27:887-892, 1988.
Wu and Wu, J. Biol. Chem., 262:4429-4432, 1987.
Wu et al., Cell Stem Cell 13, 659-662, 2013.
Wu et al., Nat Biotechnol 32, 670-676, 2014.
Xu et al., Mol Ther 24, 564-569, 2016.
Yamauchi-Takihara etal., Proc. Natl. Acad. Sci. USA, 86(10):3504-3508, 1989.
Yang et al., Proc. Natl. Acad. Sci. USA, 87:9568-9572, 1990.
Yin et al., Nat Biotechnol 32, 551-553, 2014.
Yin et al., Physiol Rev 93, 23-67, 2013.
Young et al., Cell Stem Cell 18, 533-540, 2016.
Yutzey et al. Mol. Cell. Biol., 9: 1397, 1989.
Zechner et al., Cell Metabolism 12, 633-642, 2010.
Zelenin et al., FEES Lett., 280:94-96, 1991.
Zetsche et al., Cell 163, 759-771, 2015.
Ziober and Kramer, J. Bio. Chem., 271(37):22915-22922, 1996.

## Claims

1. A nucleic acid comprising:
a sequence encoding a first DMD guide RNA targeting a first genomic target sequence,
a sequence encoding a second DMD guide RNA targeting a second genomic target sequence,
a sequence encoding a third DMD guide RNA targeting a third genomic target sequence,
a first promoter, wherein the first promoter drives expression of the sequence encoding the first DMD guide RNA,
a second promoter, wherein the second promoter drives expression of the sequence encoding the second DMD guide RNA, and
a third promoter, wherein the third promoter drives expression of the sequence encoding the third DMD guide RNA,
wherein the first genomic target sequence, the second genomic target sequence, and the third genomic target sequence comprise a dystrophin splice acceptor site,
and wherein the sequence encoding the first DMD guide RNA, the sequence encoding the second DMD guide RNA, and the sequence encoding the third DMD guide RNA are identical.

2. The nucleic acid of claim 1, wherein at least two of the first promoter, the second promoter, and the third promoter are identical.

3. The nucleic acid of claim 1, wherein at least two of the first promoter, the second promoter, and the third promoter are not identical.

4. The nucleic acid of any one of claims 1-3, wherein the dystrophin splice acceptor site is the 5' splice acceptor site of exon 51.

5. The nucleic acid of any one of claims 1-4, wherein the first promoter or the second promoter comprises a constitutive promoter.

6. The nucleic acid of any one of claims 1-5, wherein the first promoter or the second promoter comprises a constitutive promoter, an inducible promoter, or a cell-type specific promoter.

7. The nucleic acid of claim 6, wherein the cell-type specific promoter is a muscle-specific promoter.

8. The nucleic acid of any one of claims 1-7, wherein the first promoter or the second promoter comprises a U6 promoter, an H1 promoter, or a 7SK promoter.

9. The nucleic acid of any one of claims 1-8, wherein the nucleic acid comprises a DNA sequence.

10. The nucleic acid of any one of claims 1-8, wherein the nucleic acid comprises an RNA sequence.

11. The nucleic acid of any one of claims 1-10, wherein the nucleic acid further comprises a sequence encoding a 5' inverted terminal repeat (ITR) and a sequence encoding a 3' ITR.

12. The nucleic acid of any one of claims 1-11, wherein the sequence encoding the first DMD guide RNA, the sequence encoding the second DMD guide RNA, or the sequence encoding the third DMD guide RNA comprises the sequence of any one of SEQ ID NOs: 60-382, 706-708 and 712-719.

13. The nucleic acid of any one of claims 1-12, wherein the sequence encoding the first DMD guide RNA, the sequence encoding the second DMD guide RNA, and the sequence encoding the third DMD guide RNA comprises the sequence of SEQ ID NO: 714.

## Patentansprüche

1. Nukleinsäure, umfassend:
eine Sequenz, die eine erste DMD-Leit-RNA codiert, die auf eine erste genomische Zielsequenz abzielt,
eine Sequenz, die eine zweite DMD-Guide-RNA codiert, die auf eine zweite genomische Zielsequenz abzielt,
eine Sequenz, die eine dritte DMD-Leit-RNA codiert, die auf eine dritte genomische Zielsequenz abzielt, einen ersten Promotor, wobei der erste Promotor die Expression der Sequenz antreibt, die die erste DMD-Leit-RNA codiert,
einen zweiten Promotor, wobei der zweite Promotor die Expression der Sequenz antreibt, die für die zweite DMD-Leit-RNA codiert, und
einen dritten Promotor, wobei der dritte Promotor die Expression der Sequenz antreibt, die für die dritte DMD-Leit-RNA codiert,
wobei die erste genomische Zielsequenz, die zweite genomische Zielsequenz und die dritte genomische Zielsequenz eine Dystrophinspleißakzeptorstelle umfassen,
und wobei die Sequenz, die die erste DMD-Leit-RNA codiert, die Sequenz, die die zweite DMD-Leit-RNA codiert, und die Sequenz, die die dritte DMD-Leit-RNA codiert, identisch sind.

2. Nukleinsäure nach Anspruch 1, wobei mindestens zwei des ersten Promotors, des zweiten Promotors und des dritten Promotors identisch sind.

3. Nukleinsäure nach Anspruch 1, wobei mindestens zwei des ersten Promotors, des zweiten Promotors und des dritten Promotors nicht identisch sind.

4. Nukleinsäure nach einem der Ansprüche 1 bis 3, wobei die Dystrophinspleißakzeptorstelle die 5'-Spleißakzeptorstelle von Exon 51 ist.

5. Nukleinsäure nach einem der Ansprüche 1 bis 4, wobei der erste Promoter oder der zweite Promoter einen konstitutiven Promoter umfasst.

6. Nukleinsäure nach einem der Ansprüche 1 bis 5, wobei der erste Promotor oder der zweite Promotor einen konstitutiven Promotor, einen induzierbaren Promotor oder einen zelltypspezifischen Promotor umfasst.

7. Nukleinsäure nach Anspruch 6, wobei der zelltypspezifische Promotor ein muskelspezifischer Promotor ist.

8. Nukleinsäure nach einem der Ansprüche 1 bis 7, wobei der erste Promoter oder der zweite Promoter einen U6-Promoter, einen H1-Promoter oder einen 7SK-Promoter umfasst.

9. Nukleinsäure nach einem der Ansprüche 1 bis 8, wobei die Nukleinsäure eine DNA-Sequenz umfasst.

10. Nukleinsäure nach einem der Ansprüche 1 bis 8, wobei die Nukleinsäure eine RNA-Sequenz umfasst.

11. Nukleinsäure nach einem der Ansprüche 1 bis 10, wobei die Nukleinsäure ferner eine Sequenz umfasst, die eine 5' invertierte terminale Wiederholung (ITR) codiert, und eine Sequenz, die eine 3' ITR codiert.

12. Nukleinsäure nach einem der Ansprüche 1 bis 11, wobei die Sequenz, die für die erste DMD-Leit-RNA codiert, die Sequenz, die für die zweite DMD-Leit-RNA codiert, oder die Sequenz, die für die dritte DMD-Leit-RNA codiert, die Sequenz nach einer der SEQ ID NOs: 60-382, 706-708 und 712-719 umfasst.

13. Nukleinsäure nach einem der Ansprüche 1 bis 12, wobei die Sequenz, die die erste DMD-Leit-RNA codiert, die Sequenz, die die zweite DMD-Leit-RNA codiert, und die Sequenz, die die dritte DMD-Leit-RNA codiert, die Sequenz von SEQ ID NO: 714 umfasst.

## Revendications

1. Acide nucléique comprenant :
une séquence codant pour un premier ARN guide DMD ciblant une première séquence cible génomique,
une séquence codant pour un deuxième ARN guide DMD ciblant une deuxième séquence cible génomique,
une séquence codant pour un troisième ARN guide DMD ciblant une troisième séquence cible génomique,
un premier promoteur, dans lequel le premier promoteur entraîne l'expression de la séquence codant pour le premier ARN guide DMD,
un deuxième promoteur, dans lequel le deuxième promoteur entraîne l'expression de la séquence codant pour le deuxième ARN guide DMD, et
un troisième promoteur, dans lequel le troisième promoteur entraîne l'expression de la séquence codant pour le troisième ARN guide DMD,
dans lequel la première séquence cible génomique, la deuxième séquence cible génomique, et la troisième séquence cible génomique comprennent un site accepteur d'épissage de la dystrophine,
et dans lequel la séquence codant pour le premier ARN guide DMD, la séquence codant pour le deuxième ARN guide DMD, et la séquence codant pour le troisième ARN guide DMD sont identiques.

2. Acide nucléique selon la revendication 1, dans lequel au moins deux du premier promoteur, du deuxième promoteur, et du troisième promoteur sont identiques.

3. Acide nucléique selon la revendication 1, dans lequel au moins deux du premier promoteur, du deuxième promoteur, et du troisième promoteur ne sont pas identiques.

4. Acide nucléique selon l'une quelconque des revendications 1 à 3, dans lequel le site accepteur d'épissage de la dystrophine est un site accepteur d'épissage en 5' de l'exon 51.

5. Acide nucléique selon l'une quelconque des revendications 1 à 4, dans lequel le premier promoteur ou le deuxième promoteur comprend un promoteur constitutif.

6. Acide nucléique selon l'une quelconque des revendications 1 à 5, dans lequel le premier promoteur ou le deuxième promoteur comprend un promoteur constitutif, un promoteur inductible, ou un promoteur spécifique au type de cellule.

7. Acide nucléique selon la revendication 6, dans lequel le promoteur spécifique au type de cellule est un promoteur spécifique du muscle.

8. Acide nucléique selon l'une quelconque des revendications 1 à 7, dans lequel le premier promoteur ou le deuxième promoteur comprend un promoteur U6, un promoteur H1, ou un promoteur 7SK.

9. Acide nucléique selon l'une quelconque des revendications 1 à 8, dans lequel l'acide nucléique comprend une séquence d'ADN.

10. Acide nucléique selon l'une quelconque des revendications 1 à 8, dans lequel l'acide nucléique comprend une séquence d'ARN.

11. Acide nucléique selon l'une quelconque des revendications 1 à 10, dans lequel l'acide nucléique comprend en outre une séquence codant pour une répétition terminale inversée (ITR) en 5' et une séquence codant pour une ITR en 3'.

12. Acide nucléique selon l'une quelconque des revendications 1 à 11, dans lequel la séquence codant pour le premier ARN guide DMD, la séquence codant pour le deuxième ARN guide DMD, ou la séquence codant pour le troisième ARN guide DMD comprend la séquence de l'une quelconque parmi SEQ ID NO : 60-382, 706-708 et 712-719.

13. Acide nucléique selon l'une quelconque des revendications 1 à 12, dans lequel la séquence codant pour le premier ARN guide DMD, la séquence codant pour le deuxième ARN guide DMD, et la séquence codant pour le troisième ARN guide DMD comprend la séquence de SEQ ID NO : 714.
